(19) Europäisches Patentamt
European Patent Office
Office européen des brevets

(11) EP 2 125 856 B1

(12) **EUROPEAN PATENT SPECIFICATION**

(45) Date of publication and mention of the grant of the patent:
**14.09.2016 Bulletin 2016/37**

(21) Application number: **07854973.0**

(22) Date of filing: **05.12.2007**

(51) Int Cl.:
***C07H 21/04*** *(2006.01)*

(86) International application number:
**PCT/US2007/086559**

(87) International publication number:
**WO 2008/070749 (12.06.2008 Gazette 2008/24)**

(54) **PHOTOCLEAVABLE LABELED NUCLEOTIDES AND NUCLEOSIDES AND LABELED NUCLEOTIDES AND NUCLEOSIDES AND METHODS FOR THEIR USE IN DNA SEQUENCING**

DURCH LICHTEINWIRKUNG SPALTBARE MARKIERTE NUKLEOTIDE UND NUKLEOSIDE UND MARKIERTE NUKLEOTIDE UND NUKLEOSIDE UND VERFAHREN ZU IHRER VERWENDUNG BEI DER DNA-SEQUENZIERUNG

NUCLÉOTIDES ET NUCLÉOSIDES MARQUÉS PHOTOCLIVABLES, NUCLÉOTIDES ET NUCLÉOSIDES MARQUÉS, ET LEURS PROCÉDÉS D’UTILISATION DANS LE SÉQUENÇAGE D’ADN

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **05.12.2006 US 567189**
**05.12.2006 US 567193**

(43) Date of publication of application:
**02.12.2009 Bulletin 2009/49**

(73) Proprietor: **Lasergen, Inc.**
**Houston TX 77054 (US)**

(72) Inventors:
- **LITOSH, Vladislav A.**
  **Cypress, TX 77433 (US)**
- **STUPI, Brian P.**
  **Cypress, TX 77433 (US)**
- **METZKER, Michael L.**
  **Houston, TX 77054 (US)**
- **WU, Weidong**
  **Houston, TX 77096 (US)**

(74) Representative: **Dehmel, Albrecht**
**Dehmel & Bettenhausen**
**Patentanwälte PartmbB**
**Herzogspitalstraße 11**
**80331 München (DE)**

(56) References cited:
**WO-A1-2004/018493**
**WO-A2-02/29003**
**WO-A2-03/048178**
**WO-A2-2004/058791**
**WO-A2-2005/084367**

- **METZKER M.L. ET AL.: ’Emerging technologies in DNA sequencing’ GENOME RESEARCH vol. 15, no. 12, 2005, pages 1767 - 1776, XP002379405**
- **KULIKOWSKI T.: ’Structure-activity relationships and conformational features of antiherpetic pyrimidine and purine analogues’ PHARMACY WORLD & SCIENCE vol. 16, no. 2, 1994, pages 127 - 138, XP009038030**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

## TECHNICAL FIELD

The present invention relates generally to compounds and methods for DNA sequencing and other types of DNA analysis. More particularly, the invention relates to nucleotides and nucleosides labeled with photocleavable groups, nucleotides and nucleosides labeled with noncleavable groups and methods for their use in DNA sequencing and analysis.

## BACKGROUND ART

Methods for rapidly sequencing DNA have become needed for analyzing diseases and mutations in the population and developing therapies. The most commonly observed form of human sequence variation is single nucleotide polymorphisms (SNPs), which occur in approximately 1 -in-300 to 1 -in- 1000 base pairs of genomic sequence. Building upon the complete sequence of the human genome, efforts are underway to identify the underlying genetic link to common diseases by SNP mapping or direct association. Technology developments focused on rapid, high-throughput, and low cost DNA sequencing would facilitate the understanding and use of genetic information, such as SNPs, in applied medicine.

In general, 10%-to-15% of SNPs will affect protein function by altering specific amino acid residues, will affect the proper processing of genes by changing splicing mechanisms, or will affect the normal level of expression of the gene or protein by varying regulatory mechanisms. It is envisioned that the identification of informative SNPs will lead to more accurate diagnosis of inherited disease, better prognosis of risk susceptibilities, or identity of sporadic mutations in tissue. One application of an individual's SNP profile would be to significantly delay the onset or progression of disease with prophylactic drug therapies.

Moreover, an SNP profile of drug metabolizing genes could be used to prescribe a specific drug regimen to provide safer and more efficacious results. To accomplish these ambitious goals, genome sequencing will move into the resequencing phase with the potential of partial sequencing of a large majority of the population, which would involve sequencing specific regions or single base pairs in parallel, which are distributed throughout the human genome to obtain the SNP profile for a given complex disease.

Sequence variations underlying most common diseases are likely to involve multiple SNPs, which are dispersed throughout associated genes and exist in low frequency. Thus, DNA sequencing technologies that employ strategies for *de novo* sequencing are more likely to detect and/or discover these rare, widely dispersed variants than technologies targeting only known SNPs.

Traditionally, DNA sequencing has been accomplished by the "Sanger" or "dideoxy" method, which involves the chain termination of DNA synthesis by the incorporation of 2',3'-dideoxynucleotides (ddNTPs) using DNA polymerase (Sanger, F., Nicklen, S., and Coulson, A. R. (1977) DNA sequencing with chain-terminating inhibitors. Proc. Natl. Acad. Sci. USA 74, 5463-5467). The reaction also includes the natural 2'-deoxynucleotides (dNTPs), which extend the DNA chain by DNA synthesis. Balanced appropriately, competition between chain extension and chain termination results in the generation of a set of nested DNA fragments, which are uniformly distributed over thousands of bases and differ in size as base pair increments. Electrophoresis is used to resolve the nested DNA fragments by their respective size. The ratio of dNTP/ddNTP in the sequencing reaction determines the frequency of chain termination, and hence the distribution of lengths of terminated chains. The fragments are then detected *via* the prior attachment of four different fluorophores to the four bases of DNA (i.e., A, C, G, and T), which fluoresce their respective colors when irradiated with a suitable laser source. Currently, Sanger sequencing has been the most widely used method for discovery of SNPs by direct PCR sequencing (Gibbs, R. A., Nguyen, P.-N., McBride, L. J., Koepf, S. M., and Caskey, C. T. (1989) Identification of mutations leading to the Lesch-Nyhan syndrome by automated direct DNA sequencing of in vitro amplified cDNA. Pro. Natl. Acad. Sci. USA 86, 1919- 1923) or genomic sequencing (Hunkapiller, T., Kaiser, R. J., Koop, B. F. , and Hood, L. (1991) Large-scale and automated DNA sequencing Determination. Science 254, 59-67; International Human Genome Sequencing Consortium. Initial sequencing and analysis of the human genome. (2001) Nature 409, 860-921).

Another promising sequencing approach is cyclic reversible termination (CRT), which is a cyclic method of detecting the synchronistic, single base additions of multiple templates. This approach differentiates itself from the Sanger method (Metzker, M.L. (2005) Genome Rex 15, 1767-1776) in that it can be performed without the need for gel electrophoresis, a major bottleneck in advancing this field. Like Sanger sequencing, however, longer read-lengths translates into fewer sequencing assays needed to cover the entire genome.

It has remained difficult to accomplish the goal of long CRT reads because reversible terminators typically act as poor substrates with commercially available DNA polymerases. Reversible terminators are structured with a 3'-O-blocking group and a nucleobase attached fluorescent dye via a linking group. Both blocking and dye groups require removal prior to subsequent base additions. These nucleotide modifications are not well tolerated by DNA polymerases, which can be mutated by numerous strategies to improve enzymatic performance. Upon deprotection, the nucleobase

linker group is left behind, accumulating sequentially in the growing DNA duplex with subsequent CRT cycles. It is believed that poor enzyme kinetics and a sequentially modified DNA duplex limit longer read-lengths. The present invention describes, in part, novel, reversible nucleotide structures that require a single attachment of both terminating and fluorescent dye moieties, improving enzyme kinetics as well as deprotection efficiencies. These reversible terminators are incorporated efficiently by a number of commercially available DNA polymerases, with the deprotection step transforming the growing DNA duplex into its natural state.

DNA sequencing read-lengths of CRT technologies are governed by the overall efficiency of each nucleotide addition cycle. For example, if one considers the end-point of 50% of the original starting material as having an acceptable signal-to-noise ratio, the following equation can be applied to estimate the effect of the cycle's efficiency on read-length: $(RL)^{Ceff} = 0.5$, where RL is the read-length in bases and *Ceff* is the overall cycle efficiency. In other words, a read-length of 7 bases could be achieved with an overall cycle efficiency of 90%, 70 bases could be achieved with a cycle efficiency of 99% and 700 bases with a cycle efficiency of 99.9%. To achieve the goal of sequencing large stretches, the method must provide very high cycle efficiency or the recovery may fall below acceptable signal to noise ratios. Reversible terminators that exhibit higher incorporation and deprotection efficiencies will achieve higher cycle efficiencies, and thus longer read-lengths.

For CRT terminators to function properly, the protecting group must be efficiently cleaved under mild conditions. The removal of a protecting group generally involves either treatment with strong acid or base, catalytic or chemical reduction, or a combination of these methods. These conditions may be reactive to the DNA polymerase, nucleotides, oligonucleotide-primed template, or the solid support creating undesirable outcomes. The use of photochemical protecting groups is an attractive alternative to rigorous chemical treatment and can be employed in a non-invasive manner.

A number of photoremovable protecting groups including 2-nitrobenzyl, benzyloxycarbonyl, 3-nitrophenyl, phenacyl, 3,5-dimethoxybenzoinyl, 2,4-dinitrobenzenesulphenyl, and their respective derivatives have been used for the syntheses of peptides, polysaccharides, and nucleotides (Pillai, V. N. R. (1980) Photoremovable Protecting Groups in Organic Synthesis. Synthesis, 1-26). Of these, the light sensitive 2-nitrobenzyl protecting group has been successfully applied to the 2'-OH of ribonucleosides for diribonucleoside synthesis (Ohtsuka, E., Tanaka, S., and Ikehara, M. (1974) Studies on transfer ribonucleic acids and related compounds. IX(1) Ribooligonucleotide synthesis using a photosensitive o-nitrobenzyl protection at the 2'-hydroxyl group. Nucleic Acids Res. 1, 1351-1357), the 2'-OH of ribophosphoramidites in automated ribozyme synthesis (Chaulk, S. G., and MacMillan, A. M. (1998) Caged RNA: photo-control of a ribozyme. Nucleic Acids Res. 26, 3173-3178), the 3'-OH of phosphoramidites for oligonucleotide synthesis in the Affymetrix chemistry (Pease, A. C., Solas, D., Sullivan, E. J., Cronin, M. T., Holmes, C. P., and Fodor, S. P. A. (1994) Light-generated oligonucleotide arrays for rapid DNA sequence analysis. Proc. Natl. Acad. Sci. USA 91, 5022-5026), and to the 3'-OH group for DNA sequencing applications (Metzker, M. L., Raghavachari, R., Richards, S., Jacutin, S. E., Civitello, A., Burgess, K., and Gibbs, R. A. (1994) Termination of DNA synthesis by novel 3'-modified deoxyribonucleoside triphosphates. Nucleic Acids Res. 22, 4259-4267). Under deprotection conditions (ultraviolet light >300 nm), the 2-nitrobenzyl group can be efficiently cleaved without affecting either the pyrimidine or purine bases (Pease, A. C., Solas, D., Sullivan, E. J., Cronin, M. T., Holmes, C. P., and Fodor, S. P. A. (1994) Light-generated oligonucleotide arrays for rapid DNA sequence analysis. Proc. Natl. Acad. Sci. USA 91, 5022-5026) and (Bartholomew, D. G., and Broom, A. D. (1975) One-step Chemical Synthesis of Ribonucleosides bearing a Photolabile Ether Protecting Group. J. Chem. Soc. Chem. Commun., 38).

The need for developing new sequencing technologies has never been greater than today with applications spanning diverse research sectors including comparative genomics and evolution, forensics, epidemiology, and applied medicine for diagnostics and therapeutics. Current sequencing technologies are too expensive, labor intensive, and time consuming for broad application in human sequence variation studies. Genome center cost is calculated on the basis of dollars per 1,000 $Q_{20}$ bases and can be generally divided into the categories of instrumentation, personnel, reagents and materials, and overhead expenses. Currently, these centers are operating at less than one dollar per 1,000 $Q_{20}$ bases with at least 50% of the cost resulting from DNA sequencing instrumentation alone. Developments in novel detection methods, miniaturization in instrumentation, microfluidic separation technologies, and an increase in the number of assays per run will most likely have the biggest impact on reducing cost.

It is therefore an object of the invention to provide novel compounds that are useful in efficient sequencing of genomic information in high throughput sequencing reactions.

It is another object of the invention to provide novel reagents and combinations of reagents that can efficiently and affordably provide genomic information.

Described are libraries and arrays of reagents for diagnostic methods and for developing targeted therapeutics for individuals.

**DISCLOSURE OF INVENTION**

SUMMARY

PHOTOCLEAVABLE LABELED NUCLEOTIDES AND NUCLEOSIDES

Provided are nucleoside compounds as well as phosphates and salts thereof, that can be used in DNA sequencing technology. The compounds are optionally in the form of ribonucleoside triphosphate (NTP) and deoxyribonucleoside triphosphate (dNTP) compounds. The nucleotide and nucleoside compounds include a photocleavable group lableled with a fluorescent dye. The nucleotide and nucleoside compounds containing photocleavable protecting groups are designed to terminate DNA synthesis and then be cleave efficiently, so that nucleic acid oligomers can be sequenced rapidly in a parallel format. The presence of such cleavable groups lableled with fluorescent dyes on the nucleotide and nucleoside compounds can enhance the speed and accuracy of sequencing of large oligomers of DNA in parallel, to allow, for example, rapid whole genome sequencing, and the identification of polymorphisms and other valuable genetic information.

A variety of nucleotide and nucleoside compounds, containing the nucleobases adenine, cytosine, guanine, thymine, uracil, or naturally occurring derivatives thereof, are provided that include cleavable groups and/or which can be derivatized to include a detectable label such as a dye.

In one embodiment the base of the nucleoside is covalently attached with a 2-nitrobenzyl group, and the alpha carbon position of the 2-nitrobenzyl group is optionally substituted with one alkyl or aryl group as described herein. The 2-nitrobenzyl group can be functionalized to enhance the termination properties as well as the light catalyzed deprotection rate. The termination properties of the 2-nitrobenzyl, or alpha carbon substituted 2-nitrobenzyl group, attached to the nucleobase occur even when the 3'-OH group on the ribose sugar is unblocked. These 3'-OH unblocked terminators are well-tolerated by a number of commercially available DNA polymerases, representing a key advantage over 3'-O-blocked terminators. The alpha carbon substituted 2-nitrobenzyl group also can be derivatized to include a selected fluorescent dye.

In one embodiment the base of the nucleoside is covalently attached with a 2-nitrobenzyl group, and the 2-nitrobenzyl group is optionally substituted with one or more of an electron donating and electron withdrawing group as described herein. The 2-nitrobenzyl group can be functionalized to enhance the light catalyzed deprotection rate. The 2-nitrobenzyl group also can be derivatized to include a detectable fluorescent dye.

In particular, methods for DNA sequencing are provided using combinations of the four nucleoside triphosphate compounds, modified with 2-nitrobenzyl groups, and derivatives described herein and labeled with distinct fluorescent dyes, which could be used for identifying the incorporated bases to reveal the underlying DNA sequence.

LABELED NUCLEOTIDES AND NUCLEOSIDES

Also provided are nucleoside compounds as well as phosphates and salts thereof, that can be used in DNA sequencing technology. The compounds are optionally in the form of ribonucleoside triphosphate (NTP) and deoxyribonucleoside triphosphate (dNTP) compounds. The nucleotide and nucleoside compounds include a noncleavable group labeled with a fluorescent dye. The nucleotide and nucleoside compounds are designed to terminate DNA synthesis, so that nucleic acid oligomers can be sequenced rapidly in a parallel format. A variety of nucleotide and nucleoside compounds, containing the nucleobases adenine, cytosine, guanine, thymine, uracil, or naturally occurring derivatives thereof, are provided that can be derivatized to include a detectable label such as a dye.

In one embodiment the base of the nucleoside is covalently attached with a benzyl group, and the alpha carbon position of the benzyl group is optionally substituted with one alkyl or aryl group as described herein. The benzyl group can be functionalized to enhance the termination properties. The termination properties of the optionally alpha carbon substituted benzyl group attached to the nucleobase occur even when the 3'-OH group on the ribose sugar is unblocked. These 3'-OH unblocked terminators are well-tolerated by a number of commercially available DNA polymerases, representing a key advantage over 3'-O-blocked terminators. The linker group also can be derivatized to include a selected fluorescent dye.

In particular, methods for DNA sequencing are provided using combinations of the four nucleoside triphosphate compounds, modified with a non-cleavable terminating group, and derivatives described herein and labeled with distinct fluorescent dyes, which could be used for identifying the incorporated bases to reveal the underlying DNA sequence.

DETAILED DESCRIPTION

PHOTOCLEAVABLE LABELED NUCLEOTIDES AND NUCLEOSIDES

Provided are nucleotide and nucleoside compounds as well as salts, esters and phosphates thereof, that can be used in rapid DNA sequencing technology. The compounds are optionally in the form of ribonucleoside triphosphates (NTPs) and deoxyribonucleoside triphosphates (dNTP). The nucleotide and nucleoside compounds in one embodiment includes a photocleavable group labeled with a fluorescent dye. The nucleotide and nucleoside compounds include photoremovable protecting groups that are designed to terminate DNA synthesis as well as cleave rapidly, so that these monomers can be used for rapid sequencing in a parallel format. The presence of such rapidly cleavable groups, labeled with fluorescent dyes, on the nucleotide and nucleoside compounds can enhance the speed and accuracy of sequencing of large oligomers of DNA in parallel, to allow, for example, rapid whole genome sequencing, and the identification of polymorphisms and other valuable genetic information.

A variety of nucleotide and nucleoside compounds, containing the nucleobases adenine, cytosine, guanine, thymine, uracil, or naturally occurring derivatives thereof, are provided that include cleavable groups andlor which can be derivatized to include a detectable label such as a dye.

In one embodiment, the nucleobases adenine, cytosine, guanine, thymine, uracil, or naturally occurring derivatives thereof, can be covalently attached to a photoremovable protecting group such as a 2-nitrobenzyl group. The 2-nitrobenzyl group can be derivatized to enhance its termination of DNA synthesis as well as the deprotection rate, thus increasing its usefulness in DNA sequencing. The photoremovable protecting group, such as a 2-nitrobenzyl group, also can be derivatized with a fluorescent dye by covalent linkage to the photoremovable protecting group.

I. Advantages of Photocleavable Labeled Nucleotide and Nucleoside Compounds for Sequencing

Nucleotide and nucleoside compounds are provided which are useful in DNA sequencing technology. Cyclic reversible termination (CRT) is a cyclic method of detecting the synchronous, single base additions of multiple templates. This approach differentiates itself from the Sanger method in that it can be performed without the need for gel electrophoresis and highly-parallel format, which are major bottlenecks in advancing this field. Like Sanger sequencing, however, longer read-lengths translates into fewer sequencing assays needed to cover the entire genome. The CRT cycle comprises three steps, incorporation, imaging, and deprotection. For this procedure, cycle efficiency, cycle time, and sensitivity are important factors. The cycle efficiency is the product of deprotection and incorporation efficiencies and determines the CRT read-length. The CRT cycle time is the sum of incorporation, imaging, and deprotection times. For rapid CRT for whole genome sequencing, the nucleotide and nucleoside compounds as disclosed herein may be used, which can exhibit fast and efficient deprotection properties. These compounds can be labeled with fluorescent dyes, attached directly to a 2-nitrobenzyl, providing fluorescent, reversible terminators with similar deprotection properties. The read-length of CRT technologies are governed by the overall efficiency of each nucleotide addition cycle, product of deprotection and incorporation efficiencies. For example, if one considers the end-point of 50% of the original starting material as having an acceptable signal-to-noise ratio, the following equation can be applied to estimate the effect of the cycle's efficiency on read-length:

$$(\mathrm{RL})^{Ceff} = 0.5$$

where RL is the read-length in bases and *Ceff* is the overall cycle efficiency. In other words, a read-length of 7 bases could be achieved with an overall cycle efficiency of 90%, 70 bases could be achieved with a cycle efficiency of 99% and 700 bases with a cycle efficiency of 99.9%. The efficiency of incorporation of compounds according to the invention may range from about 70% to about 100% of the incorporation of the analogous native nucleoside. Preferably, the efficiency of incorporation will range from about 85% to about 100%. Photocleavage efficiencies will preferably range from about 85% to about 100%. Further, termination of nucleic acid extension will range from about 90% to about 100% upon incorporation of compounds according to the invention. Nucleotide and nucleoside compounds in one embodiment have a cycle efficiency of at least 80%, 90%, 91%, 92%, 93%, 94%, 95%, 96%, 97%, 98%, 99%, 99.1%, 99.2%, 99.3%, 99.4%, 99.5%, 99.6%, 99.7%, 99.8%, or 99.9%. When applied to genomic DNA, the compounds can be used in CRT to read directly from genomic DNA. Fragmented genomic DNA can be hybridized to a high-density oligonucleotide chip containing priming sites that span selected chromosomes. Each priming sequence is separated by the estimated read-length of the CRT method. Between base additions, a fluorescent imager can simultaneously image the entire high-density chip, marking significant improvements in speed and sensitivity. The fluorophore, which is attached to the 2-nitrobenzyl group or its derivatives described herein, is removed by uv irradiation releasing the 2-nitrobenzyl group for

the next round of base addition. After approximately 500 CRT cycles, the complete and contiguous genome sequence information can then be compared to the reference human genome to determine the extent and type of sequence variation in an individual's sample.

II. Photocleavable Labeled Nucleotide And Nucleoside Compounds

A variety of nucleosides and compounds as well as their mono, di and triphosphates are provided. The compounds are useful for DNA sequencing technology. In one embodiment, the nucleotide and nucleoside compounds include a photocleavable terminating group labeled with a fluorescent dye that can be detected and efficiently cleaved in CRT reactions. The nucleotide and nucleoside compounds can be converted into their respective natural nucleoside monophosphates for subsequent rounds of DNA polymerase reactions. In a particular embodiment, a nucleotide and nucleoside compounds are provided comprising a deoxyribose or ribose sugar and a base, wherein the base is covalently linked to a photocleavable terminating, 2-nitrobenzyl group. The 2-nitrobenzyl group can be substituted with groups that increase termination of DNA synthesis as well as the rate of deprotection. In addition, the 2-nitrobenzyl group can be detectable by attaching a reporter group, such as a dye. The dye may be optionally linked to 2-nitrobenzyl group by a bifunctional linker. Compounds according to the invention may be represented by the following formula:

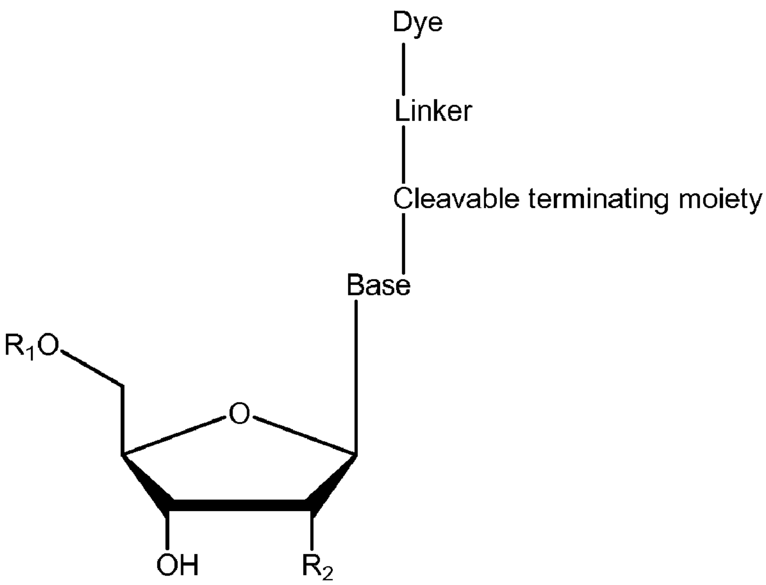

wherein the compound is selected from the group consisting of the compounds represented by formulas I-VII. Compounds according to the invention can be designed as fluorescent, photolabile reversible terminators useful in DNA synthesis sequencing. The compounds can be optimized reversible terminators, modified to have fast and efficient deprotection behavior and good fluorescent properties in aqueous solutions. A compound is provided having a structure of formulas I-VII:

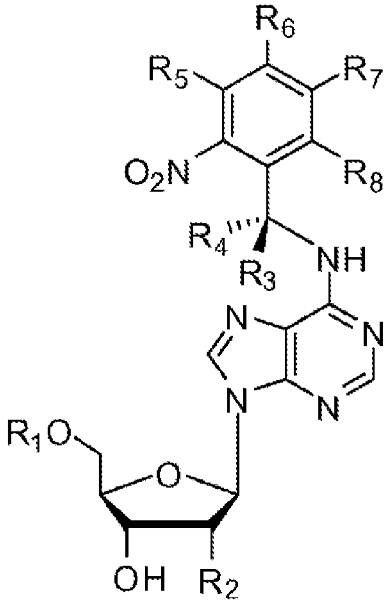

formula I

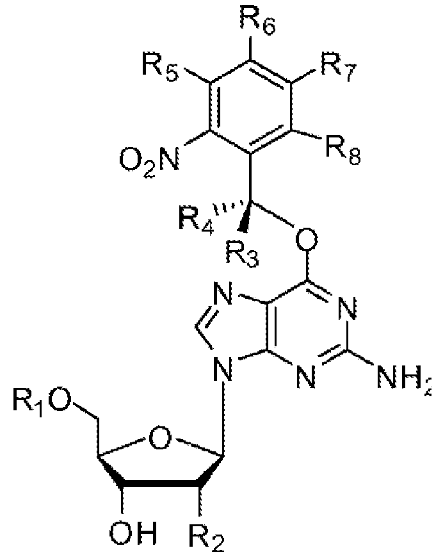

formula II

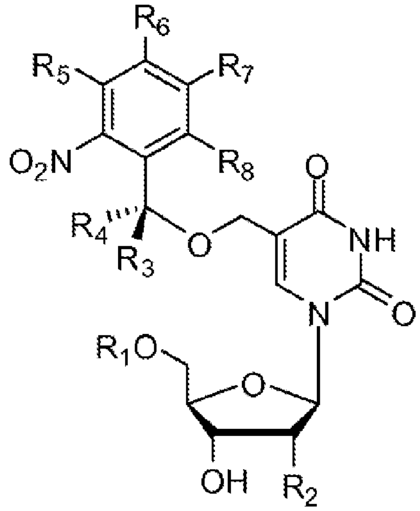

formula III

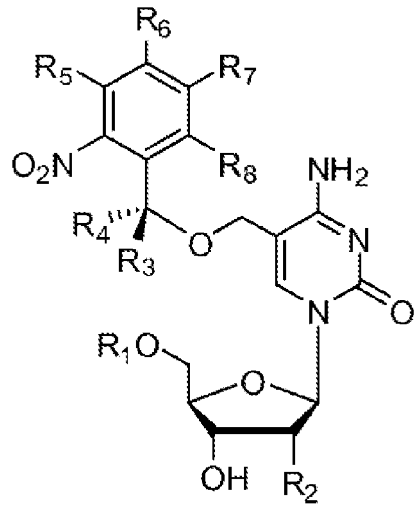

formula IV

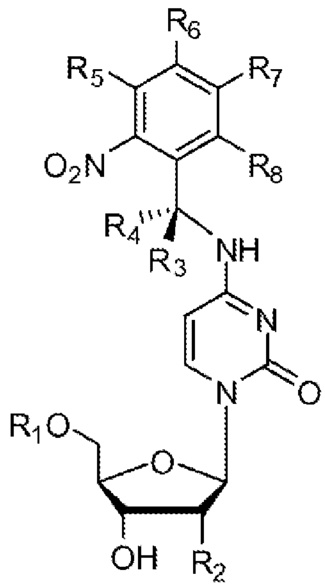

formula V

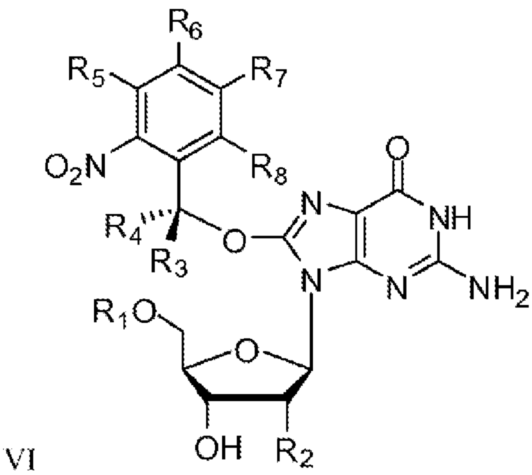

formula VI

or

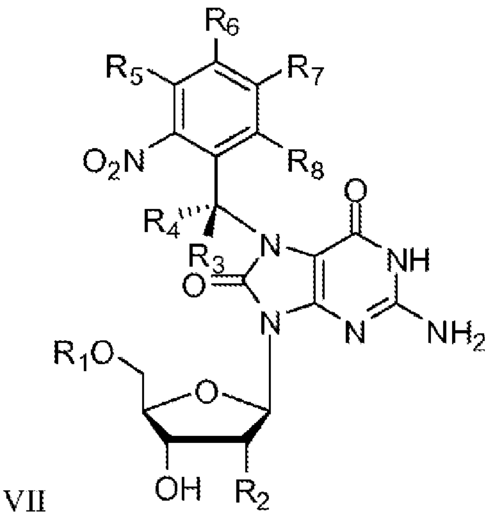

formula VII

wherein $R_1$ = H, monophosphate, diphosphate or triphosphate, $R_2$ = H or OH, $R_3$ and $R_4$ are each independently selected from the group of H, a $C_1$-$C_{12}$ straight chain or branched alkyl, a $C_2$-$C_{12}$ straight chain or branched alkenyl or polyenyl, a C2-C12 straight chain or branched alkynyl or polyalkynyl, and an aromatic group such as a phenyl, naphthyl, or pyridine ring, with the proviso that at least one of $R_3$ and $R_4$ is H; $R_5$, $R_6$, $R_7$, and $R_8$ are each independently selected from the group H, $OCH_3$, $NO_2$, CN, a halide, a $C_1$-$C_{12}$ straight chain or branched alkyl, a $C_2$-$C_{12}$ straight chain or branched alkenyl or polyenyl, a $C_2$-$C_{12}$ straight chain or branched alkynyl or polyalkynyl an aromatic group such as a phenyl, naphthyl, or pyridine ring, and/or a linker group of the general structure:

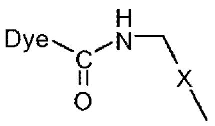

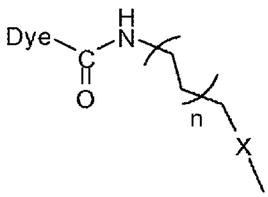

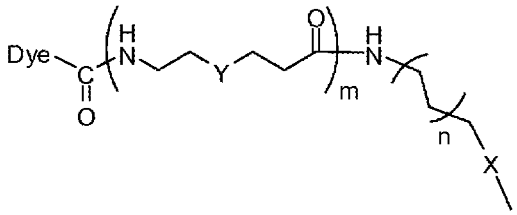

X = $CH_2$, CH=CH, C≡C, O, S, or NH, Y = $CH_2$, O, or NH, n = an integer from 0-12; m = an integer from 0-12, and Dye = a fluorophore, or pharmaceutically acceptable salt or ester thereof or enantiomer, racemic mixture, or stereoisomer thereof. In a particular embodiment, in the compounds provided herein comprising a derivatized 2-nitrophenyl ring, such as the compounds of formulas I-VII, the rate of deprotection and removal of the 2-nitrophenyl group during DNA sequencing can be enhanced by including an electron donating group at the 4-position or an electron withdrawing group at the 5-position of the 2-nitrophenyl ring.

In a preferred embodiment, $R_3$ and $R_4$ are selected from the group consisting of, but not limited to, $-CH_3$, $-CH_2CH_3$, $-CH_2CH_2CH_3$, isopropyl, tert-butyl, phenyl, 2-nitrophenyl, and 2,6-dinitrophenyl. Alternatively, $R_3$ and $R_4$ are selected from the group consisting of, but not limited to, alkyl and aromatic groups optionally containing at least one heteroatom in the alkyl or aromatic groups, and further wherein the aromatic group may optionally be an aryl such as phenyl or polycyclic such as a naphthyl group. In certain embodiments, $R_5$, $R_6$, $R_7$, and $R_8$ are selected from an aromatic group consisting of aryl and polycyclic groups.

Alternatively, photocleavable terminating moieties may have the following general structures:

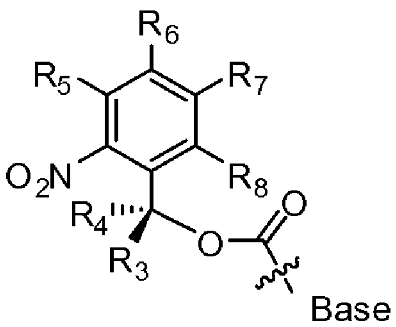

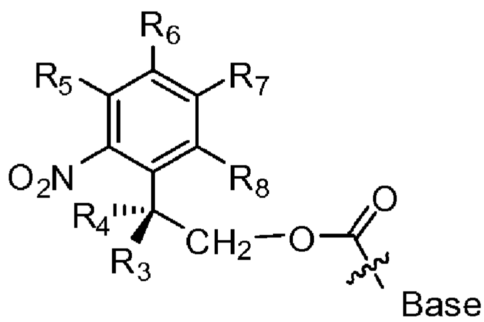

For example, compounds with such photocleavable terminating moieties could have the following structures:

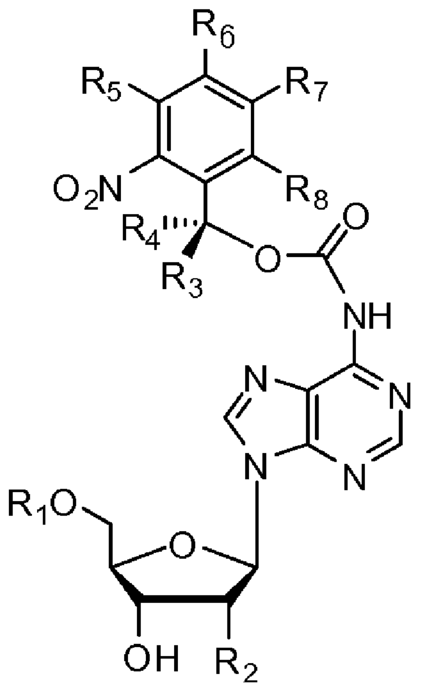

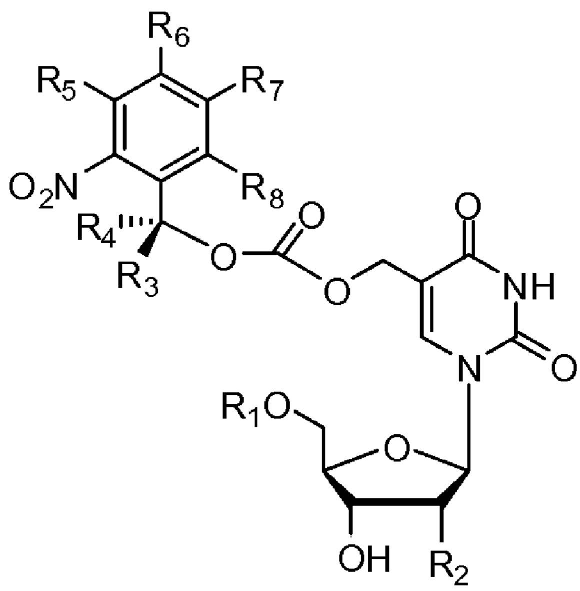

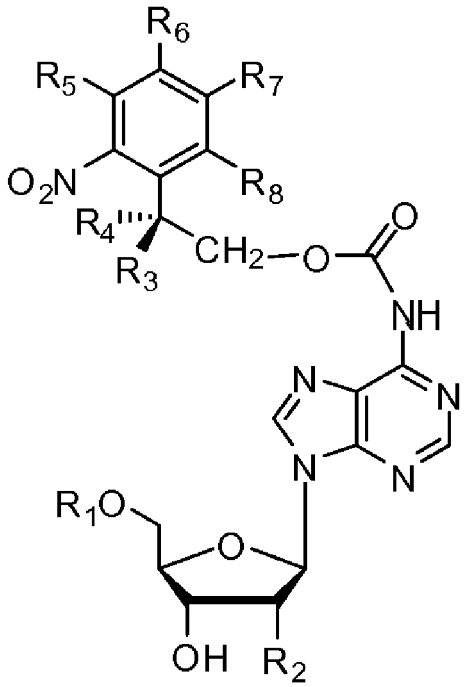

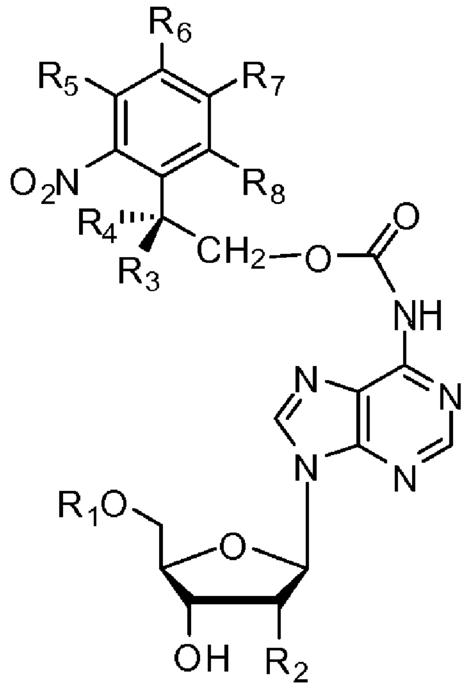

wherein the cleavable terminating moiety is attached to the base through a linkage selected from the group consisting of benzyl amine, benzyl ether, carbamate, carbonate, 2-(o-nitrophenyl)ethyl carbamate, and 2-(o-nitrophenyl)ethyl carbonate.

Fluorescent dyes are not particularly limited. For example, the fluorophore may be selected from the group consisting of, but not limited to, BODIPY, fluorescein, rhodamine, coumarin, xanthene, cyanine, pyrene, phthalocyanine, phycobiliprotein, alexa, squarene dye, combinations resulting in energy transfer dyes, and derivatives thereof.

Preferred embodiments include but are not limited to the following compounds:

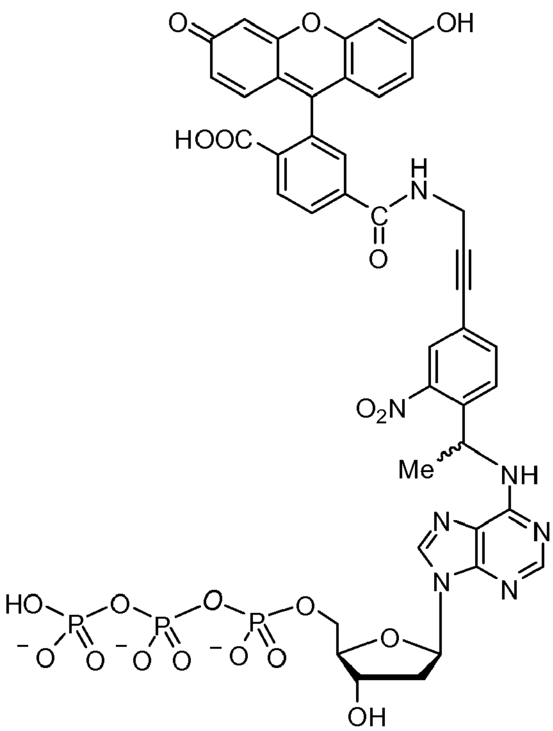

,

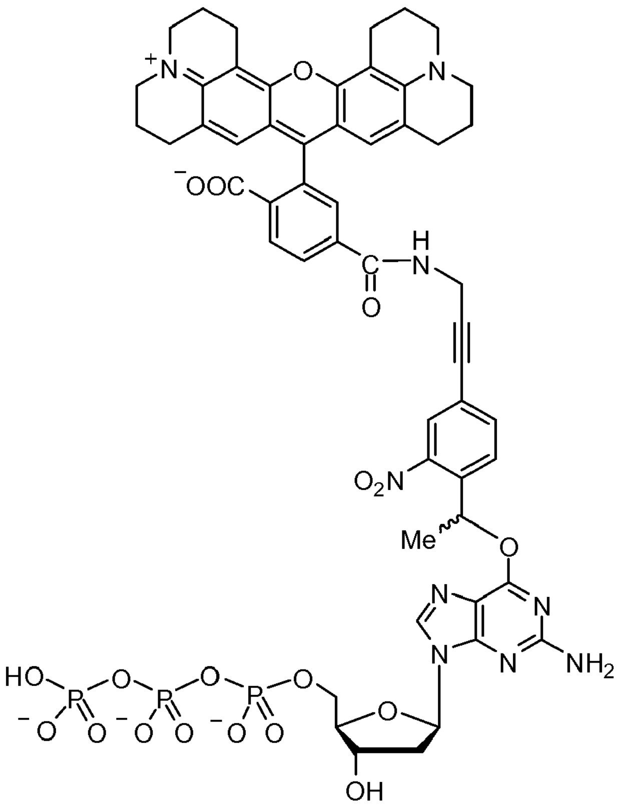

,

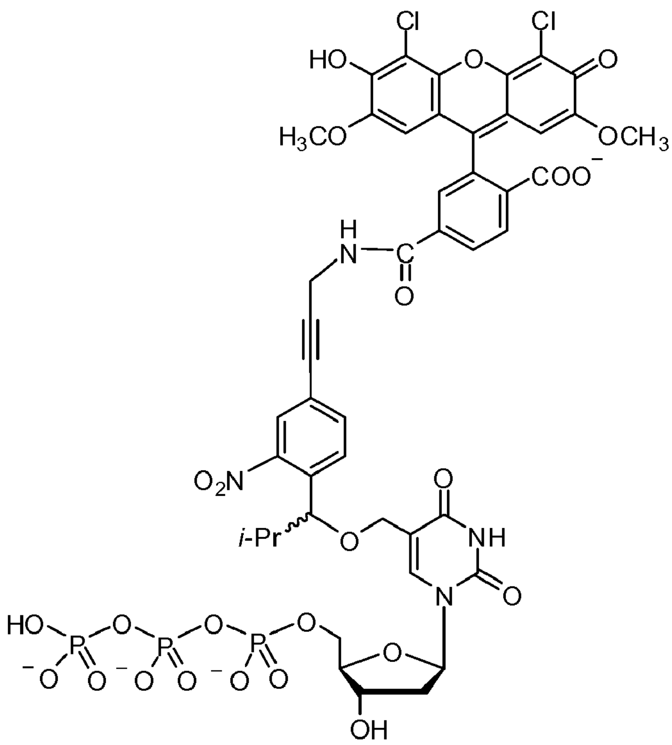

,

and

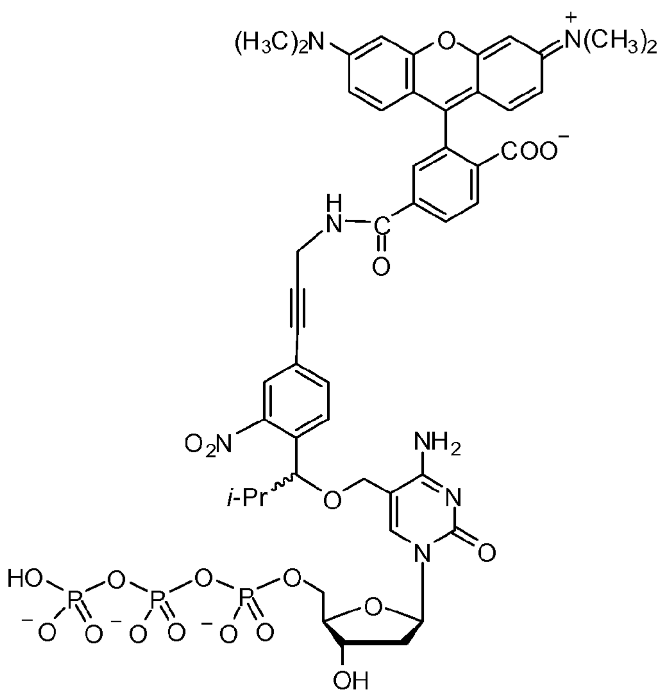

III. Synthesis of Photocleavable Nucleotide and Nucleoside Compounds

The compounds disclosed herein can be synthesized generally as disclosed herein, and using methods available in the art. For example, the following general scheme represents the synthesis of an adenosine compound:

*General Scheme for synthesis of an Adenosine N6-modified compound*

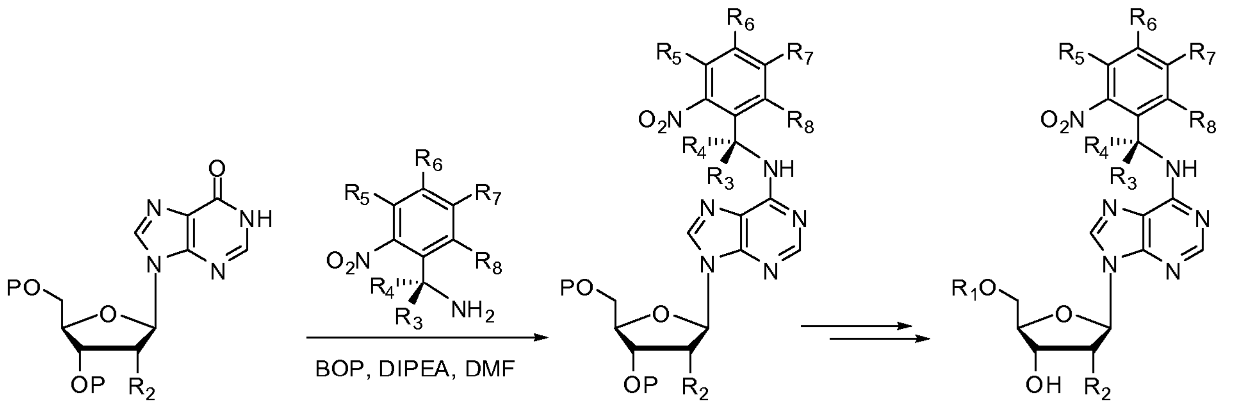


P denotes a suitable protecting group
$R_2$ = H or OH group with a suitable protecting group

$R_1$ to $R_8$ has the same definition as defined for the general structure

*General Scheme for synthesis Guanosine O6-modified compounds*

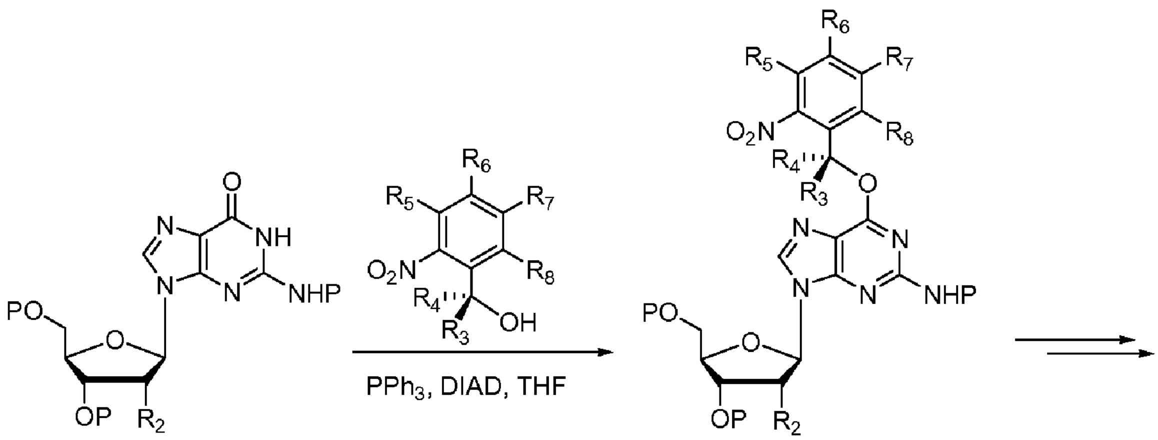


P denotes a suitable protecting group
$R_2$ = H or OH group with a suitable protecting group

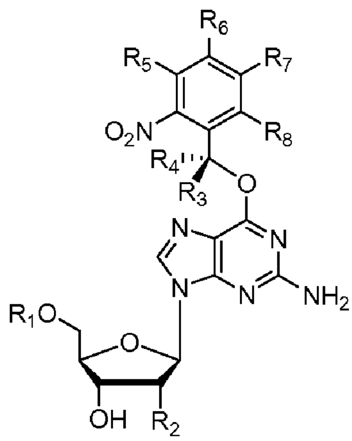

$R_1$ to $R_8$ has the same definition as defined for the general structure

*General Scheme for synthesis Guanosine 8-Oxo-modified compounds*

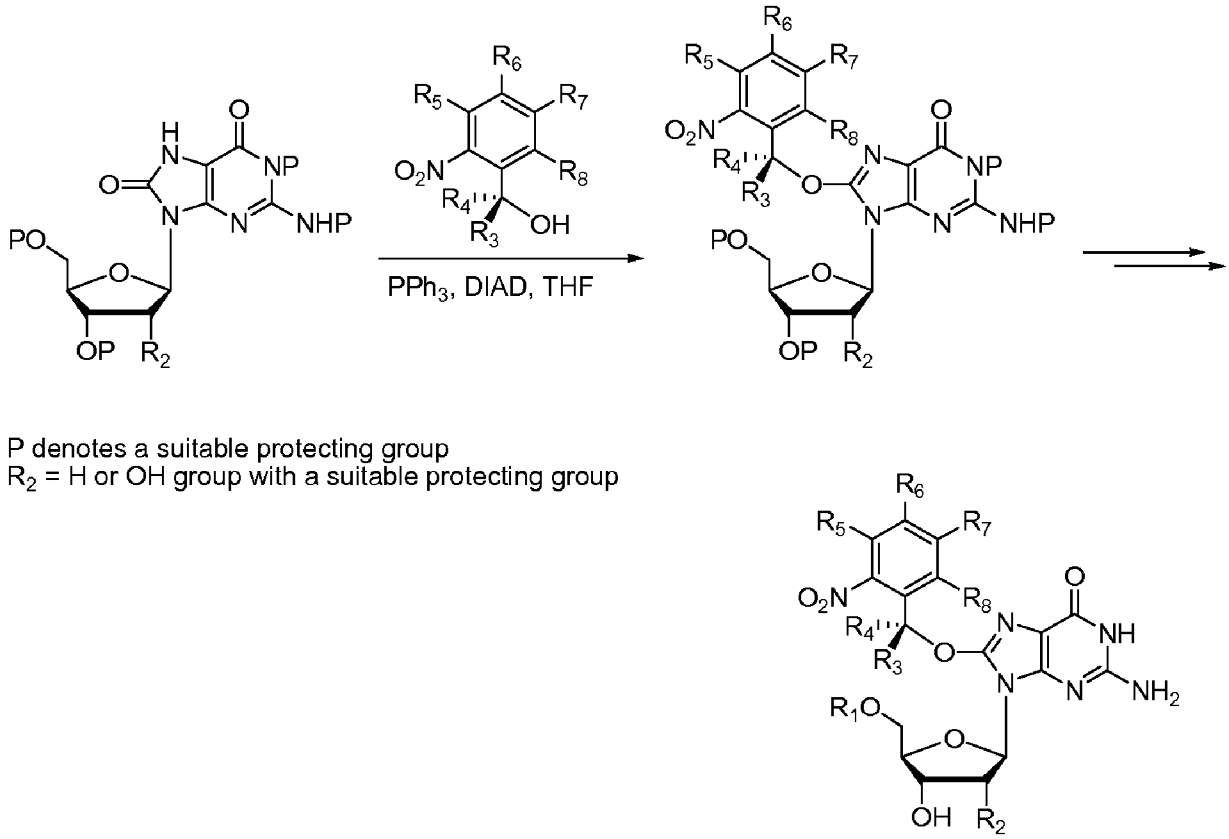


P denotes a suitable protecting group
$R_2$ = H or OH group with a suitable protecting group

$R_1$ to $R_8$ has the same definition as defined for the general structure

*General Scheme for synthesis Uridine 5-HOMe-modified compounds*

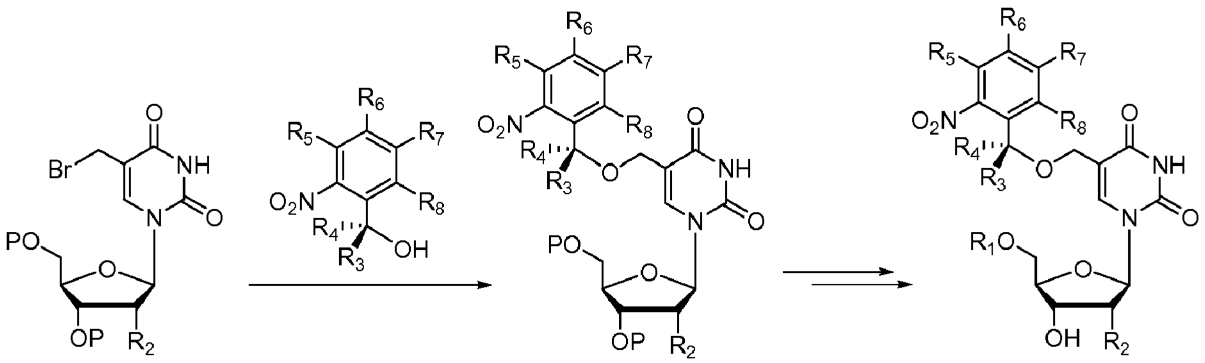

P denotes a suitable protecting group
$R_2$ = H or OH group with a suitable protecting group

$R_1$ to $R_8$ has the same definition as defined for the general structure

*General Scheme for synthesis Cytidine 5-HOMe-modified compounds*

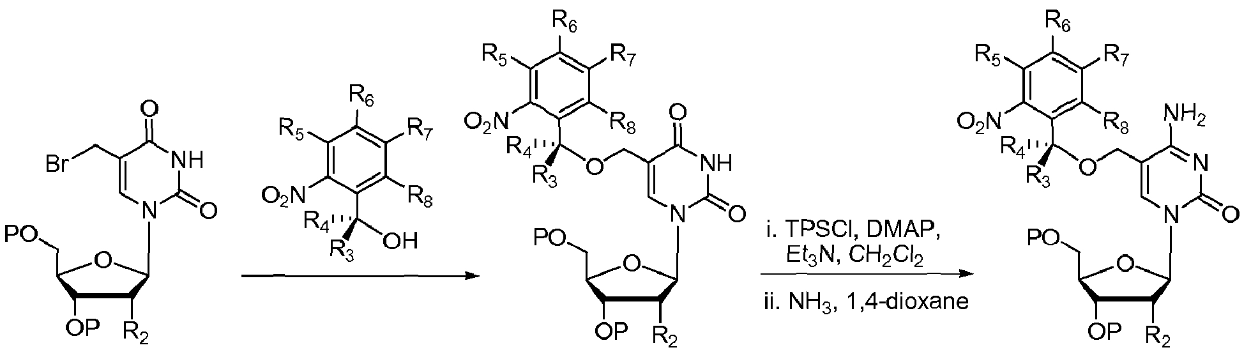


P denotes a suitable protecting group
$R_2$ = H or OH group with a suitable protecting group

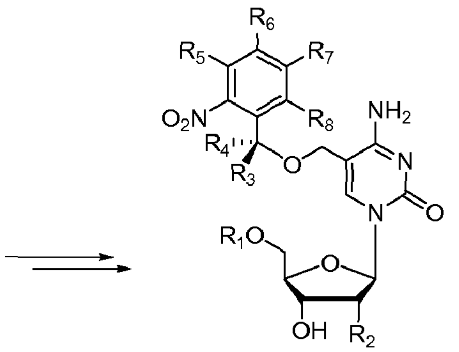

$R_1$ to $R_8$ has the same definition as defined for the general structure

General Scheme for synthesis Cytidine *N*4-modified compounds

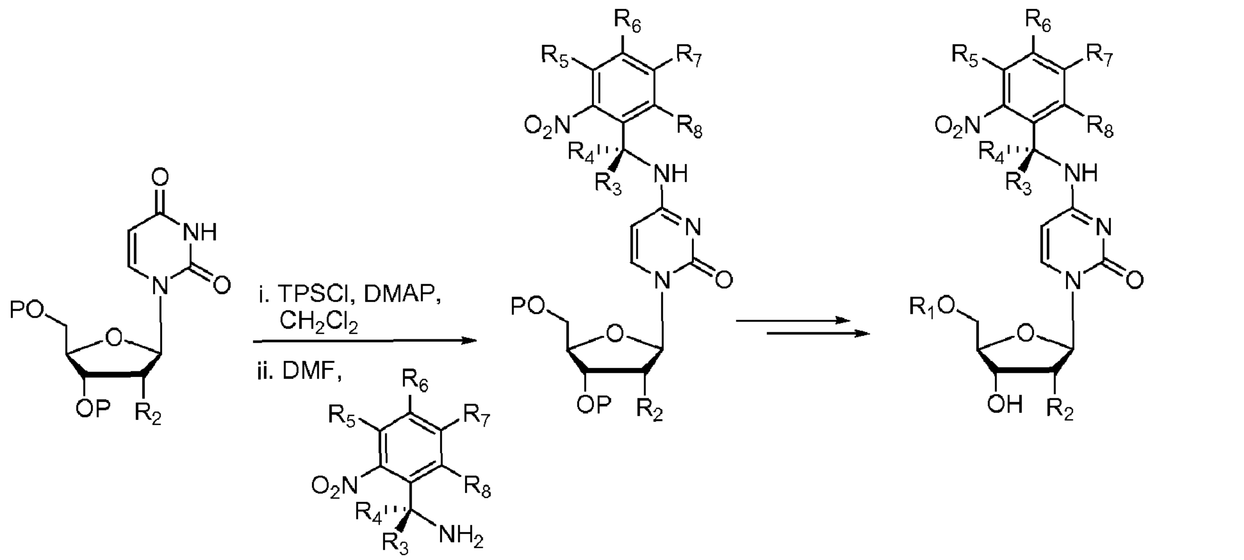


P denotes a suitable protecting group
$R_2$ = H or OH group with a suitable protecting group

$R_1$ to $R_8$ has the same definition as defined for the general structure

Additional details are provided in the Examples section.

LABELED NUCLEOTIDES AND NUCLEOSIDES

Provided are nucleotide and nucleoside compounds as well as salts, esters and phosphates thereof, that can be used in rapid DNA sequencing technology. The compounds are optionally in the form of ribonucleoside triphosphates (NTPs) and deoxyribonucleoside triphosphates (dNTP). The nucleotide and nucleoside compounds in one embodiment includes a non-cleavable group labeled with a fluorescent dye. The nucleotide and nucleoside compounds are designed to terminate DNA synthesis, so that these monomers can be used for rapid sequencing in a parallel format. The presence of such groups labeled with fluorescent dyes on the nucleotide and nucleoside compounds can enhance the speed and accuracy of sequencing of large oligomers of DNA in parallel, to allow, for example, rapid whole genome sequencing, and the identification of polymorphisms and other valuable genetic information.

A variety of nucleotide and nucleoside compounds, containing the nucleobases adenine, cytosine, guanine, thymine, uracil, or naturally occurring derivatives thereof, are provided that include non-cleavable terminating moieties and/or which can be derivatized to include a detectable label such as a dye.

In one embodiment, the nucleobases adenine, cytosine, guanine, thymine, uracil, or naturally occurring derivatives thereof, can be covalently attached to a dye via a non-cleavable terminating moiety. The non-cleavable terminating moiety can be derivatized to enhance its termination of DNA synthesis thus increasing its usefulness in DNA sequencing.

I. Advantages of Labeled Nucleotide And Nucleoside Compounds for Sequencing

Nucleotide and nucleoside compounds are provided which are useful in DNA sequencing technology. The efficiency of incorporation of compounds according to the invention may range from about 70% to about 100% of the incorporation of the analogous native nucleoside. Preferably, the efficiency of incorporation will range from about 85% to about 100%. Further, termination of nucleic acid extension will range from about 90% to about 100% upon incorporation of compounds according to the invention. Nucleotide and nucleoside compounds in one embodiment have a termination efficiency of at least 90%, 91%, 92%, 93%, 94%, 95%, 96%, 97%, 98%, 99%, 99.1%, 99.2%, 99.3%, 99.4%, 99.5%, 99.6%, 99.7%, 99.8%, or 99.9%.

II. Labeled Nucleotide And Nucleoside Compounds

A variety of nucleosides and compounds as well as their mono, di and triphosphates are provided. The compounds are useful for sequencing technology. In one embodiment, the nucleoside compound includes a fluorescent group that can be detected efficiently. The nucleoside compounds can be converted into their respective triphosphates for DNA polymerase reactions. Compounds according to the invention may be represented by the following formula:

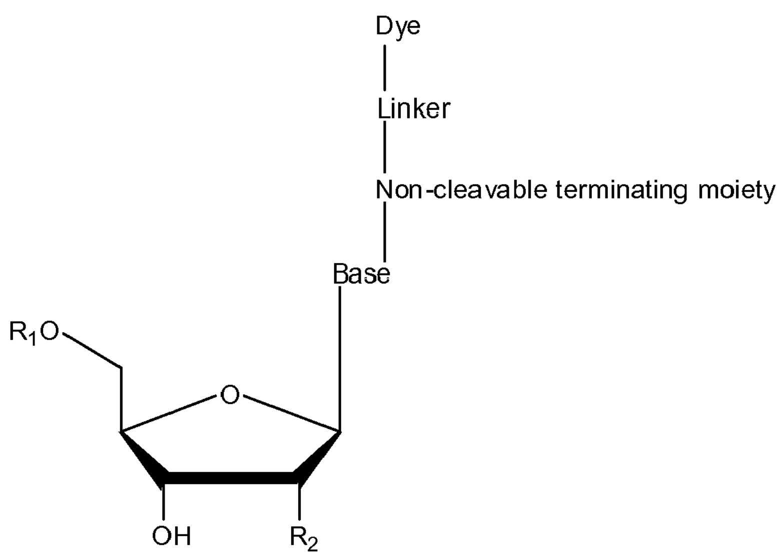

wherein the compound is selected from the group consisting of the compounds represented by formulas VIII-XIV. . Compounds according to the invention can be designed as fluorescent, non-labile nonreversible terminators useful in DNA synthesis sequencing.

A compound is provided having a structure of formulas VIII-XIV:

formula VIII

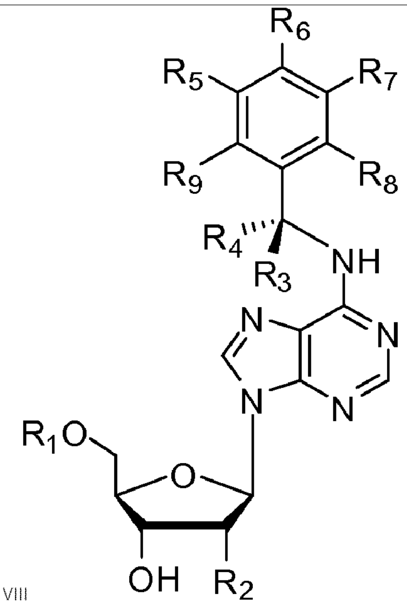

formula IX

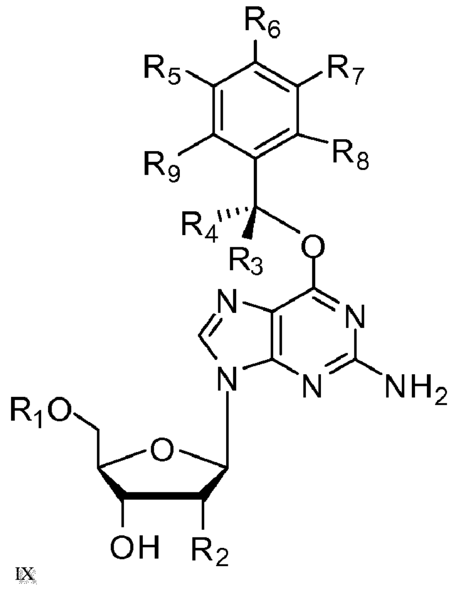

formula X

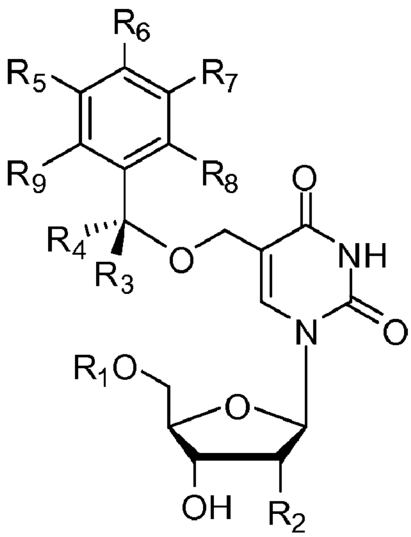

formula XI

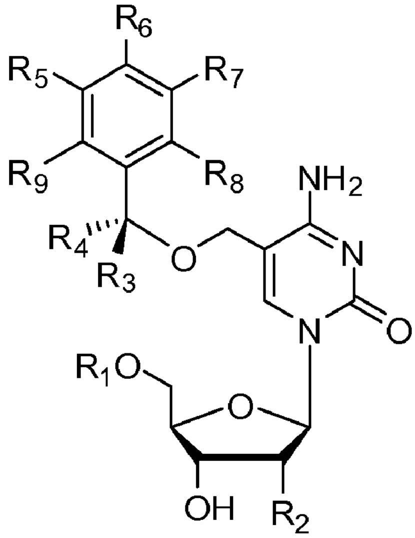

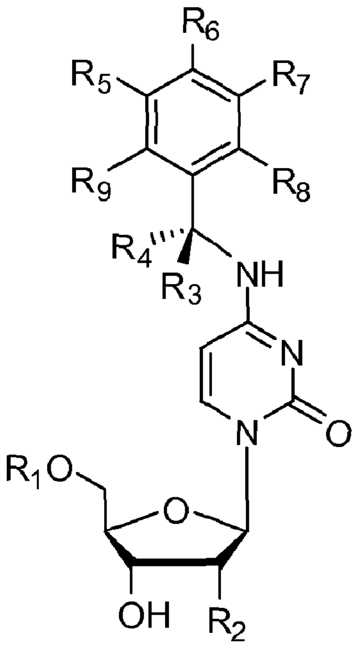

formula XII

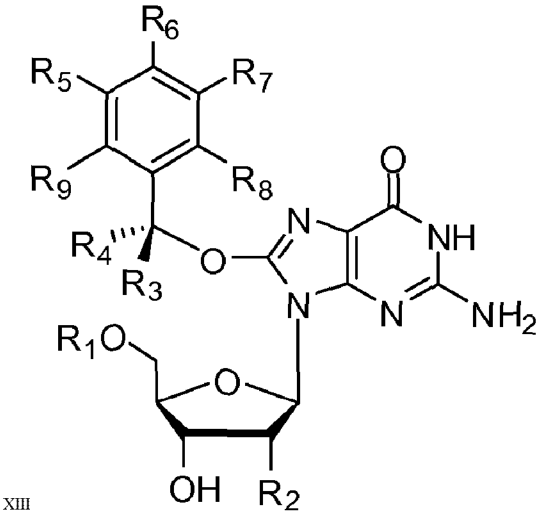

formula XIII

or

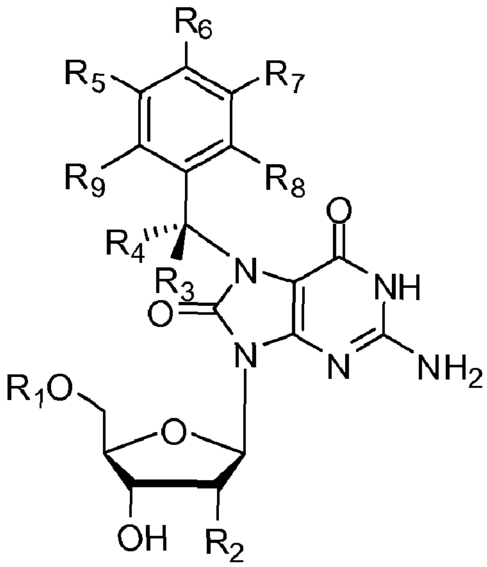

formula XIV

wherein $R_1$ = H, monophosphate, diphosphate or triphophosphate, $R_2$ = H or OH, $R_3$ and $R_4$ are each independently selected from the group of H, a $C_1$-$C_{12}$ straight chain or branched alkyl, a $C_2$-$C_{12}$ straight chain or branched alkenyl or polyenyl, a $C_2$-$C_{12}$ straight chain or branched alkynyl or polyalkynyl, and an aromatic group such as a phenyl, naphthyl, or pyridine ring; $R_5$, $R_6$, and $R_7$, are each independently selected from the group H, $OCH_3$, $NO_2$, CN, a halide, a $C_1$-$C_{12}$ straight chain or branched alkyl, a $C_2$-$C_{12}$ straight chain or branched alkenyl or polyenyl, a $C_2$-$C_{12}$ straight chain or branched alkynyl or polyalkynyl, an aromatic group such as a phenyl, naphthyl, or pyridine ring, and/or a linker group of the general structure:

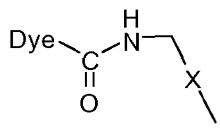

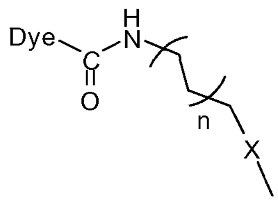

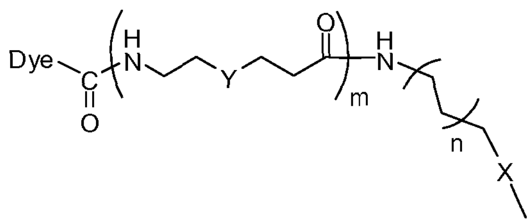

X = $CH_2$, CH=CH, C≡C, O, S, or NH, Y = $CH_2$, O, or NH, n = an integer from 0-12; m = an integer from 0-12, and Dye = a fluorophore; and $R_8$ and $R_9$ are as defined above for $R_5$, $R_6$, and $R_7$, with the proviso that $R_8$ and $R_9$ are not $NO_2$; or pharmaceutically acceptable salt or ester thereof or enantiomer, racemic mixture, or stereoisomer thereof.

In a preferred embodiment, $R_3$ and $R_4$ are selected from the group consisting of, but not limited to, $-CH_3$, $-CH_2CH_3$, $-CH_2CH_2CH_3$, isopropyl, *tert*-butyl, phenyl, 2-nitrophenyl, and 2,6-dinitrophenyl. Alternatively, $R_3$ and $R_4$ are selected from the group consisting of, but not limited to, alkyl and aromatic groups optionally containing at least one heteroatom in the alkyl or aromatic groups, and further wherein the aromatic group may optionally be an aryl such as phenyl or polycyclic such as a naphthyl group. In certain embodiments, $R_5$, $R_6$, $R_7$, and $R_8$ are selected from an aromatic group consisting of aryl and polycyclic groups.

Alternatively, linkers may have the following general structures:

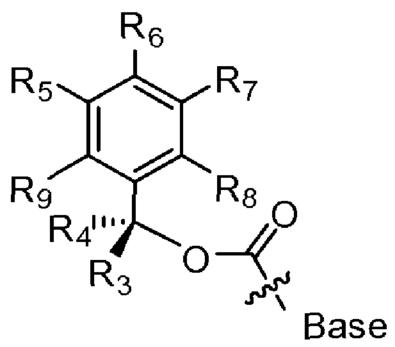

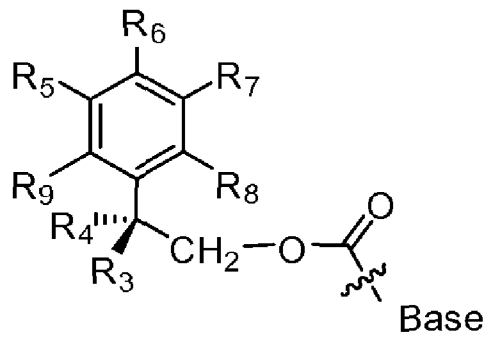

For example, compounds with such linkers could have the following structures:

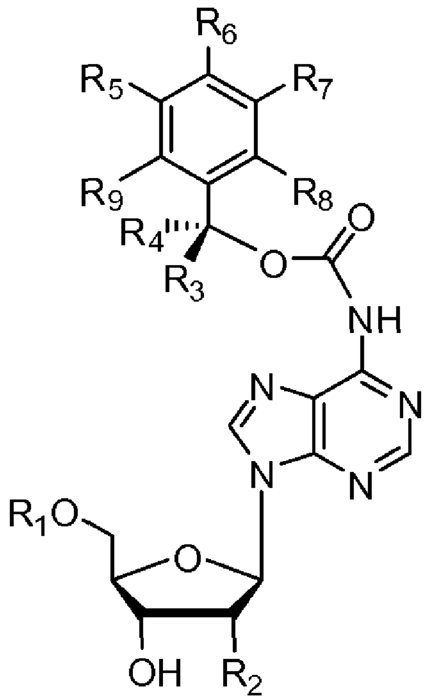

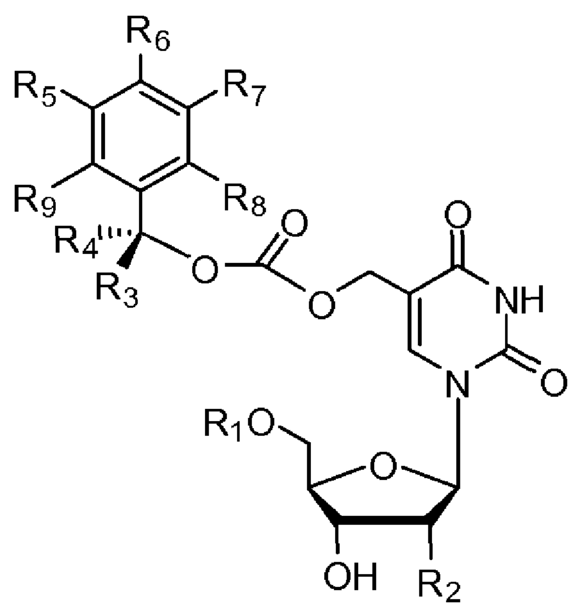

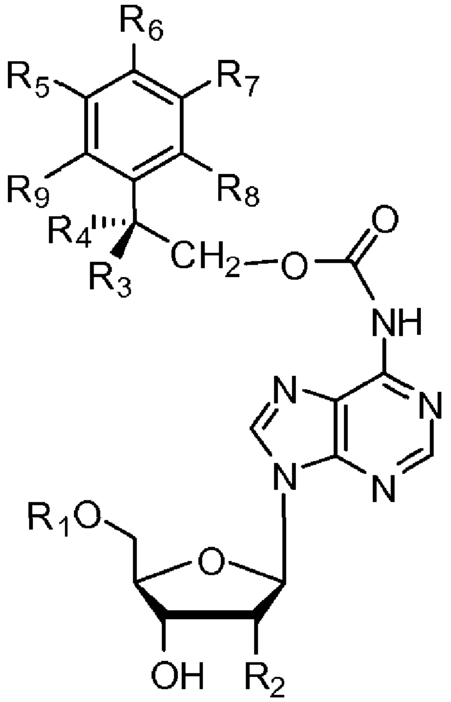

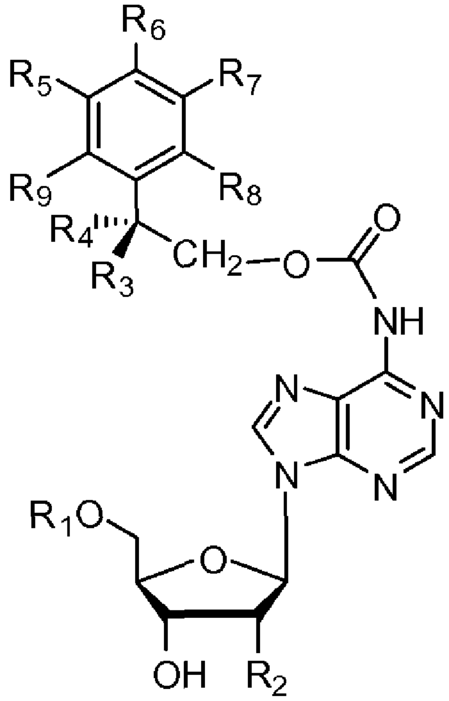

wherein the noncleavable terminating moiety can be attached to the base through a linkage such as a benzyl amine, benzyl ether, carbamate, carbonate, 2-(o-nitrophenyl)ethyl carbamate, and/or 2-(o-nitrophenyl)ethyl carbonate.

Fluorescent dyes are not particularly limited. For example, the fluorophore may be selected from the group consisting of, but not limited to, BODIPY, fluorescein, rhodamine, coumarin, xanthene, cyanine, pyrene, phthalocyanine, phycobiliprotein, alexa, squarene dye, combinations resulting in energy transfer dyes, and derivatives thereof. Preferred embodiments include but are not limited to the following compounds:

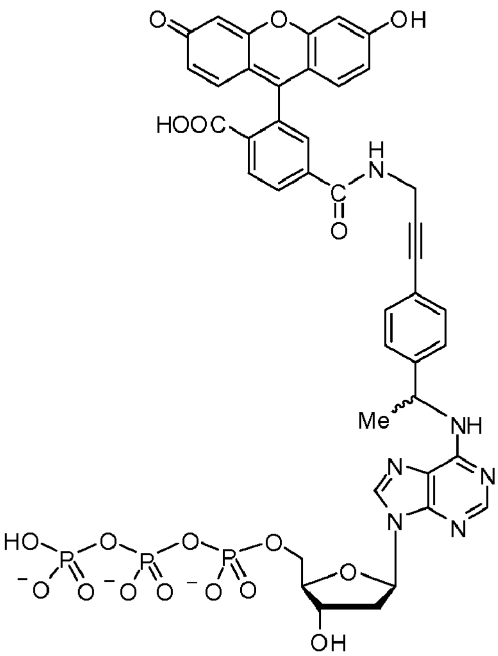

,

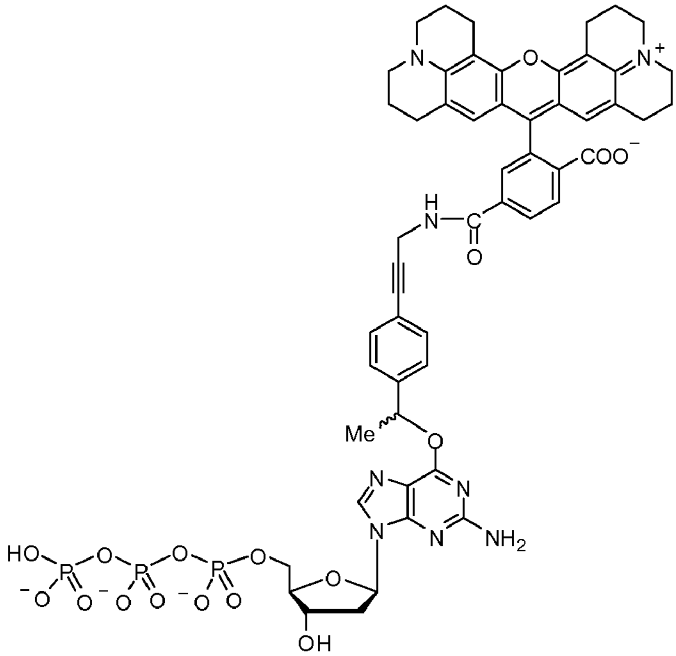

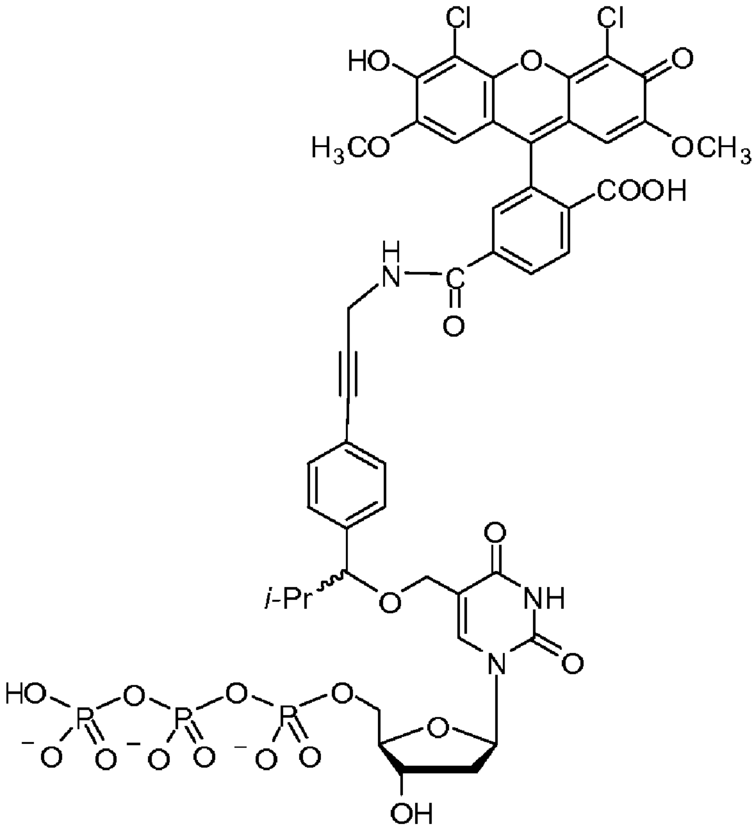

,

and

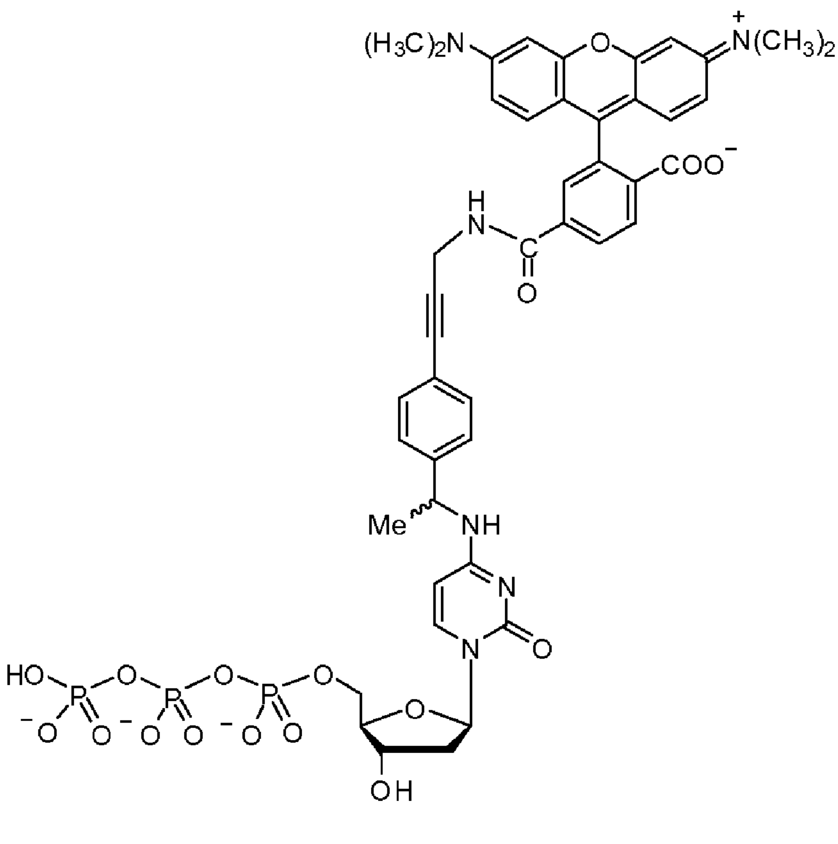

III. Synthesis of Labeled Nucleotide And Nucleoside Compounds

The compounds disclosed herein can be synthesized generally as disclosed herein, and using methods available in the art. For example, the following general scheme represents the synthesis of an adenosine compound:

*General Scheme for synthesis of an Adenosine N6-modified compound*

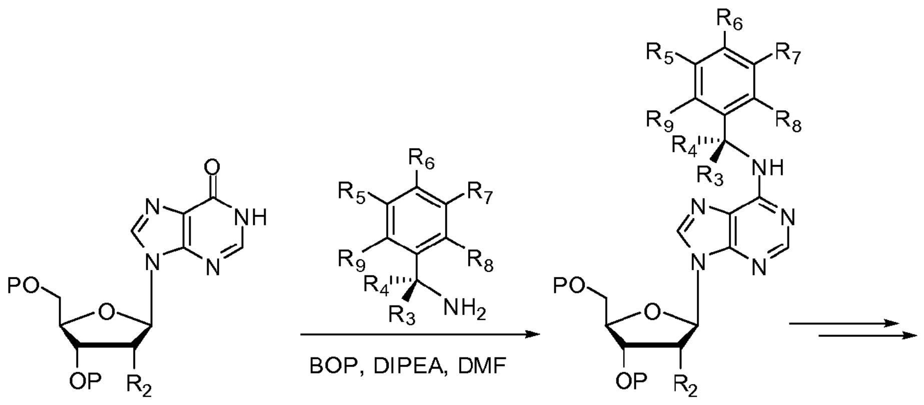


P denotes a suitable protecting group
$R_2$ = H or OH group with a suitable protecting group

$R_1$ to $R_8$ has the same definition as defined for the general structure

*General Scheme for synthesis Guanosine 06-modified compounds*

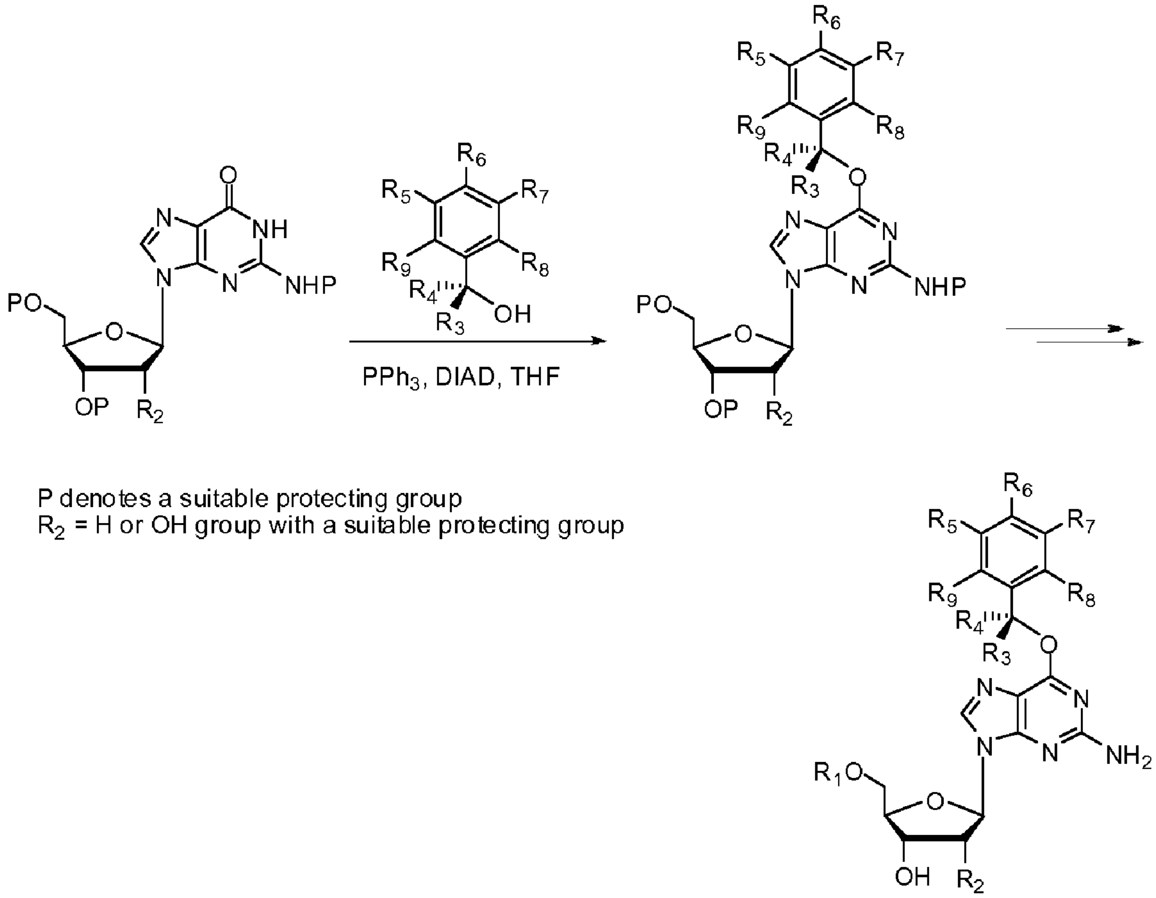


$R_1$ to $R_8$ has the same definition as defined for the general structure

*General Scheme for synthesis Guanosine 8-Oxo-modified compounds*

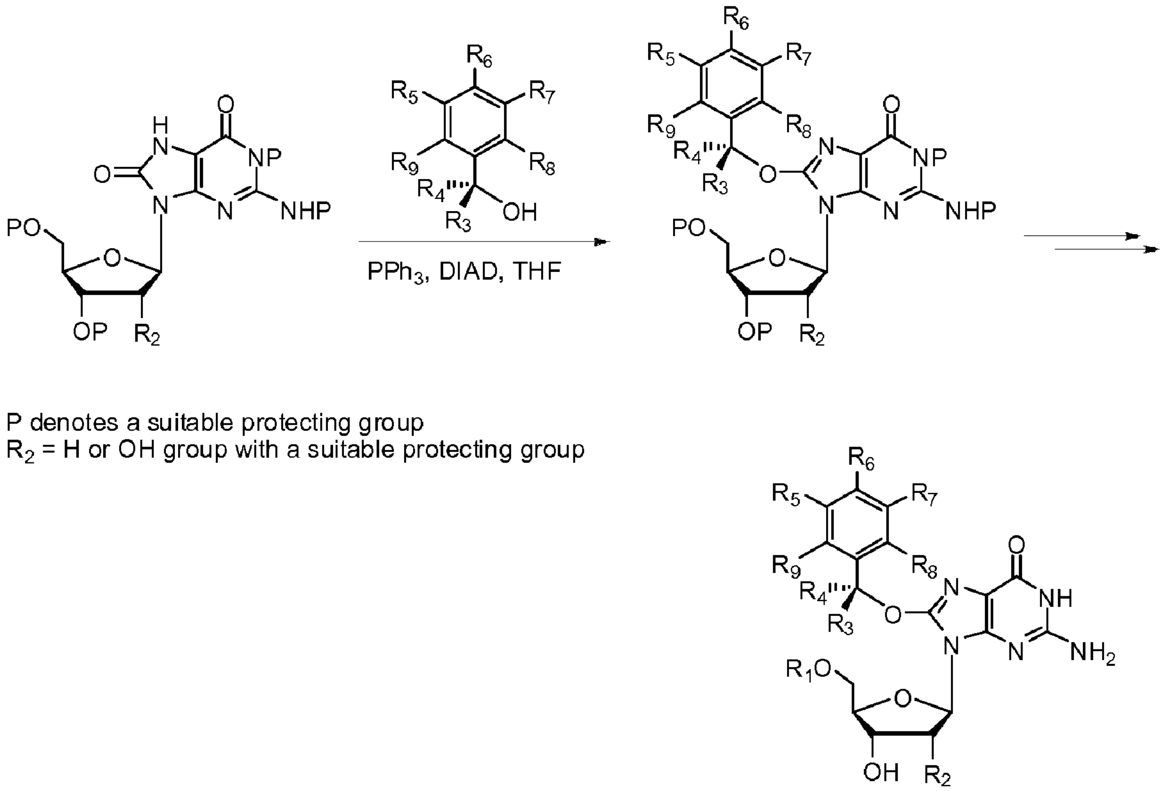


P denotes a suitable protecting group
$R_2$ = H or OH group with a suitable protecting group

$R_1$ to $R_8$ has the same definition as defined for the general structure

*General Scheme for synthesis Uridine 5-HOMe-modified compounds*

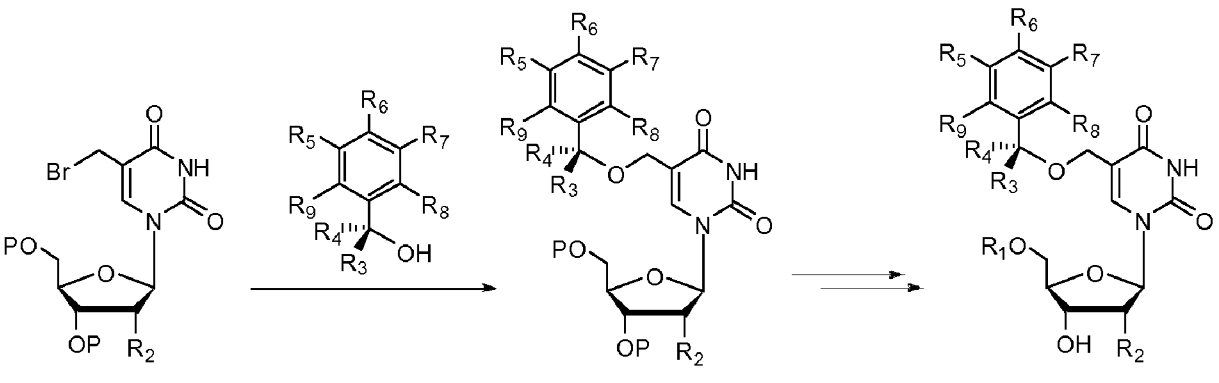

P denotes a suitable protecting group
$R_2$ = H or OH group with a suitable protecting group

$R_1$ to $R_8$ has the same definition as defined for the general structure

*General Scheme for synthesis Cytidine 5-HOMe-modified compounds*

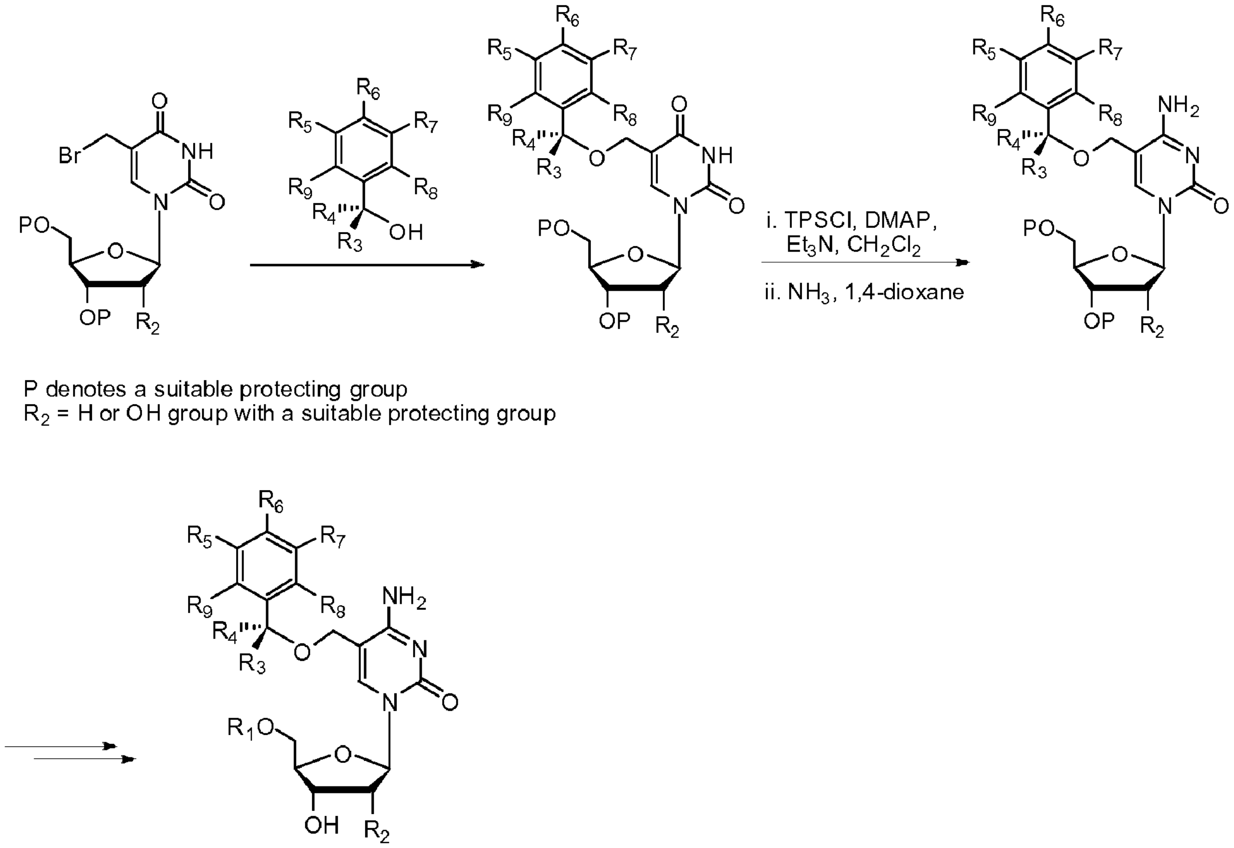


P denotes a suitable protecting group
$R_2$ = H or OH group with a suitable protecting group

$R_1$ to $R_8$ has the same definition as defined for the general structure

*General Scheme for synthesis Cytidine N4-modified compounds*

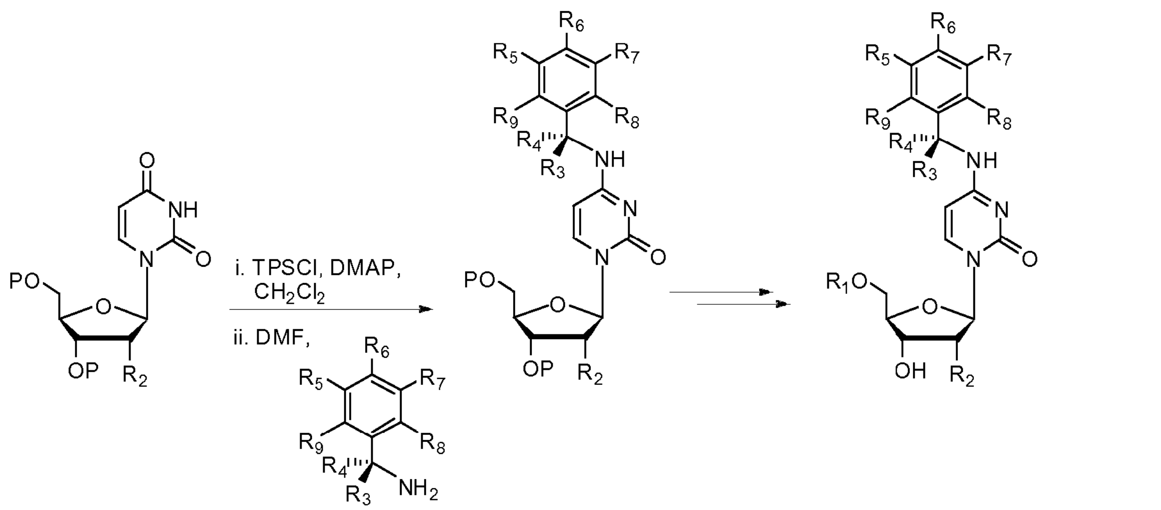


P denotes a suitable protecting group
$R_2$ = H or OH group with a suitable protecting group

$R_1$ to $R_8$ has the same definition as defined for the general structure

IV. Methods of Use of Compounds According to the Invention

The nucleotide and nucleoside compounds disclosed herein can be used in for a variety of purposes in DNA sequencing technology. Polymerases used in conjunction with the compounds according to the invention may be native polymerases or modified polymerases. Polymerases include without limitation Taq DNA polymerase, Klenow(exo-) DNA polymerase, Bst DNA polymerase, Vent(exo-) DNA polymerase, Pfu(exo-) DNA polymerase, and DeepVent(ex0-) DNA polymerase. Modified polymerases include without limitation TaqFS DNA polymerase, ThermoSequenase DNA polymerase, ThermoSequenase II DNA polymerase, Therminator DNA polymerase, Therminator II DNA polymerase, and Vent(exo-) A488L DNA polymerase. Preferably, compounds according to the invention are incorporated at levels equal to or near the incorporation levels of naturally-occurring nucleotides, thus resulting in no bias against the compounds according to the invention. Even more preferably, compounds according to the invention are compatible with commercially-available polymerases.

PHOTOCLEAVABLE LABELED NUCLEOTIDES AND NUCLEOSIDES

In one embodiment, the compounds can be used in cyclic reversible termination (CRT), which is a cyclic method of detecting the synchronous, single base additions of multiple templates. Longer read-lengths translate into fewer sequencing assays needed to cover the entire genome. A method of synthesizing a nucleic acid comprises the following steps:

a) attaching the 5'-end of a primer to a solid surface;
b) hybridizing a target nucleic acid to the primer attached to the solid surface;
c) adding one or more compounds according to the formula:

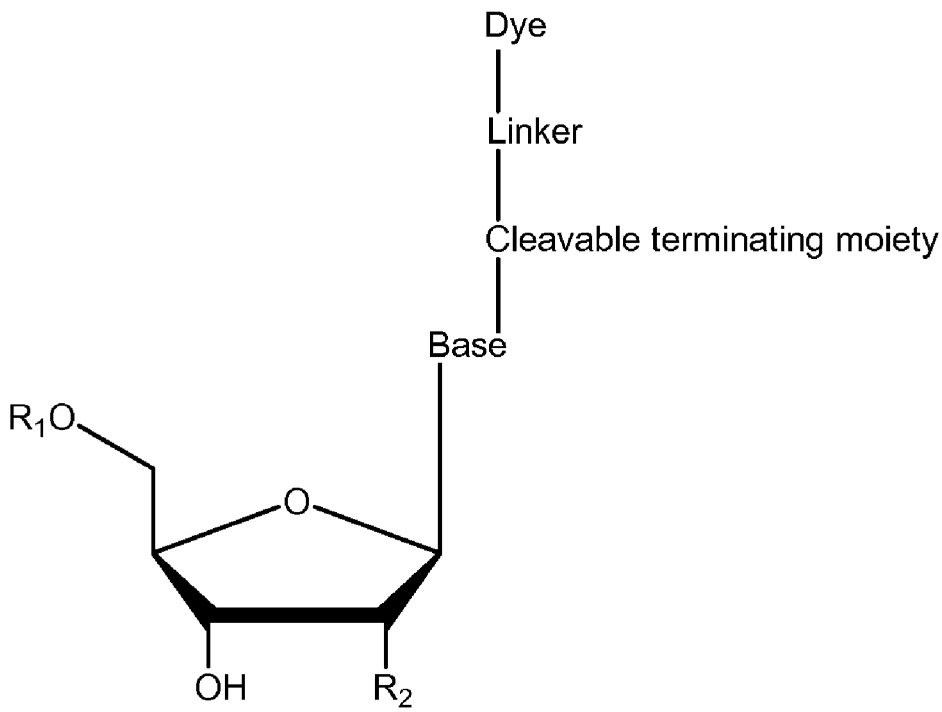

wherein the compound is selected from the group consisting of compounds represented by formulas I-VII as defined in claim 1;
d) adding DNA polymerase to the hybridized primer/target nucleic acid complex to incorporate the compound of the previous step into the growing primer strand, wherein the incorporated compound terminates the polymerase reaction at an efficiency of between about 90% to about 100%;
e) optionally washing the solid surface to remove unincorporated components;
f) detecting the incorporated fluorophore, wherein the detector is optionally a pulsed multiline excitation detector for imaging fluorescent dyes;
g) optionally adding one or more chemical compounds to permanently cap unextended primers;
h) exposing the solid support to a light source to remove the photocleavable moiety resulting in an extended primer

with naturally-occurring components; and

i) washing the solid surface to remove the cleaved protecting group; and repeating the above steps in a cyclic fashion.

In another embodiment, compounds according to the invention can be used in a method of determining the sequence of a nucleic acid molecule comprising the steps of adding a target nucleic acid molecule to a sequencing apparatus, adding one or more compounds according to the invention to the sequencing apparatus, adding a polymerase enzyme and optionally naturally-occurring nucleic acid components to the sequencing apparatus, performing a polymerase reaction to incorporate at least one of the compounds of the previous step into a growing nucleic acid strand, and analyzing the result of the polymerase reaction for incorporation of at least one compound according to the invention. The steps can be performed in any order and in any number of iterations. Preferably, the incorporation step is followed by termination of strand growth at a rate of from about 90% to about 100%. In another embodiment, the incorporation of the inventive compound occurs at about 70% to about 100% of the rate of incorporation of a native substrate of the analogous base, such that no significant bias occurs. For example, the incorporation rate may occur at about 85% to about 100% of the normal rate for the corresponding nucleotide base. An important embodiment includes the step of exposing the nucleic acid molecule resulting from incorporation of a modified nucleotide to a *uv* or other light source to remove a photocleavable terminating moiety from the nucleic acid. Preferably, the efficiency of the photocleavage step is about 85% to about 100% from exposure to the *uv* or other light source.

Methods according to the invention can be practiced individually or in combination. For example, the method of sequencing a nucleic acid molecule can be practiced in part as a method of incorporating a non-naturally occurring component into a nucleic acid molecule, or a separate method of converting a non-naturally occurring component in a nucleic acid molecule into a naturally-occurring component following incorporation of a compound according to the invention.

In one embodiment, methods according to the invention include the aspect of terminating nucleic acid synthesis following incorporation of an unprotected 3'-OH nucleotide. Advantageously, an unprotected 3'-OH nucleotide can be incorporated by polymerases at a higher level, resulting in lower levels of modified nucleotide present in the polymerase reaction and lower bias compared to natural nucleotides. Therefore, a preferred embodiment includes a method of terminating nucleic acid synthesis comprising the step of placing a 3'-OH unprotected nucleotide or nucleoside in the environment of a polymerase and allowing incorporation of the 3'-OH unprotected nucleotide or nucleoside into a nucleic acid molecule, wherein the 3'-OH unprotected nucleotide or nucleoside is a compound according to the following formula:

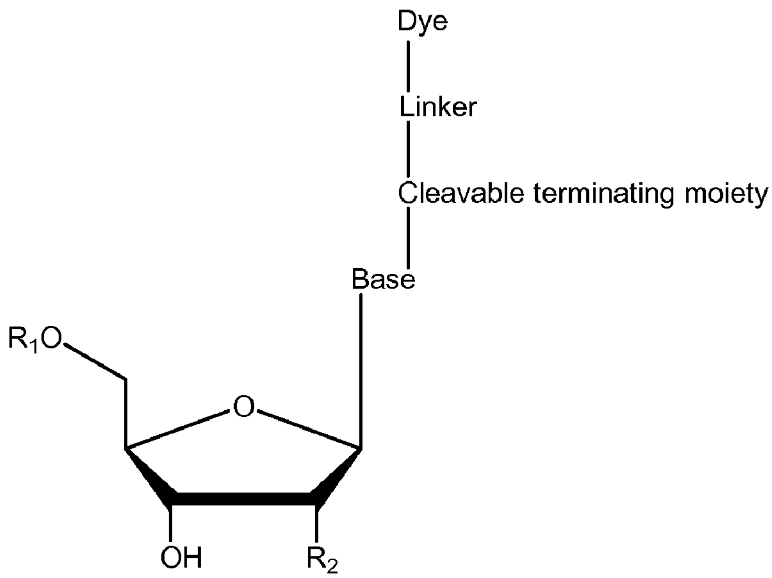

wherein the compound is selected from the group consisting of the compounds of formulas I-VII as defined in claims 1. Preferably, the method has an efficiency of termination upon incorporation of the 3'-OH unprotected nucleotide or nucleoside ranging from about 90% to about 100%. Alternatively, the method may have an efficiency of incorporation of the 3'-OH unprotected nucleotide or nucleoside ranges from about 70% to about 100% compared to the efficiency of incorporation of a naturally-occurring nucleotide or nucleoside with the same base.

The nucleotide and nucleoside compounds can be used in CRT to read directly from genomic DNA. Fragmented genomic DNA can be hybridized to a high-density oligonucleotide chip containing priming sites that span selected chromosomes. Each priming sequence is separated by the estimated read-length of the CRT method. Between base

additions, a fluorescent imager, such as a Pulsed Multiline Excitation (PME) detector, can simultaneously image the entire high-density chip, marking significant improvements in speed and sensitivity. The fluorophore, which is attached to the cleavable terminating group on the base, is removed by *uv* or other light irradiation, thereby transforming the modified nucleotide into its natural form for the next round of base addition. After approximately 500 CRT cycles, the completed and contiguous genome sequence information can then be compared to the reference human genome to determine the extent and type of sequence variation in an individual's sample. Methods for cyclic reversible termination have been developed in the art and can be used, as described, e.g., in WO 2003/021212, the disclosure of which is incorporated herein by reference.

In one embodiment, a method for sequencing a nucleic acid by detecting the identity of a nucleotide analogue after the nucleotide analogue is incorporated into a growing strand of DNA in a polymerase reaction is used, which comprises the following steps:

(a) attaching the 5' end of a nucleic acid to a solid surface;

(b) attaching a primer to the nucleic acid attached to the solid surface;

(c) adding a polymerase and one or more different nucleoside triphosphate compounds to

the nucleic acid wherein the nucleoside triphosphate compound incorporates and then terminates the polymerase reaction,
wherein each nucleoside triphosphate compound comprises a base selected from the group consisting of adenine, guanine, cytosine, thymine, and uracil, and their analogues and a photocleavable terminating group attached to the base, the photocleavable group comprising a detectable label, and a deoxyribose or ribose sugar,

(d) optionally washing the solid surface to remove unincorporated nucleotide analogues;

(e) detecting and thereby identifying the detectable label, such as a fluorescent dye or reporter molecule, attached to the terminated nucleoside triphosphate, e.g. with PME;

(f) optionally adding one or more chemical compounds to permanently cap any unreacted -OH group on the primer attached to the nucleic acid or on a primer extension strand formed by adding one or more nucleotides to the primer;

(g) exposing the solid surface to a light source to remove the photocleavable protecting group containing the unique label or reporter molecule, wherein the remaining incorporated nucleoside monophosphate unit resembles a natural, native, or unmodified nucleic acid molecule;

(h) washing the solid surface to remove the cleaved protecting group; and

(i) repeating steps (c) through (h) for determining the sequence of identified incorporated nucleotide analogs into the growing primer strand.

LABELED NUCLEOTIDES AND NUCLEOSIDES

In a preferred embodiment, methods according to the invention include a method of determining the sequence of a target nucleic acid comprising (i) adding a target nucleic acid to a Sanger or Sanger-type sequencing apparatus, (ii) adding one or more compounds according to the invention to the sequencing apparatus, with the proviso that where more than one type of base is present, each base is attached to a different fluorophore; (iii) adding a complementary primer and a polymerase enzyme, (iv) performing a polymerase reaction to incorporate at least one of the compounds of step (ii) into a growing nucleic acid strand, and (v) analyzing the result of the Sanger sequencing reaction with fluorescence sequencing instrumentation or by pulsed multiline excitation fluorescence, wherein steps (i)-(iii) can be performed in any order.

In a preferred embodiment, incorporation of at least one compound according to step (iv) is followed by termination of strand growth at an efficiency of from about 90% to about 100%. Alternatively, the incorporation of at least one compound according to step (iv) occurs at about 70% to about 100% of the efficiency of incorporation of a native substrate with the same base in the polymerase reaction, or more preferably at about 85% to about 100%.

Methods according to the invention also include a method of incorporating a nonnaturally occurring component into a nucleic acid comprising: (i) adding a target nucleic acid to a sequencing apparatus; (ii) adding one or more compounds according to the invention to the sequencing apparatus, with the proviso that where more than one type of

base is present, each base is attached to a different fluorophore; (iii) adding a polymerase enzyme; and (iv) performing a polymerase reaction to incorporate at least one of the compounds of step (ii) into a growing nucleic acid strand, wherein steps (i)-(iii) can be performed in any order. The method can further comprise (v) analyzing the result of the polymerase chain reaction for incorporation of at least one compound from step (ii).

An alternative embodiment of the invention is a method of terminating nucleic acid synthesis comprising the step of placing a 3'-OH unprotected nucleotide or nucleoside according to the invention in the environment of a polymerase and allowing incorporation of the 3'-OH unprotected nucleotide or nucleoside into a nucleic acid. Preferred embodiments of the method have an efficiency of termination upon incorporation of the 3'-OH unprotected nucleotide or nucleoside ranging from about 90% to about 100%; with the efficiency of incorporation of the 3'-OH unprotected nucleotide or nucleoside ranging from about 70% to about 100% compared to the efficiency of incorporation of a naturally-occurring nucleotide or nucleoside with the same base.

Methods of performing Sanger or Sanger-type sequencing comprising addition of a compound according to the invention to a Sanger or Sanger-type sequencing method are also encompassed. A method of performing mini-sequencing or minisequencing-type sequencing comprising addition of a compound according to the invention to a mini-sequencing or minisequencing-type sequencing method is within the scope of the invention.

*PME Detector*

In one embodiment using any of the foregoing labeled and/or photocleavable nucleosides and nucleotides, a pulsed-multiline excitation ("PME") for color-blind fluorescence detection can be used as described in US 2003/0058440 published March 27, 2003, or WO 2003/021212, published March 13, 2003. This technology provides fluorescence detection with application for high-throughput identification of informative SNPs, for more accurate diagnosis of inherited disease, better prognosis of risk susceptibilities, or identification of sporadic mutations. The PME technology has two main advantages that significantly increase fluorescence sensitivity: (1) optimal excitation of all fluorophores in the genomic assay and (2) "color-blind" detection, which collects considerably more light than standard wavelength resolved detection. This technology differs significantly from DNA sequencing instrumentation which features single source excitation and color dispersion for DNA sequence identification. The technology can be used in clinical diagnostics, forensics, and general sequencing methodologies and will have the capability, flexibility, and portability of targeted sequence variation assays for a large majority of the population.

In one embodiment, an apparatus and method for use in high-throughput DNA sequence identification is used. A pulse-multiline excitation apparatus for analyzing a sample containing one or more fluorescent species is used, comprising: one or more lasers configured to emit two or more excitation lines, each excitation line having a different wavelength; a timing circuit coupled to the one or more lasers and configured to generate the two or more excitation lines sequentially according to a timing program to produce time-correlated fluorescence emission signals from the sample; a non-dispersive detector positioned to collect the time-correlated fluorescence emission signals emanating from the sample; and an analyzer coupled to the detector and configured to associate the time-correlated fluorescence emission signals with the timing program to identify constituents of the sample.

The detector and the analyzer may be integral. In one embodiment, the two or more excitation lines intersect at the sample, or the two or more excitation lines may be configured so that they do not intersect in the sample. The two or more excitation lines may be coaxial. In one embodiment, the apparatus may further comprise an assembly of one or more prisms in operative relation with the one or more lasers and configured to render radiation of the two or more excitation lines substantially colinear andlor coaxial. The apparatus may have a plurality of excitation lines, for example 1, 2, 3, 4, 5, 6, 7, 8, 9, 10, 11, 12, 13, 14, 15, 16 or more excitation lines having 1, 2, 3, 4, 5, 6, 7, 8, 9, 10, 11, 12, 13, 14, 15, 16 or more excitation wavelengths, respectively. The sample may be comprised a plurality of vessels such as capillaries, for example in 1, 2, 3, 4, 5, 6, 7, 8, 9, 10, 11, 12, 13, 14, 15, 16, up to 20, up to 24, up to 28, up to 36, up to 48, up to 64, up to 96, up to 384 or more capillaries. A sheath flow cuvette may be used.

The timing program may comprise a delay between the firing of each laser of between about 10 fs and about 5 s, between about 1 ms and about 100 ms, or between about 50 ps and about 500 ps. One or more of the excitation lines is pulsed. The pulsed excitation line may be controlled by TTL logic or by mechanical or electronic means. In one embodiment, the apparatus may generate a sequence of discrete excitation lines that are time-correlated with the fluorescence emission signals from the sample. The lasers may independently comprise a diode laser, a semiconductor laser, a gas laser, such as an argon ion, krypton, or helium-neon laser, a diode laser, a solid-state laser such as a Neodymium laser which will include an ion-gain medium, such as YAG and yttrium vanadate (YV04), or a diode pumped solid state laser. Other devices, which produce light at one or more discrete excitation wavelengths, may also be used in place of the laser. The laser may further comprise a Raman shifter in operable relation with at least one laser beam. In one embodiment of the invention, the excitation wavelength provided by each laser is optically matched to the absorption wavelength of each fluorophore.

The detector may comprise a charged couple device, a photomultiplier tube, a silicon avalanche photodiode

or a silicon PIN detector. The footprint of the device is preferably small, such as less than 4 ft x 4 ft x 2ft, less than 1 ft x 1ft x 2ft, and could be made as small as 1 in x 3 in x 6 in. Another aspect comprises a method of identifying sample components comprising: (a) preparing a sample comprising sample components, a first dye and a second dye; (b) placing the sample in the beam path of a first excitation line and a second excitation line; (c) sequentially firing the first excitation line and the second excitation line; (d) collecting fluorescence signals from the samples as a function of time; and (e) sorting the fluorescence by each excitation line's on-time window, wherein the sample components are identified. It is an aspect of the invention that the fluorescence signals are collected from discrete time periods in which no excitation line is incident on the sample, the time periods occurring between the firing of the two excitation lines. This technique is known as "looking in the dark." Yet another aspect is that the absorption maximum of the first dye substantially corresponds to the excitation wavelength of the first excitation line. The absorption maximum of the second dye may also substantially corresponds to the excitation wavelength of the second excitation line. In yet another aspect there is a third and fourth dye and a third and fourth excitation line, wherein the absorption maxima of the third and fourth dyes substantially correspond to the excitation wavelengths of the third and four excitation lines, respectively. Similarly, there may be 5, 6, 7, 8, 9, 10, 11, 12, 13, 14, 15, 16 or more dyes wherein the absorption maxima of the dyes substantially corresponds to excitation wavelengths of a 5th, 6th, 7th, 8th, 9th, 10th, 11 th, 12th, 13th, 14th, or more excitation lines, respectively. The dyes may be a zanthene, fluorescein, rhodamine, BODIPY, cyanine, coumarin, pyrene, phthalocyanine, phycobiliprotein, Alexa, squariane dyes, or some other suitable dye.

In one embodiment, sample components enable the determination of SNPs. The method may be for the high-throughput identification of informative SNPs. The SNPs may be obtained directly from genomic DNA material, from PCR amplified material, or from cloned DNA material and may be assayed using a single nucleotide primer extension method. The single nucleotide primer extension method may comprise using single unlabeled dNTPs, single labeled dNTPs, single 3'-modified dNTPs, single base-modified 3'-dNTPs, single alpha-thiodNTPs or single labeled 2',3'-dideoxynucleotides. The mini-sequencing method may comprise using single unlabeled dNTPs, single labeled dNTPs, single 3'-modified dNTPs, single base modified 3'-dNTPs, single alpha-thio-dNTPs or single labeled 2',3'-dideoxynucleotides. The SNPs may be obtained directly from genomic DNA material, from PCR amplified material, or from cloned DNA materials. Also envisioned are methods for detecting nucleic acids. Nucleic acids may be detected in situ or in various gels, blots, and similar methods for detecting nucleic acids, such as disclosed in US Patent 7,125,660, which is incorporated herein by reference.

## EXAMPLES

PHOTOCLEAVABLE LABELED NUCLEOTIDES AND NUCLEOSIDES

Example 1 : dA compounds

Synthesis of 3'-O-(2-nitrobenzyl)-2'-deoxyadenosine triphosphate (WWlp108) **Scheme.** Synthesis of *3'-O-(2-nitrobenzyl)-2'-deoxyadenosine-5'-triphosphate.,* (i) TBSCl, imidazole, DMF, room temperature, overnight; $Boc_2O$, DMAP, DMF, room temperature, overnight, 83%; (ii) *n*-$Bu_4NF$, THF, 0°C, then gradually warmed to room temperature, 96%; (iii) TBSCl, imidazole, DMF, 83%; (iv) *n*-$Bu_4NOH$, NaI, NaOH, $CH_2Cl_2/H_2O$, 2-nitrobenzyl bromide, room temperature, 91%; (v) $SiO_2$, high vacuum, 70-80°C, 24 hours, 91%; (vi) *n*-$Bu_4NF$, THF, 0°C, then gradually warmed to room temperature, 23%; (vii) $POCl_3$, $(MeO)_3PO$, minus 20°C; (*n*-$Bu_3NH)_2H_2P_2O_7$, *n*-$Bu_3N$, DMF; 1 M $HNEt_3HCO_3$, 31%.

*$N^6$, $N^6$-Bis-tert-butyloxycarbonyl-3',5'-O-bis-tert-butyldimethylsilyl-2'-deoxyadenosine* ***(dA.01)***

Compound **dA.01** was synthesized according to the procedure described by Furrer and Giese[1]. A solution of 2'-deoxyadenosine **dA** (2.5 g, 10 mmol), imidazole (4.5 g, 66 mmol), and TBSCl (4.82 g, 32 mmol) in anhydrous DMF (25 mL) was stirred at room temperature overnight. Methanol (20 mL) was added, and the mixture was stirred for 20 minutes and then concentrated *in vacuo.* The residue was dissolved in anhydrous DMF (15 mL) followed by the addition of DMAP (3.66 g, 30 mmol) and $BoC_2O$ (6.55 g, 30 mmol). The reaction was stirred at room temperature overnight and then concentrated *in vacuo.* The residue was dissolved in $CH_2Cl_2$ (100 mL) and washed twice with saturated $NH_4Cl$ solution (50 mL each). The combined aqueous layer was extracted with $CH_2Cl_2$ (50 mL). The combined organic layer was then dried over $Na_2SO_4$, concentrated *in vacuo*, and purified by silica gel column chromatography to give *$N^6$,$N^6$*-bis-*tert*-butyloxycarbonyl-3',5'-*O*-bis-*tert*-butyldimethylsilyl-2'-deoxyadenosine **dA.01** (5.66 g, 83%) as a white foam.

*$N^6$,$N^6$-Bis-tert-butyloxycarbonyl-2'-deoxyadenosine* ***(dA. 02)***

A solution of $Bu_4NF$ (7.85 g, 30 mmol) in THF (30 mL) was added to a solution of compound **dA.01** (6.78 g, 10 mmol) in THF (30 mL) at 0°C. The reaction mixture was gradually warmed to room temperature, stirred for two hours,

and then concentrated *in vacuo.* The residue was [1] Furrer, E. and Giese, B. (2003) On the distance-independent hole transfer over long (A·T)n-sequences in DNA. Helvetica Chimica Acta, 86, 3623-3632.
dissolved in $CH_2Cl_2$ (100 mL) and washed twice with saturated $NH_4Cl$ solution (100 mL each). The organic layer was then dried over $Na_2SO_4$, concentrated *in vacuo,* and purified by silica gel column chromatography to yield *N6,N6*-bis-*tert*-butyloxycarbonyl-2'-deoxyadenosine **dA.02** (4.34 g, 96%) as a white foam.

*1H NMR (400 MHz, $CDCl_3$):* δ 8.84 (s, 1 H, H-8), 8.20 (s, 1 H, H-2), 6.41 (dd, 1 H, *J* = 5.6 Hz and 9.2 Hz, H-1'), 4.77 (d, 1 H, H-4'), 4.21 (s, 1 H, H-3'), 3.98 (dd, 1 H, H-5'a), 3.80 (m, 1 H, H-5'b), 3.00 (m, 1 H, H-2'a), 2.36 (m, 1 H, H-2'b), 1.47 (s, 18 H, $(CH_3)_3CO$).

*N6,N6-Bis-tert-butyloxycarbonyl-5'-O-tert-butyldimethylsilyl-2'-deoxyadenosine* ***(dA.03)***

A solution of TBSCl (1.88 g, 12.5 mmol) in anhydrous DMF (5 mL) was added to a solution of compound **dA.02** (4.34 g, 9.6 mmol) and imidazole (1.3 g, 19.2 mmol) in anhydrous DMF (20 mL) at 0°C. The mixture was gradually warmed to room temperature and stirred for two days. Water (50 mL) was added, and the mixture was extracted three times with ethyl acetate (40 mL each). The combined organic layer was washed with saturated $NH_4Cl$ solution (50 mL), dried over $Na_2SO_4$, and purified by silica gel column chromatography to yield *N6,N6*-bis-*tert*-butyloxycarbonyl-5'-*O*-*tert*-butyl-dimethylsilyl-2'-deoxyadenosine **dA.03** (4.68 g, 83%) as a white foam.

*1H NMR (400 MHz, $CDCl_3$):* 5 8.84 (s, 1 H, H-8), 8.42 (s, 1 H, H-2), 6.57 (t, 1 H, *J* = 6.4 Hz, H-1'), 4.69 (m, 1 H, H-4'), 4.10 (m, 1 H, H-3'), 3.89 (m, 2 H, H-5'a and H-5'b), 2.70 (m, 1 H, H-2'a), 2.58 (m, 1 H, H-2'b), 1.44 (s, 18 H, $(CH_3)_3CO$), 0.91 (s, 9 H, $(CH_3)_3CSi$), 0.10 (s, 6 H, $(CH_3)_2Si$).

*N6,N6-Bis-tert-butyloxycarbonyl-5'-O-tert-butyldimethylsilyl-3'-O-(2-nitrobenzyl)-2'-deoxyadenosine (dA.04)*

A solution of compound **dA.03** (1.13 g, 2 mmol) in $CH_2Cl_2$ (3 mL) was mixed with a solution of *n*-$Bu_4NOH$ (0.94 mL, 4 mmol, 55% aqueous solution) and NaI (20 mg, catalytic amount) in NaOH (1 M; 3 mL). To the mixture, a solution of 2-nitrobenzyl bromide (1.3 g, 6 mmol) in $CH_2Cl_2$ (2 mL) was added dropwise, and the reaction mixture was stirred at room temperature for two hours in the dark. The organic layer was separated, and the aqueous layer was extracted twice with $CH_2Cl_2$ (10 mL each). The combined organic layer was dried over $Na_2SO_4$, concentrated *in vacuo,* and purified by silica gel column chromatography to yield *N6,N6*-bis-*tert*-butyloxycarbonyl-5'-*O*-*tert*-butyldimethylsilyl-3'-*O*-(2-nitrobenzyl)-2'-deoxyadenosine **dA.04** (1.28 g, 91 %) as a white foam.

*1H NMR (400 MHz, $CDCl_3$):* δ 8.85 (s, 1 H, H-8), 8.43 (s, 1 H, H-2), 8.10 (dd, 1 H, Ph-H), 7.81 (d, 1 H, Ph-H), 7.70 (t, 1 H, Ph-H), 7.51 (t, 1 H, Ph-H), 6.57 (t, 1 H, *J* = 6.8 Hz, H-1'), 4.98 (dd, 2 H, $PhCH_2$), 4.45 (m, 1 H, H-4'), 4.33 (m, 1 H, H-3'), 3.90 (m, 2 H, H-5'a and H-5'b), 2.73 (m, 2 H, H-2'a and H-2'b), 1.46 (s, 18 H, $(CH_3)_3CO$), 0.91 (s, 9 H, $(CH_3)_3CSi$), 0.11 (s, 6 H, $(CH_3)_2Si$);

*ToF-MS (ESI):* For the molecular ion $C_{33}H_{49}N_6O_9Si$ $[M+H]^+$, the calculated mass was 701.3330, and the observed mass was 701.3317.

*5'-O-tert-Butyldimethylsilyl-3'-O-(2-nitrobenzyl)-2'-deoxyadenosine* ***(dA.05)***

Silica gel 60 (10g, 100-200 mesh, activated by heating to 70-80°C under reduced pressure for 24 hours) was added to a solution of compound **dA.04** (1.28 g, 1.8 mmol) in $CH_2Cl_2$ (50 mL), and the mixture was evaporated *in vacuo* to dryness. The residue obtained was heated to 70-80°C for two days under reduced pressure, washed three times with methanol (50 mL each), and filtered using a buchi funnel. The combined filtrate was concentrated *in vacuo* and purified by silica gel column chromatography to yield 5'-*O*-*tert*-butyldimethylsilyl-3'-*O*-(2-nitrobenzyl)-2'-deoxyadenosine **dA.05** (0.83 g, 91%) as a white foam.

*1H NMR (400 MHz, $CDCl_3$):* δ 8.34 (s, 1 H, H-8), 8.15 (s, 1 H, H-2), 8.09 (d, 1 H, Ph-H), 7.81 (d, 1 H, Ph-H), 7.67 (t, 1 H, Ph-H), 7.47 (t, 1 H, Ph-H), 6.50 (t, 1 H, *J* = 6.8 Hz, H-1'), 6.03 (bs, 2 H, 6-$NH_2$), 4.96 (dd, 2 H, $PhCH_2$), 4.43 (m, 1 H, H-4'), 4.30 (m, 1 H, H-3'), 3.88 (m, 2 H, H-5'a and H-5'b), 2.71 (m, 2 H, H-2'a and H-2'b), 0.91 (s, 9 H, $(CH_3)_3CSi$), 0.10 (s, 6 H, $(CH_3)_2Si$);

*ToF-MS (ESI):* For the molecular ion $C_{23}H_{33}N_6O_5Si$ $[M+H]^+$, the calculated mass was 501.2282, and the observed mass was 501.1702.

*3'-O-(2-Nitrobenzyl)-2'-deoxyadenosine* ***(dA.06)***

A solution of *n*-$Bu_4NF$ (314 mg, 1.2 mmol) in THF (1.2 mL) was added to a solution of compound **dA.05** (400 mg, 0.8 mmol) in THF (3 mL) at 0°C. The reaction mixture was gradually warmed to room temperature and stirred for four hours. Methanol (10 mL) was added to dissolve the precipitate formed during the reaction, followed by the addition of silica gcl 60 (1.5 g). The mixture was evaporated *in vacuo* to dryness, and the residue was purified by silica gel column

chromatography to yield 3'-*O*-(2-nitrobenzyl)-2'-deoxyadenosine **dA.06** (72 mg, 23%) as a white foam.

*$^1$H NMR (400 MHz, DMSO-$d_6$):* δ 8.35 (s, 1 H, H-8), 8.13 (s, 1 H, H-2), 8.06 (d, 1 H, Ph-H), 7.79 (m, 2 H, Ph-H), 7.60 (m, 1 H, Ph-H), 7.34 (bs, 2 H, 6-$NH_2$, $D_2O$ exchangeable), 6.33 (dd, 1 H, *J* = 4.8 and 6.8 Hz, H-1'), 5.40 (t, 1 H, $D_2O$ exchangeable, 5'-OH)), 4.92 (s, 2 H, $PhCH_2$), 4.36 (m, 1 H, H-4'), 4.12 (m, 1 H, H-3'), 3.59 (m, 2 H, H-5'a and H-5'b), 2.85 (m, 1 H, H-2'a), 2.54 (m, 1 H, H-2'b);

*ToF-MS (ESI):* For the molecular ion $C_{17}H_{19}N_6O_5$ [M+H]$^+$, the calculated mass was 387.1417, and the observed mass was 387.1350.

*3'-O-(2-Nitrobenzyl)-2'-deoxyadenosine-5'-triphosphate **(WW1p108)***

$POCl_3$ (26 μL, 0.24 mmol) was added to a solution of compound **dA.06** (72 mg, 0.18 mmol) in trimethylphosphate (1 mL), and maintained at minus 20-30°C for 2.5 hours. A solution of bis-tri-n-butylammonium pyrophosphate (427 mg, 0.9 mmol) and tri-*n*-butylamine (0.2 mL) in anhydrous DMF (2 mL) was added. After five minutes of stirring, triethylammonium bicarbonate buffer (1 M, pH 7.5; 10 mL) was added. The reaction was stirred at room temperature for one hour and then lyophilized to dryness. The residue was dissolved in water (10 mL), filtered, and purified by anion exchange chromatography using a Q Sepharose FF column (2.5 x 20 cm) with a linear gradient of $NH_4HCO_3$ (50 mM to 500 mM in 300 minutes) at a flow rate of 4.5 mL/min. The fractions containing triphosphate were combined and lyophilized to give 3'-*O*-(2-nitrobenzyl)-2'-deoxyadenosine-5'-triphosphate **WW1p108** (38 mg, 31%) as a white fluffy solid.

*$^1$H NMR (400 MHz, $D_2O$):* δ 8.50 (s, 1 H, H-8), 8.24 (s, 1 H, H-2), 8.10 (d, 1 H, Ph-H), 7.78 (d, 1 H, Ph-H), 7.62 (m, 1 H, Ph-H), 6.50 (dd, 1 H, *J* = 6.8 and 8 Hz, H-1'), 5.03 (dd, 2 H, Ph-$CH_2$), 4.65 (m, 1 H, H-4'), 4.53 (m, 1 H, H-3'), 4.22 (m, 2 H, H-5'a and H-5'b), 2.80 (m, 2 H, H-2'a and H-2'b);

*$^{31}$P NMR (162 MHz, $D_2O$):* δ -5.54 (d, *J* = 19.4 Hz), -10.85 (d, *J* = 19.4 Hz), -21.31 (t, *J* = 19.4 Hz);

*ToF-MS (ESI):* For the molecular ion $C_{17}H_{21}N_6O_{14}P_3Na$ [M+Na]$^+$, the calculated mass was 649.0226, and the observed mass was 649.0212.

The triphosphate was further purified using preparative HPLC without UV detection to give sample free from contamination of the natural nucleotide (*e.g.,* dATP). Determination of the concentration of the triphosphate solution was performed by UV/VIS measurement using the extinction coefficient of $\varepsilon_{260}$ = 20,800.

**Synthesis of *N*$^6$-(2-nitrobenzyl)-2'-deoxyadenosine triphosphate (VVW1p129)**

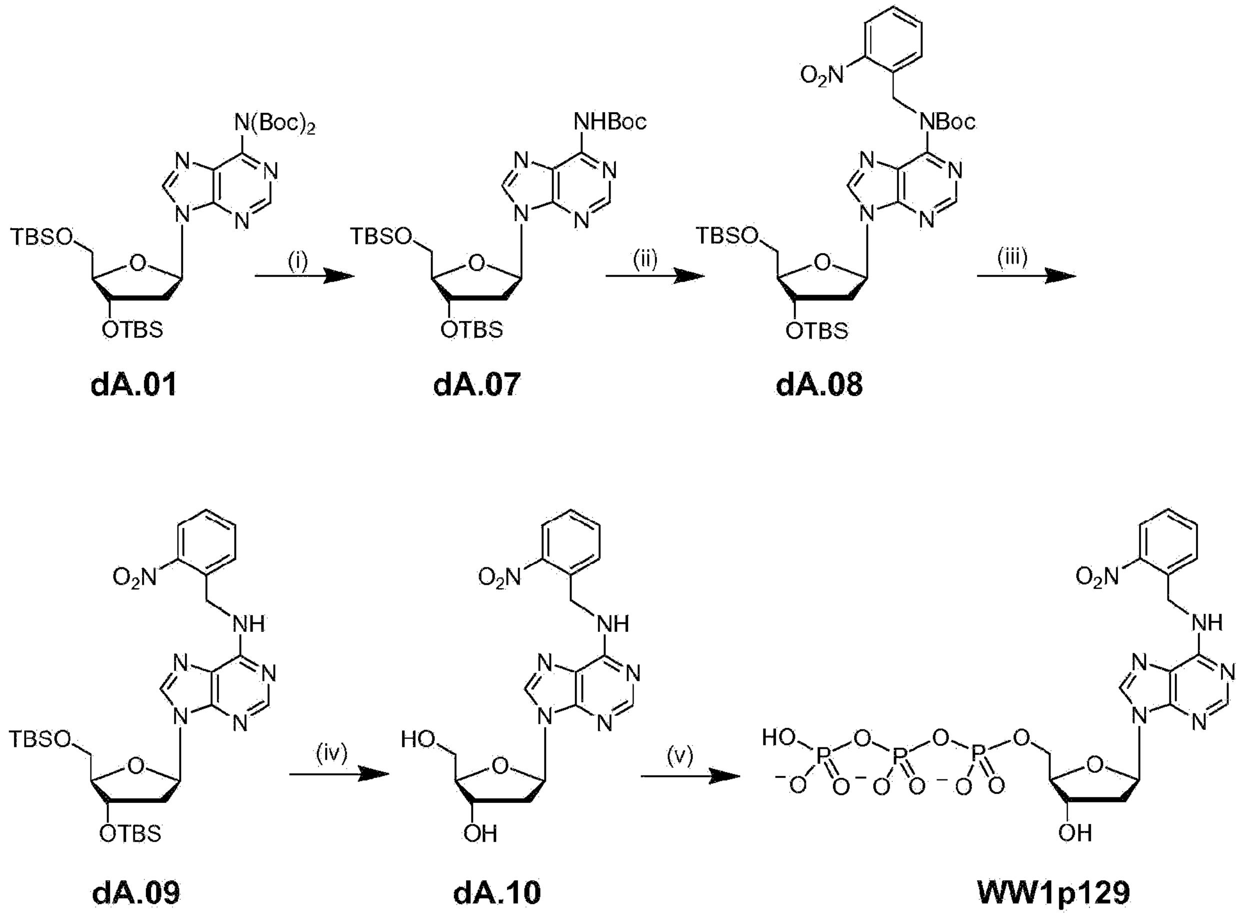


dA.01 dA.07 dA.08

dA.09 dA.10 WW1p129

**Scheme.** Synthesis of *$N^6$-(2-nitrobenzyl)-2'-deoxyadenosine-5'-triphosphate*. (i) $Mg(ClO_4)_2$, THF, 50°C, 68%; (ii) NaH, DMF, 2-nitrobenzyl bromide, 0°C, then gradually warmed to room temperature, 49%; (iii) $SiO_2$, vacuum, 70-80°C, 87%; (iv) *n*-$Bu_4NF$, THF, 43%; (v) $POCl_3$, $(MeO)_3PO$, minus 20-30°C; $(n$-$Bu_3NH)_2H_2P_2O_7$, *n*-$Bu_3N$, DMF; 1 M $HNEt_3HCO_3$; 60%.

*$N^6$-tert-Butyloxycarbonyl-3',5'-O-bis-tert-butyldimethylsilyl-2'-deoxyadenosine* ***(dA.07)***

$Mg(ClO_4)_2$ (155 mg, 0.7 mmol) was added to a solution of compound **dA.01** (2.36 g, 3.5 mmol) in anhydrous THF (35 mL) and stirred at 50°C overnight. Solvent was removed *in vacuo,* and the crude product was purified by silica gel column chromatography to give *$N^6$-tert*-butyloxycarbonyl-3',5'-*O*-bis-*tert*-butyldimethylsilyl-2'-deoxyadenosine **dA.07** (1.39 g, 68%) as a yellow foam.

*$^1H$ NMR (400 MHz, $CDCl_3$):* δ 8.74 (s, 1 H, H-8), 8.29 (s, 1 H, H-2), 8.24 (s, 1 H, 6-NHBoc), 6.49 (t, 1 H, *J* = 6.4 Hz, H-1'), 4.60 (m, 1 H, H-4'), 4.02 (m, 1 H, H-3'), 3.86 (dd, 1 H, H-5'a), 3.77 (dd, 1 H, H-5'b), 2.62 (m, 1 H, H-2'a), 2.47 (m, 1 H, H-2'b), 1.54 (s, 9 H, $(CH_3)_3CO$), 0.90 (s, 18 H, $(CH_3)_3CSi$), 0.08 (2 s, 12 H, $(CH_3)_2Si$).

*$N^6$-tert-Butyloxycarbonyl-$N^6$-(2-nitrobenzyl)-3',5'-O-bis-tert-butyldimethylsilyl-2'-deoxyadenosine (dA.08)*

NaH (5.3 mg, 0.22 mmol, dry) was added to a solution of compound **dA.07** (116 mg, 0.2 mmol) in anhydrous DMF (2 mL) at 0°C and stirred for 30 minutes. A solution of 2-nitrobenzyl bromide (43 mg, 0.2 mmol) in anhydrous DMF (0.5 mL) was added dropwise. The mixture was gradually warmed to room temperature and stirred for two hours. DMF was removed *in vacuo,* and the residue was dissolved in ethyl acetate (20 mL), washed twice with saturated $NH_4Cl$ solution (10 mL each) and once with water (10 mL). The combined aqueous layer was extracted with ethyl acetate (10 mL), and the combined organic layer was dried over $Na_2SO_4$, concentrated *in vacuo,* and purified by silica gcl column

chromatography to yield *N*[6]-*tert*-butyloxycarbonyl-*N*[6]-(2-nitrobenzyl)-3',5'-*O*-bis-*tert*-butyldimethylsilyl-2'-deoxyadenosine **dA.08** (70 mg, 49%) as a viscous oil.

*$^1$H NMR (400 MHz, $CDCl_3$):* δ 8.69 (s, 1 H, H-8), 8.38 (s, 1 H, H-2), 8.05 (dd, 1 H, Ph-H), 7.77 (d, 1 H, Ph-H), 7.56 (m, 1 H, Ph-H), 7.40 (m, 1 H, Ph-H), 6.51 (t, 1 H, *J* = 6.4 Hz, H-1'), 5.63 (s, 2 H, Ph-$CH_2$), 4.63 (m, 1 H, H-4'), 4.03 (m, 1 H, H-3'), 3.87 (m, 1 H, H-5'a), 3.78 (m, 1 H, H-5'b), 2.63 (m, 1 H, H-2'a), 2.46 (m, 1 H, H-2'b), 1.40 (s, 9 H, $(CH_3)_3CO$), 0.92 (s, 18 H, $(CH_3)_3CSi$), 0.10 (2 s, 12 H, $(CH_3)_2Si$-);

*ToF-MS (ESI):* For the molecular ion $C_{34}H_{55}N_6O_7Si_2$ $[M+H]^+$, the calculated mass was 715.3671, and the observed mass was 715.3661.

*N[6]-(2-Nitrobenzyl)-3',5'-O-bis-tert-butyldimethylsilyl-2'-deoxyadenosine* ***(dA.09)***

Silica gel 60 (3.5 g, 100-200 mesh, activated by heating to 70-80°C under reduced pressure for 24 hours) was added to a solution of compound **dA.08** (325 mg, 0.45 mmol) in $CH_2Cl_2$ (20 mL), and the mixture was evaporated *in vacuo* to dryness. The residue was heated to 70-80°C under reduced pressure for two days, washed three times with methanol (20 mL each), and filtered using a buchi funnel. The combined filtrate was concentrated *in vacuo* and purified by silica gel column chromatography to yield *N*[6]-(2-nitrobenzyl)-3',5'-*O*-bis-*tert*-butyldimethylsilyl-2'-deoxyadenosine **dA.09** (238 mg, 86%) as a yellow foam.

*$^1$H NMR (400 MHz, $CDCl_3$):* δ 8.37 (s, 1 H, H-8), 8.09 (s, 1 H, H-2), 8.07 (d, 1 H, Ph-H), 7.74 (d, 1 H, Ph-H), 7.56 (m, 1 H, Ph-H), 7.42 (m, 1 H, Ph-H), 6.57 (t, 1 H, 6-NH), 6.44 (t, 1 H, *J* = 6.4 Hz, H-1'), 5.19 (bs, 2 H, Ph-$CH_2$), 4.61 (m, 1 H, H-4'), 4.00 (m, 1 H, H-3'), 3.86 (dd, 1 H, H-5'a), 3.76 (dd, 1 H, H-5'b), 2.63 (m, 1 H, H-2'a), 2.43 (m, 1 H, H-2'b), 0.91 (s, 18 H, $(CH_3)_3CSi$), 0.09 (2s, 12 H, $(CH_3)_2Si$-);

*ToF-MS (ESI):* For the molecular ion $C_{29}H_{47}N_6O_5Si_2$ $[M+H]^+$, the calculated mass was 615.3147, and the observed mass was 615.2288.

*N[6]-(2-Nitrobenzyl)-2'-deoxyadenosine* ***(dA.10)***

A solution of *n*-$Bu_4NF$ (216 mg, 0.83 mmol) in THF (1 mL) was added to a solution of compound **dA.09** (202 mg, 0.33 mmol) in THF (5 mL) at 0°C. The reaction mixture was gradually warmed to room temperature and stirred for two hours. Silica gel 60 (1 g) was added, and the mixture was evaporated *in vacuo* to dryness. The residue was purified by silica gel column chromatography to yield *N*[6]-(2-nitrobenzyl)-2'-deoxyadenosine **dA.10** (55 mg, 43%) as a white foam.

*$^1$H NMR (400 MHz, DMSO-$d_6$):* δ 8.48 (br s, 1 H, D2O exchangeable, 6-NH), 8.41 (s, 1 H, H-8), 8.16 (s, 1 H, H-2), 8.04 (dd, 1 H, Ph-H), 7.66 (d, 1 H, Ph-H), 7.51 (m, 2 H, Ph-H), 6.35 (t, 1 H, *J* = 6.4 Hz, H-1'), 5.32 (d, 1 H, $D_2O$ exchangeable, 3'-OH), 5.17 (t, 1 H, $D_2O$ exchangeable, 5'-OH), 4.97 (bs, 2 H, Ph-$CH_2$), 4.41 (m, 1 H, H-4'), 3.87 (m, 1 H, H-3'), 3.60 (m, 1 H, H-5'a), 3.52 (m, 1 H, H-5'b), 2.71 (m, 1 H, H-2'a), 2.28 (m, 1 H, H-2'b);

*ToF-MS (ESI):* For the molecular ion $C_{17}H_{19}N_6O_5$ $[M+H]^+$, the calculated mass was 387.1417, and the observed mass was 387.1186.

*N[6]-(2-Nitrobenzyl)-2'-deoxyadenosine-5'-triphosphate* ***(WW1p129)***

$POCl_3$ (19 μL, 0.2 mmol) was added to a solution of compound **dA.10** (52 mg, 0.13 mmol) in trimethylphosphate (0.5 mL) and maintained at minus 20-30°C for 2.5 hours. A solution of bis-tri-*n*-butylammonium pyrophosphate (308 mg, 0.65 mmol) and tri-*n*-butylamine (130 μL) in anhydrous DMF (1.3 mL) was added. After five minutes of stirring, triethylammonium bicarbonate buffer (1 M, pH 7.5; 10 mL) was added. The reaction was stirred at room temperature for one hour and then lyophilized to dryness. The residue was dissolved in water (10 mL), filtered, and purified by anion exchange chromatography using a Q Sepharose FF column (2.5 x 20 cm) with a linear gradient of $NH_4HCO_3$ (50 mM to 500 mM in 300 minutes) at a flow rate of 4.5 mL/min. The fractions containing triphosphate were combined and lyophilized to give *N*[6]-(2-nitrobenzyl)-2'-deoxyadenosine-5'-triphosphate **WW1p129** (53 mg, 60%) as a white fluffy solid.

*$^1$H NMR (400 MHz, $D_2O$):* δ 8.42 (s, 1 H, H-8), 8.13 (s, 1 H, H-2), 8.09 (d, 1 H, Ph-H), 7.55 (m, 2 H, Ph-H), 7.45 (m, 1 H, Ph-H), 6.46 (t, 1 H, *J* = 6.4 Hz, H-1'), 5.05 (bs, 2 H, Ph-$CH_2$), 4.29 (s, 1 H, H-3'), 4.21 (m, 2 H, H-5'a and H-5'b), 2.78 (m, 1 H, H-2'a), 2.59 (m, 1 H, H-2'b);

*$^{31}$P NMR (162 MHz, $D_2o$):* δ -5.86 (d, *J* = 16.2 Hz), -10.78 (d, *J* = 16.2 Hz), -19.22 (t, *J* = 16.2 Hz);

*ToF-MS (ESI):* For the molecular ion $C_{17}H_{22}N_6O_{14}P_3$ $[M-H]^-$, the calculated mass was 625.0250, and the observed mass was 625.0231.

The triphosphate was further purified using preparative HPLC without UV detection to give sample free from contamination of the natural nucleotide. Determination of the concentration of the triphosphate solution was performed by UV/VIS measurement using the extinction coefficient of $\varepsilon_{260}$ = 20,800.

**Synthesis of *N*6-(4-methoxy-2-nitrobenzyl)-2'-deoxyadenosine triphosphate (WW2p005)**

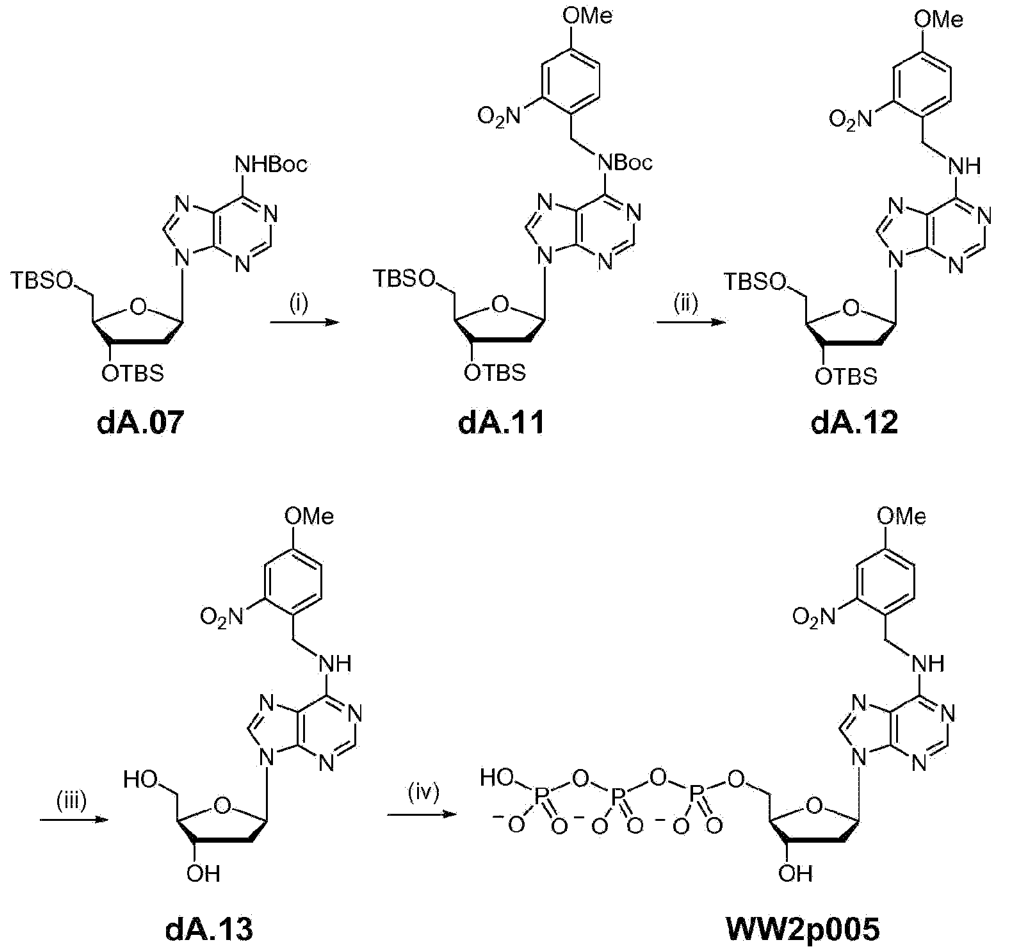


**dA.07** **dA.11** **dA.12**

**dA.13** **WW2p005**

**Scheme.** Synthesis of *$N^6$-(4-methoxy-2-nitrobenzyl)-2'-deoxyadenosine-5'-triphosphate*. (i) NaH, DMF, 4-methoxy-2-nitrobenzyl bromide, 0°C, then gradually warmed to room temperature, 64%; (ii) $SiO_2$, high vacuum, 70-80°C, 24 hours, 84%; (iii) *n*-$Bu_4NF$, THF, 99%; (iv) $POCl_3$, $(MeO)_3PO$, minus 20-30°C; (*n*-$Bu_3NH)_2H_2P_2O_7$, *n*-$Bu_3N$, DMF; 1 M $HNEt_3HCO_3$; 40%.

*$N^6$-tert-Butyloxycarbonyl-$N^6$-(4-methoxy-2-nitrobenzyl)-3',5'-O-bis-tert-butyldimethylsilyl-2'-deoxyadenosine* ***(dA.11)***

NaOH (26 mg, 1.1 mmol, dry) was added to a solution of compound **dA.07** (580 mg, 1.0 mmol) in anhydrous DMF (5 mL) at 0°C and stirred for 45 minutes. A solution of 4-methoxy-2-nitrobenzyl bromide (260 mg, 1.05 mmol) in anhydrous DMF (1.0 mL) was added dropwise. The mixture was gradually warmed to room temperature and stirred overnight. DMF was removed *in vacuo,* and the residue was dissolved in ethyl acetate (50 mL) and washed twice with saturated $NH_4Cl$ solution (30 mL each). The combined aqueous layer was extracted with ethyl acetate (20 mL), and the combined organic layer was dried over $Na_2SO_4$, concentrated *in vacuo,* and purified by silica gel column chromatography to yield *$N^6$-tert*-butyloxycarbonyl-*$N^6$*-(4-methoxy-2-nitrobenzyl)-3',5'-*O*-bis-*tert*-butyldimethylsilyl-2'-deoxyadenosine **dA.11** (480 mg, 64%) as a viscous oil.

*$^1H$ NMR (400 MHz, $CDCl_3$):* δ 8.68 (s, 1 H, H-8), 8.37 (s, 1 H, H-2), 7.66 (d, 1 H, *J* = 8.7 Hz, Ph-H), 7.55 (d, 1 H, *J* = 2.7 Hz, Ph-H), 7.10 (dd, 1 H, *J* = 2.7 and 8.7 Hz , Ph-H), 6.51 (t, 1 H, *J* = 6.4 Hz, H-1'), 5.55 (s, 2 H, Ph-$CH_2$), 4.63 (m, 1 H, H-4'), 4.03 (m, 1 H, H-3'), 3.87 (m, 1 H, H-5'a), 3.84 (s, 3 H, $OCH_3$), 3.78 (m, 1 H, H-5'b), 2.63 (m, 1 H, H-2'a), 2.44 (m, 1 H, H-2'b), 1.41 (s, 9 H, $(CH_3)_3CO$), 0.92 (s, 18 H, $(CH_3)_3CSi$), 0.10 (2 s, 12 H, $(CH_3)_2Si$-);

*ToF-MS (ESI):* For the molecular ion $C_{35}H_{57}N_6O_5Si_2$ $[M+H]^+$, the calculated mass was 745.3776, and the observed mass was 745.3782.

*$N^6$-(4-Methoxy-2-nitrobenzyl)-3',5'-O-bis-tert-butyldimethylsilyl-2'-deoxyadenosine* ***(dA.12)***

Silica gel 60 (5 g, 100-200 mesh, activated by heating to 70-80°C under reduced pressure for 24 hours) was added to a solution of compound **dA.11** (530 mg, 0.71 mmol) in $CH_2Cl_2$ (30 mL), and the mixture was evaporated *in vacuo* to dryness. The residue was heated to 70-80°C under reduced pressure for two days, washed three times with methanol (20 mL each), and filtered using a buchi funnel. The combined filtrate was concentrated *in vacuo* and purified by silica gel column chromatography to yield *$N^6$*-(4-methoxy-2-nitrobenzyl)-3',5'-*O*-bis-*tert*-butyldimethylsilyl-2'-deoxyadenosine **dA.12** (385 mg, 84%) as a yellow foam.

*$^1H$ NMR (400 MHz, $CDCl_3$):* δ 8.37 (s, 1 H, H-8), 8.08 (s, 1 H, H-2), 7.66 (d, 1 H, *J* = 8.5 Hz, Ph-H), 7.57 (m, 1 H, *J* = 2.7 Hz Ph-H), 7.09 (dd, 1 H, *J* = 2.7 and 8.7 Hz Ph-H), 6.52 (t, 1 H, 6-NH), 6.43 (t, 1 H, *J* = 6.4 Hz, H-1'), 5.07 (bs, 2 H, Ph-$CH_2$), 4.60 (m, 1 H, H-4'), 4.00 (m, 1 H, H-3'), 3.87 (m, 1 H, H-5'a), 3.85 (s, 3 H, $OCH_3$), 3.76 (dd, 1 H, H-5'b), 2.62 (m, 1 H, H-2'a), 2.43 (m, 1 H, H-2'b), 0.91 (s, 18 H, $(CH_3)_3CSi$), 0.09 (2 s, 12 H, $(CH_3)_2Si$-);

*ToF-MS (ESI):* For the molecular ion $C_{30}H_{48}N_6O_6Si_2$ $[M+H]^+$, the calculated mass was 645.3252, and the observed mass was 645.3248.

*$N^6$-(4-Methoxy-2-nitrobenzyl)-2'-deoxyadenosine* ***(dA.13)***

A solution of *n*-$Bu_4NF$ (353 mg, 1.35 mmol) in THF (2 mL) was added to a solution of compound **dA.12** (350 mg, 0.54 mmol) in THF (5 mL) at 0°C. The reaction mixture was gradually warmed to room temperature and stirred for four hours. Silica gel 60 (1.5 g) was added, and the mixture was evaporated *in vacuo* to dryness. The residue was purified by silica gel column chromatography to yield *$N^6$*-(4-methoxy-2-nitrobenzyl)-2'-deoxyadenosine **dA.13** (225 mg, 99%) as a yellow foam.

*$^1H$ NMR (400 MHz, DMSO-$d_6$):* δ 8.40 (bs, 1 H, $D_2O$ exchangeable, 6-NH), 8.39 (s, 1 H, H-8), 8.15 (s, 1 H, H-2), 7.54 (d, 1 H, *J* = 2.7 Hz, Ph-H), 7.44 (d, 1 H, *J* = 8.2 Hz, Ph-H), 7.24 (d, 1 H, *J* = 2.7 and 8.7 Hz, Ph-H), 6.33 (t, 1 H, *J* = 6.7 Hz, H-1'), 5.32 (d, 1 H, $D_2O$ exchangeable, 3'-OH), 5.17 (t, 1 H, $D_2O$ exchangeable, 5'-OH), 4.88 (bs, 2 H, Ph-$CH_2$), 4.40 (m, 1 H, H-4'), 3.87 (m, 1 H, H-3'), 3.81 (s, 3 H, $OCH_3$), 3.60 (m, 1 H, H-5'a), 3.52 (m, 1 H, H-5'b), 2.70 (m, 1 H, H-2'a), 2.26 (m, 1 H, H-2'b);

*ToF-MS (ESI):* For the molecular ion $C_{18}H_{21}N_6O_6$ $[M+H]^+$, the calculated mass was 417.1523, and the observed mass was 417.1458.

*$N^6$-(4-Methoxy-2-nitrobenzyl)-2'-deoxyadenosine-5'-triphosphate* ***(WW2p005)***

$POCl_3$ (19 μL, 0.2 mmol) was added to a solution of compound **dA.13** (42 mg, 0.1 mmol) in trimethylphosphate (0.5 mL) and maintained at minus 20-30°C for three hours. A solution of bis-tri-*n*-butylammonium pyrophosphate (237 mg, 0.5 mmol) and tri-*n*-butylamine (100 μL) in anhydrous DMF (1 mL) was added. After five minutes of stirring, triethylammonium bicarbonate buffer (1 M, pH 7.5; 10 mL) was added. The reaction was stirred at room temperature for one hour and then lyophilized to dryness. The residue was dissolved in water (10 mL), filtered, and purified by anion exchange chromatography using a Q Sepharose FF column (2.5 x 20 cm) with a linear gradient of $NH_4HCO_3$ (50 mM to 500 mM in 300 minutes) at a flow rate of 4.5 mL/min. The fractions containing triphosphate were combined and lyophilized to give *$N^6$*-(4-methoxy-2-nitrobenzyl)-2'-deoxyadenosine-5'-triphosphate **WW2p005** (28 mg, 40%) as a white fluffy solid.

*$^1H$ NMR (400 MHz, $D_2O$):* δ 8.41 (s, 1 H, H-8), 8.17 (s, 1 H, H-2), 7.64 (d, 1 H, *J* = 2.7 Hz, Ph-H), 7.47 (d, 1 H, *J* = 8.7 Hz, Ph-H), 7.15 (d, 1 H, *J* = 2.7 and 8.7 Hz, Ph-H), 6.46 (t, 1 H, *J* = 6.7 Hz, H-1'), 4.97 (bs, 2 H, Ph-$CH_2$), 4.29 (s, 1 H, H-3'), 4.20 (m, 2 H, H-5'a and H-5'b), 3.84 (s, 3 H, $OCH_3$), 2.80 (m, 1 H, H-2'a), 2.60 (m, 1 H, H-2'b);

*$^{31}P$ NMR (162 MHz, $D_2O$):* δ -5.97 (d, *J* = 19.9 Hz), -11.07 (d, *J* = 19.3 Hz), -21.76 (t, *J* = 19.3 Hz);

*ToF-MS (ESI):* For the molecular ion $C_{18}H_{21}N_6O_{15}P_3Na$ $[M-2H+Na]^-$, the calculated mass was 677.0175, and the observed mass was 677.0197.

The triphosphate was further purified using preparative HPLC without UV detection to give sample free from contamination of the natural nucleotide. Determination of the concentration of the triphosphate solution was performed by UV/VIS measurement using the extinction coefficient of $\varepsilon_{260}$ = 20,800.

**Synthesis of 6-FAM labeled *$N^6$*-[4-(3-amino-1-propyl)-2-nitrobenzyl]-2'-deoxyadenosine triphosphate (WW2p055)**

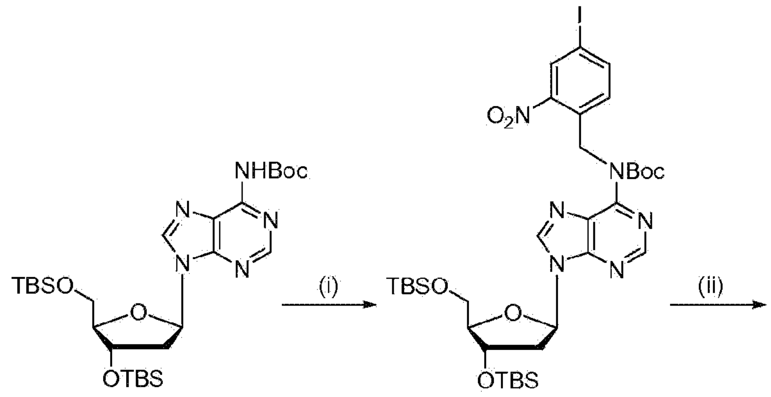


dA.07 dA.14

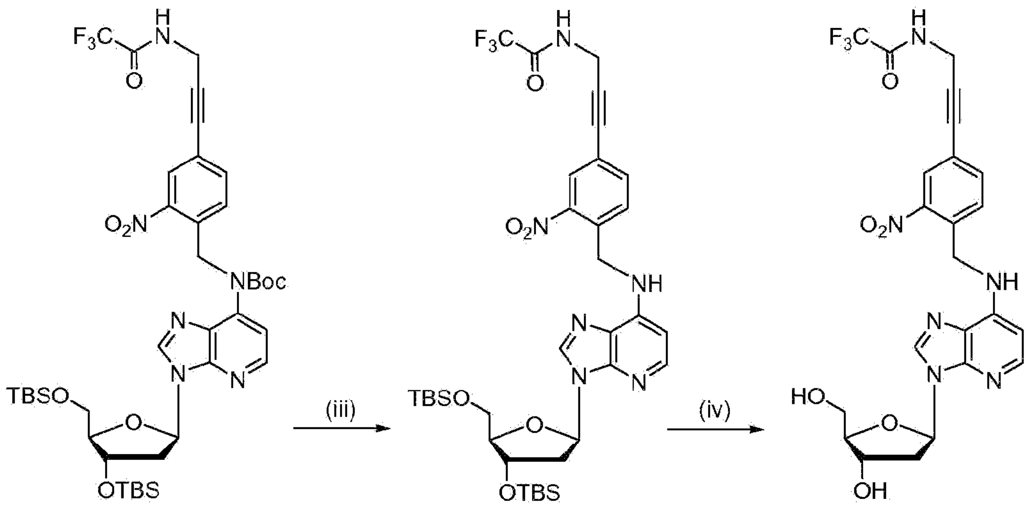


dA.15 dA.16 dA.17

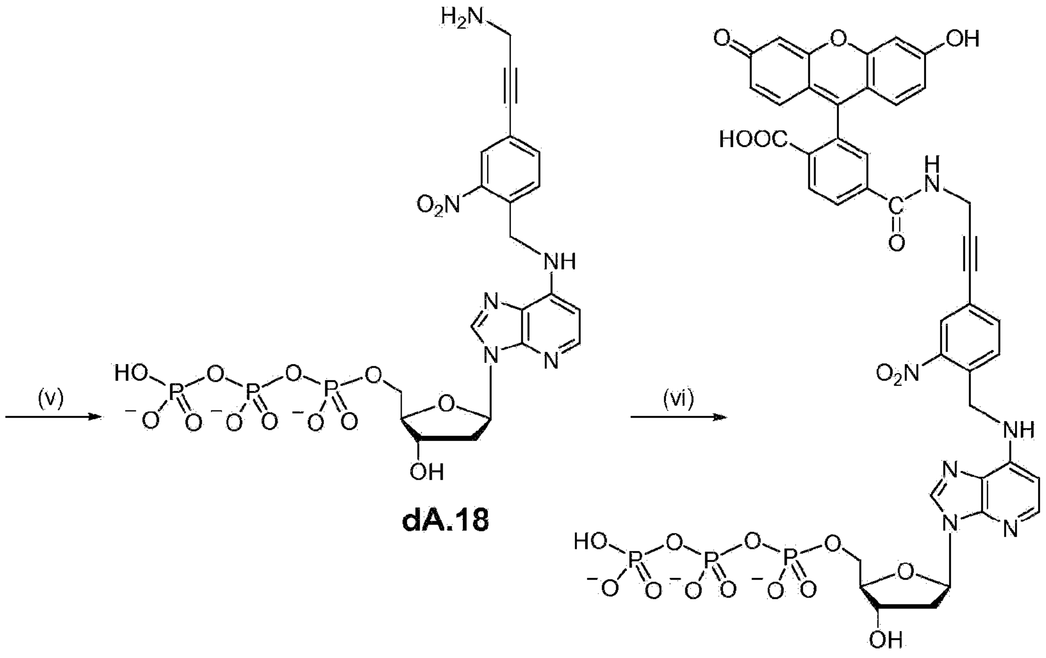


dA.18

WW2p055

**Scheme.** Synthesis of *6-FAM labeled $N^6$-[4-(3-amino-1-propyl)-2-nitrobenzyl]-2'deoxyadenosine triphosphate*. (i) NaH, DMF, 4-iodo-2-nitrobenzyl bromide, 0°C, then gradually warmed to room temperature, 61%; (ii) $PdCl_2(PPh_3)_2$, CuI, $Et_3N$, THF, reflux, 94%; (iii) $SiO_2$, vacuum, 70-80°C, 82%; (iv) *n*-$Bu_4NF$, THF, 0°C, then gradually warmed to room temperature, 33%; (v) $POCl_3$, proton sponge, $(MeO)_3PO$, minus 20-30°C, two hours; ($n$-$Bu_3NH)_2H_2P_2O_7$, *n*-$Bu_3N$, DMF, two minutes; 1 M $HNEt_3HCO_3$, one hour; $NH_4OH$, one hour; 72 %; (vi) 6-FAM-SE, 0.1 M $NaHCO_3/NaCO_3$, pH 9.2.

*$N^6$-tert-Butyloxycarbonyl-$N^6$-(4-iodo-2-nitrobenzyl)-3',5'-O-bis-tert-butyldimethylsilyl-2'-deoxyadenosine **(dA.14)***

NaH (40 mg, 1.66 mmol, dry) was added to a solution of compound **dA.07** (875 mg, 1.51 mmol) in anhydrous DMF (10 mL) at 0°C and stirred for 45 minutes. A solution of 4-iodo-2-nitrobenzyl bromide (516 mg, 1.51 mmol) in anhydrous DMF (2 mL) was added dropwise. The mixture was gradually warmed to room temperature and stirred for four hours. DMF was removed *in vacuo*, and the residue was dissolved in ethyl acetate (50 mL), washed with saturated $NH_4Cl$ solution (30 mL), and washed twice with water (30 mL each). The combined aqueous layer was extracted with ethyl acetate (20 mL), and the combined organic layer was dried over $Na_2SO_4$, concentrated *in vacuo,* and purified by silica gel column chromatography to yield $N^6$-*tert*-butyloxycarbonyl-$N^6$-(4-iodo-2-nitrobenzyl)-3',5'-*O*-bis-*tert*-butyldimethylsilyl-2'-deoxyadenosine **dA.14** (777 mg, 61 %) as a white foam.

*$^1H$ NMR (400 MHz, $CDCl_3$)* $\delta$ 8.66 (s, 1 H, H-8), 8.38 (s, 1 H, H-2), 8.34 (d, 1 H, $J$ = 1.1 Hz, Ph-H), 7.85 (dd, 1 H, $J$ = 1.1 and 8.3 Hz, Ph-H), 7.50 (d, 1 H, $J$ = 8.3 Hz, Ph-H), 6.50 (t, 1 H, $J$ = 6.3 Hz, H-1'), 5.54 (s, 2 H, Ph-$CH_2$), 4.63 (m, 1 H, H-3'), 4.03 (m, 1 H, H-4'), 3.87 (m, 1 H, H-5'a), 3.78 (m, 1 H, H-5'b), 2.62 (m, 1 H, H-2'a), 2.46 (m, 1 H, H-2'b), 1.41 (s, 9 H, $(CH_3)_3CO$), 0.92 (s, 18 H, $(CH_3)_3CSi$), 0.10 (2 s, 12 H, $(CH_3)_2Si$).

*$N^6$-tert-Butyloxycarbonyl-$N^6$-[4-(3-trifluoroacetamido-1-propynyl)-2-nitrobenzyl]-3',5'-O-bis-tert-butyldimethylsilyl-2'-deoxyadenosine **(dA.15)***

Under a $N_2$ atmosphere, a mixture of compound **dA.14** (730 mg, 0.87 mmol), *N*-propargyltrifluoroacetamide (183 mg, 1.2 mmol)), CuI (33 mg, 0.17 mmol), bis(triphenylphosphine)palladium(II) chloride (61 mg, 0.087 mmol), and $Et_3N$ (1.6 mL, 11.57 mmol) in anhydrous THF (7.5 mL) was refluxed for six hours in the dark. The mixture was concentrated *in vacuo,* and the residue was purified by silica gel column chromatography to yield $N^6$-*tert*-butyloxycarbonyl-$N^6$-[4-(3-trifluoroacetamido-1-propynyl)-2-nitrobenzyl]-3',5'-*O*-bis-*tert*-butyldimethylsilyl-2'-deoxyadenosine **dA.15** (706 mg, 94%) as a yellow foam.

*$^1H$ NMR (400 MHz, $CDCl_3$):* δ 8.67 (s, 1 H, H-8), 8.39 (s, 1 H, H-2), 8.06 (d, 1 H, *J* = 1.5 Hz, Ph-H), 7.73 (d, 1 H, *J* = 8.2 Hz, Ph-H), 7.56 (dd, 1 H, *J* = 1.5 and 8.2 Hz, Ph-H), 7.28 (br s, 1 H, NH), 6.51 (t, 1 H, *J* = 6.4 Hz, H-1'), 5.59 (s, 2 H, Ph-$CH_2$), 4.63 (m, 1 H, H-3'), 4.37 (m, 2 H, $CH_2$), 4.03 (m, 1 H, H-4'), 3.87 (m, 1 H, H-5'a), 3.78 (m, 1 H, H-5'b), 2.63 (m, 1 H, H-2'a), 2.48 (m, 1 H, H-2'b), 1.40 (s, 9 H, $(CH_3)_3CO$), 0.92 (s, 18 H, $(CH_3)_3CSi$), 0.10 (2 s, 12 H, $(CH_3)_2Si$-).

*$N^6$-[4-(3-Trifluoroacetamido-1-propynyl)-2-nitrobenzyl]-3',5'-O-bis-tert-butyldimethylsilyl-2 '-deoxyadenosine* ***(dA.16)***

Silica gel 60 (3.5 g, 100-200 mesh, activated by heating to 70-80°C under reduced pressure for 24 hours) was added to a solution of compound **dA.15** (507 mg, 0.59 mmol) in $CH_2Cl_2$ (30 mL), and the mixture was evaporated *in vacuo* to dryness. The residue was heated to 70-80°C under reduced pressure for 42 hours, washed three times with methanol (20 mL each), and filtered using a buchi funnel. The combined filtrate was concentrated *in vacuo* and purified by silica gel column chromatography to yield *N*$^6$-[4-(3-trifluoroacetamido-1-propynyl)-2-nitrobenzyl]-3',5'-*O*-bis-*tert*-butyldimethyl-silyl-2'-deoxyadenosine **dA.16** (366 mg, 82%) as a yellow foam.

*$^1H$ NMR (400 MHz, $CDCl_3$):* δ 8.35 (s, 1 H, H-8), 8.12 (s, 1 H, H-2), 8.05 (d, 1 H, *J* = 1.3 Hz, Ph-H), 7.66 (d, 1 H, *J* = 8.0 Hz, Ph-H), 7.48 (dd, 1 H, *J* = 1.3 and 8.0 Hz, Ph-H), 7.20 (bs, 1 H, NH), 6.54 (t, 1 H, NH), 6.44 (t, 1 H, *J* = 6.4 Hz, H-1'), 5.16 (bs, 2 H, Ph-$CH_2$), 4.61 (m, 1 H, H-4'), 4.39 (d, 2 H, $CH_2$), 4.00 (m, 1 H, H-3'), 3.87 (m, 1 H, H-5'a), 3.78 (m, 1 H, H-5'b), 2.62 (m, 1 H, H-2'a), 2.44 (m, 1 H, H-2'b), 1.40 (s, 9 H, $(CH_3)_3CO$), 0.91 (s, 18 H, $(CH_3)_3CSi$), 0.09 (2 s, 12 H, $(CH_3)_2Si$-).

*$N^6$-[4-(3-Trifluoroacetamido-1-propynyl)-2-nitrobenzyl]-2'-deoxyadenosine (**dA.17**)*

A solution of *n*-$Bu_4NF$ (282 mg, 1.08 mmol) in THF (2 mL) was added to a solution of compound **dA.16** (330 mg, 0.43 mmol) in THF (5 mL) at 0°C. The reaction mixture was gradually warmed to room temperature and stirred for two hours. Methanol (5 mL) and silica gel 60 (2 g) were added, and the mixture was evaporated *in vacuo* to dryness. The residue was purified by silica gel column chromatography to yield *N*$^6$-[4-(3-trifluoroacetamido-1-propynyl)-2-nitrobenzyl]-2'-deoxyadenosine **dA.17** (75 mg, 33%) as a white foam.

*$^1H$ NMR (400 MHz, DMSO-$d_6$):* δ 10.08 (t, 1 H, $D_2O$ exchangeable, NH), 8.50 (br s, 1 H, $D_2O$ exchangeable, NH), 8.42 (s, 1 H, H-8), 8.16 (s, 1 H, H-2), 8.06 (d, 1 H, *J* = 1.6 Hz, Ph-H), 7.71 (dd, 1 H, *J* = 1.6 and 8.1 Hz, Ph-H), 7.51 (m, 1 H, Ph-H), 6.35 (t, 1 H, *J* = 6.4 Hz, H-1'), 5.30 (d, 1 H, $D_2O$ exchangeable, 3'-OH), 5.13 (br s, 1 H, $D_2O$ exchangeable, 5'-OH), 4.96 (br s, 2 H, Ph-$CH_2$), 4.41 (m, 1 H, H-4'), 4.29 (d, 2 H, $CH_2$), 3.87 (m, 1 H, H-3'), 3.60 (m, 1 H, H-5'a), 3.51 (m, 1 H, H-5'b), 2.72 (m, 1 H, H-2'a), 2.27 (m, 1 H, H-2'b).

*$N^6$-[4-(3-Amino-1-propyl)-2-nitrobenzyl]-2'-deoxyadenosine-5'-triphosphate (**dA.18**)*

$POCl_3$ (8.5 μL, 0.09 mmol) was added to a solution of compound **dA.17** (32 mg, 0.06 mmol) and proton sponge (19 mg, 0.09 mmol) in trimethylphosphate (0.5 mL) and maintained at minus 20-30°C for two hours. A solution of bis-tri-*n*-butylammonium pyrophosphate (142 mg, 0.3 mmol) and tri-*n*-butylamine (60 μL) in anhydrous DMF (0.6 mL) was added. After two minutes of stirring, triethylammonium bicarbonate buffer (1 M, pH 7.5; 5 mL) was added. The reaction was stirred for one hour at room temperature, followed by the dropwise addition of concentrated ammonium hydroxide (10 mL, 27%) at 0°C. The mixture was stirred for an additional hour at room temperature and then lyophilized to dryness. The residue obtained was dissolved in water (10 mL), filtered, and purified by anion exchange chromatography using a Q Sepharose FF column (2.5 × 20 cm) with a linear gradient of $NH_4HCO_3$ (50 mM to 500 mM in 300 minutes) at a flow rate of 4.5 mL/min. The fractions containing triphosphate were combined and lyophilized to give triphosphate **dA.18** (31 mg, 72%) as a white fluffy solid.

*$^1H$ NMR (400 MHz, $D_2O$):* δ 8.47 (s, 1 H, H-8), 8.23 (s, 1 H, Ph-H), 8.20 (s, 1 H, H-2), 7.65 (d, 1 *H, J* = 8.2 Hz, Ph-H), 7.57 (d, 1 H, *J* = 8.2 Hz, Ph-H), 6.52 (t, 1 H, *J* = 6.8 Hz, H-1'), 5.14 (br s, 2 H, Ph-$CH_2$), 4.31 (s, 1 H, H-4'), 4.21 (m, 2 H, H-5'a and H-5'b), 3.60 (s, 2 H, $CH_2$), 2.82 (m, 1 H, H-2'a), 2.62 (m, 1 H, H-2'b);

*$^{31}P$ NMR (162 MHz, $D_2O$)*: δ -5.43 (d, *J* = 15.4 Hz), -10.46 (d, *J* = 15.6 Hz), -18.85 (t, *J* = 15.6 Hz);

*ToF-MS (ESI):* For the molecular ion $C_{20}H_{23}N_7O_{14}P_3$ $[M-H]^-$, the calculated mass was 678.0516, and the observed mass was 678.0857.

*6-FAM labeled $N^6$-[4-(3-Amino-1-propyl)-2-nitrobenzyl]-2'-deoxyadenosine-5'-triphosphate (**WW2p055**)*

A solution of 6-FAM-SE (6.7 mg, 0.014 mmol) in anhydrous DMSO (70 μL) was added to a solution of triphosphate **dA.18** (4.4 μmol) in $Na_2CO_3/NaHCO_3$ buffer (0.1 M, pH 9.2; 3 mL) and incubated at room temperature for one hour. The reaction was purified by reverse-phase HPLC using a Perkin Elmer OD-300 $C_{18}$ column (4.6 × 250 mm) to yield the 6-FAM labeled triphosphate **WW2p055** (2.6 mg, 49%). Mobile phase: A, 100 mM triethylammonium acetate (TEAA) in water (pH 7.0); B, 100 mM TEAA in water/$CH_3CN$ (30:70). Elution was performed with a linear gradient of

5-20% B for 20 minutes and then 20-90% B for 20 minutes. The concentration of **WW2p055** was estimated by adsorption spectroscopy using the extinction coefficient of the 6-FAM dye *(i.e.,* 68,000 at 494 nm).

*$^{31}P$ NMR (162 MHz, $D_2O$):* δ -5.87 (d, *J* = 19.8 Hz), -11.01 (d, *J* = 19.1 Hz), -21.76 (t, *J* = 19.8 Hz);

*ToF-MS (ESI):* For the molecular ion $C_{41}H_{35}N_7O_{20}P_3$ $[M+H]^+$, the calculated mass was 1038.1150, and the observed mass was 1138.1281.

**Synthesis of 5(6)-SFX labeled *N*$^6$-[4-(3-amino-1-propyl)-2-nitrobenzyl]-2'deoxy-aclenosine triphosphate (WW2p0.52)**

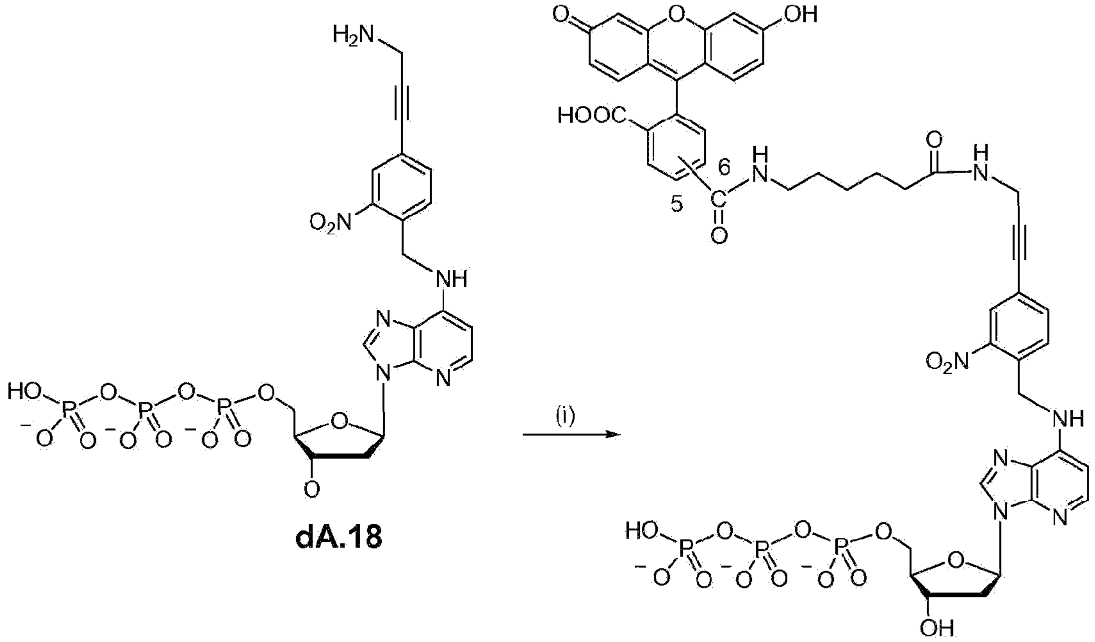


**dA.18**

**WW2p052**

**Scheme.** Synthesis of *5(6)-SFX labeled N$^6$-[4-(3-amino-1-propyl)-2-nitrobenzyl]-2'-deoxyadenosine triphosphate.* (i) 5(6)-SFX, 0.1 M $NaHCO_3/NaCO_3$, pH 9.2.

*5(6)-SFX lableled N$^6$-[4-(3-Amino-1-propyl)-2-nitrobenzyl]-2'-deoxyadenosine-5 '-triphosphate* (***WW2p052***)

A solution of 5(6)-SFX (1.5 mg, 2.55 μmol) in anhydrous DMSO (30 μL) was added to a solution of triphosphate **dA.18** (0.54 μmol) in $Na_2CO_3/NaHCO_3$ buffer (0.1 M, pH 9.2; 0.8 mL) and incubated at room temperature for one hour. The reaction was purified by reverse-phase HPLC using a Perkin Elmer OD-300 $C_{18}$ column (4.6 × 250 mm) to yield the 5(6)-SFX labeled triphosphate **WW2p052.** Mobile phase: A, 100 mM triethylammonium acetate (TEAA) in water (pH 7.0); B, 100 mM TEAA in water/$CH_3CN$ (30:70). Elution was performed with a linear gradient of 5-20% B for 20 minutes and then 20-90% B for 20 minutes. The concentration of **WW2p052** was estimated by adsorption spectroscopy using the extinction coefficient of the 6-FAM dye *(i.e.,* 68,000 at 494 nm).

Synthesis of ***N*$^6$-[1-(2-nitrophenyl)ethyl]-2'-deoxyadenosine** triphosphate (WW3p006)

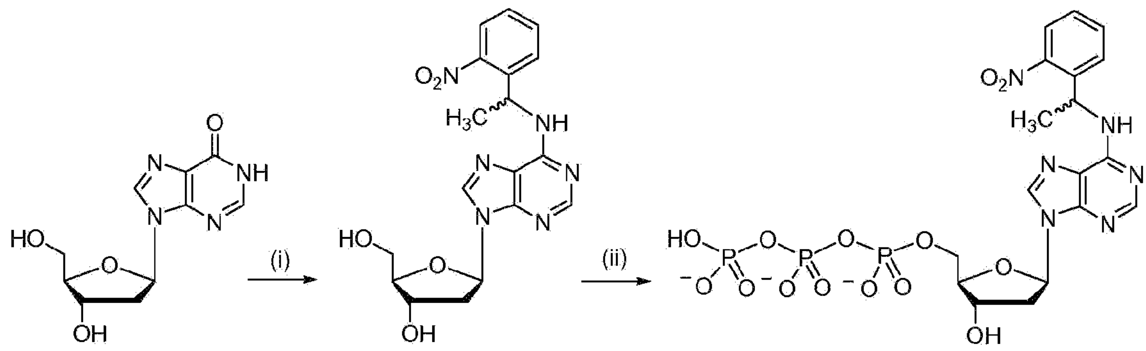


dI dA.19 WW3p006

**Scheme.** Synthesis of *$N^6$-[1-(2-nitrophenyl)ethyl]-2'-deoxyadenosine triphosphate* (i) 1-(2-nitrophenyl)ethylamine, BOP, DIPEA, DMF, room temperature, overnight, 42%; (ii) $POCl_3$, proton sponge, $(MeO)_3PO$, 0°C, two hours; $(n\text{-}Bu_3NH)_2H_2P_2O_7$, $n$-$Bu_3N$, DMF, five minutes; 1 M $HNEt_3HCO_3$, one hour.

*$N^6$-[1-(2-Nitrophenyl)ethyl]-2'-deoxyadenosine (dA. 19)*

To a suspension of 2'-deoxyinosine (100 mg, 0.4 mmol) and benzotriazol-1-yloxytris(dimethylamino)phosphonium hexafluorophosphate (BOP, 210 mg, 0.48 mmol) in anhydrous DMF (1 mL), *N,N*-diisopropylethylamine (100 µL, 0.6 mmol) was added followed by the addition of a solution of 1-(2-nitrophenyl)ethylamine (250 mg, 1.51 mmol) in DMF (1 mL). The reaction was stirred at room temperature for 64 hours. Silica gel 60 (1 g, 60 -200 mesh) was added, and the mixture was evaporated *in vacuo* to dryness. The residue was purified by silica gel column chromatography to yield $N^6$-[1-(2-nitrophenyl)ethyl]-2'-deoxyadenosine **dA.19** (67 mg, 42%, 1:1 mixture of diastereomers) as a white foam.

*$^1H$ NMR (400 MHz, DMSO-$d_6$) for diastereomers:* δ 8.68 (br s, 1 H, $D_2O$ exchangeable, NH), 8.42 (br s, 1H, H-8), 8.16 and 8.06 (2 s, 1H, H-2), 7.88 (m, 2 H, Ph-H), 7.69 (m, 1 H, Ph-H), 7.46 (m, 1 H, Ph-H), 6.34 (m, 1 H, H-1'), 5.79 (m, 1 H, Ph-CH), 5.32 (br s, 1 H, $D_2O$ exchangeable, 3'-OH), 5.16 (br s, 1 H, $D_2O$ exchangeable, 5'-OH), 4.42 (m, 1 H, H-3'), 3.88 (m, 1 H, H-4'), 3.61 (m, 1H, H-5'a), 3.53 (m, 1 H, H-5'b), 2.71 (m, 2 H, H-2'a), 2.25 (m, 1 H, H-2'b), 1.68 (d, 3 H, $J$ = 6.8 Hz, $CH_3$)

*$N^6$-[1-(2-Nitrophenyl)ethyl]-2'-deoxyadenosine-5'-triphosphate **(WW3p006)***

Compound **dA.19** (30 mg, 0.075 mmol) and proton sponge (32 mg, 0.15 mmol) were evaporated three times from anhydrous pyridine (2 mL) and dissolved in trimethylphosphate (0.5 mL). $POCl_3$ (10.5 µL, 0.11 mmol) was added, and the mixture was stirred for two hours at 0°C. A solution of bis-tri-*n*-butylammonium pyrophosphate (178mg, 0.38 mmol) and tri-*n*-butylamine (75 µL) in anhydrous DMF (0.75 mL) was added. After five minutes of stirring, triethylammonium bicarbonate buffer (1 M, pH 7.5; 10 mL) was added. The reaction was stirred for one hour at room temperature and then lyophilized to dryness. The residue was dissolved in water (10 mL), filtered, and part of the solution was purified with reverse-phase HPLC using a Perkin Elmer OD-300 $C_{18}$ column (4.6 × 250 mm) to yield $N^6$-[1-(2-nitrophenyl)ethyl]-2'-deoxyadenosine-5'-triphosphate **WW3p006** (1:1 mixture of diastereomers). Mobile phase: A, 100 mM triethylammonium acetate (TEAA) in water (pH 7.0); B, 100 mM TEAA in water/$CH_3CN$ (30:70). Elution was performed with a linear gradient of 5-50% B for 40 minutes and then 50-90% B for 10 minutes.

*$^1H$ NMR (400 MHz, $D_2O$) for diastereomers:* δ 8.47 (s, 1 H, H-8), 8.12 (2 s, 1 H, H-2), 8.02 (d, 1 H, $J$ = 8.2 Hz, Ph-H), 7.78 (d, 1 H, $J$ = 7.8 Hz, Ph-H), 7.67 (t, 1 H, $J$ = 7.6 Hz, Ph-H), 7.49 (t, 1 H, $J$ = 8.1 Hz, Ph-H), 6.49 (t, 1 H, $J$ = 6.4 Hz, H-1'), 5.89 (bs, 1 H, Ph-CH), 4.29 (m, 1 H, H-4'), 4.23 - 4.15 (m, 2 H, H-5'a and H-5'b), 2.81 (m, 1 H, H-2'a), 2.58 (m, 1 H, H-2'b), 1.74 (d, 3 H, $J$ = 6.8 Hz, $CH_3$);

*$^{31}P$ NMR (162 MHz, $D_2O$) for diastereomers:* δ -5.65 (m), -10.52 (d, $J$ = 19.6 Hz), -21.32 (m);

*ToF-MS (ESI):* For the molecular ion $C_{18}H_{21}N_6O_{14}P_3Na$ $[M-2H+Na]^-$, the calculated mass was 661.0226, and the observed mass was 661.0492.

**Synthesis of 6-FAM labeled *N6*-{1-[4-(3-amino-1-propynyl)-2-nitrophenyl]ethyl}-2'-deoxyadenosine triphosphate (WW3p015)**

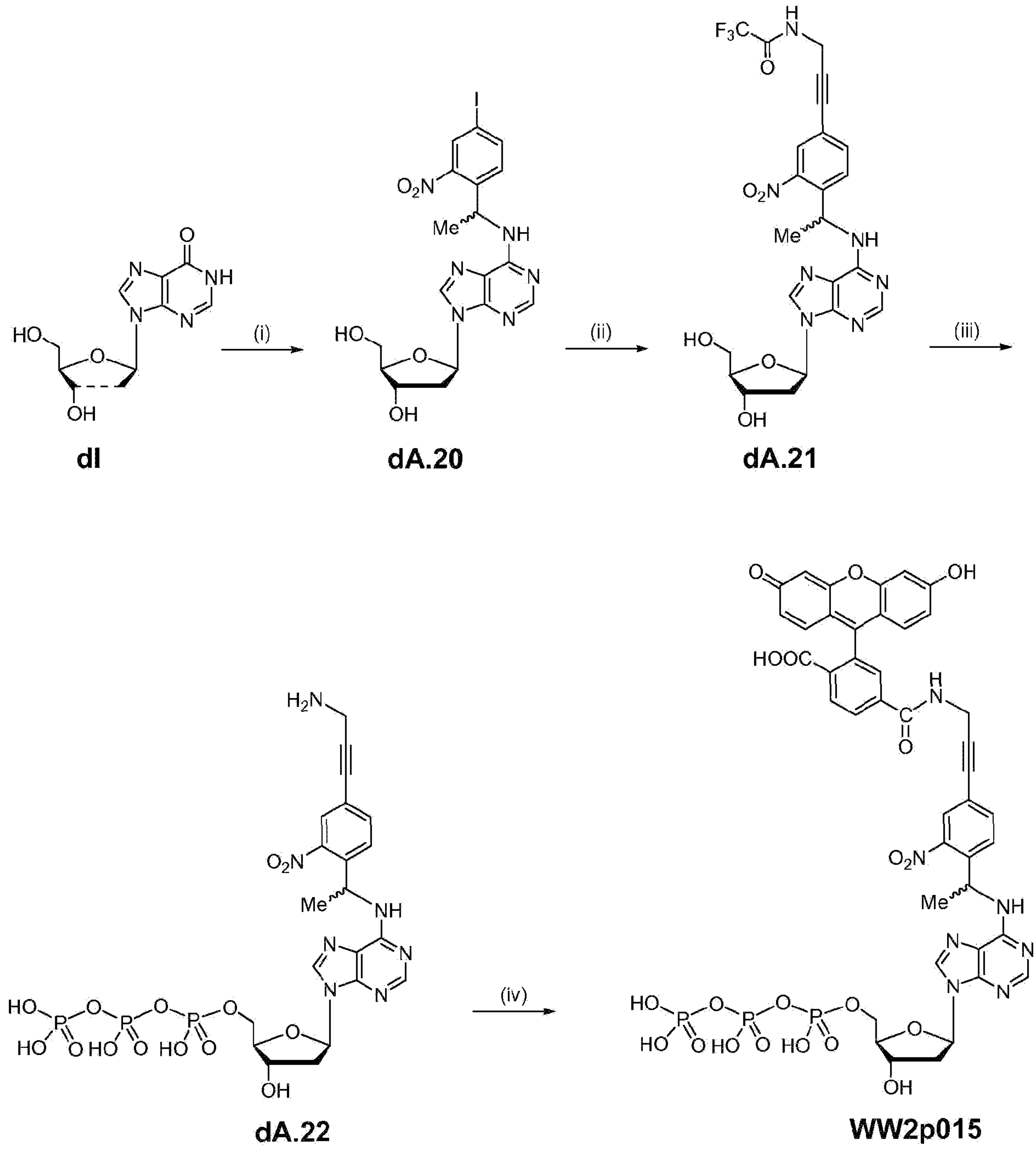


dI dA.20 dA.21

dA.22 WW2p015

**Scheme.** Synthesis of *6-FAM labeled $N^6$-{1-[4-(3-amino-1-propynyl)-2-nitrophenyl]ethyl}-2'-deoxyadenosine triphosphate.* (i) 1-(4-iodo-2-nitrophenyl)ethylamine, BOP, DIPEA, DMF, room temperature, 65%; (ii) *N*-propargyltrifluoroacetamide, $Pd(PPh_3)_4(0)$, CuI, $Et_3N$, anhydrous DMF, 4.5 h, 94%; (iii) $POCl_3$, proton sponge, $(MeO)_3PO$, 0°C, two hours; $(n\text{-}Bu_3NH)_2H_2P_2O_7$, $n$-$Bu_3N$, DMF, five minutes; 1 M $HNEt_3HCO_3$, one hour; $NH_4OH$, one hour; (iv) 6-FAM-SE, 0.1 M $NaHCO_3/Na_2CO_3$, pH 9.2, one hour.

*$N^6$-[1-(4-Iodo-2-nitrophenyl)ethyl]-2'-deoxyadenosine (**dA.20**)*

To a suspension of 2'-deoxyinosine (150 mg, 0.6 mmol) and benzotriazol-1-yloxytris(dimethylamino)phosphonium hexafluorophosphonate (BOP, 379 mg, 0.86 mmol) in anhydrous DMF (1.5 mL), *N,N*-diisopropylethylamine (186 μL, 1.1 mmol) was added followed by the addition of a solution of 1-(4-iodo-2-nitrophenyl)ethylamine (460 mg, 1.57 mmol) in anhydrous DMF (0.5 mL). The mixture was stirred under nitrogen atmosphere for 48 hours. DMF was removed *in vacuo*, and the crude product was purified by silica gel column chromatography to yield $N^6$-[1-(4-iodo-2-nitrophenyl)ethyl]-2'-deoxyadenosine **dA.20** (204 mg, 65%, 1:1 mixture of diastereomers) as a white foam.

*$^1H$ NMR (400 MHz, DMSO-$d_6$) for diastereomers:* δ 8.72 (br m, 1 H, $D_2O$ exchangeable, NH), 8.42 (br s, 1 H, H-8), 8.20 (s, 1 H, H-2), 8.06 (m, 2 H, Ph-H), 7.65 (m, 1 H, Ph-H), 6.34 (m, 1 H, H-1'), 5.70 (br s, 1 H, PhCH), 5.32 (br s, 1 H, $D_2O$ exchangeable, 3'-OH), 5.15 (br m, 1 H, $D_2O$ exchangeable, 5'-OH), 4.42 (m, 1 H, H-4'), 3.88 (m, 1 H, H-3'), 3.61 (m, 1 H, H-5'a), 3.53 (m, 1 H, H-5'b), 2.71 (m, 1 H, H-2'a), 2.25 (m, 1 H, H-2'b), 1.65 (d, 3 H, *J* = 6.9 Hz, $CH_3$);

*$^{13}C$ NMR (100 MHz, MeOH-$d_4$) for diastereomers:* δ 153.39 (CH), 151.94 (C), 150.65 (C), 143.44 (CH), 141.82 (C), 141.40 (C), 133.77/133.69 (CH), 130.55/130.52 (CH), 121.37 (C), 92.09 (C), 91.87 (C), 89.97 (CH), 87.21 (CH), 73.18/73.15 (CH), 65.78/65.76 ($CH_2$), 47.12 (CH), 41.62 ($CH_2$), 37.14/37.10 ($CH_3$);

*ES-MS (ESI):* m/e 525 $[M\text{-}H]^-$.

*$N^6$-{1-[4-(3-Trifluoroacetamido-1-propynyl)-2-nitrophenyl]ethyl}-2'-deoxyadenosine (**dA.21**)*

A solution of compound **dA.20** (108 mg, 0.21 mmol), *N*-propargyltrifluoroacetamide (127 mg, 0.84 mmol), CuI (11 mg, 0.06 mmol), tetrakis(triphenylphosphine)-palladium(0) (33 mg, 0.03 mmol), and $Et_3N$ (80 μL, 0.56 mmol) in anhydrous DMF (1.5 mL) was stirred at room temperature for four and a half hours. The mixture was concentrated *in vacuo* and purified by silica gcl column chromatography to yield $N^6$-{1-[4-(3-trifluoroacetamido-1-propynyl)-2-nitrophenyl]ethyl]}-2'-deoxyadenosine **dA.21** (106 mg, 94%, 1:1 mixture of diastereomers) as a waxy solid.

*$^1H$ NMR (400 MHz, DMSO-$d_6$) for diastereomers:* δ 10.10 (br m, 1 H, $D_2O$ exchangeable, NH), 8.71 (br m, 1 H, $D_2O$ exchangeable, NH), 8.43 (br s, 1 H, H-8), 8.15 and 8. 06 (2 s, 1 H, H-2), 7.93 (s, 1 H, Ph-H), 7.86 (m, 1 H, Ph-H), 7.75 (m, 1 H, Ph-H), 6.35 (br m, 1 H, H-1'), 5.73 (br m, 1 H, Ph-CH), 5.31 (br s, 1 H, $D_2O$ exchangeable, 3'-OH), 5.15 (br m, 1 H, $D_2O$ exchangeable, 5'-OH), 4.40 (m, 1 H, H-4'), 4.31 (d, 2 H, *J* = 5.3 Hz, $CH_2$), 3.88 (m, 1 H, H-3'), 3.62 (m, 1 H, H-5'a), 3.51 (m, 1 H, H-5'b), 2.71 (m, 1 H, H-2'a), 2.25 (m, 1 H, H-2'b), 1.67 (d, 3 H, *J* = 6.8 Hz, $CH_3$);

*$^{13}C$ NMR (100 MHz, MeOH-$d_4$) for diastereomers:* δ 157.85/157.48 (C), 152.26 (CH), 149.50 (C), 140.27 (C), 136.04 (CH), 128.02 (CH), 127.07 (CH), 122.56 (C), 120.25 (C), 117.81 (C), 114.96 (C), 88.85 (CH), 86.08 (CH), 85.81 (C), 80.67 (C), 72.07/72.04 (CH), 62.66/62.63 ($CH_2$), 48.30 (CH), 40.47 ($CH_2$), 29.46 ($CH_2$), 24.26 ($CH_3$);

*$N^6$-{1-[4-(3-Amino-1-propynyl)-2-nitrophenyl]ethyl}-2'-deoxyadenosine -5'-triphosphate (**dA.22**)*

Compound **dA.21** (44 mg, 0.08 mmol) and proton sponge (34 mg, 0.16 mmol) were evaporated three times from anhydrous pyridine (2 mL) and dissolved in trimethylphosphate (0.5 mL). $POCl_3$ (11 μL, 0.12 mmol) was added, and the mixture was stirred for two hours at 0°C. A solution of bis-tri-*n*-butylammonium pyrophosphate (190 mg, 0.4 mmol) and tri-*n*-butylamine (80 μL) in anhydrous DMF (0.8 mL) was added. After five minutes of stirring, triethylammonium bicarbonate buffer (1 M, pH 7.5; 10 mL) was added. The reaction was stirred for one hour at room temperature and then lyophilized to dryness. The residue was dissolved in water (10 mL), filtered, and part of the solution was purified with reverse-phase HPLC using a Perkin Elmer OD-300 $C_{18}$ column (4.6 x 250 mm) to yicld $N^6$-{1-[4-(3-trifluoroacetamido-1-propynyl)-2-nitrophenyl]ethyl}-2'-deoxyadenosine-5'-triphosphate. Mobile phase: A, 100 mM triethylammonium acetate (TEAA) in water (pH 7.0); B, 100 mM TEAA in water/$CH_3CN$ (30:70). HPLC purification was achieved using a linear gradient of 5-50% B for 20 minutes and then 50-90% B for 10 minutes. $N^6$-{1-[4-(3-trifluoroacetamido-1-propynyl)-2-nitrophenyl]ethyl}-2'-deoxyadenosine-5'-triphosphate was then treated with concentrated ammonium hydroxide (1 mL, 27%) at room temperature for one hour to yield $N^6$-{1-[4-(3-amino-1 -propynyl)-2-nitrophenyl]ethyl}-2'-deoxyadenosine-5'-triphos-phate **dA.22** (1:1 mixture of diastereomers).

*$^1H$ NMR (400 MHz, $D_2O$):* δ 8.45 (s, 1 H, H-8), 8.08 (2 s, 1 H, H-2), 7.95 (s, 1 H, Ph-H), 7.65 (m, 1 H, Ph-H), 7.53 (m, 1 H, Ph-H), 6.45 (t, 1 H, *J* = 6.4 Hz, H-1'), 5.80 (br s, 1 H, Ph-CH), 4.28 (s, 1 H, H-4'), 4.18 (m, 2 H, H-5'a and H-5'b), 3.64 (s, 2 H, $CH_2$), 2.78 (m, 1 H, H-2'a), 2.57 (m, 1 H, H-2'b), 1.69 (d, 3 H, *J* = 6.8 Hz, $CH_3$);

*$^{31}P$ NMR (162 MHz, $D_2O$):* δ -5.29 (d, *J* = 20.1 Hz), -10.45 (d, *J* = 19.1 Hz), -21.08 (t, *J* = 19.6 Hz);

*ToF-MS (ESI):* For the molecular ion $C_{21}H_{25}N_7O_{14}P_3$ $[M-H]^-$, the calculated mass was 692.0672 and the observed mass was 692.0757.

*6-FAM labeled $N^6$-{1-[4-(3-Amino-1-propynyl)-2-nitrophenyl]ethyl}-2'-deoxyadenosine-5'-triphosphate* ***(WW3p015)***

A solution of 6-FAM-SE (1.5 mg, 3.15 μmol) in anhydrous DMSO (30 μL was added to a solution of triphosphate **dA.22** (0.34 μmol) in $Na_2CO_3NaHCO_3$ buffer (0.1 M, pH 9.2; 100 μL) and incubated at room temperature for one hour. The reaction was purified by reverse-phase HPLC using a Perkin Elmer OD-300 $C_{18}$ column (4.6 × 250 mm) to yield the 6-FAM labeled triphosphate **WW3p015.** Mobile phase: A, 100 mM triethylammonium acetate (TEAA) in water (pH 7.0); B, 100 mM TEAA in water/$CH_3CN$ (30:70). HPLC purification was achieved using a linear gradient of 5-50% B for 40 minutes and then 50-90% B for 10 minutes. The concentration of **WW3p015** was estimated by adsorption spectroscopy using the extinction coefficient of the 6-FAM dye *(i.e.,* 68,000 at 494 nm).

**Separation of diastereoisomers *$N^6$*-{1-[4-(3-amino-1-propynyl)-2-nitrophenyl]-ethyl}-2'-deoxyadenosine-5'-triphosphate (dA.22 dS1 and dA.22 ds2)**

Separation of the two diastereoisomers of **dA.22** was performed by reverse-phase HPLC using a Perkin Elmer OD-300 $C_{18}$ column (4.6 × 250 mm) to yield *$N^6$*-{(*R or S*)-1-[4-(3-amino-1-propynyl)-2-nitrophenyl]ethyl}-2'-deoxyadenosine triphosphate **dA.22 dS1** (single diastereoisomer, absolute configuration not determined) and *$N^6$*-{(*S or R*)-1-[4-(3-amino-1-propynyl)-2-nitrophenyl]ethyl}-2'-deoxyadenosine triphosphate **dA.22 dS2** (single diastereoisomer, absolute configuration not determined). Mobile phase: A, 100 mM triethylammonium acetate (TEAA) in water (pH 7.0); B, 100 mM TEAA in water/$CH_3CN$ (30:70). HPLC purification was achieved using a linear gradient of 5-25% B for 50 minutes and then 25-50% B for 30 minutes.

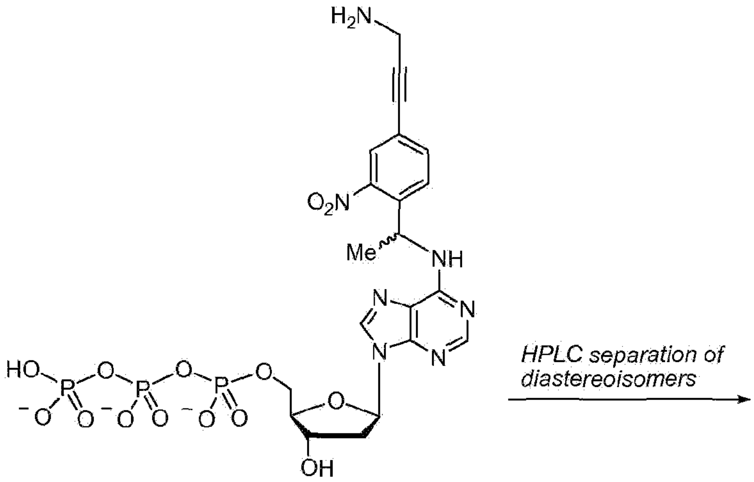


**dA.22**
*1:1 mixture of diastereoisomers*

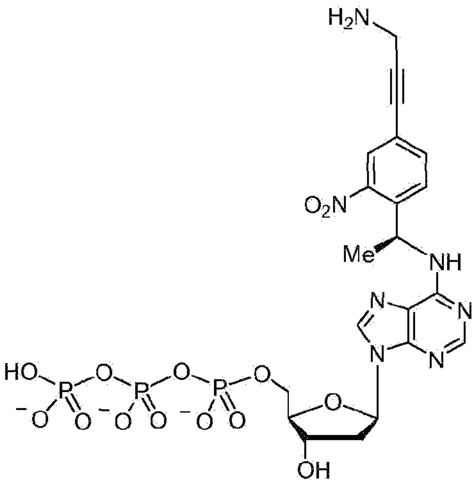

+

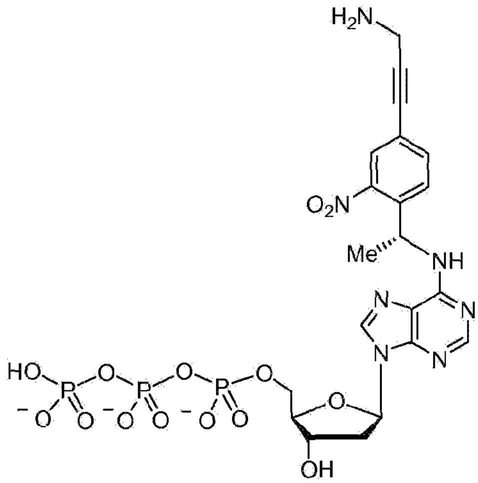

**dA.22 ds1**
*Fast eluting single diastereoisomer, absolute configuration not determined, drawing is representative*

**dA.22 ds2**
*Slow eluting single diastereoisomer, absolute configuration not determined, drawing is representative*

**Synthesis of 6-FAM labeled single diastereoisomer *N*$^6$-{(*R or S*)-1-[4-(3-amino-1-propynyl)-2-nitrophenyl]ethyl}-2'-deoxyadenosine-5'-triphosphate (WW3p021)**

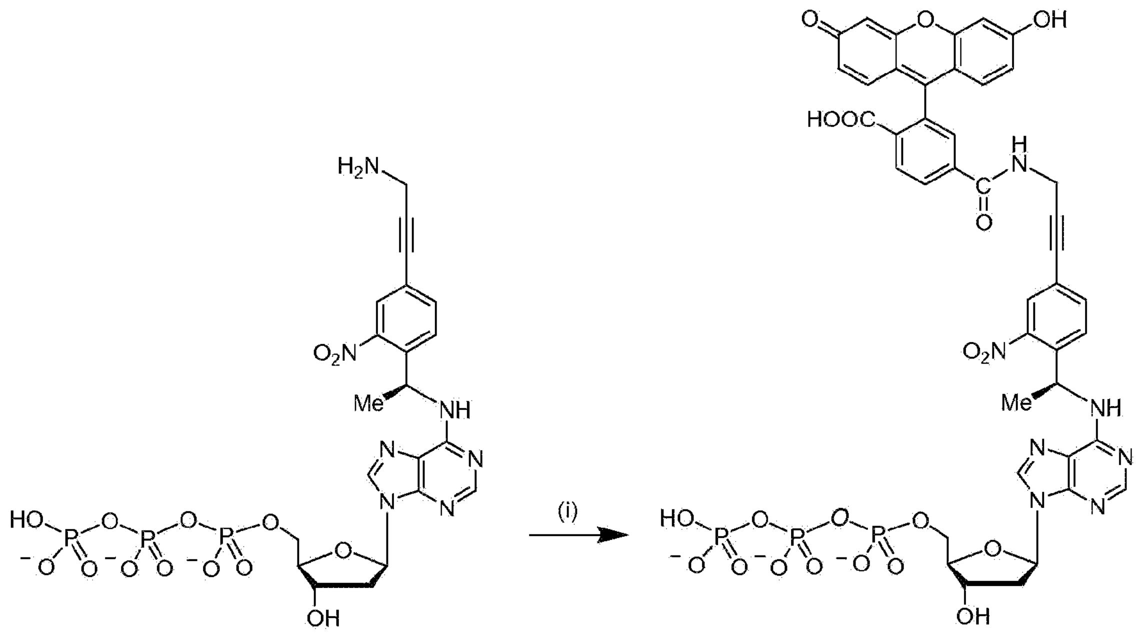


**dA.22 ds1**
*absolute configuration undetermined, drawing is representative*

**WW3p021**
*absolute configuration undetermined, drawing is representative*

**Scheme.** Synthesis of *6-FAM labeled single diastereoisomer $N^6$-{(R or S)-1-[4-(3-amino-1-propynyl)-2-nitrophenyl]ethyl}-2'-deoxyadenosine-5'-triphosphate.* (i) 6-FAM-SE, 0.1 M $NaHCO_3/Na_2CO_3$, pH 9.2, one hour.

*6- FAM labeled single diastereoisomers $N^6$-{(R or S)-1-[4-(3-amino-1-propynyl)-2-nitrophenyl]ethyl}-2'-deoxyadenosine-5'-triphosphate (**WW3p021**)*

A solution of 6-FAM-SE (0.75 mg, 1.57 μmol) in anhydrous DMSO (15 μL) was added to a solution of triphosphate **dA.22 ds1** (0.26 μmol, single diastereoisomer, absolute configuration not determined) in $Na_2CO_3/NaHCO_3$ buffer (0.1 M, pH 9.2; 150 μL) and incubated at room temperature for one hour. The reaction was purified by reverse-phase HPLC using a Perkin Elmer OD-300 $C_{18}$ column (4.6 × 250 mm) to yield the 6-FAM labeled single diastereoisomer triphosphate **WW3p021.** Mobile phase: A, 100 mM triethylammonium acetate (TEAA) in water (pH 7.0); B, 100 mM TEAA in water/$CH_3CN$ (30:70). HPLC purification was achieved using a linear gradient of 5-50% B for 40 minutes and then 50-90% B for 10 minutes. The concentration of **WW3p021** was estimated by adsorption spectroscopy using the extinction coefficient of the 6-FAM dye *(i.e.,* 68,000 at 494 nm).

**Synthesis of 6-FAM labeled single diastereoisomer *N6*-{(*R or S*)-1-{4-[3-(6-aminocaproyl)amino-1-propynyl]-2-nitrophenyl}ethyl}-2'-deoxyadenosine-5'- triphosphate (WW3p032)**

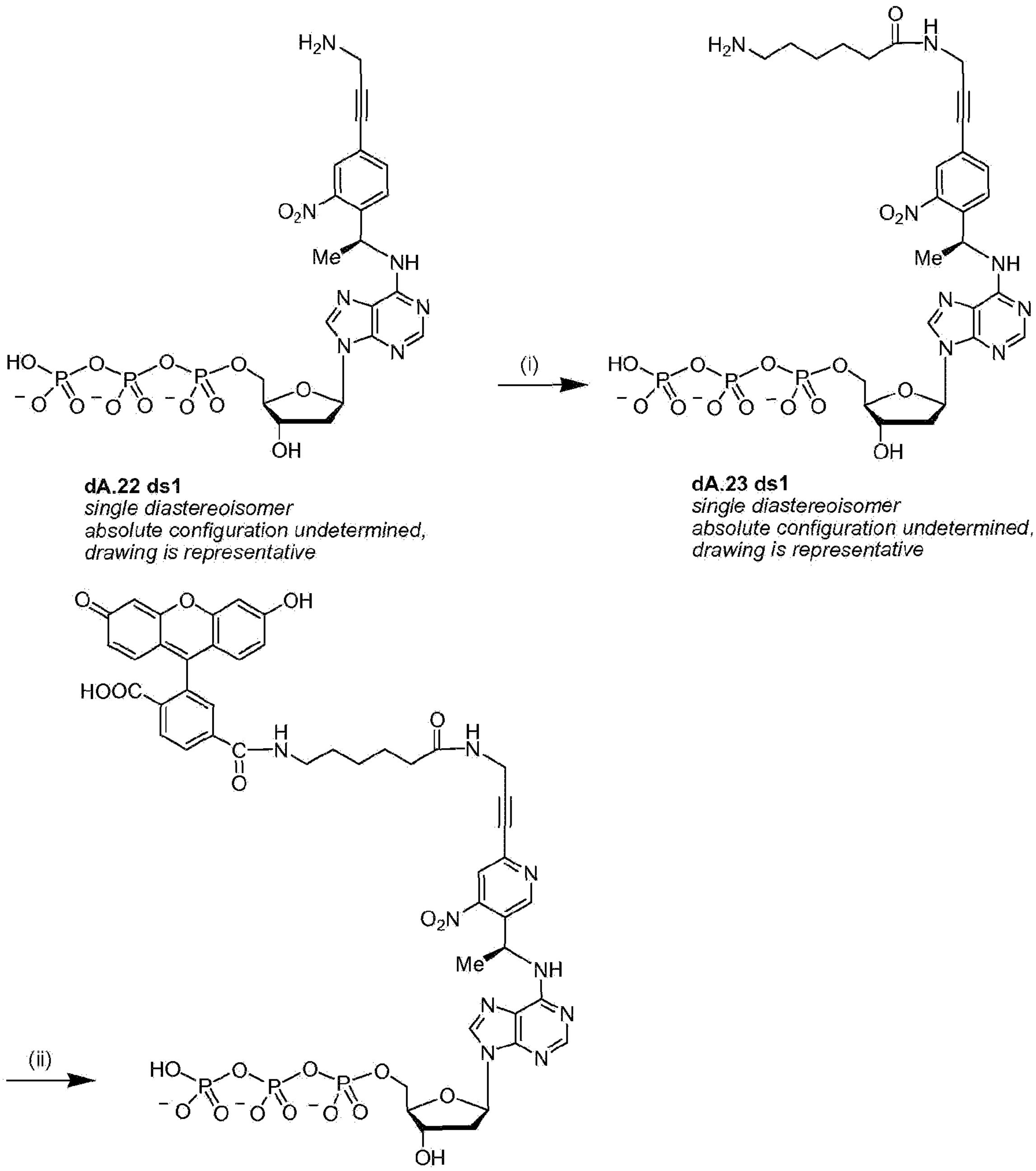


**dA.22 ds1**
*single diastereoisomer*
*absolute configuration undetermined,*
*drawing is representative*

**dA.23 ds1**
*single diastereoisomer*
*absolute configuration undetermined,*
*drawing is representative*

**WW3p032**
*single diastereoisomer absolute configuration undetermined, drawing is representative*

**Scheme.** Synthesis of *6-FAM labeled single diastereoisomer $N^6$-{(R or S)-1-{4-[3-(6-aminocaproyl)amino-1-propynyl]-2-nitrophenyl}ethyl}-2'-deoxyadenosine triphosphate.* (i) 6-*N*-(trifluoroacetyl)aminocaproic acid *N*-succinimidyl ester, 0.1 M $NaHCO_3/Na_2CO_3$, pH 9.2, one hour; NH4OH, one hour; (ii) 6-FAM-SE, 0.1 M $NaHCO_3/Na_2CO_3$ pH 9.2, one hour.

*$N^6$-{(R or S)-1-{4-[3-(6-Aminocaproyl)amino-1-propynyl]-2-nitrophenyl}ethyl}-2'-deoxyadenosine-5'-triphosphate (single diastereoisomer* ***dA.23 ds1****)*

A solution of 6-*N*-(trifluoroacetyl)aminocaproic acid *N*-succinimidyl ester (0.5 mg, 1.54 μmol) in anhydrous DMSO (10 μL) was added to a solution of triphosphate **dA.22 ds1** (0.25 μmol, single diastereoisomer, absolute config-

uration not determined) in $Na_2CO_3$/$NaHCO_3$ buffer (0.1 M, pH 9.2; 200 μL) and incubated at room temperature for one hour. $NH_4OH$ (500 uL, 25% aq) was added and the mixture was incubated at room temperature for another hour. The reaction was purified by reverse-phase HPLC using a Perkin Elmer OD-300 $C_{18}$ column (4.6 × 250 mm) to yield triphosphate **dA.23 ds1** (single diastereoisomer, absolute configuration not determined). Mobile phase: A, 100 mM triethylammonium acetate (TEAA) in water (pH 7.0); B, 100 mM TEAA in water/$CH_3CN$ (30:70). HPLC purification was achieved using a linear gradient of 5-50% B for 20 minutes and then 50-90% B for 10 minutes.

*Synthesis of 6-FAM labeled single diastereoisomer $N^6$-{(R or S)-1-{4-[3-(6-aminocapryoyl)amino-1 propynyl]-2-nitrophenyl}ethyl}-2'-deoxyadenosine-5'- triphosphate* ***(WW3p032)***

A solution of 6-FAM-SE (0.5 mg, 1.05 μmol) in anhydrous DMSO (10 μL) was added to a solution of triphosphate **dA.23 ds1** (0.196 μmol, single diastereoisomer, absolute configuration not determined) in $Na_2CO_3$/$NaHCO_3$ buffer (0.1 M, pH 9.2; 200 μL) and incubated at room temperature for one hour. The reaction was purified by reverse-phase HPLC using a Perkin Elmer OD-300 $C_{18}$ column (4.6 × 250 mm) to yield the 6-FAM labeled single diastereoisomer triphosphate **WW3p032.** Mobile phase: A, 100 mM triethylammonium acetate (TEAA) in water (pH 7.0); B, 100 mM TEAA in water/$CH_3CN$ (30:70). HPLC purification was achieved using a linear gradient of 5-50% B for 40 minutes and then 50-90% B for 10 minutes. The concentration of **WW3p032** was estimated by adsorption spectroscopy using the extinction coefficient of the 6-FAM dye *(i.e.,* 68,000 at 494 nm).

Example 2: dT compounds

**SYNTHESIS OF *$N^3$*-(2-NITOBENZYL)-THYMIDINE-5'-TRIPHOSPHATE (WW1P050)**

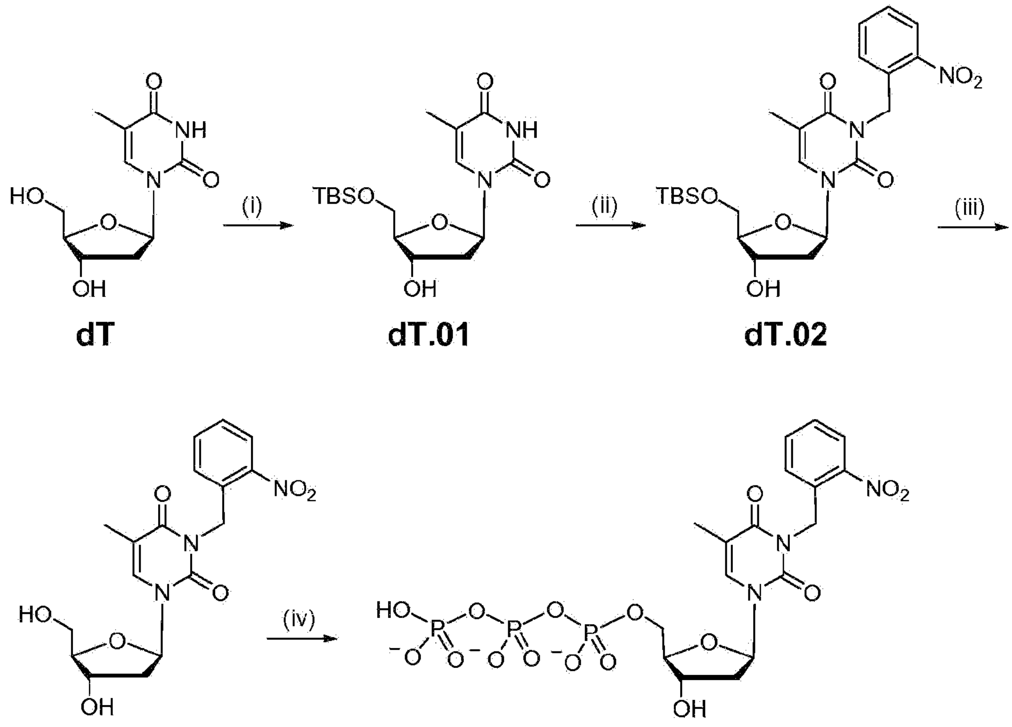


dT dT.01 dT.02

dT.03 WW1p050

**Scheme.** Synthesis of *$N^3$-(2-nitrobenzyl)-thymidine-5'-triphosphate*. (i) TBSCl, imidazole, anhydrous $CH_2Cl_2$, room temperature, overnight, 58%; (ii) 2-nitrobenzyl bromide, *n*-$Bu_4NOH$, NaI, NaOH (1 M), $CHCl_3$, room temperature, overnight, 37%; (iii) *n*-$Bu_4NF$, THF, 25°C, 45 minutes, 80%; (iv) $POCl_3$, proton sponge, $(MeO)_3PO$, 0°C, six hours; $(n\text{-}Bu_3NH)_2H_2P_2O_7$, *n*-$Bu_3N$, DMF, five minutes; 1 M $HNEt_3HCO_3$, one hour, 56%.

*5'-O-tert-butyldimethylsilyl-thymidine* ***(dT.01)***

A solution of thymidine **dT** (2.85 g, 11.76 mmol), imidiazole (2.44 g, 35.80 mmol) and TBSC1 (1.77 g, 11.76 mmol) in anhydrous $CH_2Cl_2$ (20 mL) was stirred at room temperature overnight under a $N_2$ atmosphere. The reaction mixture was then concentrated *in vacuo* to a viscous oil, followed by the addition of ethyl ether (60 mL) and water (60 mL). The organic layer was separated and washed twice with water (20 mL each), and the combined aqueous layer was extracted with ethyl ether (20 mL). The combined organic layer was dried over $Na_2SO_4$, concentrated *in vacuo* and purified by silica gel column chromatography to give *5'-O-tert*-butyldimethylsilyl-thymidine **dT.01** (3.44 g, 82%) as a white solid.

*$^1H$ NMR (400 MHz, $CDCl_3$):* δ 9.35 (br s, 1 H, H-3), 7.53 (d, 1 H, *J* = 1.2 Hz, H-6), 6.39 (dd, 1 H, *J* = 8.3, 5.6 Hz, H-1'), 4.45 (m, 1 H, H-4'), 4.07 (m, 1 H, H-3'), 3.87 (m, 2 H, H-5'a and H-5'b), 2.99 (br. s, 1 H, 3'-OH), 2.38 (m, 1 H, H-2'b), 2.09 (m, 1 H, H-2'b), 1.91 (d, 3 H, *J* = 1.2 Hz, 5-Me), 0.92 (s, 9 H, $(CH_3)_3CSi$), 0.11 (s, 6 H, $(CH_3)_2Si$).

*$N^3$-(2-Nitrohenzyl)-5'-O-tert-butyldimethylsilyl-thymidine* ***(dT.02)***

To a vigorously stirred mixture of compound **dT.01** (660 mg, 1.85 mmol), tetrabutylammonium hydroxide (0.5 mL), sodium iodide (55 mg) in $CHCl_3$ (5 mL), and NaOH (1 M; 5 mL), a solution of 2-nitrobenzyl bromide (400 mg, 1.85 mmol) in $CHCl_3$ (5 mL) was added dropwise and stirred at room temperature overnight. The organic layer was separated, and the aqueous layer was extracted twice with chloroform (5 mL each). The combined organic layer was washed with water (5 mL), brine (5 mL), and dried over $Na_2SO_4$. The solvent was evaporated *in vacuo,* and the residue was purified by silica gel column chromatography to yield *$N^3$*-(2-nitrobenzyl)-5'-*O-tert*-butyldimethylsilyl-thymidine dT.02 (562 mg, 58%) as a white foam.

*$^1H$ NMR ($CDCl_3$):* δ 7.98 (dd, 1 H, *J* = 7.2, 1.2 Hz, Ph-H), 7.60 (d, 1 H, *J* = 1.2 Hz, H-6), 7.49 (dt, 1 H, *J* = 7.6,

1.2 Hz, Ph-H), 7.36 (dt, 1 H, *J* = 8.1, 1.4 Hz, Ph-H), 7.16 (dd, 1 H, *J* = 7.8, 1.1 Hz, Ph-H), 6.31 (dd, 1 H, *J* = 8.2, 5.7 Hz, H-1'), 5.50 (d, 1 H, *J* = 16.2 Hz, $PhCH_2$), 5.44 (d, 1 H, *J* = 16.2 Hz, $PhCH_2$), 4.40 (m, 1 H, H-4'), 3.97 (q, 1 H, *J* = 2.4 Hz, H-3'), 3.82 (dq, 2 H, *J* = 11.4, 2.4 Hz, H-5'a and H-5'b), 2.98 (s, 1 H, 3'-OH), 2.29 (m, 1 H, H-2'a), 2.05 (m, 1 H, H-2'b), 1.93 (d, 3 H, *J* = 1.2 Hz, 5-Me), 0.90 (s, 9 H, $(CH_3)_3CSi$), 0.09 (s, 3 H, $(CH_3)Si$), 0.08 (s, 3 H, $(CH_3)Si$).

*$N^3$-(2-Nitrobenzyl)-thymidine* ***(dT.03)***

A solution of *n*-$Bu_4NF$ (1.0 M in THF, 1.125 mL), 1.125 mmol) was added dropwise to a solution of compound **dT.02** (369 mg, 0.75 mmol) in THF (3.75 mL). The reaction mixture was stirred at room temperature for 45 minutes, concentrated *in vacuo*, and purified by silica gel column chromatography to yield $N^3$-(2-nitrobenzyl)-thymidine **dT.03** (225 mg, 80%) as a white foam.

*$^1H$ NMR ($CDCl_3$):* δ 8.01 (dd, 1 H, *J* = 8.2, 1.3 Hz, Ph-H), 7.51 (m, 2 H, H-6 and Ph-H), 7.40 (m, 1 H, Ph-H), 7.21 (dd, 1 H, *J* = 7.8, 0.9 Hz, Ph-H), 6.21 (t, 1 H, *J* = 6.7 Hz, H-1'), 5.49 (dd, 2 H, $PhCH_2$), 4.53 (m, 1 H, H-4'), 3.97 (m, 1 H, H-3'), 3.78 (m, 2 H, H-5'a and H-5'b), 2.30 (m, 2 H, H-2'a and H-2'b), 1.94 (s, 3 H, 5-$CH_3$).

*$N^3$-(2-Nitrobenzyl)-thymidine-5'-triphosphate* ***(WW1p050)***

$POCl_3$ (30 μL, 0.33 mmol) was added to a solution of compound **dT.03** (38 mg, 0.11 mmol) and proton sponge (32 mg, 0.15 mmol) in trimethylphosphate (0.5 mL) at 0°C and stirred for six hours. A solution of bis-tri-*n*-butylammonium pyrophosphate (237 mg, 0.5 mmol) and tri-n-butylamine (100 μL) in anhydrous DMF (1 mL) was added. After five minutes of stirring, triethylammonium bicarbonate buffer (1 M, pH 7.5; 10 mL) was added to the solution. The reaction was stirred at room temperature for one hour and then lyophilized to dryness. The residue obtained was dissolved in water (10 mL), filtered, and purified by anion exchange chromatography using a Q Sepharose FF column (2.5 × 20 cm) with a linear gradient of $NH_4HCO_3$ (50 mM to 500 mM in 240 minutes) at a flow rate of 4.5 mL/min. The fractions containing triphosphate were combined and lyophilized to give triphosphate $N^3$-(2-nitrobenzyl)-thymidine-5'-triphosphate **WW1p050** (38 mg, 56%) as a white fluffy solid.

*$^1H$ NMR (400 MHz, $D_2O$):* 5 8.15 (d, 1 H, *J* = 8.2 Hz, Ph-H), 7.82 (s, 1 H, H-6), 7.64 (t, 1 H, *J* = 7.6 Hz, Ph-H), 7.54 (t, 1 H, *J* = 7.6 Hz, Ph-H), 7.24 (d, 1 H, *J* = 7.8 Hz, Ph-H), 6.35 (t, 1 H, *J* = 6.7 Hz, H-1'), 5.47 (s, 2 H, Ph-$CH_2$), 4.64 (m, 1 H, H-4'), 4.25 (m, 3 H, H-3', H-5'a and H-5'b), 2.40 (m, 2 H, H-2'a and H-2'b), 1.98 (s, 3 H, 5-$CH_3$);

*$^{31}P$ NMR (162 MHz, $D_2O$):* δ -6.12 (d, *J* = 15.6 Hz), -11.21 (d, *J* = 15.4 Hz), -19.565 (d, *J* = 15.6 Hz);

*ToF-MS (ESI):* For the molecular ion $C_{17}H_{20}N_3O_{16}P_3Na$ $[M-2H+Na]^-$, the calculated mass was 637.9954, and the observed mass was 637.9802.

**Synthesis of 5-(2-nitrobenzyloxymethyl)-2'-deoxyuridine-5'-triphosphate (VL3p03085)**

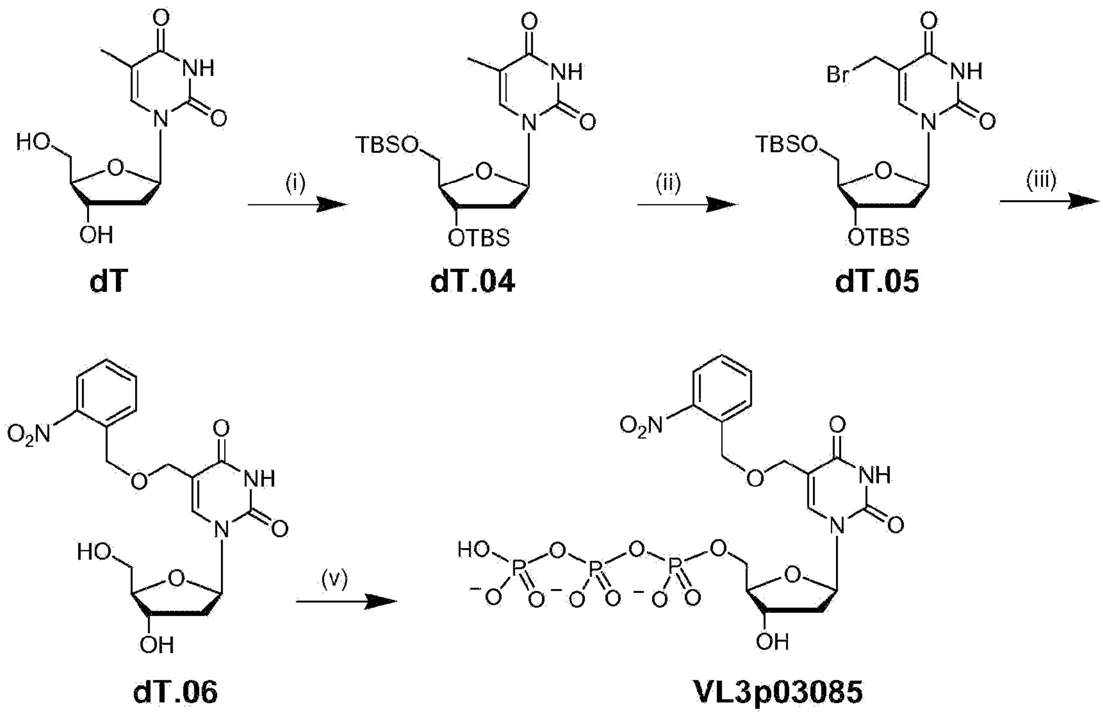


dT dT.04 dT.05

dT.06 VL3p03085

**Scheme.** *Synthesis of 5-(2-nitrobenzyloxymethyl)-2'-deoxyuridine-5'-triphosphate.* (i) TBSCl, imidazole, anhydrous DMF, room temperature, overnight, 90%; (ii) NBS, benzoyl peroxide, $CCl_4$, reflux, one hour, 44%; (iii) 2-nitrobenzyl alcohol, 110-115°C, 10 minutes, 35%; (iv) $POCl_3$, proton sponge, $(MeO)_3PO$, 0°C, two hours; $(n\text{-}Bu_3NH)_2H_2P_2O_7$, *n*-$Bu_3N$, DMF; 1 M $HNEt_3HCO_3$, one hour, 22%.

*3',5'-O-Bis-tert-butyldimethylsilyl-thymidine* ***(dT.04)***

To a solution of thymidine (5.00 g, 20.64 mmol) and imidiazole (9.0 g, 132.1 mmol) in anhydrous DMF (11 mL), a solution of TBSC1 (9.96 g, 66.05 mmol) in DMF (11 mL) was added dropwise, and the mixture was stirred at room temperature overnight under a $N_2$ atmosphere. After the mixture was diluted with water (100 mL), the formed precipitate was filtered and dissolved in ethyl ether (125 mL). The ether solution was washed twice with water (25 mL each) and once with brine (25 mL), dried over $Na_2SO_4$, and concentrated under reduced pressure to a waxy solid, which was re-crystallized from hexane/ethyl either (10:1) to yield 3',5'-O-bis-*tert*-butyldimethylsilyl-thymidine **dT.04** (10.64 g, 90%).

*$^1H$ NMR (400 MHz, $CDCl_3$):* δ 8.51 (br s, 1 H, H-3), 7.48 (d, 1 H, *J* = 1.2 Hz, H-6), 6.34 (dd, 1 H, *J* = 5.8 and 8.0 Hz, H-1'), 4.41 (m, 1 H, H-3'), 3.93 (m, 2 H, H-4'), 3.87 (dd, 1 H, *J* = 2.6 and 11.4 Hz, H-5'a), 3.76 (dd, 1 H, *J* = 2.6 and 11.4 Hz, H-5'b), 2.17 (m, 1 H, H-2'a), 2.01 (m, 1 H, H-2'b), 1.92 (d, 3 H, *J* = 1.2 Hz, $CH_3$), 0.93 (s, 9 H, $(CH_3)_3CSi$), 0.88 (s, 9 H, $(CH_3)_3CSi$), 0.11 (s, 6 H, $(CH_3)_2Si$), 0.08 (s, 6 H, $(CH_3)_2Si$).

*5-Bromomethyl-3',5'-bis-O-tert-butyldimethylsilyl-2'-deoxyuridine* ***(dT.05)***

A solution of compound **dT.04** (4.63 g, 9.83 mmol), *N*-bromosuccinimide (3.68 g, 20.68 mmol), and benzoyl peroxide (0.10 g, 75% aqueous solution) in $CCl_4$ (100 mL) was refluxed for one hour. The mixture was filtered and the filtrate was concentrated *in vacuo* and purified by silica gel column chromatography to yield 5-bromomethyl-3',5'-*O*-bis-*tert*-butyldimethylsilyl-2'-deoxyuridine **dT.05** (2.40 g, 44%).

*$^1H$ NMR (400 MHz, $CDCl_3$):* δ 9.16 (br s, 1 H, H-3), 7.89 (s, 1 H, H-6), 6.30 (dd, 1 H, *J* = 5.8 and 7.7 Hz, H-1'), 4.41 (m, 1 H, H-3'), 4.29 (d, 1 H, *J* = 10.6 Hz, $CH_2Br$), 4.23 (d, 1 H, *J* = 10.6 Hz, $CH_2Br$), 3.98 (m, 2 H, H-4'), 3.89 (dd, 1 H, *J* = 2.6 and 11.4 Hz, H-5'b), 3.78 (dd, 1 H, *J* = 2.6 and 11.4, Hz, H-5'a), 2.30 (m, 1 H, H-2'a), 2.01 (m, 1 H, H-2'b), 0.95 (s, 9 H, $(CH_3)_3CSi$), 0.91 (s, 9 H, $(CH_3)_3CSi$), 0.15 (s, 6 H, $(CH_3)_2Si$), 0.09 (s, 6 H, $(CH_3)_2Si$).

*5-(2-Nitrobenzyloxymethyl)-2'-deoxyuridine **(dT.**06)*

Compound **dT.05** (238 mg, 0.43 mmol) and 2-nitrobenzyl alcohol (331 mg, 2.17 mmol) was heated neat at 110-115°C for 10 minutes under a $N_2$ atmosphere. The mixture was cooled down to room temperature, dissolved in ethyl acetate, and purified by silica gel chromatography to yield 5-(2-nitrobenzyloxymethyl)-2'-deoxyuridine **dT.06** (60 mg, 35%).

*$^1H$ NMR (400 MHz, DMSO-$d_6$):* δ 11.41 (br s, 1 H, $D_2O$ exchangeable, NH), 8.05 (d, *J* = 8.0 Hz, 1 H, Ph-H), 7.96 (s, 1 H, H-6), 7.78 (m, 2 H, Ph-H), 7.56 (m, 1 H, Ph-H), 6.17 (t, 1 H, *J* = 6.8 Hz, H-1'), 5.25 (d, 1 H, $D_2O$ exchangeable, 3'-OH), 5.00 (t, 1 H, *J* = 5.0 Hz, $D_2O$ exchangeable, 5'-OH), 4.48 (s, 2 H, $CH_2$), 4.24 (m, 1 H, H-3'), 4.23 (s, 2 H, $CH_2$), 3.79 (m, 1 H, H-4'), 3.57 (m, 2 H, H-5'), 2.11 (m, 2 H, H-2');

*$^{13}C$ NMR (100 MHz, MeOH-$d_4$):* δ 163.38 (C), 147.18 (C), 139.53 (CH), 134.80 (C), 133.81 (C), 132.87 (CH), 128.53 (CH), 127.63 (CH), 123.75 (CH), 110.24 (C), 87.16 (CH), 82.83 (CH), 70.41 (CH), 68.26 ($CH_2$), 64.73 ($CH_2$), 61.05 ($CH_2$), 39.58 ($CH_2$);

*ToF-MS (ESI):* For the molecular ion $C_{17}H_{20}N_3O_8$ $[M+H]^-$, the calculated mass was 394.1250, and the observed mass was 394.1286.

*5-(2-Nitrobenyloxymethyl)-2'-deoxyuridine-5'-triphosphate **(VL3p03085)***

$POCl_3$ (22 μL, 0.24 mmol) was added to a solution of compound **dT.06** (48 mg, 0.12 mmol) and proton sponge (39 mg, 0.18 mmol) in trimethylphosphate (0.5 mL) at 0°C and stirred for two hours. A solution of bis-tri-*n*-butylammonium pyrophosphate (285 mg, 0.6 mmol) and tri-*n*-butylamine (120 μL) in anhydrous DMF (1.2 mL) was added. After two minutes of stirring, triethylammonium bicarbonate buffer (1 M, pH 7.5; 10 mL) was added. The reaction was stirred at room temperature for one hour and then lyophilized to dryness. The residue was dissolved in water (10 mL), filtered, and purified by anion exchange chromatography using a Q Sepharose FF column (2.5 × 20 cm) with a linear gradient of $NH_4HCO_3$ (50 mM to 500 mM in 300 minutes) at a flow rate of 4.5 mL/min. The fractions containing triphosphate were combined and lyophilized to give 5-(2-nitrobenzyloxymethyl)-2'-deoxyuridine-5'-triphosphate **VL3p03085** (16 mg, 22%) as a white fluffy solid.

*$^1H$ NMR (400 MHz, $D_2O$):* δ 8.01 (d, *J* = 8.0 Hz, 1 H, Ph-H), 7.78 (s, 1 H, H-6), 7.65 (m, 2 H, Ph-H), 7.52 (m, 1 H, Ph-H), 6.26 (t, *J* = 6.8 Hz, 1 H, H-1'), 4.93 (m, 2 H, $CH_2$), 4.57 (m, 1 H, H-3'), 4.41 (s, 2 H, $CH_2$), 4.21 (m, 3 H, H-4' and H-5'), 2.34 (m, 2 H, H-2');

*$^{31}P$ NMR (162 Hz, $D_2O$):* δ -5.58 (d, *J* = 18.5 Hz), -10.91 (d, *J* = 18.5 Hz), -20.80 (br);

*TOF-MS (ESI):* For the molecular ion $C_{17}H_{21}N_3O_{17}P_3$ $[M-H]^-$, the calculated mass was 632.0084, and the observed mass was 631.9779.

Synthesis of **5-[1-(2-nitrophenyl)ethyloxymethyl]-2'-deoxyuridine-5'-triphosphate (WW2p043)**

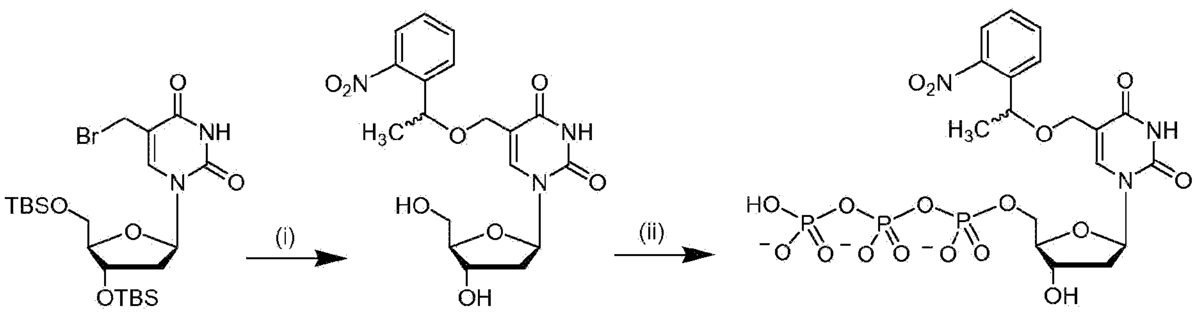


**dT.05** **dT.07** **WW2p043**

**Scheme.** Synthesis of *5-[1-(2-nitrophenyl)ethyloxymethyl]-2'-deoxyuridine-5'-triphosphate.* (i) 1-(2-nitrophenyl)ethanol (1.25 g, 7.50 mmol), 110-115°C, 10 minutes, 8%; (ii) $POCl_3$, proton sponge, $(MeO)_3PO$, 0°C, three hours; $(n\text{-}Bu_3NH)_2H_2P_2O_7$, $n\text{-}Bu_3N$, DMF; 1 M $HNEt_3HCO_3$, one hour, 55%.

*5-[1-(2-Nitrophenyl)ethyloxymethyl]-2'-deoxyuridine* ***(dT.07)***

Compound **dT.05** (0.81 g, 1.5 mmol) and 1-(2-nitrophenyl)ethanol (1.25 g, 7.50 mmol) were heated neat at 110-115°C for 10 minutes under a $N_2$ atmosphere. The mixture was cooled down to room temperature, dissolved in ethyl acetate, and purified by silica gel chromatography to yield 5-[1-(2-nitrophenyl)ethyloxymethyl]-2'-deoxyuridine **dT.07** (48 mg, 8%, 1:1 mixture of diastereomers).

*$^1H$ NMR (400 MHz, DMSO-$d_6$) for diastereomers:* $\delta$ 11.36 and 11.35 (2 br s, 1 H, $D_2O$ exchangeable, NH), 7.95 (m, 1 H, Ph-H), 7.87 (m, 1 H, Ph-H), 7.76 (m, 2 H, H-6 and Ph-H), 7.54 (m, 1 H, Ph-H), 6.14 (m, 1 H, H-1'), 5.25 (d, 1 H, $D_2O$ exchangeable, 3'-OH), 5.00 (m, 2 H, among them 1 H $D_2O$ exchangeable, 5'-OH and CH), 4.24 (m, 1 H, H-3'), 3.96 (m, 2 H, $CH_2$), 3.78 (m, 1 H, H-4'), 3.57 (m, 2 H, H-5'), 2.08 (m, 2 H, H-2'), 1.43 (d, *J* =6.3 Hz, 3 H, $CH_3$);

*ToF-MS (ESI):* For the molecular ion $C_{18}H_{22}N_3O_9$ $[M+H]^+$, the calculated mass was 408.1407, and the observed mass was 408.1446.

*5-[1-(2-Nitrophenyl)ethytoxymethyl]-2'-deoxyuridine-5'-triphosphate* ***(WW2p043)***

$POCl_3$ (15 $\mu$L, 0.17 mmol) was added to a solution of compound **dT.07** (34 mg, 0.08 mmol) and proton sponge (27 mg, 0.12 mmol) in trimethylphosphate (0.5 mL) at 0°C and stirred for three hours. A solution of tri-*n*-butylammonium pyrophosphate (197 mg, 0.4 mmol) and tri-*n*-butylamine (100 $\mu$L) in anhydrous DMF (0.8 mL) was added. After five minutes of stirring, triethylammonium bicarbonate buffer (1 M, pH 7.5, 10 mL) was added. The reaction was stirred at room temperature for one hour and then lyophilized to dryness. The residue was dissolved in water (10 mL), filtered, and purified by anion exchange chromatography using a Q Sepharose FF column (2.5 $\times$ 20 cm) with a linear gradient of $NH_4HCO_3$ (50 mM to 500 mM in 300 minutes) at a flow rate of 4.5 mL/min. The fractions containing triphosphate were combined and lyophilized to give 5-[1-(2-nitrophenyl)ethyloxymethyl]-2'-deoxyuridine-5'-triphosphate **WW2p043** (32 mg, 55%, 1:1 mixture of diastereomers) as a white fluffy solid.

*$^1H$ NMR (400 MHz, $D_2O$) for diastereomers:* $\delta$ 7.93 (m, 1 H, Ph-H), 7.71 - 7.61 (m, 3 H, H-6 and Ph-H), 7.49 (m, 1 H, Ph-H), 6.18 and 6.12 (2 t, *J* = 6.6 Hz, 1 H, H-1'), 5.13 (m, 1 H, CH), 4.53 (m, 1 H, H-3'), 4.39 (m, 1 H, H-4'), 4.20 (m, 4 H, $CH_2$ and H-5'), 2.28 (m, 2 H, H-2'), 1.54 (d, 3 H, *J* = 6.3 Hz, $CH_3$);

*$^{31}P$ NMR (162 MHz, $D_2O$):* 5 -7.98 (br), -12.64 (br), -23.33 (br);

*ToF-MS (ESI):* For the molecular ion $C_{18}H_{24}N_3O_{17}P_3Na$ $[M+Na]^+$, the calculated mass was 670.0216, and the observed mass was 670.0176.

**Synthesis of 6-JOE labeled 5-[4-(3-amino-1-propynyl)-2-nitrobenzyloxymethyl]-2'-deoxyuridine-5'-triphosphate (WW2p075)**

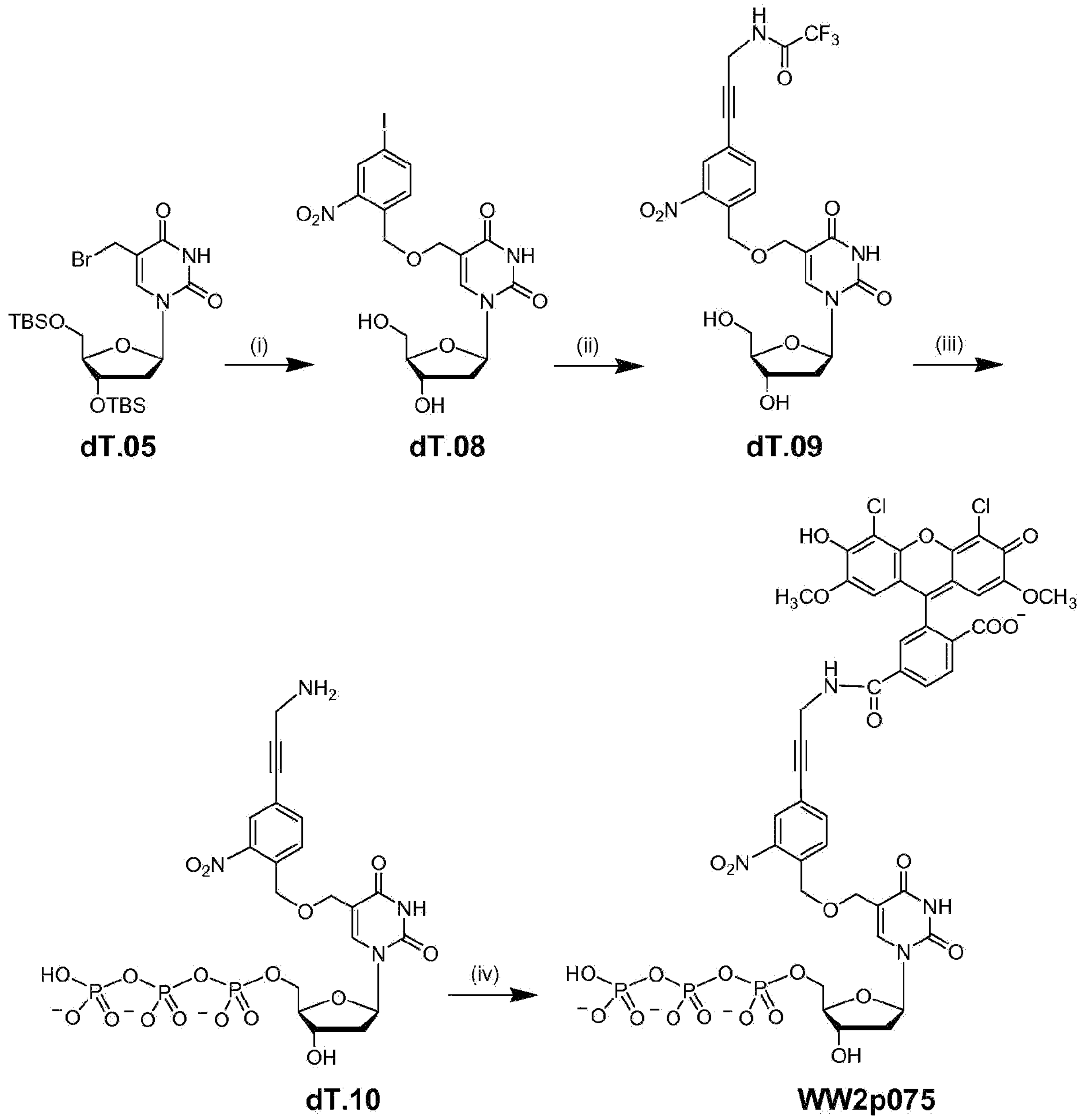


dT.05 dT.08 dT.09

dT.10 WW2p075

**Scheme.** Synthesis of *6-JOE labeled 5-[4-(3-amino-1-propynyl)-2-nitrobenzyloxymethyl]-2'-deoxyuridine-5'-triphosphate.* (i) 4-iodo-2-nitrobenzyl alcohol, neat, 110-115°C, 10 minutes, 10%; (ii) *N*-propargyltrifluoroacetamide, $Pd(PPh_3)_4$ (0), CuI, $Et_3N$, anhydrous DMF, four hours, 50%; (iii) $POCl_3$, proton sponge, $(MeO)_3PO$, 0°C, four hours; $(n\text{-}Bu_3NH)_2H_2P_2O_7$, $n\text{-}Bu_3N$, DMF, five minutes; 1 M $HNEt_3HCO_3$, one hour; $NH_4OH$, one hour; 31%; (iv) 6-JOE-SE, 0.1 M $Na_2CO_3/NaHCO_3$ buffer (pH 9.2), one hour.

*5-(4-Iodo-2-nitrobenzyloxymethyl)-2'-deoxyuridine* ***(dT.08)***

Compound **dT.05** (0.59 g, 1.06 mmol) and 4-iodo-2-nitrobenzyl alcohol (1.09 g, 3.9 mmol) were heated neat at 110-115°C for 10 minutes under a $N_2$ atmosphere. The mixture was cooled down to room temperature, dissolved in ethyl acetate, and purified by silica gel chromatography to yield 5-(4-iodo-2-nitrobenzyloxymethyl)-2'-deoxyuridine **dT.08** (52 mg, 10%) as a low-melting solid.

*$^1H$ NMR (400 MHz, DMSO-$d_6$):* δ 11.42 (s, 1 H, $D_2O$ exchangeable, N-H), 8.34 (d, 1 H, *J* = 1.7 Hz, Ph-H), 8.09 (dd, 1 H, *J* = 1.7 and 8.2 Hz, Ph-H), 7.96 (s, 1 H, H-6), 7.56 (d, 1 H, *J* = 8.2, Ph-H), 6.16 (t, 1 H, *J* = 6.8 Hz, H-1'), 5.25 (d, 1 H, $D_2O$ exchangeable, 3'-OH), 5.02 (t, 1 H, $D_2O$ exchangeable, 5'-OH), 4.77 (s, 2 H, $CH_2$), 4.20 (m, 1 H, H-3'), 4.22 (s, 2 H, $CH_2$), 3.79 (m, 1 H, H-4'), 3.57 (m, 2 H, H-5'), 2.11 (m, 2 H, H-2').

*5-[4-(3-Trifluoroacetamido-1-propynyl)-2-nitrobenzyloxymethyl]-2'-deoxyuridine **(dT.09)***

A solution of compound **dT.08** (51 mg, 0.1 mmol), *N*-propargyltrifluoroacetylamide (45 mg, 0.3 mmol), tetrakis(triphenylphosphine)-palladium(0) (12 mg, 0.01 mmol), CuI (4 mg, 0.02 mmol), and $Et_3N$ (28 μL, 0.2 mmol) in anhydrous DMF (1.2 mL) was stirred at room temperature for four hours. The mixture was concentrated *in vacuo* and purified by silica gel column chromatography to yield 5-[4-(3-trifluoroacetamido-1-propynyl)-2-nitrobenzyloxymethyl]-2'-deoxyuridine **dT.09** (27 mg, 50%) as a waxy solid.

*$^1H$ NMR (400 MHz, DMSO-$d_6$):* δ 11.44 (s, 1 H, $D_2O$ exchangeable, N3-H), 10.44 (1 H, $D_2O$ exchangeable, N-H ($COCF_3$)), 8.06 (s, 1 H, Ph-H), 7.97 (s, 1 H, H-6), 7.79 (s, 2 H, Ph-H), 6.16 (t, *J* = 6.8 Hz, 1 H, H-1'), 5.25 (d, 1 H, $D_2O$ exchangeable, 3'-OH), 5.02 (t, 1 H, $D_2O$ exchangeable, 5'-OH), 4.83 (s, 2 H, Ph-$CH_2$), 4.30 (s, 2 H, $CH_2$), 4.23 (m, 3 H, $CH_2$ and H-3'), 3.79 (m, 1 H, H-4'a), 3.57 (m, 2 H, H-5'), 2.10 (m, 2 H, H-2'a and H-2'b);

*ES+ MS (ESI):* 543 $[M+H]^+$; *ES- MS (ESI):* 541 $[M+H]^+$

*5-[4-(3-Amino-1-propynyl)-2-nitrobenzyloxymethyl]-2'-deoxyuridine-5'-triphosphate **(dT.10)***

$POCl_3$ (8 μL, 88 μmol) was added to a solution of compound **dT.09** (24 mg, 44 μmol) and proton sponge (14 mg, 66 μmol) in trimethylphosphate (0.5 mL) at 0°C and stirred for two hours. Additional $POCl_3$ (8 μL, 88 μmol) was added and stirred for another two hours. A solution of bis-tri-*n*-butylammonium pyrophosphate (104 mg, 0.22 mmol) and tri-*n*-butylamine (50 μL) in anhydrous DMF (0.5 mL) was added. After five minutes of stirring, tricthylammonium bicarbonate buffer (1 M, pH 7.5; 10 mL) was added. The reaction was stirred at room temperature for one hour, followed by the dropwise addition of concentrated ammonium hydroxide (5 mL, 27%) at 0°C. The mixture was stirred for an additional hour at room temperature and then lyophilized to dryness. The residue was dissolved in water (10 mL), filtered, and purified by anion exchange chromatography using a Q Sepharose FF column (2.5 × 20 cm) with a linear gradient of $NH_4HCO_3$ (50 mM to 500 mM in 300 minutes) at a flow rate of 4.5 mL/min. The fractions containing triphosphate were combined and lyophilized to give triphosphate **dT.10** (10 mg, 31%) as a white fluffy solid.

*$^1H$ NMR (400 MHz, $D_2O$) :* δ 8.10 (d, *J* = 5.1 Hz, 1 H, Ph-H), 7.75 (m, 2 H, H-6 and Ph-H), 7.65 (m, 1 H, *J* = 8.0 Hz, Ph-H), 6.27 (t, *J* = 6.8 Hz, 1 H, H-1'), 4.95 (m, 2 H, $CH_2$), 4.58 (m, 1 H, H-3'), 4.43 (s, 2 H, $CH_2$), 4.22 (m, 3 H, H-4' and H-5'), 3.64 (s, 2 H, $CH_2$), 2.33 (m, 2 H, H-2');

*$^{31}P$ NMR (162 Hz, $D_2O$):* δ -6.51 (d, *J* = 15.0 Hz), -11.56 (d, *J* = 15.6 Hz), -19.82 (t, *J* = 15.0 Hz);

*TOF-MS (ESI):* For the molecular ion $C_{20}H_{23}N_4O_{17}P_3Na$ $[M-2H+Na]^-$, the calculated mass was 707.0169, and the observed mass was 707.0321.

*6-JOE labeled 5-[4-(3-Amino-1-propynyl)-2-nitrobenzyloxymethyl]-2'-deoxyuridine-5'-triphosphates (WW2p075)*

A solution of 6-JOE-SE (1.25 mg, 2 μmol) in anhydrous DMSO (50 μL) was added to a solution of triphosphate **dT.10** (1.4 μmol) in $Na_2CO_3$/$NaHCO_3$ buffer (0.1 M, pH 9.2; 0.5 mL) and incubated at room temperature for one hour. The reaction was purified by reverse-phase HPLC using a Perkin Elmer OD-300 $C_{18}$ column (4.6 x 250 mm) to yield the 6-JOE labeled triphosphate **WW2p075.** Mobile phase: A, 100 mM triethylammonium acetate (TEAA) in water (pH 7.0); B, 100 mM TEAA in water/$CH_3CN$ (30:70). Elution was performed with a linear gradient of 5-38% B for 40 minutes and then 38-90% B for 10 minutes. The concentration of **WW2p075** was estimated by adsorption spectroscopy using the extinction coefficient of the 6-JOE dye *(i.e.,* 75,000 at 520 nm).

**Synthesis of 6-JOE labeled 5-{1-[4-(3-amino-1-propynyl)-2-nitrophenyl]ethyl-oxymethyl}-2'-deoxyuridine-5'-triphosphate (WW2p113)**

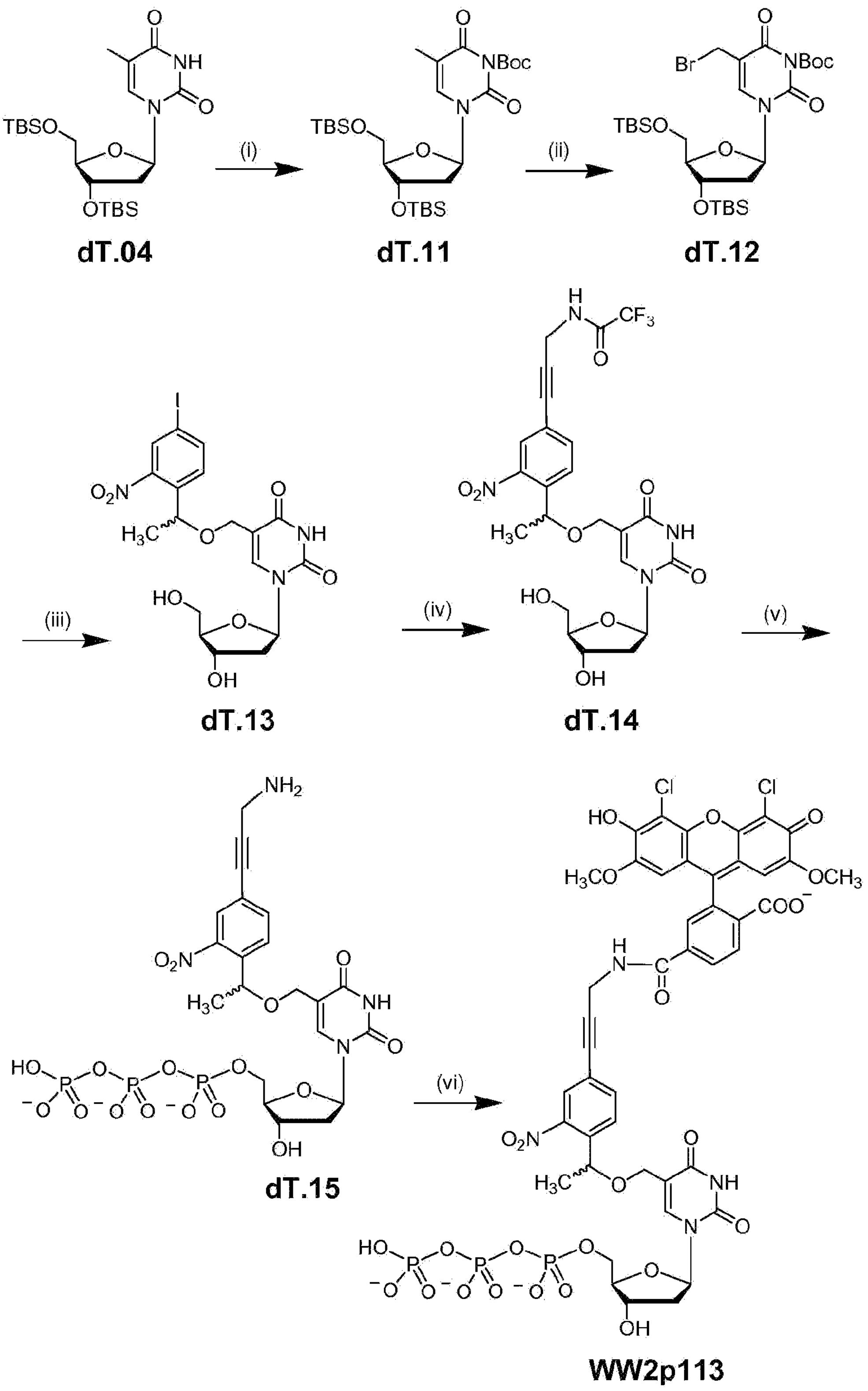


dT.04

dT.11

dT.12

dT.13

dT.14

dT.15

WW2p113

**Scheme**. Synthesis of *6-JOE labeled 5-{1-[4-(3-Amino-1-propynyl)-2-nitrophenyl]-ethyloxy-methyl}-2'-deoxyuridine-5'-triphosphate*. (i) $Boc_2O$, DMAP, anhydrous DMF, room temperature, 16 hours, 78%; (ii) NBS, benzoyl peroxide, $CCl_4$, reflux, one hour, 43%; (iii) 1-(4-iodo-2-nitrophenyl)ethanol, neat, 95°C, 50 minutes, 13%; (iv) *N*-propargyltrifluoroacetamide, $Pd(PPh_3)_4$ (0), CuI, $Et_3N$, anhydrous DMF, four hours, 76%; (v) $POCl_3$, proton sponge, $(MeO)_3PO$, 0°C; $(n\text{-}Bu_3NH)_2H_2P_2O_7$, *n*-$Bu_3N$, DMF; 1 M $HNEt_3HCO_3$, one hour; $NH_4OH$, one hour; 31%; (vi) 6-JOE-SE, 0.1 M $Na_2CO_3/NaHCO_3$ buffer (pH 9.2), one hour.

*$N^3$-tert-Butyloxycarbonyl-3',5'-O-bis-tert-butyldimethylsilyl-thymidine* ***(dT.11)***

To a solution of compound **dT.04** (2.43 g, 5.15 mmol) and DMAP (1.39 g, 11.34 mmol) in anhydrous DMF (45 mL), a solution of di-*tert*-butyldicarbonate (2.47 g, 11.34 mmol) in DMF (9 mL) was added dropwise. The mixture was stirred at room temperature for 16 hours under a $N_2$ atmosphere. The mixture was concentrated *in vacuo,* and the crystalline residue was dissolved in $CH_2Cl_2$ (80 mL), washed with saturated $NH_4Cl$ solution (10 mL), dried over $Na_2SO_4$, and *concentrated in vacuo.* The residue was purified by silica gel column chromatography to yield $N^3$-*tert*-butyloxycarbonyl-3',5'-*O*-bis-*tert*-butyldimethylsilyl-thymidine **dT.11** (2.30 g, 78%) as a white solid.

*$^1H$ NMR (400 MHz, $COCl_3$):* δ 7.50 (d, 1 H, *J* = 1.1 Hz, H-6), 6.34 (dd, 1 H, *J* = 5.8 and 7.9 Hz, H-1'), 4.42 (m, 1 H, H-3'), 3.95 (m, 2 H, H-4'), 3.87 (dd, 1 H, *J* = 2.5 and 11.4 Hz, H-5'a), 3.76 (dd, 1 H, *J* = 2.5 and 11.4 Hz, H-5'b), 2.17 (m, 1 H, H-2'a), 2.01 (m, 1 H, H-2'b), 1.92 (d, 3 H, *J* = 1.2 Hz, $CH_3$), 1.60 (s, 9 H, $(CH_3)_3COCON$), 0.93 (s, 9 H, $(CH_3)_3CSi$), 0.88 (s, 9 H, $(CH_3)_3CSi$), 0.11 (s, 6 H, $(CH_3)_2Si$), 0.08 (s, 6 H, $(CH_3)_2Si$).

*$N^3$-tert-Buyloxycarbonyl-5-bromomethyl-3',5'-bis-O-tert-butyldimethylsilyl-2'-deoxyuridine* ***(dT.12)***

A solution of compound **dT.11** (570 mg, 1.00 mmol), *N*-bromosuccinimide (0.37 g, 2.10 mmol), and benzoyl peroxide (10 mg, 75% aqueous solution) in $CCl_4$ (10 mL) was refluxed for one hour. The mixture was filtered, concentrated *in vacuo*, and purified by silica gel column chromatography to yield $N^3$-*tert*-butyloxycarbonyl-5-bromomethyl-3',5'-*O*-bis-*tert*-butyldimethylsilyl-2'-deoxyuridine **dT.12** (281 mg, 43%) as a waxy solid.

*$^1H$ NMR (400 MHz, $CDCl_3$):* δ 7.89 (s, 1 H, H-6), 6.27 (dd, 1 H, *J* = 5.7 and 7.7 Hz, H-1'), 4.39 (m, 1 H, H-3'), 4.27 (d, 1 H, *J* = 10.6 Hz, $CH_2Br$), 4.20 (d, 1 H, *J* = 10.6 Hz, $CH_2Br$), 3.98 (m, 2 H, H-4'), 3.89 (dd, 1 H, *J* = 2.5 and 11.4, Hz, H-5'b), 3.78 (dd, 1 H, *J* = 2.6 and 11.4 Hz, H-5'a), 2.30 (m, 1 H, H-2'a), 2.04 (m, 1 H, H-2'b), 1.61 (s, 9 H, $(CH_3)_3COCON$), 0.95 (s, 9 H, $(CH_3)_3CSi$), 0.89 (s, 9 H, $(CH_3)_3CSi$), 0.14 (s, 6 H, $(CH_3)_2Si$), 0.07 (s, 6 H, $(CH_3)_2Si$);

*$^{13}C$ NMR (100 MHz, $CDCl_3$):* δ 159.21 (C), 147.99 (C), 147.32 (C), 138.46 (CH), 111.30 (C), 88.34 (CH), 87.22 (C), 86.00 (CH), 72.28 (CH), 63.04 ($CH_2$), 41.93 ($CH_2$), 27.42 ($CH_3$), 25.99 ($CH_3$), 25.71 ($CH_3$), 25.65 ($CH_3$), 24.91 ($CH_2$), 18.47 (C), 17.97 (C), - 3.58 ($CH_3$), -4.65 ($CH_3$), -4.86 ($CH_3$), -5.32 ($CH_3$).

*5-[1-(4-Iodo-2-nitrophenyl)ethyloxymethyl]-2'-deoxyuridine* ***(dT.13)***

Compound **dT.12** (323 mg, 0.50 mmol) and 1-(4-iodo-2-nitrophenyl)ethanol (293 mg, 2.23 mmol) were heated neat at 95-97°C for 50 minutes under a $N_2$ atmosphere. The mixture was cooled down to room temperature, dissolved in ethyl acetate, and purified by silica gel chromatography to yield 5-[1-(4-iodo-2-nitrophenyl)ethyloxymethyl]-2'-deoxyuridine **dT.13** (34 mg, 13%, 1:1 mixture of diastereomers) as a waxy solid.

*$^1H$ NMR (400 MHz, MeOH-$d_4$) for diastereomers:* δ 8.26 (t, 1 H, *J* = 1.7 Hz, H-6), 8.05 (dd, 1 H, *J* = 1.6, 8.3 Hz, Ph-H), 8.02 (d, 1 H, *J* = 10.6 Hz, Ph-H), 7.60 (dd, 1 H, *J* = 1.1, 8.3 Hz, Ph-H), 6.26 (m, 1 H, H-1'), 5.03 (m, 1 H, PhC<u>H</u>), 4.41 (m, 1 H, H-3'), 4.10 (m, 2 H, $CH_2$), 3.94 (m, 1 H, H-4'), 3.80 (m, 1 H, H-5'a), 3.74 (m, 1 H, H-5'b), 2.30 (m, 1 H, H-2'a), 2.20 (m, 1 H, H-2'b), 1.50 (m, 3 H, $CH_3$).

*5-{1-[4-(3-Trifluoroacetamido-1-propynyl)2-nitrophenyl]ethyloxymethyl}-2'-deoxyuridine* ***(dT.14)***

A solution of compound **dT.13** (52 mg, 0.1 mmol), *N*-propargyltrifluoroacetylamide (44 mg, 0.29 mmol), tetrakis(triphenylphosphine)-palladium(O) (12 mg, 0.01 mmol), CuI (4 mg, 0.02 mmol), and $Et_3N$ (27 μL, 0.2 mmol) in anhydrous DMF (1.5 mL) was stirred at room temperature for four hours. The mixture was concentrated *in vacuo* and purified by silica gel column chromatography to yield 5-[1-(4-{3-trifluoroacetamido-1-propynyl}-2-nitrophenyl)ethyloxymethyl]-2'-deoxy-uridine **dT.14** (41 mg, 76%, 1:1 mixture of diastereomers) as a waxy solid.

*$^1H$ NMR (400 MHz, DMSO-$d_6$) for diastereomers:* δ 11.36, 11.35(2 s, 1 H, $D_2O$ exchangeable, NH), 10.11 (t, 1 H, *J* = 5.6 Hz, $D_2O$ exchangeable, NH), 7.98 (s, 1 H, H-6), 7.88 (d, 1 H, *J* = 8.1 Hz, Ph-H), 7.78 (m, 2 H, Ph-H), 6.14 (t, *J* = 7.0 Hz, 1 H, H-1'), 5.25 (m, 1 H, $D_2O$ exchangeable, 3'-OH), 5.01 (m, 1 H, $D_2O$ exchangeable, 5'-OH), 4.98 (m, 1 H, PhCH), 4.33 (m, 2 H, $CH_2$), 4.24 (m, 1 H, H-3'), 4.00 (m, 1 H, H-5'a), 3.94 (m, 1 H, H-5'b), 3.78 (m, 1 H, H-4'), 3.57 (m, 2 H, $CH_2$), 2.08 (m, 2 H, H-2'a and H-2'b), 1.41 (d, *J* = 8.1 Hz, 3 H, $CH_3$);

*$ES^+$* MS *(ESI):* 579 $[M+Na]^+$.

*5-{1-[4-(3-Amino-1-propynyl)-2-nitrophenyl]ethyloxymethyl}-2'-deoxyuridine-5'-triphosphate **(dT.15)***

$POCl_3$ (10 μL, 0.11 mmol) was added to a solution of compound **dT.14** (30 mg, 0.054 mmol) and proton sponge (17 mg, 0.08 mmol) in trimethylphosphate (0.5 mL) at 0°C and stirred for two hours. Additional $POCl_3$ (2.5 μL, 0.03 mmol) was added and stirred for another hour. A solution of bis-tri-*n*-butylammonium pyrophosphate (128 mg, 0.27 mmol) and tri-n-butylamine (60 μL) in anhydrous DMF (0.54 mL) was added. After five minutes of stirring, triethylammonium bicarbonate buffer (1 M, pH 7.5; 10 mL) was added. The reaction was stirred at room temperature for one hour, followed by the dropwise addition of concentrated ammonium hydroxide (5 mL, 27%) at 0°C. The mixture was stirred at room temperature for an additional hour and then lyophilized to dryness. The residue was dissolved in water (10 mL), filtered, and purified by anion exchange chromatography using a Q Sepharose FF column (2.5 x 20 cm) with a linear gradient of $NH_4HCO_3$ (50 mM to 500 mM in 300 minutes) at a flow rate of 4.5 mL/min. The fractions containing triphosphate were combined and lyophilized to give triphosphate **dT.15** (16 mg, 40%, 1:1 mixture of diastereomers) as a white fluffy solid.

*$^1H$ NMR (400 MHz, $D_2O$) for diastereomers:* δ 8.01 ( m, 1 H, Ph-H), 7.74 - 7.55 (m, 3 H, H-6 and Ph-H), 6.18 and 6.12 (2 t, *J* = 6.4 Hz, 1 H, H-1'), 5.11 (m, 1 H, PhCH), 4.53 (m, 1 H, H-3'), 4.37 (m, 1 H, H-4'), 4.20 (m, 4 H, $CH_2$ and H-5'), 3.65 (s, 2 H, $CH_2$), 2.35 (m, 1 H, H-2'a), 2.25 (m, 1 H, H-2'b), 1.54 (d, 3 H, *J* = 6.4 Hz, $CH_3$);

*$^{31}P$ NMR (162 MHz, $D_2O$) for diastereomers:* δ -5.87 (d, *J* = 19.8 Hz), -11.18 and - 11.30 (2 d, *J* = 19.4 Hz), -21.62 (t, *J* = 19.6 Hz);

*ToF-MS (ESI):* For the molecular ion $C_{21}H_{27}N_4O_{17}P_3Na$ $[M+Na]^+$, the calculated mass was 723.0482, and the observed mass was 723.0497.

*6-JOE labeled 5-{1-[4-(3-Amino-1-propynyl)-2-nitrophenyl]ethyloxymethyl)-2'-deoxy-uridine-5'-triphosphate **(WW2p113)***

A solution of 6-JOE-SE (0.75 mg, 1.2 μmol) in anhydrous DMSO (30 μL) was added to a solution of triphosphate **dT.15** (0.56 μmol) in $Na_2CO_3$/$NaHCO_3$ buffer (0.1 M, pH 9.2; 200 μL) and incubated at room temperature for one hour. The reaction was purified by reverse-phase HPLC using a Perkin Elmer OD-300 $C_{18}$ column (4.6 x 250 mm) to yield the 6-JOE labeled triphosphate **WW2p113.** Mobile phase: A, 100 mM TEAA in water (pH 7.0); B, 100 mM TEAA in water/$CH_3CN$ (30:70). Elution was performed with a linear gradient of 5-50% B for 40 minutes and then 50-90% B for 10 minutes. The concentration of **WW2p113** was estimated by adsorption spectroscopy using the extinction coefficient of the 6-JOE dye *(i.e.,* 75,000 at 520 nm).

Synthesis of **5-[1-(2-nitrophenyl)-2-(methyl)propyloxymethyl]-2'-deoxyuridine-5'-triphosphate (WW2p148)**

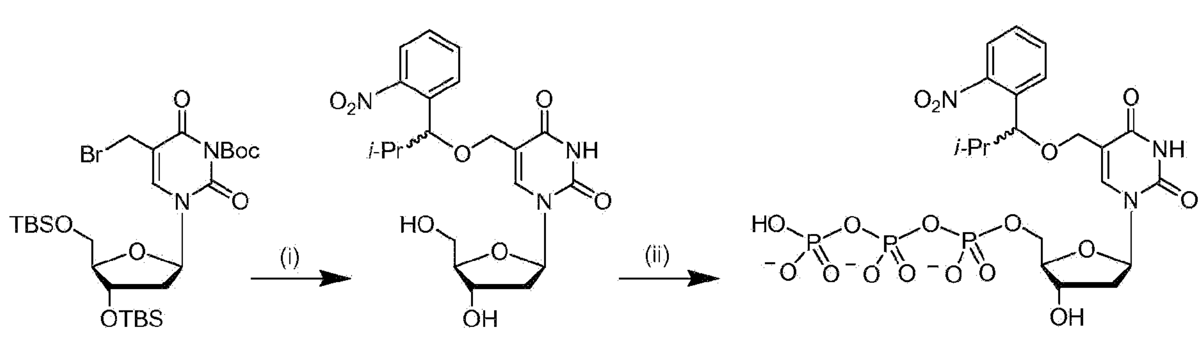


**dT.12** **dT.16** **WW2p148**

**Scheme**. Synthesis of *5-[1-(2-nitrophenyl)-2-(methyl)propyloxymethyl]-2'-deoxyuridine-5'-triphosphate*. (i) 1-(2-nitrophenyl)-2-methylpropanol, 100-110°C, 35 minutes, 14%; (ii) $POCl_3$, proton sponge, $(MeO)_3PO$, 0°C, three hours; $(n\text{-}Bu_3NH)_2H_2P_2O_7$, $n\text{-}Bu_3N$, DMF; 1 M $HNEt_3HCO_3$, one hour.

*5-[1-(2-Nitrophenyl)-2-(methyl)propyloxymethyl]-2'-deoxyuridine* ***(dT.16)***

Compound **dT.12** (0.316 g, 0.486 mmol) and 1-(2-nitrophenyl)-2-methylpropanol (0.706 g, 3.62 mmol) were heated neat at 100-110°C for 35 minutes under a nitrogen atmosphere. The mixture was cooled down to room temperature, dissolved in ethyl acetate, and purified by silica gel chromatography to yield 5-[1-(2-nitrophenyl)-2-(methyl)propyloxymethyl]-2'-deoxyuridine **dT.16** (30 mg, 14%, 1:1 mixture of diastereomers).

*$^1H$ NMR (400 MHz, DMSO-$d_6$) for diastereomers:* δ 11.33 and 11.34 (2 br s, 1 H, $D_2O$ exchangeable, NH), 7.95 (m, 1 H, Ph-H), 7.83 (m, 1 H, Ph-H), 7.72 (m, 1 H, Ph-H), 7.68 (m, 1 H, H-6), 7.54 (m, 1 H, Ph-H), 6.14 (m, 1 H, H-1'), 5.26 (d, 1 H, $D_2O$ exchangeable,
3'-OH), 4.97 (m, 1 H, 1 H $D_2O$ exchangeable, 5'-OH), 4.66 (m, 1 H, CH), 4.22 (m, 1 H, H-3'), 3.98 (m, 2 H, $CH_2$), 3.78 (m, 1 H, H-4'), 3.55 (m, 2 H, H-5'), 2.08 (m, 2 H, H-2'), 1.72 (m, 1 H, CH), 0.85 (m, 3 H, $CH_3$), 0.80 (m, 3 H, $CH_3$);

*$^{13}C$ NMR (100 MHz, $COCl_3$) for diastereomers:* δ 165.04 (C), 152.20/151.09 (C), 141.19/141.04 (CH), 139.28 (C), 138.05 (C), 134.08 (CH), 130.57/130.51 (CH), 129.60 (CH), 125.25/125.19 (CH), 112.62/112.43 (C), 89.12 (CH)/86.64 (CH), 82.72/82.40 (CH), 72.45 (CH), 65.78/65.61 ($CH_2$), 63.01 ($CH_2$), 41.50/41.45 ($CH_2$), 36.21 (CH), 26.68 (CH), 19.85/19.80 ($CH_3$), 18.20/18.14 ($CH_3$);

*5-[1-(2-Nitrophenyl)-2-(methyl)propyloxymethyl]-2'-deoxyuridine-5'-triphosphate* ***(WW2p148)***

$POCl_3$ (13 μL, 0.17 mmol) was added to a solution of compound **dT.16** (30 mg, 0.07mmol) and proton sponge (30 mg, 0.14 mmol) in trimethylphosphate (0.5 mL) at 0°C and stirred for three hours. A solution of tri-*n*-butylammonium pyrophosphate (166 mg, 0.35 mmol) and tri-*n*-butylamine (70 μL) in anhydrous DMF (0.7 mL) was added. After five minutes of stirring, triethylammonium bicarbonate buffer (1 M, pH 7.5; 10 mL) was added. The reaction was stirred at room temperature for one hour and then lyophilized to dryness. The residue was dissolved in water (10 mL), filtered, and part of the solution was purified by anion exchange chromatography using a Q Sepharose FF column (2.5 x 20 cm) with a linear gradient of $NH_4HCO_3$ (50 mM to 500 mM in 240 minutes) at a flow rate of 4.5 mL/min. The fractions containing triphosphate were combined and lyophilized to give *5-[1-(2-nitrophenyl)-2-(methyl)propyloxymethyl]-2'-deoxyuridine-5'-triphosphate* **WW2p148** (1:1 mixture of diastereomers) as a white fluffy solid.

*$^1H$ NMR (400 MHz, $D_2O$) for diastereomers:* δ 7.93 ( m, 1 H, Ph-H), 7.74-7.64 (m, 3 H, H-6 and Ph-H), 7.52 (m, 1 H, Ph-H), 6.19 and 6.13 (2 t, *J* = 6.6 Hz, 1 H, H-1'), 4.55 (m, 1 H, H-3'), 4.40 (m, 1 H, H-4'), 4.21 (m, 4 H, $CH_2$ and H-5'), 2.38 - 2.22 (m, 2 H, H-2'), 1.99 (m, 1 H, CH), 1.01 (m, 3 H, $CH_3$), 0.78 (m, 3 H, $CH_3$).

*$^{31}P$ NMR (162 MHz, $D_2O$) for diastereomers:* δ -5.26 (d, *J* = 20.1 Hz), -10.66 and - 10.72 (2 d, *J* = 19.6 Hz), -21.17 (t, *J* = 19.6 Hz).

*ToF-MS (ESI):* For the molecular ion $C_{20}H_{27}N_3O_{17}P_3$ $[M-H]^-$, the calculated mass was 674.0553, and the observed mass was 674.0470.

Synthesis of **6-JOE labeled 5-{1-[4-(3-amino-1-propynyl)-2-nitrophenyl]-2-(methyl)propyloxymethyl}-2'-deoxyuridine-5'-triphosphate (WW2p150)**

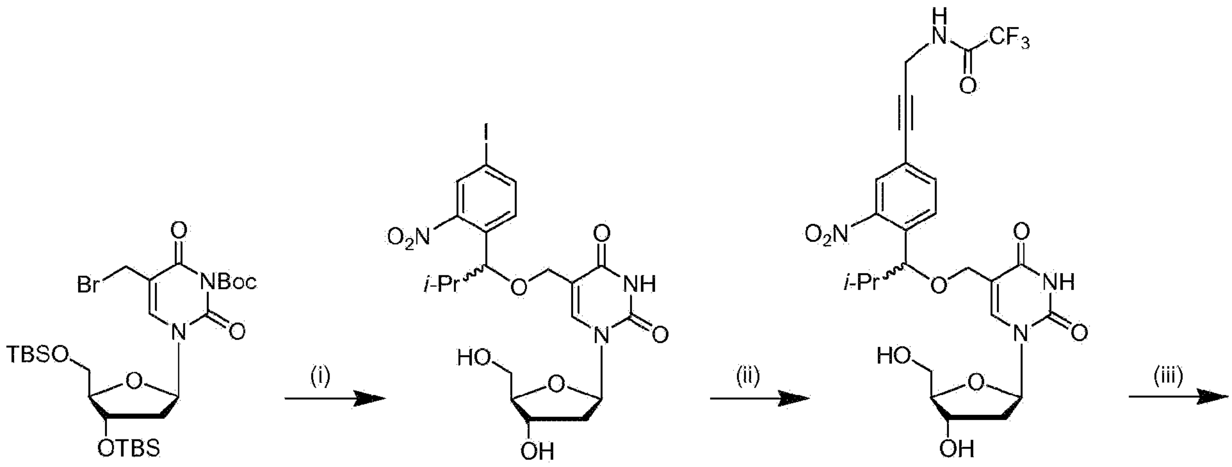


**dT.12** **dT.17** **dT.18**

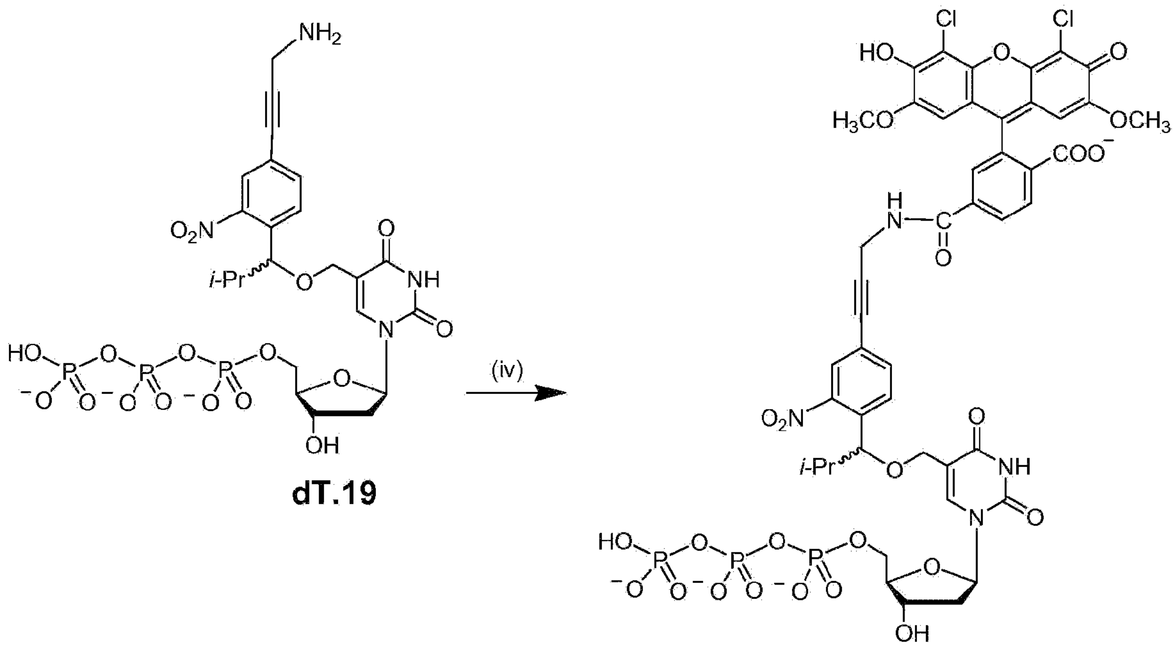


**dT.19**

**WW2p150**

**Scheme**. Synthesis of *6-JOE labeled 5-{1-[4-(3-Amino-1-propynyl)-2-nitrophenyl]-2-(methyl)propyloxymethyl}-2'-deoxyuridine-5'-triphosphate*. (i) 1-(4-iodo-2-nitrophenyl)-2-methylpropanol, neat, 108°C, 45 minutes, 18%; (ii) *N*-propargyltrifluoroacetamide, $Pd(PPh_3)_4$ (0), CuI, $Et_3N$, anhydrous DMF, 4.5 hours, 99%; (iii) $POCl_3$, proton sponge, $(MeO)_3PO$, 0°C, three hours; ($n$-$Bu_3NH)_2H_2P_2O_7$, $n$-$Bu_3N$, DMF; 1 M $HNEt_3HCO_3$, one hour; $NH_4OH$, one hour; (iv) 6-JOE-SE, 0.1 M $Na_2CO_3/NaHCO_3$ buffer (pH 9.2), one hour.

*5-[1-(4-Iodo-2-nitrophenyl)-2-(methyl)propyloxymethyl]-2'-deoxyuridine* ***(dT.17)***

Compound **dT.12** (400 mg, 0.615 mmol) and 1-(4-iodo-2-nitrophenyl)-2-methylethanol (800 mg, 2.49 mmol) were heated neat at 108°C for 45 minutes under a nitrogen atmosphere. The mixture was cooled down to room temperature, dissolved in ethyl acetate, and purified by silica gel chromatography to yield 5-[1-(4-iodo-2-nitrophenyl)-2-(methyl)propyloxymethyl]-2'-deoxyuridine **dT.17** (64 mg, 18%, 1:1 mixture of diastereomers) as a waxy solid.

*$^1H$ NMR (400 MHz, MeOH-$d_4$) for diastereomers:* δ 8.22 (m, 1 H, H-6), 8.02 (m, 2 H, Ph-H), 7.49 (m, 1 H, Ph-H), 6.22 (m, 1 H, H-1'), 4.69 (m, 1 H, CH), 4.41 (m, 1 H, H-3'), 4.10 (m, 2 H, $CH_2$), 3.92 (m, 1 H, H-4'), 3.75 (m, 2 H, H-5'a), 2.17 (m, 1 H, H-2'a), 2.15 (m, 1 H, H-2'b), 1.90(m, 1 H, CH), 0.92 (m, 3 H, $CH_3$), 0.85 (m, 3 H, $CH_3$);

*$^{13}C$ NMR (100 MHz, $CDCl_3$) for diastereomers:* δ 165.11 (C), 152.16/151.18 (C), 143.12 (CH), 141.44/141.32 (CH), 137.91/137.88 (C), 133.83/133.77 (CH), 132.37/132.33 (CH), 130.80/129.67 (C), 112.40/112.24 (C), 92.75 (C), 89.12 (CH)/86.90 (CH), 82.43/82.18 (CH), 72.39/72.37 (CH), 65.83/65.70 ($CH_2$), 62.96 ($CH_2$), 41.56/41.49 ($CH_2$), 36.01 (CH), 27.83/26.37 (CH), 19.82/19.78 ($CH_3$), 17.91/17.88 ($CH_3$);

*5-{1-[4-(3-Trifluoroacetamido-1-propynyl)-2-nitrophenyl]-2-(methyl)propyloxymethyl}-2'-deoxyuridine* ***(dT.18)***

A solution of compound **dT.17** (60 mg, 0.107 mmol), *N*-propargyl-trifluoroacetylamide (48.5 mg, 0.321 mmol), tetrakis(triphenylphosphine)-palladium(O) (12.4 mg, 0.01 mmol), CuI (4 mg, 0.02 mmol), and $Et_3N$ (30 μL, 0.214 mmol) in anhydrous DMF (1.5 mL) was stirred at room temperature for 4.5 hours. The mixture was concentrated *in vacuo* and purified by silica gel column chromatography to yield 5-[1-(4-{3-trifluoroacetamido-1-propynyl}-2-nitrophenyl)-2-(methyl)propyloxymethyl]-2'-deoxyuridine **dT.18** (62 mg, 99%, 1:1 mixture of diastereomers) as a waxy solid.

*$^1H$ NMR (400 MHz, DMSO-$d_6$) for diastereomers:* δ 11.36, 11.35(2 s, 1 H, $D_2O$ exchangeable, $N^3$-H), 10.11 (t, 1 H, *J* = 5.3 Hz, $D_2O$ exchangeable, NHTFA), 7.99 (m, 1 H, H-6), 7.86 (m, 1 H, Ph-H), 7.75 (m, 1 H, Ph-H), 7.65 (m, 1 H, Ph-H), 6.12 (m, 1 H, H-1'), 5.26 (m, 1 H, $D_2O$ exchangeable, 3'-OH), 4.97 (m, 1 H, $D_2O$ exchangeable, 5'-OH), 4.62 (m, 1 H, CH), 4.31 (m, 2 H, $CH_2$), 4.30 (m, 1 H, H-3'), 3.98 (m, 2 H, $CH_2$), 3.78 (m, 1 H, H-4'), 3.55 (m, 2 H, H-5'a and H-5'b), 2.08 (m, 2 H, H-2'a and H-2'b), 1.77 (m, 1 H, CH), 0.82 (m, 6 H, 2 $CH_3$);

*5-{1-[4-(3-Amino-1-propynyl)-2-nitrophenyl]-2-(methyl)propyloxymethyl}-2'-deoxyuridine-5'-triphosphate* ***(dT.19)***

$POCl_3$ (8 μL, 0.09 mmol) was added to a solution of compound **dT.18** (34 mg, 0.06 mmol) and proton sponge (26 mg, 0.12 mmol) in trimcthylphosphatc (0.3 mL) at 0°C and stirred for two hours. Additional POCl3 (4 μL, 0.045 mmol) was added and stirred for another hour. A solution of bis-tri-*n*-butylammonium pyrophosphate (142 mg, 0.3 mmol) and tri-*n*-butylamine (60 μL) in anhydrous DMF (0.6 mL) was added. After five minutes of stirring, triethylammonium bicarbonate buffer (1 M, pH 7.5; 10 mL) was added. The reaction was stirred at room temperature for one hour, followed by the dropwise addition of concentrated ammonium hydroxide (5 mL, 27%) at 0°C. The mixture was stirred at room temperature for an additional hour and then lyophilized to dryness. The residue was dissolved in water (10 mL), filtered, and part of the solution was purified by anion exchange chromatography using a Q Sepharose FF column (2.5 x 20 cm) with a linear gradient of $NH_4HCO_3$ (50 mM to 500 mM in 240 minutes) at a flow rate of 4.5 mL/min. The fractions containing triphosphate were combined and lyophilized to give triphosphate **dT.19** (1:1 mixture of diastereomers) as a white fluffy solid.

*$^1H$ NMR (400 MHz, $D_2O$) for diastereomers:* δ 8.06 and 8.04 (2 s, 1 H, Ph-H), 7.78 (m, 1 H, Ph-H), 7.69 - 7.59 (m, 2 H, H-6 and Ph-H), 6.13 (m, 1 H, H-1'), 4.55 (m, 1 H, H-3'), 4.46 and 4.34 (2 d, 2 H, $CH_2$), 4.20 (m, 3 H, H-4' and H-5'), 3.87 and 3.83 (2 s, 2 H, $CH_2$), 2.40 -2.20 (m, 2 H, H-2'), 1.99 (m, 1 H, CH), 1.02 (m, 3 H, $CH_3$), 0.79 (m, 3 H, $CH_3$);

*$^{31}P$ NMR (162 MHz, $D_2O$) for diastereomers:* δ -5.15 (d, *J* = 19.8 Hz), -10.48 and - 10.54 (2 d, *J* = 19.6 Hz), -21.0 (m);

*ToF-MS (ESI):* For the molecular ion $C_{23}H_{30}N_4O_{17}P_3$ $[M-H]^-$, the calculated mass was 727.0819, and the observed mass was 727.0828.

*6-JOE labeled 5-{1-[4-(3-amino-1-propynyl)-2-nitrophenyl]-2-(methyl)propyloxymethyl}-2'-deoxyuridine-5'-triphosphate* ***(WW2p150)***

A solution of 6-JOE-SE (0.75 mg, 1.2 μmol) in anhydrous DMSO (30 μL) was added to a solution oftriphosphate **WW2p145** (0.47 μmol) in $Na_2CO_3$/$NaHCO_3$ buffer (0.1 M, pH 9.2; 0.3 mL) and incubated at room temperature for one hour. The reaction was purified by reverse-phase HPLC using a Perkin Elmer OD-300 $C_{18}$ column (4.6 x 250 mm) to yield the 6-JOE labeled triphosphate **WW2p150.** Mobile phase: A, 100 mM TEAA in water (pH 7.0); B, 100 mM TEAA in water/$CH_3CN$ (30:70). HPLC purification was achieved using a linear gradient of 5-50% B for 40 minutes and then 50-90% B for 10 minutes. The concentration of **WW2p150** was estimated by adsorption spectroscopy using the extinction coefficient of the 6-JOE dye *(i.e.,* 75,000 at 520 nm).

**Synthesis of 6-JOE labeled 5-{(*R* or *S*)-1-[4-(3-amino-1-propynyl)-2-nitrophenyl]-2-(methyl)propyloxymethyl}-2'-deoxyuridine-5'-triphosphate (WW3p024)**

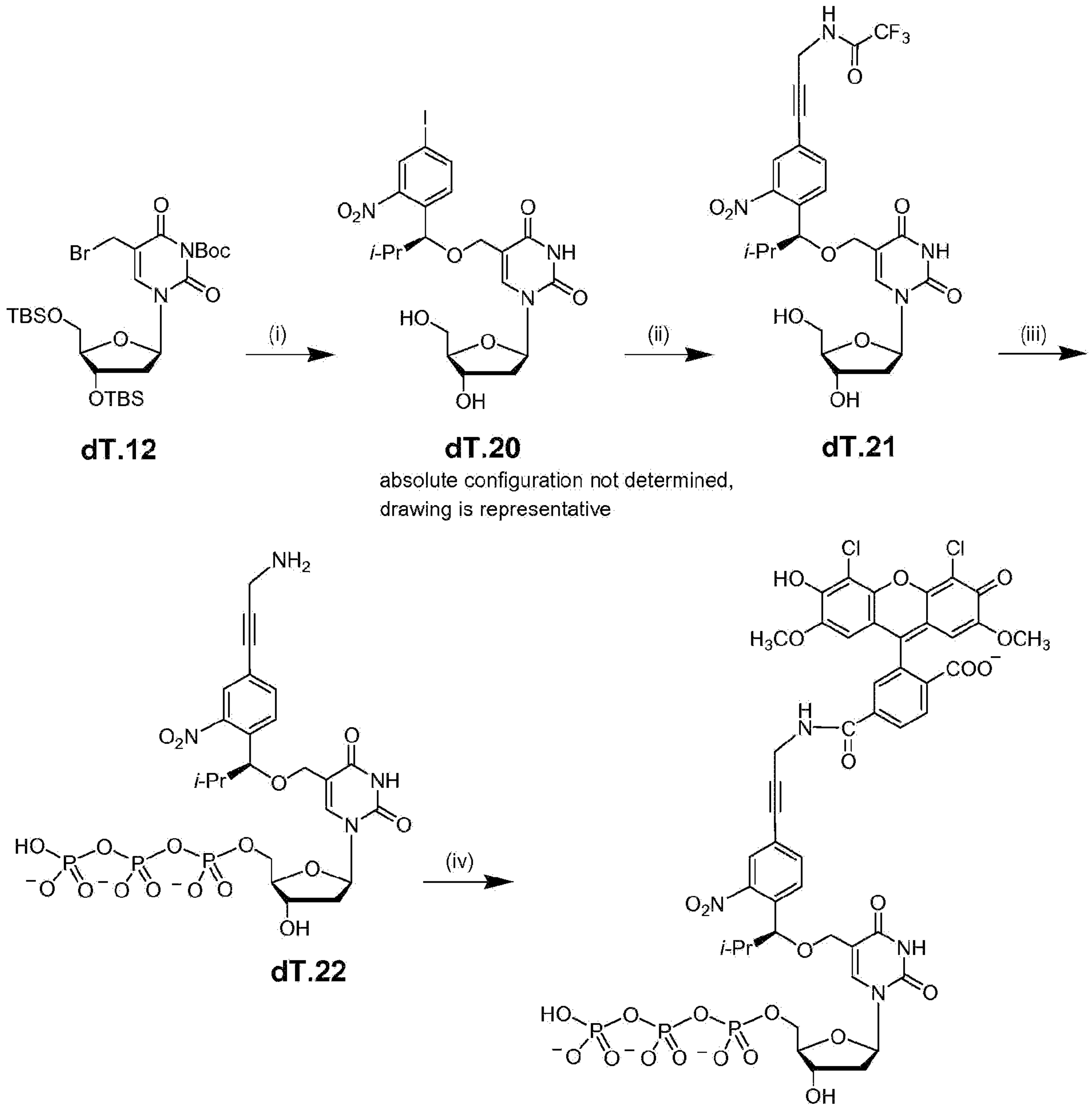


dT.12

dT.20

absolute configuration not determined, drawing is representative

dT.21

dT.22

WW3p024

**Scheme.** Synthesis of *6-JOE labeled 5-{(R or S)-1-[4-(3-Amino-1-propynyl)-2-nitrophenyl]-2-(methyl)propyloxymethyl}-2'-deoxyuridine-5'-triphosphate*. (i) (S or R)-1-(4-iodo-2-nitrophenyl)-2-methylpropanol, neat, 108°C, 45 minutes, 20%; (ii) *N*-propargyltrifluoroacetamide, $Pd(PPh_3)_4$ (0), CuI, $Et_3N$, anhydrous DMF, 4.5 hours, 81%; (iii) $POCl_3$, proton sponge, $(MeO)_3PO$, 0°C, two hours; (*n*-

U.S. Patent Application to be determined Attorney Docket 11997.105001a $Bu_3NH)_2H_2P_2O_7$, *n*-$Bu_3N$, DMF; 1 M $HNEt_3HCO_3$, one hour; $NH_4OH$, two hours; (iv) 6-JOE-SE, 0.1 M $Na_2CO_3$/$NaHCO_3$ buffer (pH 9.2), one hour.

*5-[(R or S)-1-(4-Iodo-2-nitrophenyl)-2-(methyl)propyloxymethyl]-2'-deoxyuridine **(dT.20)***

Compound **dT.12** (143 mg, 0.22 mmol) and enantio-pure (S or *R*)-1-(4-iodo-2-nitrophenyl)-2-methylpropanol (282 mg, 0.88 mmol), absolute configuration not determined) were heated neat at 108°C for 45 minutes under a nitrogen atmosphere. The mixture was cooled down to room temperature, dissolved in ethyl acetate, and purified by silica gel chromatography to yield *5-[(R or S*)-1-(4-iodo-2-nitrophenyl)-2-(methyl)propyloxymethyl]-2'-deoxyuridine dT.20 (25 mg, 20%, absolute configuration not determined) as a waxy solid.

*$^1$H NMR (400 MHz, MeOH-$d_4$)* δ 8.22 (d, 1 H, *J* = 1.8 Hz, H-6), 8.01 (m, 2 H, Ph-H), 7.50 (d, 1 H, J = 8.3 Hz, Ph-H), 6.25 (t, 1 H, *J* = 7.2 Hz, H-1'), 4.69 (d, 1 H, *J* = 5.8 Hz, PhCH), 4.41 (m, 1 H, H-3'), 4.10 (m, 2 H, $CH_2$), 3.92 (m, 1 H, H-4'), 3.75 (m, 2 H, H-5'a), 2.17 (m, 1 H, H-2'a), 2.15 (m, 1 H, H-2'b), 1.90(m, 1 H, CH), 0.92 (m, 3 H, $CH_3$), 0.85 (m, 3 H, $CH_3$);

*5-{(R or S)-1-[4-(3-Trifluoroacetamido-1-propynyl)-2-nitrophenyl]-2-(methyl)-propyloxymethyl}-2'-deoxyuridine (dT.21)*

A solution of compound dT.20 (24 mg, 0.043 mmol), *N*-propargyl-trifluoroacetylamide (28 mg, 0.186 mmol), tetrakis(triphenylphosphine)-palladium(O) (7.2 mg, 0.006 mmol), CuI (2.4 mg, 0.012 mmol), and $Et_3N$ (17 μL, 0.124 mmol) in anhydrous DMF (1.5 mL) was stirred at room temperature for 4.5 hours. The mixture was *concentrated in vacuo* and purified by silica gel column chromatography to yield 5-{(*R or S*)-1-[4-(3-trifluoro-acetamido-1-propynyl)-2-nitrophenyl]-2-(methyl)propyloxymethyl]-2'-deoxyuridine **dT.21**(19.8 mg, 81%, absolute configuration not determined) as a waxy solid.

*$^1$H NMR (400 MHz, MeOH-$d_4$):* δ 8.01 (br s, 1 H, H-6), 7.95 (d, 1 H, *J* = 1.2 Hz, Ph-H), 7.72 (m, 2 H, Ph-H), 6.25 (t, 1 H, *J* = 6.7 Hz, H-1'), 4.74 (d, 1 H, *J* = 5.8 Hz, PhCH), 4.38 (m, 1 H, H-3'), 4.34 (s, 2 H, $CH_2$), 4.05 (m, 2 H, $CH_2$), 3.55 , 3.93 (m, 1 H, H-4'), 3.77 (m, 2 H, H-5'a and H-5'b), 2.15 (m, 2 H, H-2'a and H-2'b), 1.90 (m, 1 H, CH), 0.92 (m, 6 H, 2 x $CH_3$);

*5-{(R or S)-1-[4-(3-Amino-1-propynyl)-2-nitrophenyl]-2-(methyl)propyloxymethyl}-2'-deoxyuridine-5'-triphosphate **(dT.22)***

$POCl_3$ (6 μL, 0.06 mmol) was added to a solution of compound **dT.21** (18 mg, 0.03 mmol) and proton sponge (13 mg, 0.06 mmol) in trimethylphosphate (0.3 mL) at 0°C and stirred for two hours. A solution of bis-tri-n-butylammonium pyrophosphate (73 mg, 0.15 mmol) and tri-*n*-butylamine (30 μL) in anhydrous DMF (0.3 mL) was added. After five minutes of stirring, triethylammonium bicarbonate buffer (1 M, pH 7.5; 5 mL) was added. The reaction was stirred for one hour at room temperature and then lyophilized to dryness. The residue was dissolved in water (5 mL), filtered, and part of the solution was purified with reverse-phase HPLC using a Perkin Elmer OD-300 $C_{18}$ column (4.6 x 250 mm) to yield 5-{(R or S)-1-[4-(3-trifluoroacetamido-1-propynyl)-2-nitrophenyl]-2-(methyl)propyloxymethyl}-2'-deoxyuridine-5'triphosphate. Mobile phase: A, 100 mM triethylammonium acetate (TEAA) in water (pH 7.0); B, 100 mM TEAA in water/$CH_3CN$ (30:70). HPLC purification was achieved using a linear gradient of 5-50% B for 40 minutes and then 50-90% B for 10 minutes. The purified triphosphate was then treated with concentrated ammonium hydroxide (27%; 0.5 mL) at room temperature for two hours to yield *5-{(R or S*)-1-[4-(3-amino-1-propynyl)-2-nitrophenyl]-2-(methyl)-propyloxymethyl}-2'-deoxyuridine-5'-triphosphate **(dT.22,** absolute configuration not determined).

*$^1$H NMR (400 MHz, $D_2O$):* δ 8.01 (s, 1 H, Ph-H), 7.76 (d, 1 H, *J* = 6.9 Hz, Ph-H), 7.62 (m, 2 H, H-6 and Ph-H), 6.17 (t, 1 H, *J* = 6.4 Hz, H-1'), 4.55 (m, 1 H, H-3'), 4. 39 and 4.29 (2 d, 2 H, *J* = 6.4 Hz, $CH_2$), 4.17 (m, 3 H, H-4' and H-5'), 3.74 (s, 2 H, $CH_2$), 2.28 (m, 2 H, H-2'), 2.00 (m, 1 H, CH), 0.79 (m, 3 H, $CH_3$);

*$^{31}$P NMR (162 MHz, $D_2O$):* δ -5.40 (d, *J* = 19.4 Hz), -10.75 (d, *J* = 19.4 Hz), -21.23 (t, *J* = 19.4 Hz).

*6-JOE labeled 5-{(R or S)-1-[4-(3-amino-1-propynyl)-2-nitrophenyl]-2-(methyl)-propyloxymethyl}-2'-deoxyuridine-5'-triphosphate **(WW3p024)***

A solution of 6-JOE-SE (0.625 mg, 1 μmol) in anhydrous DMSO (25 μL) was added to a solution of triphosphate **dT.22** (0.31 μmol) in $Na_2CO_3$/$NaHCO_3$ buffer (0.1 M, pH 9.2; 180 μL) and incubated at room temperature for one hour. The reaction was purified by reverse-phase HPLC using a Perkin Elmer OD-300 $C_{18}$ column (4.6 x 250 mm) to yield the 6-JOE labeled triphosphate **WW3p024.** Mobile phase: A, 100 mM TEAA in water (pH 7.0); B, 100 mM TEAA in water/$CH_3CN$ (30:70). HPLC purification was achieved using a linear gradient of 5-50% B for 40 minutes and then 50-90% B for 10 minutes. The concentration of **WW3p024** was estimated by adsorption spectroscopy using the extinction coefficient of the 6-JOE dye (*i.e.*, 75,000 at 520 nm).

Example 3: dC compounds

**Synthesis of *N*4-(2-nitrobenzyl)-2'-deoxycytidine-5'-triphosphate (WW2p044)**

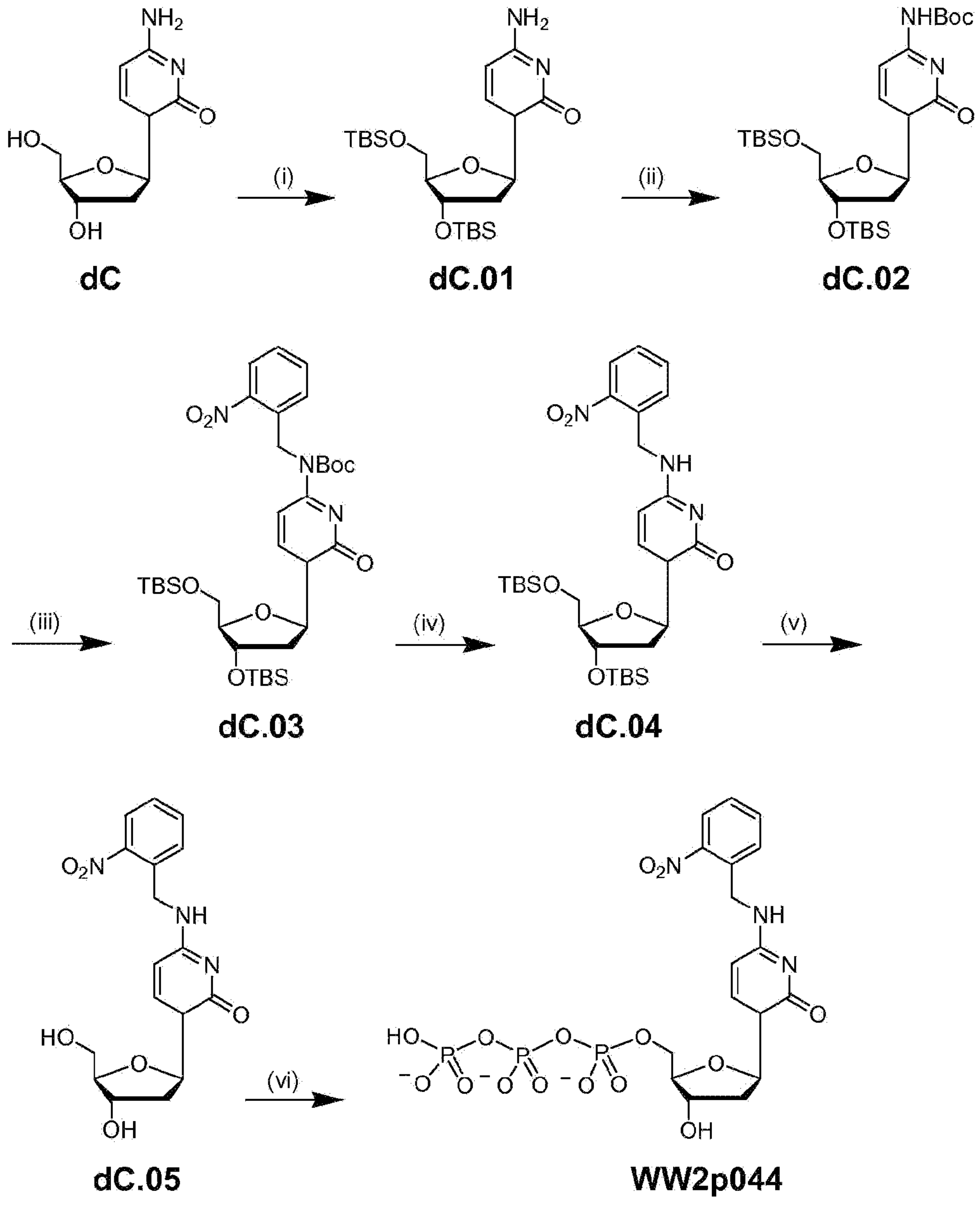


dC, dC.01, dC.02, dC.03, dC.04, dC.05, WW2p044

**Scheme.** Synthesis of *$N^4$-(2-nitrobenzyl)-2'-deoxycytidine triphosphate.* (i) TBSCl, imidazole, anhydrous DMF, room temperature, overnight, 84%; (ii) $Boc_2O$, DMAP, $Et_3N$, $CH_2Cl_2$, room temperature, overnight, 56%; (iii) 2-nitrobenzyl bromide, NaH, anhydrous DMF, 0°C, then gradually warmed to room temperature, overnight, 37%; (iv) $SiO_2$, vacuum, 70-80°C, 48 hours, 79%; (v) *n*-$Bu_4NF$, THF, 0°C, then gradually warmed to room temperature, two hours, 59%;

(vi) Pock, proton sponge, $(MeO)_3PO$, 0°C, two hours; $(n\text{-}Bu_3NH)_2H_2P_2O_7$, $n\text{-}Bu_3N$, DMF, five minutes; 1 M $HNEt_3HCO_3$, one hour, 52%.

*3',5'-O-Bis-tert-butyldimethylsilyl-2'-deoxycitidine **(dC.01)***

2'-deoxycytidine **dC** (2.85 g, 12.54 mmol), imidiazole (6.49 g, 95.31 mmol), and TBSCl (7.18 g, 47.65 mmol) were added to anhydrous DMF (27 mL) and stirred at room temperature overnight under a $N_2$ atmosphere. Methanol (20 mL) was added, and the mixture was stirred for 30 minutes and then concentrated *in vacuo.* Ethyl acetate (60 mL ) and water (60 mL) were then added. The organic layer was separated and washed twice with water (20 mL), and the combined aqueous layer was extracted with ethyl acetate (20 mL). The combined organic layer was dried with $Na_2SO_4$, concentrated *in vacuo*, and purified by silica gel chromatography to give 3',5'-*O*-bis-*tert*-butyldimethylsilyl-2'-deoxycytidine **dC.01** (4.79 g, 84%) as a white foam.

*$^1H$ NMR (400 MHz, $CDCl_3$):* δ 7.95 (d, 1 H, *J* = 7.4 Hz, H-6), 6.26 (t, 1 H, *J* = 5.6 Hz, H-1'), 5.69 (d, 1 H, *J* = 7.4 Hz, H-5), 4.37 (m, 1 H, H-3'), 3.90 (m, 2 H, H-4' and H-5'a), 3.76 (m, 1 H, H-5'b), 2.40 (m, 1 H, H-2'a), 2.08 (m, 1 H, H-2'b), 0.91 (s, 9 H, $(CH_3)_3CSi$), 0.87 (s, 9 H, $(CH_3)_3CSi$), 0.10 (s, 6 H, $(CH_3)_2Si$), 0.05 (s, 6 H, $(CH_3)_2Si$).

*$N^4$-tert-Butyloxycarbonyl-3',5'-O-bis-tert-butyldimethylsilyl-2'-deoxycytidine **(dC.02)***

Under a nitrogen atmosphere, a solution of di-*tert*-butyldicarbonate (0.34 g, 1.58 mmol) in anhydrous $CH_2Cl_2$ (3 mL) was slowly added to a solution of compound **dC.01** (0.5 g, 1.10 mmol), $Et_3N$ (0.15 mL, 1.10 mmol), and DMAP (0.13 g, 1.10 mmol) in anhydrous $CH_2Cl_2$ (5 mL). The mixture was stirred overnight at room temperature, concentrated *in vacuo,* and purified by silica gel chromatography to yield *$N^4$-tert*-butyloxycarbonyl-3',5'-*O*-bis-*tert*butyldimethylsilyl-2'-deoxycytidine **dC.02** (0.34 g, 56%) as a white foam.

*$^1H$ NMR (400 MHz, $CDCl_3$):* δ 8.30 (d, 1 H, *J* = 7.4 Hz H-6), 7.47 (bs, 1 H, NH), 7.14 (d, 1 H, *J* = 7.4 Hz, H-5), 6.25 (t, 1 H, *J* = 5.6 Hz, H-1'), 4.38 (m, 1 H, H-3'), 3.95 (m, 2 H, H-4' and H-5'a), 3.78 (m, 1 H, H-5'), 2.50 (m, 1 H, H-2'a), 2.10 (m, 1 H, H-2'b), 1.51 (s, 9 H, $(CH_3)_3CO$), 0.93 (s, 9 H, $(CH_3)_3CSi$), 0.88 (s, 9 H, $(CH_3)_3CSi$), 0.11 (s, 6 H, $(CH_3)_2Si$), 0.06 (s, 6 H, $(CH_3)_2Si$).

*N'-tert-Butyloxycarbonyl-$N^4$-(2-nitrobenzyl)-3',5'-O-bis-tert-butyldimethylsilyl-2'-deoxycytidine **(dC.03)***

NaH (32 mg, 1.26 mmol, dry) was added to a solution of compound dC.02 (540 mg, 0.97 mmol) in anhydrous DMF (6 mL) at 0°C and stirred for 30 minutes under a nitrogen atmosphere. A solution of 2-nitrobenzyl bromide (313 mg, 1.45 mmol) in anhydrous DMF (1.5 mL) was added dropwise. The reaction mixture was gradually warmed to room temperature and stirred overnight. Following the addition of ethyl acetate (60 mL), the mixture was washed three times with saturated $NH_4Cl$ solution (40 mL), and the combined aqueous layer was extracted with ethyl acetate (40 mL). The combined organic layer was dried with $Na_2SO_4$, concentrated *in vacuo*, and purified by silica gel chromatography to yield *$N^4$-tert*-butyloxycarbonyl-*$N^4$*-(2-nitrobenzyl)-3',5'-*O*-bis-*tert*-butyldimethylsilyl-2'-deoxycytidine dC.03 (250 mg, 37%) as a white foam.

*$^1H$ NMR (400 MHz, $CDCl_3$):* δ 8.29 (d, 1 *H, J* = 7.6 Hz, H-6), 8.05 (d, 1 *H, J* = 8.0 Hz, Ph-H), 7.53 (t, 1 H, *J* = 7.5 Hz, Ph-H), 7.38 (t, 1 H, *J* = 7.6 Hz, Ph-H), 7.28 (m, 2 H, Ph-H and H-5), 6.26 (t, 1 H, *J* = 5.6 Hz, H-1'), 5.60 (q, 2 H, $Ph\text{-}CH_2$), 4.41 (m, 1 H, H-3'), 3.96 (m, 2 H, H-4' and H-5'a), 3.80 (m, 1 H, H-5'b), 2.51 (m, 1 H, H-2'a), 2.15 (m, 1 H, H-2'b), 1.28 (s, 9 H, $(CH_3)_3CO$), 0.95 (s, 9 H, $(CH_3)_3CSi$), 0.88 (s, 9 H, $(CH_3)_3CSi$), 0.14 (s, 6 H, $(CH_3)_2Si$), 0.07 (s, 6 H, $(CH_3)_2Si$).

*$N^4$-(2-Nitrobenzyl)-3',5'-O-bis-tert-butyldimethylsilyl-2'-deoxycitidine **(dC.04)***

Silica gel 60 (2.5 g, 100-200 mesh, activated by heating to 50-60°C under reduced pressure for 24 hours) was added to a solution of compound dC.03 (250 mg, 0.36 mmol) in $CH_2Cl_2$ (5 mL), and the mixture was evaporated *in vacuo* to dryness. The residue was heated to 60-70°C under reduced pressure for 48 hours, washed three times with MeOH (30 mL), and filtered using a buchi funnel. The combined filtrate was concentrated *in vacuo* and purified by silica gel chromatography to yield *$N^4$*-(2-nitrobenzyl)-3',5'-*O*-bis-*tert*-butyldimethylsilyl-2'-deoxycytidine **dC.04** (0.185 g, 79%) as a white foam.

*$^1H$ NMR (400 MHz, $CDCl_3$):* δ 8.02 (d, 1 H, *J* = 8.0 Hz, Ph-H), 7.92 (d, 1 H, *J* = 7.2 Hz, H-6), 7.79 (d, 1 H, *J* = 7.5 Hz, Ph-H), 7.57 (t, 1 H, *J* = 7.3 Hz, Ph-H), 7.41 (t, 1 *H, J* = 7.5 Hz, Ph-H), 6.38 (bs, 1 H, NH), 6.26 (t, 1 H, *J* = 5.2 Hz, H-1'), 5.68 (d, 1 H, *J* = 7.2 Hz, H-5), 4.92 (m, 2 H, $Ph\text{-}CH_2$), 4.36 (m, 1 H, H-3'), 3.88 (m, 2 H, H-4' and H-5'a), 3.75 (m, 1 H, H-5'b), 2.39 (m, 1 H, H-2'a), 2.07 (m, 1 H, H-2'b), 0.91 (s, 9 H, $(CH_3)_3CSi$), 0.87 (s, 9 H, $(CH_3)_3CSi$), 0.09 (s, 6 H, $(CH_3)_2Si$), 0.05 (s, 6 H, $(CH_3)_2Si$).

*$N^4$-(2-Nitrobenzyl)-2'-deoxycytidine* ***(dC.05)***

A solution of *n*-$NBu_4F$ (190 mg, 0.73 mmol) in THF (1.4 mL) was added dropwise to a solution of compound **dC.04** (170 mg, 0.29 mmol) in THF (3.4 mL) at 0°C under a nitrogen atmosphere. The reaction mixture was stirred for two hours, concentrated *in vacuo*, and purified by silica gel chromatography to yield $N^4$-(2-nitrobenzyl)-2'-deoxycytidine **dC.05** (62 mg, 59%) as a white foam.

*$^1H$ NMR (400 MHz, DMSO-$d_6$):* δ 8.23 (t, 1 H, $D_2O$ exchangeable, NH), 8.07 (d, 1 H, *J* = 8.0 Hz, Ph-H), 7.83 (d, 1 *H, J* = 7.4 Hz, H-6), 7.74 (t, 1 H, *J* = 7.5 Hz, Ph-H), 7.54 (m, 2 H, Ph-H), 6.13 (t, 1 H, *J* = 6.8 Hz, H-1'), 5.91 (d, 1 H, *J* = 7.4 Hz, H-5), 5.19 (d, 1 H, $D_2O$ exchangeable, 3'-OH), 4.97 (t, 1 H, $D_2O$ exchangeable, 5'-OH), 4.78 (m, 2 H, Ph-$CH_2$), 4.19 (m, 1 H, H-3'), 3.76 (m, 1 H, H-4'), 3.55 (m, 2 H, H-5'a and H-5'b), 2.09 (m, 1 H, H-2'a), 1.93 (m, 1 H, H-2'b);

*$^{13}C$ NMR (100 MHz, $CD_3OD$):* δ 165.71, 158.51, 149.70, 141.75, 135.09, 134.73, 131.30, 129.43, 125.94, 96.75, 88.83, 87.57, 72.03, 62.78, 42.71, 42.00.

*$N^4$-(2-Nitrobenzyl)-2'-deoxycytidine-5'-triphosphate* ***(WW2p044)***

$POCl_3$ (17 μL, 0.2 mmol) was added to a solution of compound **dC.05** (36 mg, 0.1 mmol) and proton sponge (32 mg, 0.15 mmol) in trimethylphosphate (0.5 mL) at 0°C and stirred for two hours. Additional $POCl_3$ (9 μL, 0.1 mmol) was added and stirred for another hour. A solution of bis-tri-*n*-butylammonium pyrophosphate (237 mg, 0.5 mmol) and tri-n-butylamine (100 μL) in anhydrous DMF (1 mL) was added. After five minutes of stirring, triethylammonium bicarbonate buffer (1 M, pH 7.5; 10 mL) was added. The reaction was stirred for one hour at room temperature and then lyophilized to dryness. The residue was dissolved in water (10 mL), filtered, and purified by anion exchange chromatography using a Q Sepharose FF column (2.5 x 20 cm) with a linear gradient of $NH_4HCO_3$ (50 mM to 500 mM in 300 minutes) at a flow rate of 4.5 mL/min. The fractions containing triphosphate were combined and lyophilized to give the triphosphate **WW2p044** (34 mg, 52%) as a white fluffy solid.

*$^1H$ NMR (400 MHz, $D_2O$):* δ 8.12 (d, 1 H, *J* = 8.0 Hz, Ph-H), 7.83 (d, 1 H, *J* = 7.6 Hz, H-6), 7.69 (t, 1 H, *J* = 7.6 Hz, Ph-H), 7.60 (d, 1 H, *J* = 7.6 Hz, Ph-H), 7.53 (t, 1 H, *J* = 8.0 and 7.6 Hz, Ph-H), 6.29 (t, 1 H, *J* = 6.8 Hz, H-1'), 6.15 (d, 1 H, *J* = 7.6 Hz, H-5), 4.85 (bs, 2 H, Ph-$CH_2$), 4.58 (m, 1 H, H-3'), 4.21 (m, 3 H, H-4', H-5'a and H-5'b), 2.38 (m,

1 H, H-2'a), 2.28 (m, 1 H, H-2'b);

*$^{31}P$ NMR (162 MHz, $D_2O$):* δ -5.61 (d, *J* = 15.9 Hz), -10.60 (d, *J* = 15.4 Hz), -19.26 (br);

*ToF-MS (ESI):* For the molecular ion $C_{16}H_{21}N_4O_{15}P_3Na$ $[M+Na]^+$, the calculated mass was 625.0114, and the observed mass was 624.9993.

**Synthesis of *N^4*-[4-(3-amino-1-propynyl)-2-nitrobenzyl]-2'-deoxycytidine-5'-triphosphate and dye labeling (WW2p080)**

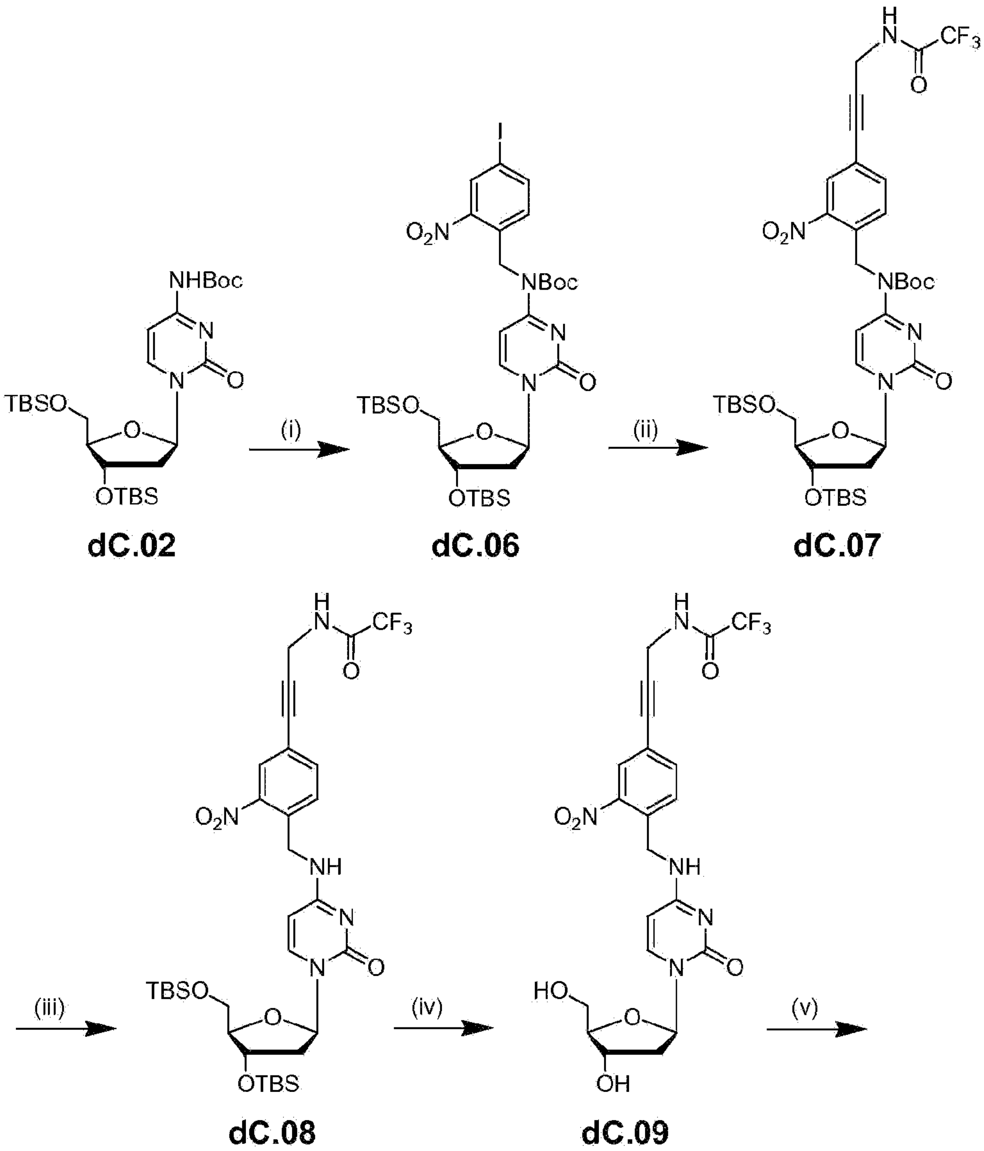


dC.02 dC.06 dC.07

dC.08 dC.09

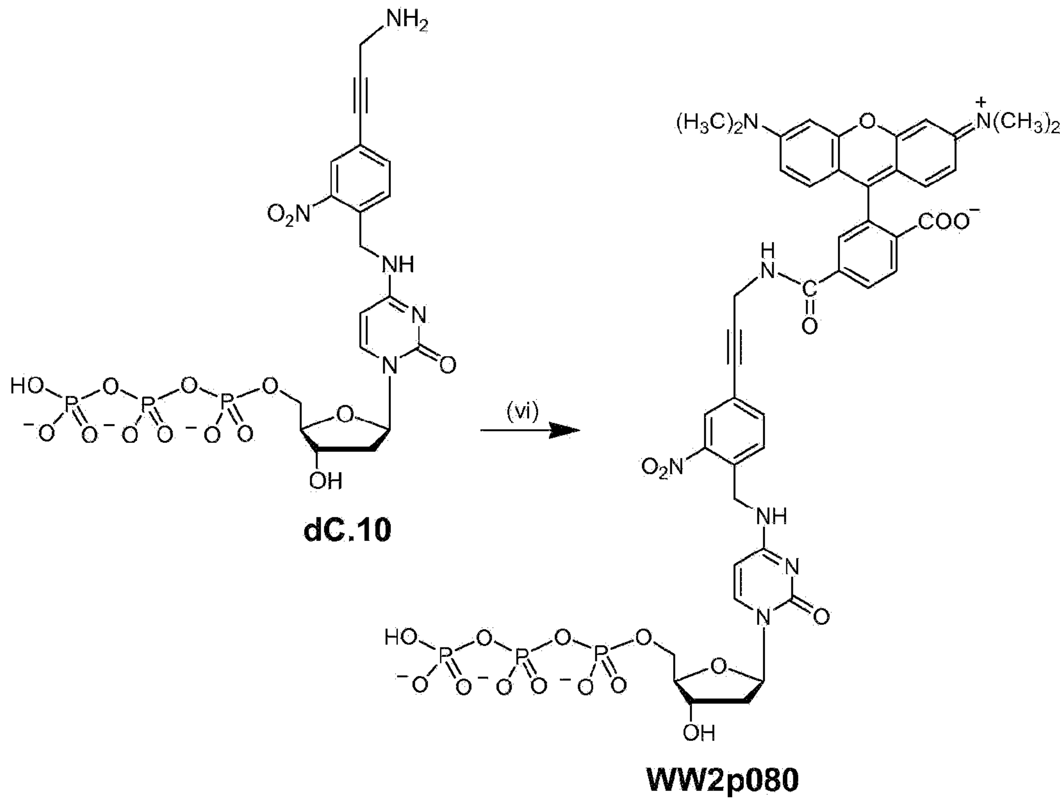


dC.10

WW2p080

**Scheme.** Synthesis of *6-TAMRA labeled $N^4$-[4-(3-amino-1-propynyl)-2-nitrobenzyl]-2'-deoxycytidine-5'-triphosphate*. (i) 4-iodo-2-nitrobenzyl bromide, *n*-$Bu_4NBr$, $CH_2Cl_2$, 1 M NaOH, room temperature, four hours, 45%; (ii) *N*-propargyltrifluoroacetamide, $PdCl_2(PPh_3)_2$, CuI, $Et_3N$, THF, reflux, three hours, 82%; (iii) $SiO_2$, vacuum, 70-80°C, 48 hours, 81%; (iv) *n*-$Bu_4NF$, THF, 0°C, two hours, then room temperature overnight, 39%; (v) $POCl_3$, proton sponge, $(MeO)_3PO$, 0°C, two hours; $(n\text{-}Bu_3NH)_2H_2P_2O_7$, *n*-$Bu_3N$, DMF, five minutes; 1 M $HNEt_3HCO_3$, one hour; $NH_4OH$, one hour; 39%; (vi) 6-TAMRA-SE, 0.1 M $Na_2CO_3$/$NaHCO_3$ buffer (pH 9.2), one hour.

*$N^4$-tert-Butyloxycarbonyl-$N^4$-(4-iodo-2-nitrobenzyl)-3',5'-O-bis-tert-butyldimethylsilyl-2'-cleoxycytidine* ***(dC.06)***

A solution of 4-iodo-2-nitrobenzyl bromide (461 mg, 1.35 mmol) in $CH_2Cl_2$ (4 mL) was added dropwise to a mixture of compound **dC.02** (250 mg, 0.45 mmol) and *n*-$Bu_4NBr$ (145 mg, 0.45 mmol) in $CH_2Cl_2$ (4 mL) and NaOH (1 M; 4.5 mL). The reaction was stirred vigorously at room temperature for four hours. The organic layer was separated, and the aqueous layer was extracted twice with $CH_2Cl_2$ (4 mL each). The combined organic layer was dried with $Na_2SO_4$, concentrated *in vacuo,* and purified by silica gel chromatography to give *$N^4$-tert*-butyloxycarbonyl-*$N^4$*-(4-iodo-2-nitrobenzyl)-3',5'-*O*-bis-*tert*-butyldimethylsilyl-2'-deoxycytidine **dC.06** (167 mg, 45%) as a white foam.

*$^1H$ NMR (400 MHz, $CDCl_3$):* δ 8.37 (d, 1 H, *J* = 1.8 Hz, Ph-H), 8.30 (d, 1 H, *J* = 7.5 Hz, H-6), 7.82 (dd, 1 H, *J* = 1.8 Hz and 8.3 Hz, Ph-H), 7.26 (d, 1 H, *J* = 7.5 Hz, H-5), 6.99 (d, 1 H, *J* = 8.4 Hz, Ph-H), 6.24 (dd, 1 H, *J* = 6.3 and 5.0 Hz, H-1'), 5.52 (q, 2 H, Ph-$CH_2$), 4.41 (m, 1 H, H-3'), 3.96 (m, 2 H, , H-4' and H-5'a), 3.80 (m, 1 H, H-5'b), 2.51 (m, 1 H, H-2'a), 2.14 (m, 1 H, H-2'b), 1.34 (s, 9 H, $(CH_3)_3CO$), 0.95 (s, 9H, $(CH_3)_3CSi$), 0.89 (s, 9H, $(CH_3)_3CSi$), 0.14 (s, 3 H, $(CH_3)Si$), 0.13 (s, 3 H, $(CH_3)Si$), 0.08 (s, 3 H, $(CH_3)Si$), 0.07 (s, 3 H, $(CH_3)$ Si).

*$N^4$-tert-Butyloxycarbonyl-$N^4$-[4-(3-trifluoroacetamido-1-propynyl)-2-nitrobenzyl]-3'5'-O-bis-tert-butyldimethylsilyl2'-deoxycytidine* ***(dC.07)***

Under a nitrogen atmosphere, a solution of bis(triphenylphosphine)palladium(II) dichloride (41 mg, 0.058 mmol) in anhydrous THF (3 mL) was quickly added to a mixture of compound **dC.06** (315 mg, 0.39mmol), CuI (15 mg, 0.078 mmol), $Et_3N$ (0.73 mL, 5.2 mmol) and *N*-propargyltrifluoroacetamide (82 mg, 0.54 mmol) in anhydrous THF (7 mL). The reaction mixture was refluxed for two hours, concentrated *in vacuo,* and purified by silica gel chromatography to yield *$N^4$-tert*-butyloxycarbonyl-*$N^4$*-[4-(3-trifluoroacetamido-1-propynyl)-2-nitrobenzyl]-3',5'-*O*-bis-*tert*-butyldimethylsilyl-2'-deoxycytidine **dC.07** (268 mg, 82%) as a white foam.

*$^1H$ NMR (400 MHz, $CDCl_3$):* δ 8.33 (d, 1 H, *J* = 7.7 Hz, H-6), 8.03 (d, 1 H, *J* = 1.5 Hz, Ph-H), 7.61 (bs, 1 H, NH), 7.50 (dd, 1 H, *J* = 1.5 Hz and 8.2 Hz, Ph-H), 7.32 (d, 1 H, *J* = 7.7 Hz, H-5), 7.18 (d, 1 H, *J* = 8.2 Hz, Ph-H), 6.24 (t, 1 H, *J* = 6.0 Hz, H-1'), 5.56 (q, 2 H, $PhCH_2$), 4.42 (m, 1 H, H-3'), 4.35 (d, 2 H, $CH_2$), 3.97 (m, 2 H, H-5'a and H-4'), 3.80 (m, 1 H, H-5'b), 2.50 (m, 1 H, H-2'a), 2.05 (m, 1 H, H-2'b), 1.31 (s, 9 H, $(CH_3)_3CO$), 0.96 (s, 9H, $(CH_3)_3CSi$), 0.89 (s, 9H, $(CH_3)_3CSi$), 0.15 (s, 3 H, $(CH_3)Si$), 0.14 (s, 3 H, $(CH_3)$ Si), 0.07 (s, 6 H, $(CH_3)_2Si$).

*$4^i$-[4-(3-Trifluoroacetamido-1-propynyl)-2-nitrobenzyl]-3',5'-O-bis-tert-batyldimethylsilyl-2-cleoxycytidine* ***(dC.08)***

Silica gel 60 (3.1 g, 100-200 mesh, activated by heating to 50-60°C under reduced pressure for 24 hours) was added to a solution of compound **dC.07** (305 mg, 0.36 mmol) in $CH_2Cl_2$ (4 mL), and the mixture was *evaporated in vacuo* to dryness. The residue was heated to 60-70°C under reduced pressure for 24 hours, washed three times with MeOH (30 mL), and filtered using a buchi funnel. The combined filtrate was concentrated *in vacuo* and purified by silica gel chromatography to yield *$N^4$*-[4-(3-trifluoroacetamido-1-propynyl)-2-nitrobenzyl]-3',5'-*O*-bis-*tert*-butyldimethylsilyl-2'-deoxycytidine **dC.08** (219 mg, 81%) as a white foam.

*$^1H$ NMR (400 MHz, $CDCl_3$):* δ 8.25 (bs, 1 H, N-H), 7.94 (d, 1 H, *J* = 7.4 Hz, H-6), 7.84 (s, 1 H, Ph-H), 7.62 (d, 1 H, *J* = 7.7 Hz, Ph-H), 7.41 (d, 1 H, *J* = 7.8 Hz, Ph-H), 6.25 (m, 2 H, N-H and H-1'), 5.67 (d, 1 H, *J* = 7.3 Hz, H-5'), 4.83 (m, 2 H, Ph-$CH_2$), 4.38 (m, 2 H, $CH_2$ and H-3'), 3.90 (m, 2 H, H-4' and H-5'a), 3.75 (m, 1 H, H-5'b), 2.36 (m, 1 H, H-2'a), 2.05 (m, 1 H, H-2'b), 0.90 (s, 9H, $(CH_3)_3CSi$), 0.87 (s, 9H, $(CH_3)_3CSi$), 0.08 (s, 6 H, $(CH_3)_2Si$), 0.05 (s, 6 H, $(CH_3)_2Si$).

*$N^4$-(4-(3-Trifluoroacetamido-1-propynyl)-2-nitrobenzyl]-2'-deoxycytidine* ***(dC.09)***

A solution of *n*-$Bu_4NF$ (94 mg, 0.36 mmol) in THF (2.5 mL) was added dropwise to a solution of compound **dC.08** (200 mg, 0.27 mmol) in THF (1 mL) at 0°C under a $N_2$ atmosphere. The reaction mixture was stirred at 0°C for two hours and then at room temperature overnight, concentrated *in vacuo*, and purified by silica gel chromatography to yield *$N^4$*-[4-(3-trifluoroacetamido-1-propynyl)-2-nitrobenzyl]-2'-deoxycytidine **dC.09** (54 mg, 39%) as a white foam.

*$^1H$ NMR (400 MHz, (DMSO-$d_6$):* δ 10.12 (t, 1 H, N-H), 8.26 (t, 1 H, N-H), 8.08 (s, 1 H, Ph-H), 7.84 (d, 1 H, *J* = 7.5 Hz, H-6), 7.78 (d, 1 H, *J* = 8.1 Hz, Ph-H), 7.50 (d, 1 H, *J* = 8.1 Hz, Ph-H), 6.13 (t, 1 H, *J* = 6.8 Hz, H-1'), 5.91 (d, 1 H, *J* = 7.4 Hz, H-5), 5.20 (d, 1 H, 3'-OH), 5.10 (t, 1 H, 5'-OH), 4.77 (d, 2 H, Ph-$CH_2$), 4.35 (m, 1 H, H-3'), 4.31 (m, 2 H, $CH_2$), 3.80 (m, 1 H, H-4'), 3.55 (m, 2H, H-5'), 2.10 (m, 1 H, H-2'a), 1.92 (m, 1 H, H-2'b).

*$N^4$-[4-(3-Amino-1-propynyl)-2-nitrobenzyl]-2'-deoxycytidine-5'-triphosphate* ***(dC.10)***

$POCl_3$ (18 μL, 0.2 mmol) was added to a solution of compound **dC.09** (49 mg, 0.1 mmol) and proton sponge (32 mg, 0.15 mmol) in trimethylphosphate (0.5 mL) at 0°C and stirred for two hours. A solution of bis-tri-*n*-butylammonium pyrophosphate (237 mg, 0.5 mmol) and tri-*n*-butylamine (100 uL) in anhydrous DMF (1 mL) was added. After five minutes of stirring, triethylammonium bicarbonate buffer (1 M, pH 7.5; 10 mL) was added. The reaction was stirred for one hour at room temperature, followed by the dropwise addition of concentrated ammonium hydroxide (5 mL, 27%) at 0°C. The mixture was stirred for an additional hour at room temperature and then lyophilized to dryness. The residue was dissolved in water (10 mL), filtered, and purified by anion exchange chromatography using a Q Sepharose FF column (2.5 x 20 cm) with a linear gradient of $NH_4HCO_3$ (50 mM to 500 mM in 300 minutes) at a flow rate of 4.5 mL/min. The fractions containing triphosphate were combined and lyophilized to give the triphosphate **dC.10** (28 mg, 39%) as a white fluffy solid.

*$^1H$ NMR (400 MHz, $D_2O$):* δ 8.22 (s, 1 H, Ph-H), 7.88 (d, 1 *H, J* = 7.6 Hz, H-6), 7.74 (d, 1 H, *J* = 8.0 Hz, Ph-H), 7.59 (d, 1 *H, J* = 8.0 Hz, Ph-H), 6.33 (t, 1 H, *J* = 6.8 Hz, H-1'), 6.18 (d, 1 H, *J* = 7.6 Hz, H-5), 4.61 (m, 1 H, H-3'), 4.24 (m, 3 H, H-4', H-5'), 3.63 (s, 2 H, $CH_2$), 2.42 (m, 1 H, H-2'a), 2.29 (m, 1 H, H-2'b);

*$^{31}P$ NMR (162 MHz, $D_2O$):* δ -5.88 (d, *J* = 15.6 Hz), -10.69 (d, *J* = 15.6 Hz), -19.25 (t, *J* = 15.6 Hz);

*ToF-MS (ESI):* For the molecular ion $C_{19}H_{22}N_5O_{15}P_3Na$ $[M-2H+Na]^-$, the calculated mass was 676.0223, and the observed mass was 676.0563.

*6-TAMRA labeled $N^4$-[4-(3-Amino-1-propynyl)-2-nitrobenzyl]-2'-deoxycytidine-5'-triphosphate* ***(WW2p080)***

A solution of 6-TAMRA-SE (0.75 mg, 1.4 μmol) in anhydrous DMSO (30 μL) was added to a solution of triphosphate **doc.10** (1.6 μmol) in $Na_2CO_3$/$NaHCO_3$ buffer (0.1 M, pH 9.2; 0.3 mL) and incubated at room temperature for 30 minutes. The reaction was purified with reverse-phase HPLC using a Perkin Elmer OD-300 $C_{18}$ column (4.6 x 250 mm) to yield the 6-TAMRA labeled triphosphate **WW2p080.** Mobile phase: A, 100 mM triethylammonium acetate (TEAA) in water (pH 7.0); B, 100 mM TEAA in water/$CH_3CN$ (30:70). HPLC purification was achieved a linear gradient of 5-50% B for 40 minutes and then 50-90% B for 10 minutes. The concentration **of WW2p080** was estimated by adsorption spectroscopy using the extinction coefficient of the 6-TAMRA dye (*i.e.,* 65,000 at 555 nm).

**Synthesis of $N^4$-[1-(2-nitrophenyl)ethyl]-2'-deoxycytidine-5'-triphosphate (WW2p115)**

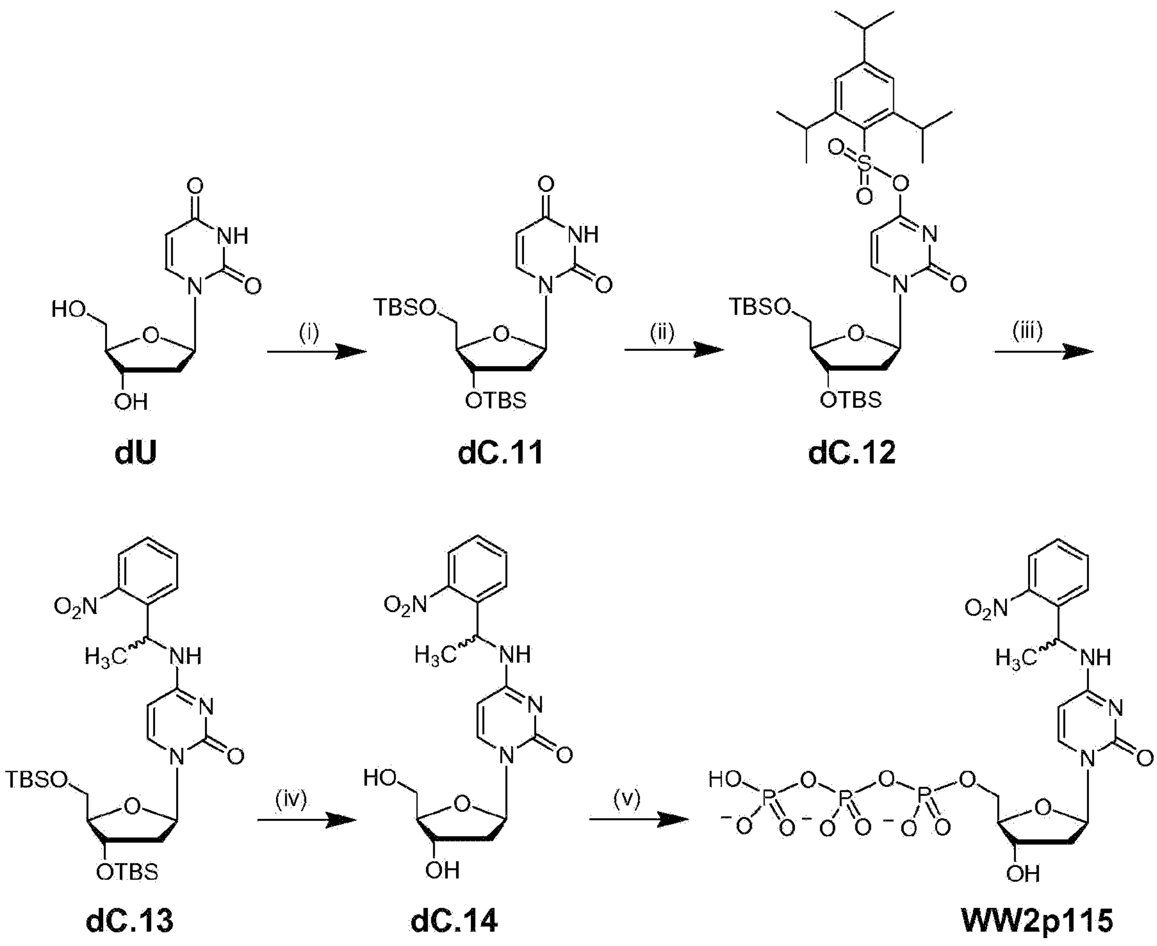


dU dC.11 dC.12

dC.13 dC.14 WW2p115

**Scheme.** Synthesis of *$N^4$-[1-(2-nitrophenyl)ethyl]-2'-deoxycytidine-5'-triphosphate*. (i) TBSCl, imidiazole, DMF, room temperature, three hours, 92%; (ii) TPSCl, DMAP, $CH_2Cl_2$, room temperature, 88%; (iii) 1-(2-nitrophenyl)ethylamine **dC.13c**, DMF, 90°C, 1.5 hours, 39%; (iv) *n*-$Bu_4NF$, THF, 0°C, two hours, then room temperature, one hour, 90%; (v) $POCl_3$, proton sponge, $(MeO)_3PO$, 0°C, two hours; (*n*-$Bu_3NH)_2H_2P_2O_7$, *n*-$Bu_3N$, DMF, five minutes; 1 M $HNEt_3HCO_3$, one hour.

*3',5'-O-Bis-tert-butyldimethylsilyl-2'-deoxyuiridine* ***(dC.11)***

Under a nitrogen atmosphere, a mixture of 2'-deoxyuridine **dU** (2.5 g, 10.95 mmol), TBSCl (7.26 g, 48.2 mmol) and imidiazole (6.56 g, 96.4 mmol) in anhydrous DMF (25 mL) was stirred at 0°C for two hours and then warmed to room temperature over one hour. The reaction mixture was concentrated *in vacuo* and purified by silica gel column chromatography to yield 3',5'-*O*-bis-*tert*-butyldimethylsilyl-2'-deoxyuridine **dC.11** (4.62 g, 92%) as a white foam.

*$^1H$ NMR (400 MHz, $CDCl_3$):* δ 9.48 (s, 1 H, NH), 7.80 (d, 1 H, *J* = 8.1 Hz, H-6), 6.19 (t, 1 H, *J* = 6.4 Hz, H-1'), 5.59 (d, 1 H, *J* = 8.1 Hz, H-5), 4.31 (m, 1 H, H-3'), 3.81 (m, 2 H, H-4' and H-5'a), 3.65 (m, 1 H, H-5'b), 2.21 (m, 1 H, H-2'a), 1.97 (m, 1 H, H-2'b), 0.81 (s, 9 H, $(CH_3)_3CSi$), 0.79 (s, 9 H, $(CH_3)_3CSi$), 0.00 (s, 6 H, $(CH_3)_2Si$), -0.02 (2s, 3 H each, $(CH_3)_2Si$).

*$O^4$-(2,4,6-Triisopropylbenzenesulfonyl)-3',5'-O-bis-tert-butyldimethylsilyl-2'-deoxyuridine* ***(dC.12)***

2,4,6-Triisopropylbenzenesulfonyl chloride (660 mg, 2.19 mmol) was added to a solution of compound **dC.11** (500 mg, 1.09 mmol), DMAP (6.7 mg, catalytic amount) and $Et_3N$ (0.62 mL, 4.38 mmol) in anhydrous $CH_2Cl_2$ (6 mL) under a nitrogen atmosphere. The reaction mixture was stirred overnight at room temperature, concentrated *in vacuo,* and purified by silica gel column chromatography to yield $O^4$-(2,4,6-triisopropylbenzenesulfonyl)-3',5'-*O*-bis-*tert*-butyldimethylsilyl-2'-deoxyuridine **dC.12** (690 mg, 88%) as a white foam.

*$^1H$ NMR (400 MHz, $CDCl_3$):* $\delta$ 8.46 (d, 1 H, *J* = 7.3 Hz, H-6), 7.20 (s, 2 H, Ph-H), 6.08 (dd, 1 H, *J* = 4.3 Hz, H-1'), 6.01 (d, 1 H, *J* = 7.3 Hz, H-5), 4.33 (m, 1 H, H-3'), 4.25 (m, 2 H, CH), 3.94 (m, 2 H, H-4' and H-5'a), 3.76 (m, 1 H, H-5'b), 2.91 (m, 1 H, CH), 2.48 (m, 1H, H-2'a), 2.12 (m, 1 H, H-2'b), 1.31 (d, 6 H, $CH_3$), 1.26 (dd, 12 H, $CH_3$), 0.91 (s, 9 H, $(CH_3)_3CSi$), 0.86 (s, 9 H, $(CH_3)_3CSi$), 0.10 and 0.09 (2 s, 6 H, $(CH_3)_2Si$), 0.04 (s, 6 H, $(CH_3)_2Si$).

*$N^4$-[1-(2-nitrophenyl)ethyl]-3',5'-O-bis-tert-butyldimethylsilyl-2'deoxycytidine* ***(dC.13)***

A solution of compound **dC.12** (410 mg, 0.56 mmol) and 1-(2-nitrophenyl)ethylamine **dC.13c** (470 mg, 2.82 mmol) in anhydrous DMF (3 mL) was heated to 90°C for 1.5 hours and then *concentrated in vacuo.* The residue was dissolved in $CH_2Cl_2$ (50 mL), washed with saturated $NH_4Cl$ solution (30 mL), with water (30 mL), and with saturated $NaHCO_3$ solution (30 mL). The organic layer was dried with $Na_2SO_4$, filtered, concentrated *in vacuo,* and purified by silica gel column chromatography to yield $N^4$-[1-(2-nitrophenyl)ethyl]-3',5'-*O*-bis-*tert*-butyldimethylsilyl-2'deoxycytidine **dC.13** (130 mg, 39%, 1:1 mixture of diastereomers) as a white foam.

*$^1H$ NMR (400 MHz, $CDCl_3$) for diastereomers:* $\delta$ 7.97 (m, 1 H, Ph-H), 7.89 (d, 1 H, *J* = 7.1 Hz, H-6), 7.65 (m, 2 H, Ph-H), 7.41 (m, 1 H, Ph-H), 6.19 (m, 1 H, H-1'), 5.91 (m, 1 H, H-5), 5.37 (m, 1 H, Ph-CH), 4.34 (m, 1 H, H-3'), 3.86 (m, 2 H, H-4' and H-5'a), 3.72 (m, 1 H, H-5'b), 2.05 (m, 1 H, H-2'a), 1.83 (m, 1 H, H-2'b), 1.59 (bs, 3 H, $CH_3$), 0.86 (s, 12 H, $(CH_3)_3CSi$), 0.03 (s, 12 H, $(CH_3)_2Si$).

*$N^4$-[1-(2-nitrophenyl)ethyl]-2'-deoxycytidine* ***(dC.14)***

A solution of *n*-$Bu_4NF$ (142 mg, 0.54 mmol) in THF (3 mL) was added to a solution of compound **dC.13** (130 mg, 0.22 mmol) in THF (2 mL) at 0°C. The reaction mixture was stirred at 0°C for two hours and then at room temperature for one hour, concentrated *in vacuo,* and purified by silica gel column chromatography to yield $N^4$-[1-(2-nitrophenyl)ethyl]-2'-deoxycytidine **doc.14** (80 mg, 90%, 1:1 mixture of diastereomers) as a white powder.

*$^1H$ NMR (400 MHz, $CD_3OD$) for diastereomers:* $\delta$ 7.95 (m, 2 H, Ph-H and H-5), 7.65 (m, 2 H, Ph-H), 7.45 (m, 1 H, Ph-H), 6.22 (m, 1 H, H-1'), 5.97 (m, 1 H, H-5), 5.74 (m, 1 H, Ph-CH), 4.34 (m, 1 H, H-3'), 3.93 (m, 1 H, H-4'), 3.75 (m, 2 H, H-5'), 2.32 (m, 1 H, H-2'a), 2.09 (m, 1 H, H-2'b), 1.59 (m, 3 H, $CH_3$).

*$N^4$-[1-(2-nitrophenyl)ethyl]-2'-deoxycytidine-5'-triphosphate* ***(WW2p115)***

$POCl_3$ (14 $\mu$L, 0.15 mmol) was added to a solution of compound **dC.14** (28 mg, 0.08 mmol) and proton sponge (23 mg, 0.11 mmol) in trimethylphosphate (0.5 mL) at 0°C and stirred for two hours. A solution of bis-tri-*n*-butylammonium pyrophosphate (180 mg, 0.38 mmol) and tri-*n*-butylamine (80 $\mu$L) in anhydrous DMF (0.8 mL) was added. After five minutes of stirring, triethylammonium bicarbonate buffer (1 M, pH 7.5; 10 mL) was added. The reaction was stirred for one hour at room temperature and then lyophilized to dryness. The residue was dissolved in water (10 mL), filtered, and purified by anion exchange chromatography using a Q Sepharose FF column (2.5 x 20 cm) with a linear gradient of $NH_4HCO_3$ (50 mM to 500 mM in 300 minutes) at a flow rate of 4.5 mL/min. The fractions containing triphosphate were combined and lyophilized to give the triphosphate **WW2p115** (24 mg, 47%, 1:1 mixture of diastereomers) as a white fluffy solid.

*$^1H$ NMR (400 MHz, $D_2O$) for diastereomers:* $\delta$ 7.98 (m, 1 H, Ph-H), 7.80 (m, 1 H, H-6), 7.66 (m, 2 H, Ph-H), 7.49 (m, 1 H, Ph-H), 6.24 (m, 1 H, H-1'), 6.11 (m, 1 H, H-5), 5.60 (m,1 H, Ph-CH), 4.55 (m, 1 H, H-3'), 4.19 (m, 3 H, H-4' and H-5'), 2.35 (m, 1 H, H-2'a), 2.24 (m, 1 H, H-2'b), 1.59 (d, 3 H, *J* = 6.7 Hz, $CH_3$);

*$^{31}P$ NMR (162 MHz, $D_2O$) for diastereomers:* $\delta$ -6.00 (d, *J* = 14.1 Hz), -10.82 (d, *J* = 15.6 Hz), -19.36 (t, *J* = 15.9 Hz);

*ToF-MS (ESI):* For the molecular ion $C_{17}H_{23}N_4O_{15}P_3Na$ $[M+Na]^+$, the calculated mass was 639.0271, and the observed mass was 639.0332.

**Synthesis of 1-(2-nitrophenyl)ethylamine (dC.13c)**

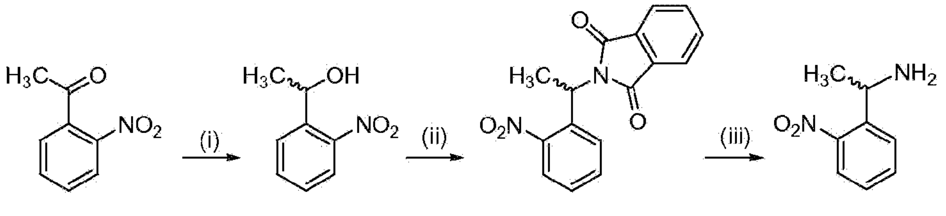


NAP dC.13a dC.13b dC.13c

**Scheme for dC.13c.** Synthesis of *1-(2-nitrophenyl)ethylamine.* (i) $NaBH_4$, MeOH, dioxane, room temperature, one hour, 92%; (ii) Phthalimide, DIAD, $Ph_3P$, THF, 0°C, three hours, 99%; (iii) $NH_2NH_2$, $CH_3CH_2OH$, reflux, one hour, 80%.

*1-(2-Nitrophenyl)ethanol **(dC.13a)***

$NaBH_4$ (3.24 g, 85.60 mmol) was slowly added to a solution of 2'-nitroacetophenone **NAP** (3.74 g, 22.65 mmol) in a mixture of methanol (34 mL) and dioxane (22 mL). The reaction mixture was stirred at room temperature for one hour and then diluted with ethyl acetate (100 mL) and washed with water (25 mL). The organic layer was dried with $Na_2SO_4$, filtered, and concentrated *in vacuo* to yield 1-(2-nitrophenyl)ethanol **dC.13a** (3.49 g, 92%) as a white powder.

*$^1H$ NMR (400 MHz, $CDCl_3$):* δ 7.89 (d, 1 H, *J* = 8.1 Hz, Ph-H), 7.83 (d, 1 H, *J* = 7.4 Hz, Ph-H), 7.65 (t, 1 H, *J* = 7.4 Hz, Ph-H), 7.42 (t, 1 H, *J* = 8.1 Hz, Ph-H), 5.41 (q, 1 H, *J* = 6.0 Hz, Ph-CH), 2.48 (s, 1 H, OH), 1.57 (d, 3 H, *J* = 6.4 Hz, $CH_3$).

*N-[1-(2-nitrophenyl)ethyl]phthalimide **(dC.13b)***

Phthalimide (660 mg, 4.5 mmol) was added to a solution of compound **dC.13a** (750 mg, 4.5 mmol) and $Ph_3P$ (1.41 g, 5.4 mmol) in THF (12 mL). The suspension was cooled to 0°C and stirred for 10 minutes, and then diisopropyl azodicarboxylate (1.1 mL, 5.4 mmol) was added dropwise. After stirring at 0°C for three hours, the reaction mixture was concentrated *in vacuo* and purified by silica gel column chromatography to yield *N*-[1-(2-nitrophenyl)ethyl]phthalimide **dC.13b** (1.33 g, 99%) as a brown oil.

*$^1H$ NMR (400 MHz, $CDCl_3$):* δ 7.93 (d, 1 H. *J* = 7.9 Hz, Ph-H), 7.81 (m, 3 H, Ph-H), 7.71 (m, 2 H, Ph-H), 7.61 (t, 1 H, *J* = 7.6 Hz, Ph-H), 7.44 (t, 1 H, *J* = 7.6 Hz, Ph-H), 6.08 (q, 1 H, *J* = 7.2 Hz, Ph-CH), 1.97 (d, 3 H, *J* = 7.2 Hz, $CH_3$).

*1-(2-nitrophenyl)ethylamine **(dC.13c)***

Compound **dC.13b** (1.33 g, 4.5 mmol) was dissolved in ethanol (21 mL) upon heating to 50°C and then cooled to room temperature. Hydrazine (0.55 mL, 11.22 mmol) was added, and the reaction mixture was refluxed for one hour and then cooled with ice. Diethyl ether (40 mL) was added to precipitate the compound, which was isolated by filtration and washed two times with diethyl ether (40 mL each). The combined filtrate was washed two times with water (40 mL each) and then with brine (40 mL). The organic layer was dried over $Na_2SO_4$ and concentrated *in vacuo* to yield 1-(4-iodo-2-nitrophenyl)ethylamine **dC.13c** (600 mg, 80%) as a brown oil.

*$^1H$ NMR (400 MHz, $CDCl_3$):* δ 7.78 (m, 2 H. Ph-H), 7.61 (t, 1H, *J* = 7.6 Hz, Ph-H), 7.37 (t, 1 H, *J* = 7.6 Hz, Ph-H), 4.59 (q, 1 H, *J* = 6.4 Hz, Ph-CH), 1.45 (d, 3 H, *J* = 6.4 Hz, $CH_3$).

**Synthesis of *N*[4]-[1-(2-nitrophenyl)ethyl]-cytidine-5'-triphosphate (WW2p152 and and WW3p026)**

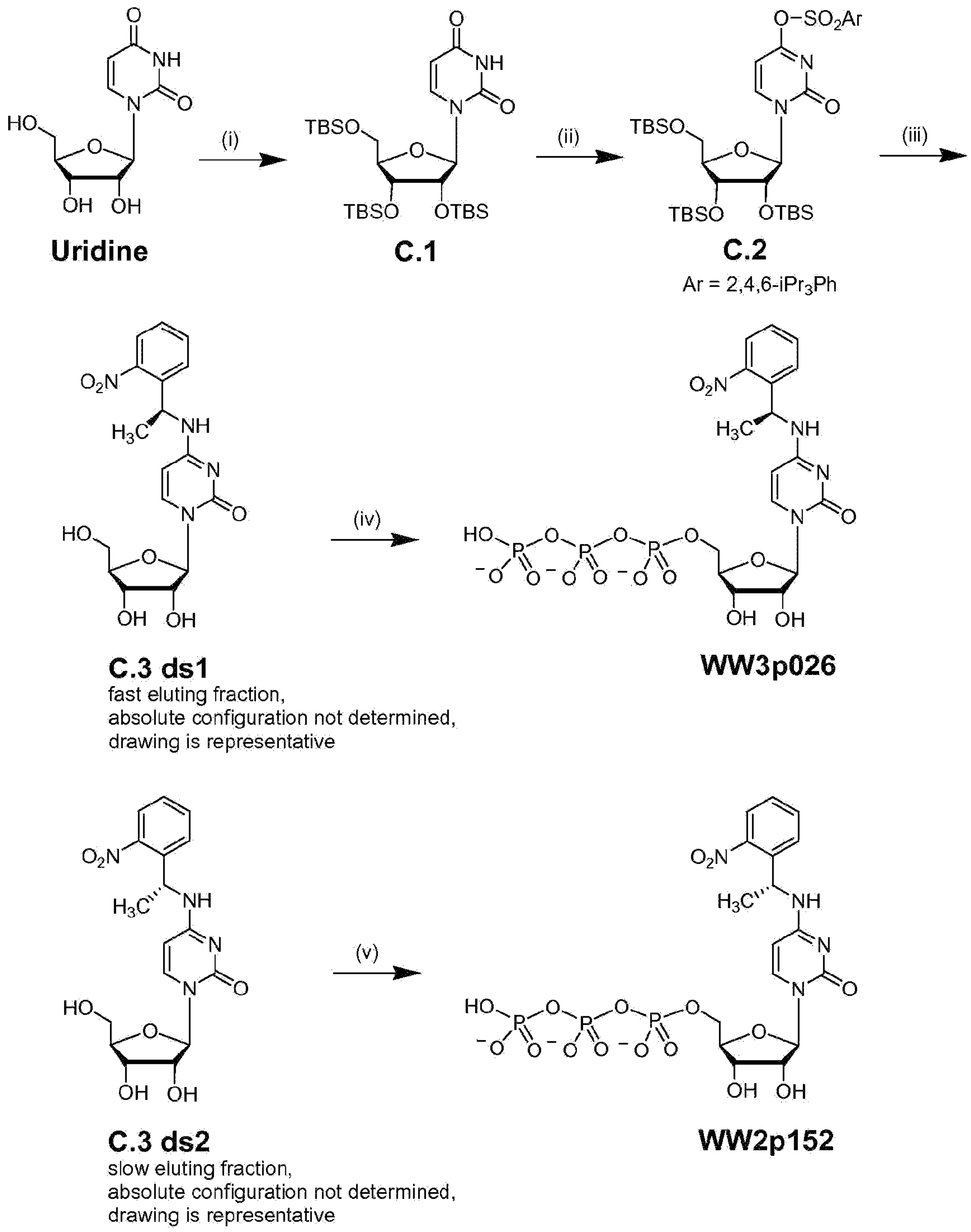


**Scheme.** Synthesis of *$N^4$-[1-(2-nitrophenyl)ethyl]-cytidine-5'-triphosphate.* (i) TBSCl, imidiazole, DMF, room temperature, 60 hours, 95%; (ii) TPSCl, $Et_3N$, DMAP, $CH_2Cl_2$, room temperature, overnight, 80%; (iii) 1-(2-nitrophenyl)ethylamine, DMF, 90°C, 45 minutes; *n*-$Bu_4NF$, THF, 0°C, then gradually warmed to room temperature, two hours; 60% for two diastereoisomers; (iv) $POCl_3$, proton sponge, $(MeO)_3PO$, 0°C, two hours; $(n\text{-}Bu_3NH)_2H_2P_2O_7$, *n*-$Bu_3N$, DMF, five minutes; 1 M $HNEt_3HCO_3$, one hour.

*2',3',5'-O-Tris-tert-butyldimethylsilyl-uridine **(C.1)***

TBSCl (995 mg, 6.6 mmol) was added to a solution of uridine (244 mg, 1 mmol) and imidiazole (898 mg, 13.2 mmol) in anhydrous DMF (5 mL) at 0°C under a nitrogen atmosphere. The mixture was warmed to room temperature and stirred for 60 hours. Ethyl acetate (30 mL) was added, and the mixture was washed two times with saturated $NH_4Cl$ solution (20 mL cach) and with water (20 mL), dried over $Na_2SO_4$, concentrated and purified by silica gel column chromatography to yield 2',3',5'-O-tris-*tert*-butyldimethylsilyl-uridine **C.1** (558 mg, 95%) as a white foam.

*$^1H$ NMR (400 MHz, $CDCl_3$):* δ 8.79 (s, 1 H, NH), 8.03 (d, 1 H, *J* = 8.2 Hz, H-6), 5.87 (d, 1 *H, J* = 3.5 Hz, H-1'), 5.68 (d, 1 H, *J* = 8.2 Hz, H-5), 4.08 (m, 3 H, H-2', H-3' and H-4'), 3.99 (d, 1 H, *J* = 11.6 Hz, H-5'a), 3.77 (d, 1 H, *J* = 11.6 Hz, H-5'b), 0.95 (s, 9 H, $(CH_3)_3CSi$), 0.91 and 0.90 (2 s, 18 H, $(CH_3)_3CSi$), 0.09 (5 s, 18 H, $(CH_3)_2Si$).

*$O^4$-(2,4,6-Triisopropylbenzenesufonyl)-2',3',5'-O-tris-tert-butyldimethylsilyl-uridine **(C.2)***

2,4,6-Triisopropylbenzenesulfonyl chloride (557 mg, 1.84 mmol) was added to a solution of compound **C.1** (540 mg, 0.92 mmol), DMAP (12 mg, catalytic amount) and $Et_3N$ (0.5 mL, 3.68 mmol) in anhydrous $CH_2Cl_2$ (10 mL) under a nitrogen atmosphere. The mixture was stirred overnight at room temperature, $CH_2Cl_2$ (20 mL) was added, washed with saturated $NH_4Cl$ solution (15 mL), dried over $Na_2SO_4$, concentrated and purified by silica gel column chromatography to yield *$O^4$*-(2,4,6-triisopropyl-benzenesulfonyl)-2',3',5'-*O*-tris-*tert*-butyldimethylsilyl-uridine **C.2** (629 mg, 80%) as a white foam.

*$^1H$ NMR (400 MHz, $CDCl_3$):* δ 8.62 (d, 1 H, *J* = 7.3 Hz, H-6), 7.20 (s, 2 H, Ph-H), 5.99 (d, 1 *H, J* = 7.3 Hz, H-5), 5.68 (d, 1 H, *J* = 1.0 Hz, H-1'), 4.23 (m, 2 H, CH), 4.09 (m, 3 H, H-2', H-3' and H-4'), 4.00 (m, 1 H, H-5'a), 3.78 (1 H, d, *J* = 11.8 Hz, H-5'b), 2.90 (m, 1 H, CH), 1.31 (d, 6 H, $CH_3$), 1.26 (dd, 12 H, $CH_3$), 0.94 (s, 9 H, $(CH_3)_3CSi$), 0.88 (2 s, 18 H, $(CH_3)_3CSi$), 0.17 - 0.05 (6 s, 18 H, $(CH_3)_2Si$).

*$N^4$-[1-(2-Nitrophenyl)ethyl]-cytidine (single diastereoisomer **C.3 ds1** and **C.3** ds2)*

A solution of compound C.2 (476 mg, 0.56 mmol) and 1-(2-nitrophenyl)ethylamine **dC.13c** (498 mg, 3 mmol) in anhydrous DMF (4 mL) was heated at 90°C for 45 minutes. Ethyl acetate (40 mL) was added, the mixture was washed with saturated $NH_4Cl$ solution (20 mL) and water (20 mL), and dried with $Na_2SO_4$, concentrated. The two diastereoisomers were separated by silica gel column chromatography to yield *$N^4$*-[1-(2-nitrophenyl)ethyl]-2',3',5'-*O*-bis-*tert*-butyldimethylsilyl-cytidine *single diastereo-isomer ds1 (fast eluting, 290 mg) and ds2 (slow eluting, 203 mg).* Both *single diastereoisomers* were used in the next step without further purification.

*$N^4$-[(R or S)-1-(2-nitrophenyl)ethyl]-cytidine (single diastereoisomer **C.3 ds1)***

A solution of *n*-$Bu_4NF$ (235 mg, 0.9 mmol) in THF (3 mL) was added to a solution of *$N^4$*-[1-(2-nitrophenyl)ethyl]-2',3',5'-*O*-bis-*tert*-butyldimethylsilyl-cytidine single diastereo-isomer ds1 (264 mg) in THF (4 mL) at 0°C. The reaction mixture was gradually warmed to room temperature and stirred for two hours. Silica gel 60 (1 g) was added, and the mixture was evaporated *in vacuo* to dryness. The residue was purified by silica gel column chromatography to yield *$N^4$*-[*(R or S)*-1-(2-nitrophenyl)ethyl]-cytidine single diastereoisomer **C.3 ds1** (65 mg, ca. 30% for two steps, absolute configuration not determined) as a white foam.

*$^1H$ NMR (400 MHz, DMSO-$d_6$):* δ 8.39 (d, 1 H, $D_2O$ exchangeable, NH), 7.92 (dd, 1 H, *J* = 1.1 and 8.1 Hz, Ph-H), 7.82 (d, 1 H, *J* = 7.5 Hz, H-6), 7.70 (dt, 1 H, *J* = 1.1 and 7.5 Hz, Ph-H), 7.64 (dd, 1 H, *J* = 1.2 and 7.5 Hz, Ph-H), 7.49 (dt, 1 H, *J* = 1.3 and 7.5 Hz, Ph-H), 1 H, Ph-H), 5.80 (d, 1 H, *J* = 7.5 Hz, H-5), 5.65 (d, 1 H, *J* = 3.4 Hz, H-1'), 5.50 (m, 1 H, Ph-CH), 5.30 (d, 1 H, $D_2O$ exchangeable, 3'-OH), 5.02 (t, 1 H, $D_2O$ exchangeable, 5'-OH), 4.96 (d, 1 H, $D_2O$ exchangeable, 2'-OH), 3.90 (m, 2 H, H-2' and H-3'), 3.78 (m, 1 H, H-4'), 3.59 (m, 1 H, H-5'a), 3.50 (m, 1 H, H-5'b), 1.48 (d, 3 H, *J* = 6.9 Hz, $CH_3$).

*$N^4$-[(S or R)-1-(2-nitrophenyl)ethyl]-cytidine (single diastereoisomer **C.3** ds2)*

A solution of *n*-$Bu_4NF$ (167 mg, 0.64 mmol) in THF (2 mL) was added to a solution of *$N^4$*-[1-(2-nitrophenyl)ethyl]-2',3',5'-*O*-bis-*tert*-butyldimethylsilyl-cytidine single diastereo-isomer ds2 (188 mg) in THF (3 mL) at 0°C. The reaction mixture was gradually warmed to room temperature and stirred for one hour. Silica gel 60 (1 g) was added, and the mixture was evaporated *in vacuo* to dryness. The residue was purified by silica gel column chromatography to yield *$N^4$*-[(*S or R*)-1-(2-nitrophenyl)ethyl]-cytidine single diastereoisomer **C.3 ds2** (67 mg, ca. 30% for two steps, absolute configuration not determined) as a white foam.

*$^1H$ NMR (400 MHz, DMSO-$d_6$):* δ 8.38 (d, 1 H, $D_2O$ exchangeable, NH), 7.92 (dd, 1 H, *J* = 1.1 and 8.1 Hz, Ph-H), 7.80 (d, 1 H, *J* = 7.5 Hz, H-6), 7.72 (dt, 1 H, *J* = 1.0 and 7.5 Hz, Ph-H), 7.63 (dd, 1 H, *J* = 1.1 and 7.5 Hz, Ph-H), 7.49

(dt, 1 H, *J* = 1.3 and 7.5 Hz, Ph-H), 5.79 (d, 1 H, *J* = 7.5 Hz, H-5), 5.68 (d, 1 H, *J* = 4.0 Hz, H-1'), 5.51 (m, 1 H, Ph-CH), 5.20 (d, 1 H, $D_2O$ exchangeable, 3'-OH), 5.01 (t, 1 H, $D_2O$ exchangeable, 5'-OH), 4.93 (d, 1 H, $D_2O$ exchangeable, 2'-OH), 3.87 (m, 2 H, H-2' and H-3'), 3.78 (m, 1 H, H-4'), 3.59 (m, 1 H, H-5'a), 3.50 (m, 1 H, H-5'a), 1.49 (d, 3 H, *J* = 6.9 Hz, $CH_3$).

*$N^4$-[(R or S)-1-(2-Nitrophenyl)ethyl]-cytidine-5'-triphosphate single diastereoisomer,* ***(WW3p026)***

$POCl_3$ (14 μL, 0.15 mmol) was added to a solution of compound **C.3 ds1** (29 mg, 0.074 mmol) and proton sponge (32 mg, 0.15 mmol) in trimethylphosphate (0.5 mL) at 0°C and stirred for two hours. A solution of bis-tri-*n*-butylammonium pyrophosphate (175 mg, 0.37 mmol) and tri-*n*-butylamine (74 μL) in anhydrous DMF (0.74 mL) was added. After five minutes of stirring, triethylammonium bicarbonate buffer (1 M, pH 7.5; 10 mL) was added. The reaction was stirred for one hour at room temperature and then lyophilized to dryness. The residue was dissolved in water (10 mL), and part of the solution was purified with reverse-phase HPLC using a Perkin Elmer OD-300 $C_{18}$ column (4.6 x 250 mm) to yield $N^4$-[(*R or S*)-1-(2-nitrophenyl)ethyl]-cytidine-5'-triphosphate single diastereoisomer **WW3p026** (absolute configuration not determined). Mobile phase: A, 100 mM triethylammonium acetate (TEAA) in water (pH 7.0); B, 100 mM

TEAA in water/$CH_3CN$ (30:70). HPLC purification was achieved a linear gradient of 5-50% B for 20 minutes and then 50-90% B for 10 minutes.

*$^1H$ NMR (400 MHz, $D_2O$):* δ 8.0 (d, 1 H, *J* = 8.1 Hz, Ph-H), 7.92 (d, 1 H, *J* = 7.6 Hz, H-6), 7.70 (m, 2 H, Ph-H), 7.50 (t, 1 H, *J* = 8.0 Hz, Ph-H), 6.14 (d, 1 H, *J* = 7.6 Hz, H-5), 5.92 (d, 1 H, *J* = 4.1 Hz, H-1'), 5.61 (q, 1 H, *J* = 6.8 Hz, Ph-CH), 4.33 - 4.21 (m, 3 H, H-2',

H-3' and H-4'), 4.01 (m, 2 H, H-5'), 1.60 (d, 3 H, *J* = 6.8 Hz, $CH_3$);

*$N^4$-[(S or R)-1-(2-nitrophenyl)ethyl]-cytidine-5'-triphosphate single diastereoisomer* ***(WW2p152)***

$POCl_3$ (11 μL, 0.12 mmol) was added to a solution of compound **C.3 ds2** (31 mg, 0.08 mmol) and proton sponge (26 mg, 0.12 mmol) in trimethylphosphate (0.5 mL) at 0°C and stirred for two hours. A solution of bis-tri-*n*-butylammonium pyrophosphate (190 mg, 0.4 mmol) and tri-*n*-butylamine (80 μL) in anhydrous DMF (0.8 mL) was added. After five minutes of stirring, triethylammonium bicarbonate buffer (1 M, pH 7.5; 10 mL) was added. The reaction was stirred for one hour at room temperature and then lyophilized to dryness. The residue was dissolved in water (10 mL), filtered, and purified by anion exchange chromatography using a Q Sepharose FF column (2.5 x 20 cm) with a linear gradient of $NH_4HCO_3$ (50 mM to 500 mM in 240 minutes) at a flow rate of 4.5 mL/min. The fractions containing triphosphate were combined and lyophilized to yield $N^4$-[(*S or R*)-1-(2-nitrophenyl)ethyl]-cytidine-5'-triphosphate single diastereoisomer **WW2p152** (26 mg, 47%, absolute configuration not determined) as a white fluffy solid.

*$^1H$ NMR (400 MHz, $D_2O$):* δ 7.99 (d, 1 H, *J* = 8.2 Hz, Ph-H), 7.82 (d, 1 H, *J* = 7.6 Hz, H-6), 7.68 (m, 2 H, Ph-H), 7.50 (t, 1 H, *J* = 7.8 Hz, Ph-H), 6.12 (d, 1 H, *J* = 7.5 Hz, H-5), 5.91 (d, 1 H, *J* = 4.4 Hz, H-1'), 5.60 (q,1 H, *J* = 6.8 Hz, Ph-CH), 4.26 (m, 5 H, H-2', H-3', H-4' and H-5'), 1.60 (d, 3 H, *J* = 6.8 Hz, $CH_3$);

*$^{31}P$ NMR (162 MHz, $D_2O$):* δ -5.18(d, *J* = 19.8 Hz), -10.46 (d, *J* = 19.1 Hz), -20.98 (t, *J* = 19.6Hz);

*ToF-MS (ESI):* For the molecular ion $C_{17}H_{21}N_4O_{16}P_3Na$ $[M-2H+Na]^-$, the calculated mass was 653.0063, and the observed mass was 652.9975.

**Synthesis of 5-[1-(2-nitrophenyl)ethyloxymethyl]-2'-deoxycytidine-5'-triphosphate (WW3p###)**

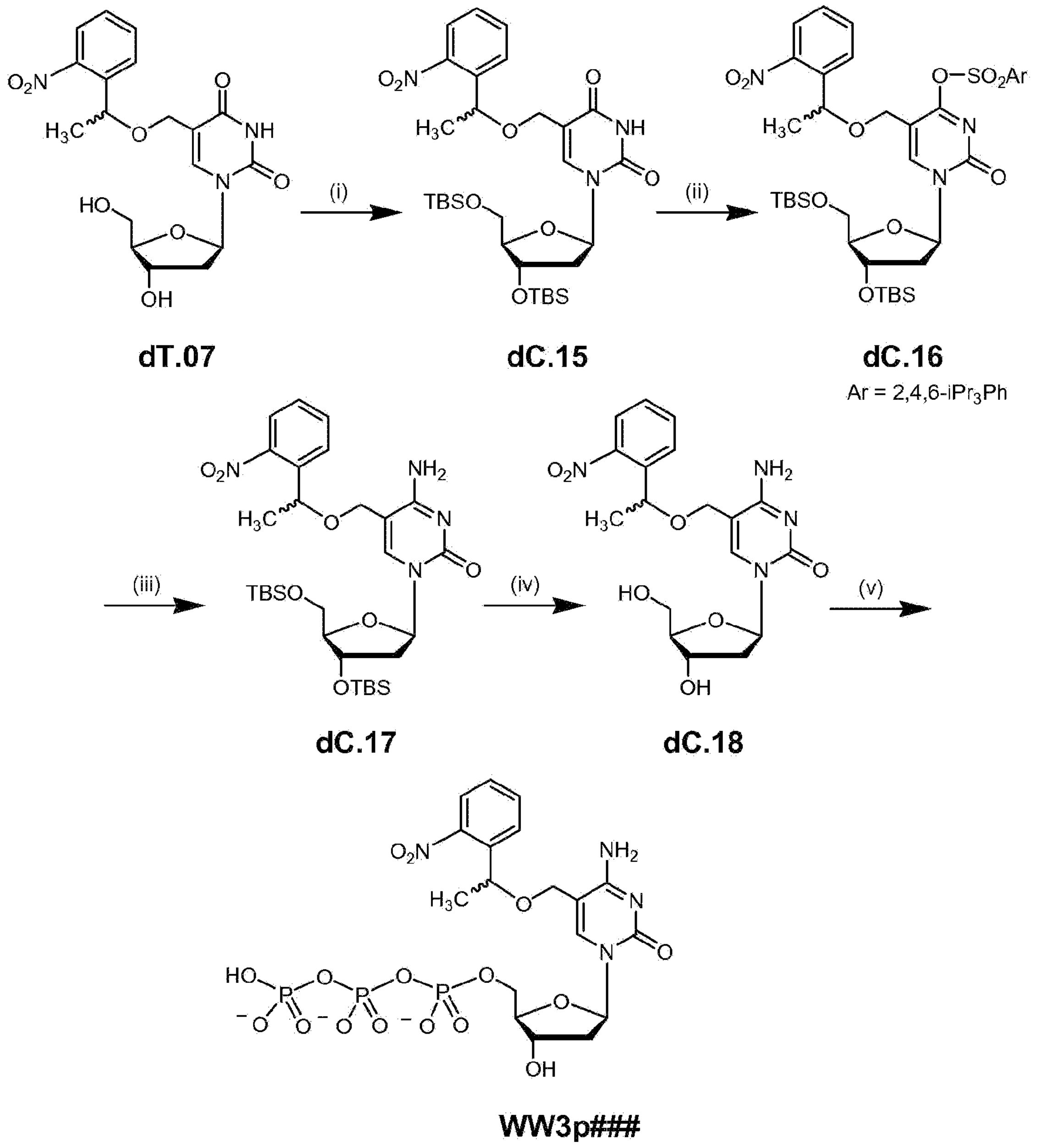


dT.07 dC.15 dC.16

dC.17 dC.18

WW3p###

**Scheme.** Synthesis of *5-[1-(2-nitrophenyl)ethyloxymethyl]-2'-deoxycytidine-5'-triphosphate.* (i) TBSCl, imidazole, DMF, room temperature, 24 hours, 42%; (ii) TPSCl, DMAP, $Et_3N$, $CH_2Cl_2$, room temperature, 48 hours, 56%; (iii) $NH_3$, 1,4-dioxane, 80°C, two hours, 56%; (iv) *n*-$Bu_4NF$, THF, room temperature,; (v) $POCl_3$, proton sponge, $(MeO)_3PO$, 0°C,; $(n\text{-}Bu_3NH)_2H_2P_2O_7$, *n*-$Bu_3N$, DMF, five minutes; 1 M $HNEt_3HCO_3$, one hour.

*3',5'-Bis-tert-butyldimethylsilyl-5-[1-(2-nitrophenyl)ethyloxymethyl]-2'-deoxyurdine* ***(dC.15)***

A solution of TBSCl (114 mg, 0.76 mmol) in anhydrous DMF (0.5 mL) was added to a solution of compound **dT.07** (97 mg, 0.24 mmol) and imidiazole (103 mg, 1.52 mmol) in anhydrous DMF (1 mL). The mixture was stirred at room temperature for 24 hours under a nitrogen atmosphere, then concentrated *in vacuo* and purified by silica gel column

chromatography to yield 3',5'-bis-*tert*-butyldimethylsilyl-5-[1-(2-nitrophenyl)-ethyloxymethyl]-2'-deoxyurdine **dC.15** (64 mg, 42%, 1:1 mixture of diastereomers).

*$^1$H NMR (400 MHz, $CDCl_3$) for diastereomers:* δ 9.39 and 9.33 (2 br s, 1 H, NH), 7.92 (m, 2 H, Ph-H), 7.70 (m, 2 H, H-6 and Ph-H), 7.42 (m, 1 H, Ph-H), 6.29 (m, 1 H, H-1'), 5.11 (m, 1 H, Ph-CH), 4.41 (m, 1 H, H-3'), 4.04 (m, 2 H, $CH_2$), 3.96 (m, 1 H, H-4'), 3.80 (m, 2 H, H-5'), 2.38 (m, 1 H, H-2'a), 2.04 (m, 1 H, H-2'b), 1.55 (m, 3 H, $CH_3$), 0.91 (s, 18 H, $(CH_3)_3CSi$), 0.10 (s, 12 H, $(CH_3)_2Si$);

*$^{13}$C NMR (100 MHz, $CDCl_3$) for diastereomers:* δ 162.89/162.76 (C), 150.15 (C), 148.40 (C), 139.21/139.17 (C), 138.94/138.68 (CH), 133.86/133.76 (CH), 128.22/128.19 (CH), 128.17/128.07 (CH), 124.23 (CH), 111.30/111.22 (C), 87.97/87.94 (CH), 85.43/85.37 (CH), 73.46/73.43 (CH), 72.34/72.28 (CH), 63.92/63.77 ($CH_2$), 63.07 ($CH_2$), 41.27/41.17 ($CH_2$), 25.97/25.93 ($CH_3$), 23.69/23.57 ($CH_3$), 18.43/18.41 (C), -4.64/-4.82 ($CH_3$), -5.37/-5.40 ($CH_3$).

*ES+ MS (ESI):* 658 $[M+Na]^+$;

*$O^4$-(2,4,6-Triisopropylbenzenesulfonyl)-3';5'-bis-tert-butyldimethylsilyl-5-[1-(2-nitrophenyl)ethyloxymethyl]-2'-deoxyurdine **(dC.16)***

A solution of 2,4,6-triisopropylbenzenesulfonyl chloride (61 mg, 0.20 mmol) was added to a solution of **dC.15** (64 mg, 0.10 mmol) and DMAP (6 mg, 0.05 mmol) in anhydrous $CH_2Cl_2$ (3 mL) followed by $Et_3N$ (63 μL, 0.45 mmol). The mixture was stirred at room temperature for 48 hours under a nitrogen atmosphere, then concentrated *in vacuo* and purified by silica gel column chromatography to give $O^4$-(2,4,6-triisopropylbenzenesulfonyl)-3',5'-bis-*tert*-butyldimethylsilyl-5-[1-(2-nitrophenyl)ethyloxy-methyl]-2'-deoxyurdine **dC.16** (50 mg, 56%, 1:1 mixture of diastereomers).

*$^1$H NMR (400 MHz, $CDCl_3$) for diastereomers:* δ 8.33 and 8.28 (2 s, 1 H, Ph-H), 7.90 (m, 3 H, Ph-H), 7.67 (m, 2 H, H-6 and Ph-H), 7.44 (m, 1 H, Ph-H), 6.27 (m, 1 H, H-1'), 5.11 (m, 1 H, Ph-CH), 4.40 (m, 1 H, H-3'), 4.06 (m, 2 H, $CH_2$), 3.97 (m, 1 H, H-4'), 3.79 (m, 2 H, H-5'), 2.38 (m, 1 H, H-2'a), 2.04 (m, 1 H, H-2'b), 1.54 (m, 3 H, $CH_3$), 1.60 (m, 3 H, CH), 0.91 (s, 18 H, $(CH_3)_3CSi$), 0.80 (m, 18 H, $CH_3$), 0.09 (s, 12 H, $(CH_3)_2Si$);

*3',5'-Bis-tert-butyldimethylsilyl-5-[1-(2-nitrophenyl)ethyloxymethyl]-2'-deoxycytidine* **(*dC.17*)**

A solution of $NH_3$ (1 mL, 0.5 M in dioxane) was added to a solution of compound **dC.16** (48 mg, 0.05 mmol) in anhydrous 1,4-dioxane (1 mL). The mixture was stirred at 80°C for two hours, then concentrated *in vacuo* and purified by silica gel column chromatography to give 3',5'-bis-tert-butyldimethylsilyl-5-[1-(2-nitrophenyl)ethyloxy-methyl]-2'-deoxycytidine **dC.18** (25 mg, 58%, 1:1 mixture of diastereomers).

Example 4: dG compounds

**Synthesis of *$N^2$*-(2-nitrobenzyl)-2'-deoxyguanidine-5'-triphosphate (WW2p067)**

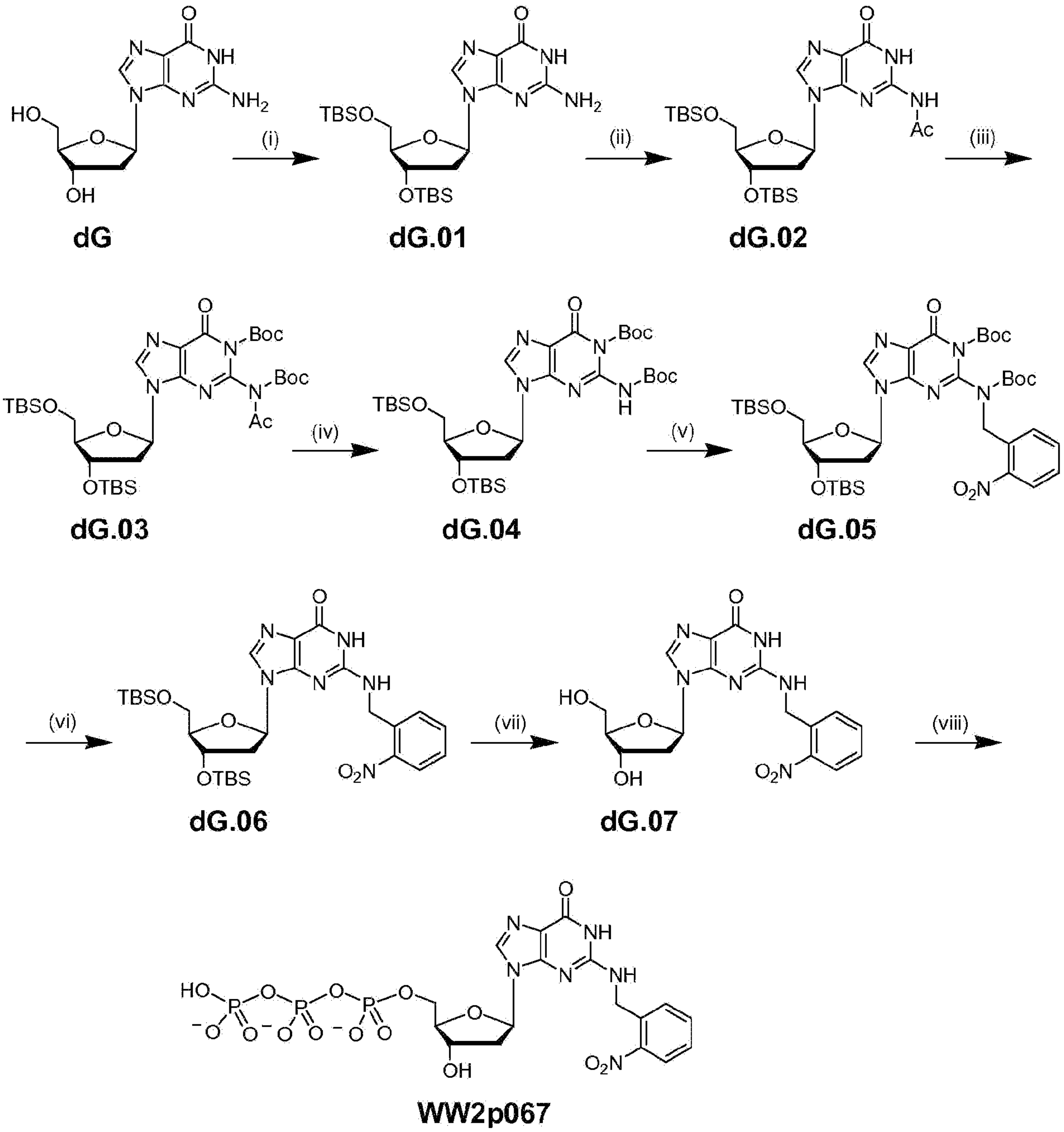


dG dG.01 dG.02

dG.03 dG.04 dG.05

dG.06 dG.07

WW2p067

**Scheme**. Synthesis of *$N^2$-(2-nitrobenzyl)-2'-deoxyguanidine-5'-triphosphate*. (i) TBSCl, imidazole, anhydrous DMF, 0°C, then gradually warmed to room temperature, overnight, 96%; (ii) $Ac_2O$, anhydrous pyridine, room temperature, 100°C, 72%; (iii) $Boc_2O$, $Et_3N$, DMAP, $CH_2Cl_2$, reflux, two hours, 54%; (iv) $K_2CO_3$, MeOH, 95%; (v). 2-nitrobenzylbromide, NaH, DMF, 0°C, then gradually warmed to room temperature, one hour, 91%; (vi) $SiO_2$, vacuum, 70-80°C, 48 hours, 97%; (vii) *n*-$Bu_4NF$, THF, 0°C, one hour, then room temperature, two hours, 83%; (viii) $POCl_3$, $(MeO)_3PO$, minus 20-30°C, two hours; $(n\text{-}Bu_3NH)_2H_2P_2O_7$, *n*-$Bu_3N$, DMF, five minutes; 1 M $HNEt_3HCO_3$, one hour, 62%.

*3',5'-O-Bis-tert-butyldimethylsilyl-2'-deoxyguanosine* ***(dG.01)***

2'-deoxyguanosine **dG** (0.89 g, 3.30 mmol), imidiazole (2.0 g, 29.32 mmol), and TBSCl (2.34 g, 15.55 mmol) were added to anhydrous DMF (8 mL) at 0°C under a $N_2$ atmosphere. The reaction mixture was gradually warmed to room temperature and stirred overnight. The mixture was then concentrated *in vacuo*, treated with a mixture of $CHCl_3$ (8 mL) and MeOH (8 mL), concentrated *in vacuo*, and purified by silica gel chromatography to yield 3',5'-*O*-bis-*tert*-butyldimethylsilyl-2'-deoxyguanosine **dG.01** (1.57 g, 96%) as a white powder.

*$^1H$ NMR (400 MHz, DMSO-$d_6$):* δ 10.60 (br s, 1 H, H-1), 7.88 (s, 1 H, H-8), 6.47 (br s, 2 H, $NH_2$), 6.10 (t, 1 *H, J* = 6.8 Hz, H-1'), 4.48 (m, 1 H, H-3'), 3.80 (m, 1 H, H-4'), 3.70 (m, 2 H, H-5'a and H-5'b), 2.64 (m, 1 H, H-2'a), 2.18 (m, 1 H, H-2'b), 0.89 (s, 9 H, $(CH_3)_3CSi$), 0.86 (s, 9 H, $(CH_3)_3CSi$), 0.11 (s, 6 H, $(CH_3)_2Si$), 0.03 (s, 6 H, $(CH_3)_2Si$).

*$N^2$-Acetyl-3',5'-O-bis-tert-butyldimethylsilyl-2'-deoxyguanosine* ***(dG.02)***

Acetic anhydride (1.22 mL, 12.92 mmol) was slowly added to a solution of compound **dG.01** (0.51 g, 1.03 mmol) in anhydrous pyridine (10 mL) at room temperature and stirred at 100°C for three hour. The reaction was *concentrated in vacuo*, co-evaporated three times with anhydrous ethanol (15 mL), and purified by silica gel chromatography to yield *$N^2$*-acetyl-3',5'-*O*-bis-*tert*-butyldimethylsilyl-2'-deoxyguanosine **dG.02** (0.34 g, 56%) as a white foam.

*$^1H$ NMR (400 MHz, DMSO-$d_6$):* δ 12.03 (s, 1 H, NH), 11.70 (s. 1 H, NH), 8.20 (s, 1 H, H-8), 6.19 (t, 1 H, *J* = 6.2 Hz, H-1'), 4.51 (m, 1 H, H-3'), 3.84 (m, 1 H, H-4'), 3.68 (m, 2 H, H-5'a and H-5'b), 2.71 (m, 1 H, H-2'a), 2.17 (m, 1 H, H-2'b), 0.89 (s, 9 H, $(CH_3)_3CSi$), 0.86 (s, 9 H, $(CH_3)_3CSi$), 0.11 (s, 6 H, $(CH_3)_2Si$), 0.04 (s, 6 H, $(CH_3)_2Si$).

*$N^1$,$N^2$-Bis-tert-butyloxycarbonyl-$N^2$-acetyl-3',5'-O-bis-tert-butyldimethylsilyl-2'-deoxyguanidine* ***(dG.03)***

A solution of di-*tert*-butyldicarbonate (8.75 g, 40 mmol) in anhydrous $CH_2Cl_2$ (27 mL) was added to a solution of compound **dG.02** (1.85 g, 3.44 mmol), $Et_3N$ (12.17 mL, 15.48 mmol), and DMAP (1.72 g, 14.10 mmol) in anhydrous $CH_2Cl_2$ (20 mL) under a $N_2$ atmosphere. The reaction mixture was refluxed for two hours, concentrated *in vacuo,* and purified by silica gel chromatography to yield *$N^1$,$N^2$*-bis-*tert*-butyloxy-carbonyl-*$N^2$*-acetyl-3',5'-*O*-bis-*tert*-butyldimethylsilyl-2'-deoxyguanidine **dG.03** (1.37 g, 54%) as a white foam.

*$^1H$ NMR (400 MHz, $CDCl_3$):* δ 8.25 (s, 1 H, H-8), 6.43 (t, 1 H, *J* = 6.4 Hz, H-1'), 4.59 (m, 1 H, H-3'), 3.99 (m, 1 H, H-4'), 3.87 (m, 1 H, H-5'a), 3.87 (m, 1 H, H-5'b), 2.60 (a, 3 H, $CH_3CO$), 2.52 (m, 1 H, H-2'a), 2.41 (m, 1 H, H-2'b), 1.71 (s, 9H, $(CH_3)_3CO$), 1.36 (s, 9H, $(CH_3)_3CO$), 0.92 (s, 9 H, $(CH_3)_3CSi$), 0.91 (s, 9 H, $(CH_3)_3CSi$), 0.10 (s, 6 H, $(CH_3)_2Si$), 0.09 (s, 6 H, $(CH_3)_2Si$).

*$N^1$,$N^2$-Bis-tert-butyloxycarbonyl-3',5'-O-bis-tert-butyldimethylsilyl-2'-deoxyguanidine* ***(dG.04)***

Compound **dG.03** (1.31 g, 1.78 mmol) was treated with $K_2CO_3$ (1.23 g, 8.90 mmol) in MeOH (20.5 mL) at room temperature for 30 minutes. The reaction was concentrated *in vacuo,* dissolved in ethyl acetate (50 mL), and washed twice with water (10 mL). The organic layer was dried with $Na_2SO_4$ and concentrated *in vacuo* to give *$N^1$*,*$N^2$*-bis-*tert*-butyloxycarbonyl-3',5'-*O*-bis-*tert*-butyldimethylsilyl-2'-deoxyguanidine **dG.04** (1.18 g, 95%) as a white foam.

*$^1H$ NMR (400 MHz, DMSO-d6):* δ 9.82 (bs, 1 H, N-H), 8.24 (s, 1 H, H-8), 6.27 (t, 1 H, *J* = 6.6 Hz, H-1'), 4.71 (m, 1 H, H-3'), 3.82 (m, 1 H, H-4'), 3.79 (m, 1 H, H-5'a), 3.73 (m, 1 H, H-5'b), 2.95 (m, 1 H, H-2'a), 2.30 (m, 1 H, H-2'b),

1.66 (s, 9 H, $(CH_3)_3CO$), 1.48 (s, 9 H, $(CH_3)_3CO$), 0.89 (s, 9H, $(CH_3)_3CSi$), 0.83 (s, 9H, $(CH_3)_3CSi$), 0.11 (2 s, 6 H, $(CH_3)_2Si$), -0.01 (2 s, 6 H, $(CH_3)_2Si$).

*$N^1$,$N^2$-Bis-tert-butyloxycarbonyl-$N^2$-(2-nitrobenzyl)-3',5'-O-bis-tert-butyldimethylsilyl-2'-deoxyguanidine* ***(dG.05)***

NaH (22 mg, 0.93 mmol, dry) was added to a solution of compound **dG.04** (500 mg, 0.72 mmol) in anhydrous DMF (6 mL) at 0°C and stirred for 30 minutes under a $N_2$ atmosphere. A solution of 2-nitrobenzyl bromide (202 mg, 0.94 mmol) in anhydrous DMF (3 mL) was added dropwise. The reaction was stirred at 0°C for one hour and then concentrated *in vacuo.* Ethyl acetate (50 mL) was added, and the mixture was washed twice with saturated $NH_4Cl$ solution (20 mL). The combined aqueous layer was extracted with ethyl acetate (20 mL), and the combined organic layer was dried with $Na_2SO_4$, concentrated in *vacuo,* and purified by silica gel chromatography to yield *$N^1$,$N^2$*-bis-*tert*-butyloxycarbonyl-*$N^2$*-(2-nitrobenzyl)-3',5'-*O*-bis-*tert*-butyldimethylsilyl-2'-deoxyguanidine **dG.05** (543 mg, 91%) as a white foam.

*$^1H$ NMR (400 MHz, $CDCl_3$):* $\delta$ 8.12 (s, 1 H, H-8), 8.06 (m, 1 H, Ph-H), 7.68 (m, 1 H, Ph-H), 7.56 (m, 1 H, Ph-H), 7.38 (m, 1 H, Ph-H), 6.41 (6, *J* = 6.2 Hz, 1 H, H-2'), 5.49 (s, 2 H, Ph-$CH_2$), 4.56 (m, 1 H, H-3'), 4.00 (m, 1 H, H-4'), 3.80 (m, 2 H, H-5'), 2.52 (m, 1 H, H-2'a), 2.39 (m, 1 H, H-2'b), 1.54 (s, 9 H, $(CH_3)_3CO$), 1.45 (s, 9 H, $(CH_3)_3CO$), 0.92 (s, 18 H, $(CH_3)_3CSi$), 0.10 (s, 12 H, $(CH_3)_2Si$).

*$N^2$-(2-Nitrobenzyl)-3',5'-O-bis-tert-butyldimethylsilyl-2'-deoxyguanidine* ***(dG.06)***

Silica gel 60 (4.5 g, 100-200 mesh, activated by heating to 50-60°C under reduced pressure for 24 hours) was added to a solution of compound **dG.05** (450 mg, 0.54 mmol) in $CH_2Cl_2$ (5 mL), and the mixture was evaporated *in vacuo* to dryness. The residue was heated to 60-70°C under reduced pressure for 48 hours, washed three times with MeOH (50 mL), and filtered using a buchi funnel. The combined filtrate was concentrated *in vacuo* and purified by silica gel chromatography to yield *$N^2$*-(2-nitrobenzyl)-3',5'-*O*-bis-*tert*-butyldimethylsilyl-2'-deoxyguanidine **dG.06** (333 mg, 97%) as a white foam.

*$^1H$ NMR (400 MHz, DMSO-d6):* $\delta$ 10.85 (s, 1 H, N-H), 8.05 (m, 1 H, Ph-H), 7.85 (s, 1 H, H-8), 7.69 (m, 1 H, Ph-H), 7.62 (m, 1 H, Ph-H), 7.54 (m, 1 H, Ph-H), 7.10 (t, 1 H, N-H), 6.07 (t, 1 H, *J* = , H-1'), 4.78 (m, 2 H, Ph-$CH_2$), 4.40 (m, 1 H, H-3'), 3.79 (m, 1 H, H-4'), 3.62 (m, 2 H, H-5'), 2.60 (m, 1 H, H-2'a), 2.15 (m, 1 H, H-2'b), 0.86 (s, 9H, $(CH_3)_3CSi$), 0.85 (s, 9H, $(CH_3)_3CSi$), 0.06 (s, 6 H, $(CH_3)_2Si$), 0.01 (s, 6 H, $(CH_3)_2Si$).

*$N^2$-(2-Nitrobenzyl)-2'-deoxyguanidine* ***(dG.07)***

A solution of *n*-$Bu_4NF$ (393 mg, 1.5 mmol) in THF (6 mL) was added dropwise to a solution of compound **dG.06** (313 mg, 0.5 mmol) in THF (12 mL) at 0°C. The reaction mixture was stirred at 0°C for one hour and then at room temperature for two hours. The reaction was *concentrated in vacuo* and purified by silica gel chromatography to yield *$N^2$*-(2-nitrobenzyl)-2'-deoxyguanidine **dG.07** (165 mg, 83%) as a yellow foam.

*$^1H$ NMR (400 MHz, DMSO-d6):* $\delta$ 10.82 (s, 1 H, $D_2O$ exchangeable, N-H), 8.06 (m, 1 H, Ph-H), 7.90 (s, 1 H, H-8), 7.72 (m, 1 H, Ph-H), 7.64 (m, 1 H, Ph-H), 7.55 (m, 1 H, Ph-H), 7.06 (t, 1 H, $D_2O$ exchangeable, N-H), 6.08 (t, 1 H, *J* = 6.4 Hz, H-1'), 5.23 (d, 1 H, $D_2O$ exchangeable, 3'-OH), 4.80 (t, 3 H, among them 1 H $D_2O$ exchangeable, 5'-OH and Ph-$CH_2$), 4.25 (m, 1 H, H-3'), 3.77 (m, 1 H, H-4'), 3.42 (m, 2 H, H-5'), 2.45 (m, 1 H, H-2'a), 2.12 (m, 1H, H-2'b).

*$N^2$-(2-Nitrobenzyl)-2'-deoxyguanidine-5'-triphosphate* ***(WW2p067)***

$POCl_3$ (17 $\mu$L, 0.18 mmol) was added dropwise to a solution of compound **dG.07** (50 mg, 0.12 mmol) in trimethylphosphate (0.5 mL) and maintained at minus 20-30°C for two hours. A solution of bis-tri-*n*-butylammonium pyrophosphate (205 mg, 0.6 mmol) and tri-*n*-butylamine (120 $\mu$l) in anhydrous DMF (1.2 mL) was added. After five minutes of stirring, triethylammonium bicarbonate buffer (1 M, pH 7.5; 10 mL) was added. The reaction was stirred for one hour at room temperature and then lyophilized to dryness. The residue was dissolved in water (10 mL), filtered, and purified by anion exchange chromatography using a Q Sepharose FF column (2.5 x 20 cm) with a linear gradient of $NH_4HCO_3$ (50 mM to 500 mM in 300 minutes) at a flow rate of 4.5 mL/min. The fractions containing triphosphate were combined and lyophilized to give *$N^2$*-(2-nitrobenzyl)-2'-deoxyguanidine-5'-triphosphate **WW2p067** (52 mg, 62%) as a white fluffy solid.

*$^1H$ NMR (400 MHz, $D_2O$):* $\delta$ 8.08 (d, 1 H, Ph-H), 8.04 (s, 1 H, H-8), 7.68 (m, 2 H, Ph-H), 7.51 (t, 1 H, Ph-H), 6.27 (t, 1 *H, J* = 6.8 Hz, H-1'), 4.63 (m, 1 H, H-3'), 4.21 (m, 1 H, H-4'), 4.10 (m, 2 H, H-5'), 2.66 (m, 1 H, H-2'a), 2.41 (m, 1 H, H-2'b);

*$^{31}P$ NMR (162 MHz, $D_2O$):* $\delta$ -6.48 (d, *J* = 15.7 Hz), -11.40 (d, *J* = 15.2 Hz), -19.94 (br);

*ToF-MS (ESI):* For the molecular ion $C_{17}H_{19}N_6O_{15}P_3Na$ $[M-2H+Na]^-$, the calculated mass was 663.0019, and the observed mass was 663.0143.

## Synthesis of $O^6$-(2-nitrobenzyl)-2'-deoxyguanosine-5'-triphosphate (WW2p077)

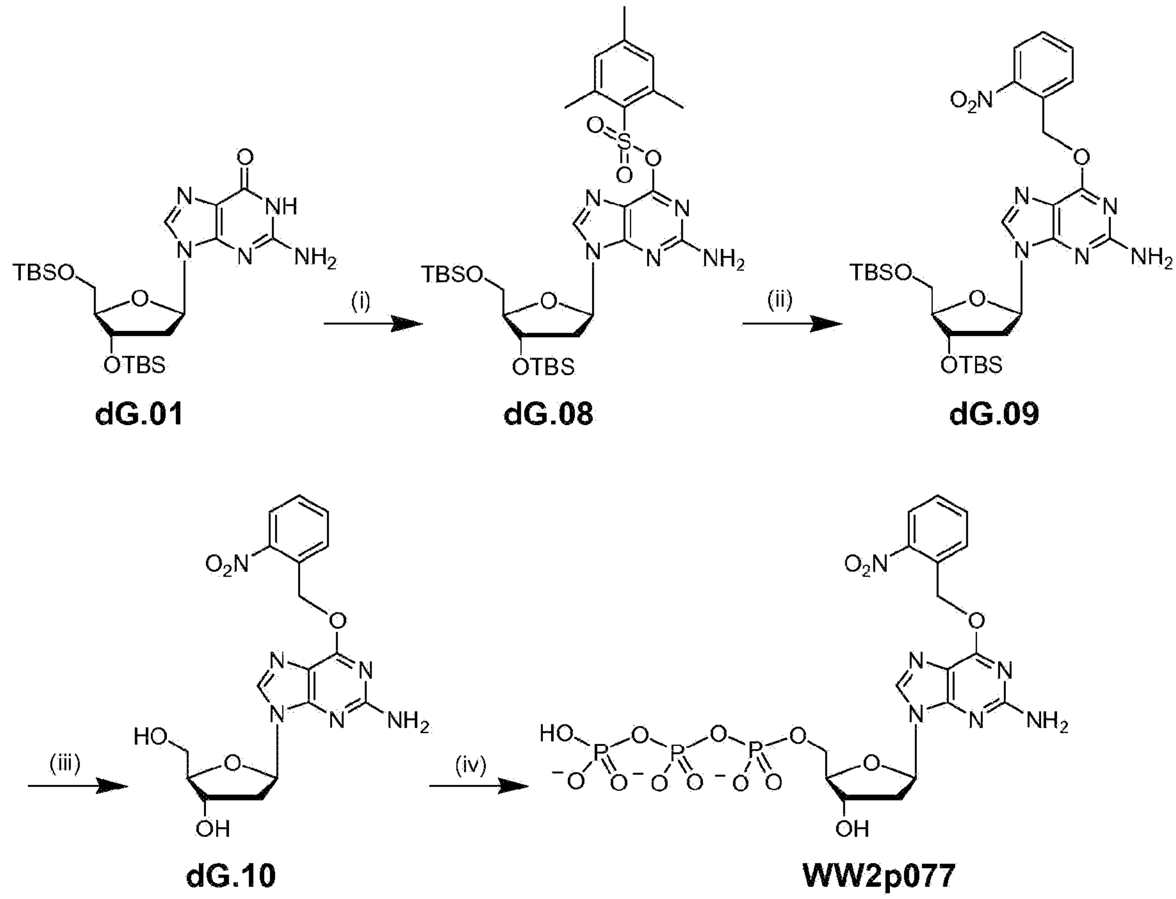


dG.01 dG.08 dG.09

dG.10 WW2p077

**Scheme**. Synthesis of *$O^6$-(2-nitrobenzyl)-2'-deoxyguanosine-5'-triphosphate*. (i) 2-mesitylene-sulfonyl chloride, $Et_3N$, DMAP, anhydrous $CH_2Cl_2$, HMPA, room temperature, overnight, 98%; (ii) 2-nitrobenzyl alcohol, DABCO, DBU, molecular sieves, anhydrous 1,2-DME, 0°C, then gradually warmed to room temperature, 24 hours, 77%; (iii) *n*-$Bu_4NF$, THF, room temperature, 1.5 hours, 94%; (iv) $POCl_3$, $(MeO)_3PO$, minus 20-30°C, two hours; (*n*-$Bu_3NH)_2H_2P_2O_7$, *n*-$Bu_3N$, DMF, five minutes; 1 M $HNEt_3HCO_3$, one hour, 35%.

*$O^6$-(2-Mesitylenesulfonyl)-3',5'-bis-O-(tert-butyldimethylsilyl)-2-deoxyguanosine* ***(dG.08)***

2-Mesitylenesulfonyl chloride (510 mg, 2.34 mmol), $Et_3N$ (0.56 mL, 4.0 mmol), and DMAP (27 mg, 0.197 mmol) were added to a solution of compound **dG.01** (500 mg, 1.0 mmol) in a mixture of hexmethylphosphoramide (1.5 mL) and anhydrous $CH_2Cl_2$ (7 mL). The reaction was stirred at room temperature overnight and then diluted with ethyl ether (25 mL). The ether solution was washed twice with a saturated solution of $NaHCO_3$ (10 mL each) and then with brine (10 mL). The organic layer was dried over $Na_2SO_4$ and *concentrated in vacuo* to give a semi-solid, which was dissolved in ethyl ether (2 mL) and gradually diluted with hexane (40 mL). The precipitate was collected by filtration to yield $O^6$-(2-mesitylenesulfonyl)-3',5'-bis-*O*-(*tert*-butyldimethylsilyl)-2'-deoxyguanosine **dG.08** (737 mg, 98%).

*$^1H$ NMR (400 MHz, $CDCl_3$):* δ 7.98 (s, 1 H, H-8), 6.98 (s, 2 H, Ph-H), 6.28 (t, 1 H, *J* = 6.5 Hz, H-1'), 4.84 (br s,

2 H, $NH_2$), 4.57 (m, 1 H, H-3'), 3.97 (m, 1 H, H-4'), 3.78 (dd, 1 *H, J* = 2.9 and 11.0 Hz, H-5'a), 3.75 (dd, 1 H, *J* = 2.9 and 11.0 Hz, H-5'b), 2.75 (s, 6 H, $CH_3$), 2.53 (m, 1 H, H-2'a), 2.34 (m, 1 H, H-2'b), 2.31 (s, 3 H, $CH_3$), 0.91 (s, 9 H, $(CH_3)_3CSi$), 0.90 (s, 9 H, $(CH_3)_3CSi$), 0.09 (s, 6 H, $(CH_3)_2Si$), 0.06 (s, 6 H, $(CH_3)_2Si$).

*$O^6$-(2-Nitrobenzyl)-3',5'-bis-O-(tert-butyldimethylsilyl)-2'-deoxyguanosine* ***(dG.09)***

DABCO (139 mg, 1.24 mmol) and 2-nitrobenzyl alcohol (474 mg, 3.10 mmol) were added to a solution of compound **dG.08** (420 mg, 0.62 mmol) and 4 Å molecular sieves (200 mg) in anhydrous 1,2-DME (6.2 mL) at 0°C. The mixture was warmed to room temperature and stirred for 30 minutes. DBU (139 μL, 0.93 mmol) was added, and the reaction was stirred at room temperature for 24 hours. Ethyl acetate (100 mL) was added, and the organic layer was washed with water (20 mL) and brine (20 mL), dried over $Na_2SO_4$, concentrated *in vacuo,* and purified by silica gel chromatography to yield $0^6$-(2-nitrobenzyl)-3',5'-bis-*O*-(*tert*butyldimethylsilyl)-2'-deoxyguanosine **dG.09** (300 mg, 77%).

*$^1H$ NMR (400 MHz, $CDCl_3$):* δ 8.09 (dd, 1 H, *J* = 1.1 and 8.2 Hz, Ph-H), 7.94 (s, 1 H, H-8), 7.86 (d, 1 H, *J* = 7.7 Hz, Ph-H), 7.60 (dt, 1 H, *J* = 1.1 and 7.7 Hz, Ph-H), 7.45 (dt, 1 H, *J* = 1.0 and 8.2 Hz, Ph-H), 6.32 (t, 1 H, *J* = 6.5 Hz, H-1'), 5.94 (s, 2H, $NH_2$), 4.89 (s, 2 H, $PhCH_2$), 4.59 (m, 1 H, H-3'), 3.98 (m, 1 H, H-4'), 3.81 (dd, 1 H, *J* = 2.9 and11.0 Hz, H-5'a), 3.75 (dd, 1 H, *J* = 2.9 and 11.0 Hz, H-5'b), 2.58 (m, 1 H, H-2'a), 2.37 (m, 1 H, H-2'b), 0.91 (s, 18 H, $(CH_3)_3CSi$), 0.10 (s, 6 H, $(CH_3)_2Si$), 0.08 (s, 6 H, $(CH_3)_2Si$).

*$O^6$-(2-Nitrobenzyl)-2'-deoxyguanosine (dG.10)*

A solution of *n*-$Bu_4NF$ (252 mg, 0.80 mmol) in THF (4 mL) was added to a solution of compound **dG.09** (200 mg, 0.32 mmol) in THF (4 mL) at room temperature. The mixture was stirred for 1.5 hours, concentrated *in vacuo*, and purified by silica gel chromatography to yield *$O^6$*-(2-nitrobenzyl)-2'-deoxyguanosine **dG.10** (119 mg, 94%).

*$^1H$ NMR (400 MHz, DMSO-$d_6$):* δ 8.16 (dd, 1 H, *J* = 1.0 and 8.2, Hz, Ph-H), 8.13 (s, 1 H, H-8), 7.79 (m, 2 H, Ph-H), 7.63 (m, 1 H, Ph-H), 6.49 (br s, 2 H, $D_2O$ exchangeable, $NH_2$), 6.22 (dd, 1 H, *J* = 6.1 and 7.7 Hz, H-1'), 5.87 (s, 2 H, $PhCH_2$), 5.28 (br, 1 H, $D_2O$ exchangeable, 5'-OH), 4.99 (br, 1 H, $D_2O$ exchangeable, 3'-OH), 4.35 (m, 1 H, H-3'), 3.82 (m, 1 H, H-4'), 3.55 (m, 1 H, H-5'b), 3.52 (m, 1 H, H-5'a), 2.58 (m, 1 H, H-2'a), 2.23 (m, 1 H, H-2'b).

*$O^6$-(2-Nitrobenzyl)-2'-deoxyguanosine-5'-triphosphate* ***(WW2p077)***

$POCl_3$ (14 μL, 0.1 mmol) was added dropwise to a solution of compound **dG.10** (43 mg, 0.1 mmol) in trimethylphosphate (0.5 mL) and maintained at minus 20-30°C for two hours. A solution of bis-tri-*n*-butylammonium pyrophosphate (237 mg, 0.5 mmol) and tri-*n*-butylamine (100 μL) in anhydrous DMF (1.0 mL) was added. After five minutes of stirring, triethylammonium bicarbonate buffer (1 M, pH 7.5; 10 mL) was added. The reaction was stirred for one hour at room temperature and then lyophilized to dryness. The residue was dissolved in water (10 mL), filtered, and purified by anion exchange chromatography using a Q Sepharose FF column (2.5 x 20 cm) with a linear gradient of $NH_4HCO_3$ (50 mM to 500 mM in 300 minutes) at a flow rate of 4.5 mL/min. The fractions containing triphosphate were combined and lyophilized to give *$O^6$*-(2-nitrobenzyl)-2'-deoxyguanosine-5'-triphosphate **WW2p077** (24 mg, 35%) as a white fluffy solid.

*$^1H$ NMR (400 MHz, $D_2O$):* δ 8.21 (s, 1 H, H-8), 8.13 (d, *J* = 8.2 Hz, 1 H, Ph-H), 7.83 (d, 1 H, J = 7.8 Hz, Ph-H), 7.74 (t, 1 H, *J* = 7.8 Hz, Ph-H), 7.51 (t, *J* = 7.8 Hz, 1 H, Ph-H), 6.35 (t, 1 H, *J* = 6.8 Hz, H-1'), 5.86 (s, 2 H, Ph-$CH_2$), 4.28 (m, 1 H, H-4'), 4.23 (m, 2 H, H-5'), 2.82 (m, 1 H, H-2'a), 2.57 (m, 1 H, H-2'b);

*$^{31}P$ NMR (162 MHz, $D_2O$):* δ -6.48 (br), -10.96 (br), -21.83 (br);

*ToF-MS (ESI):* For the molecular ion $C_{17}H_{19}N_6O_{15}P_3Na$ $[M-2H+Na]^-$, the calculated mass was 663.0019, and the observed mass was 663.0228.

**Synthesis of 6-ROX labeled O$^6$-[4-(3-amino-1-propynyl)-2-nitrobenzyl]-2'-deoxyguanosine-5'-triphosphate (WW2p121)**

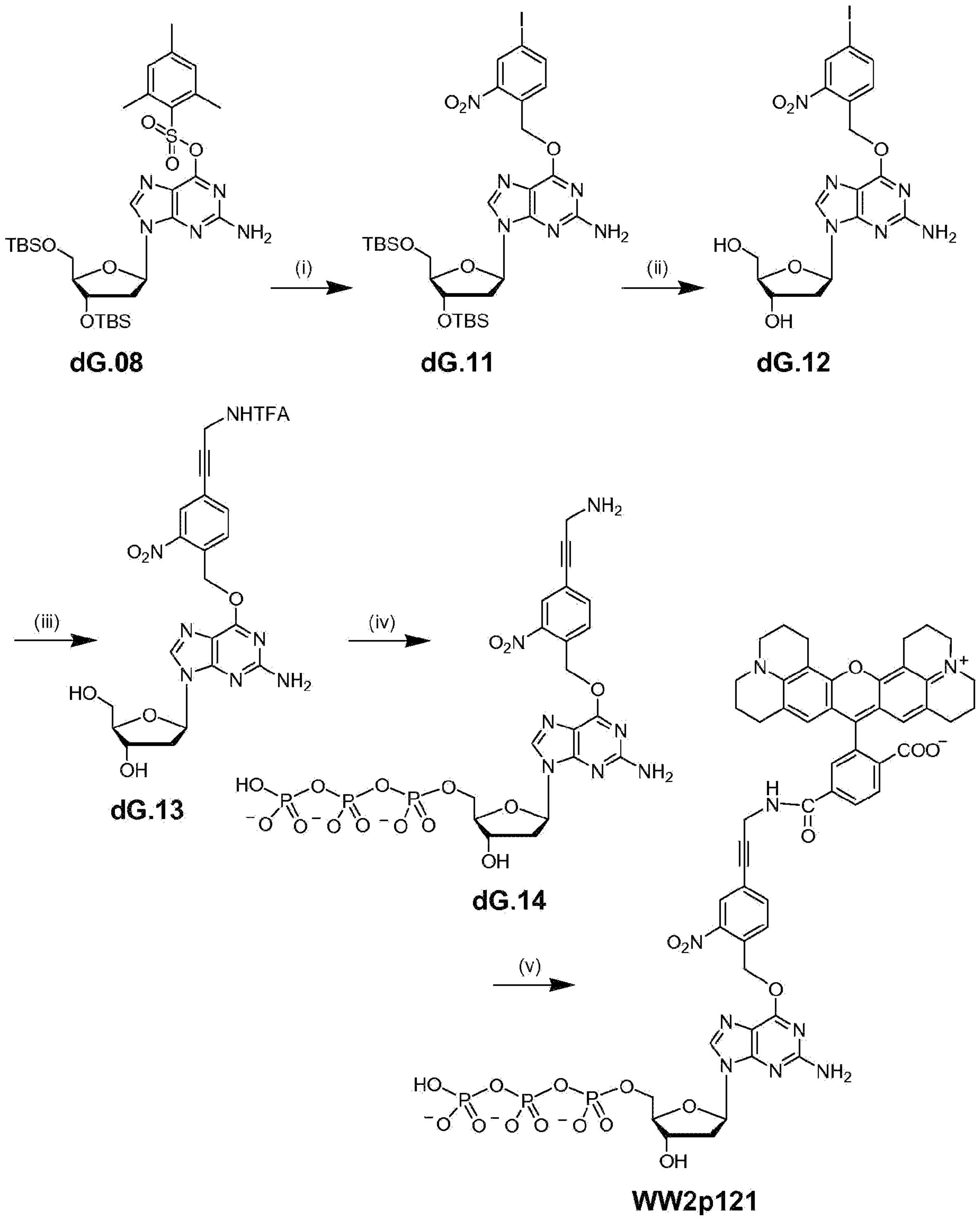


dG.08 dG.11 dG.12

dG.13

dG.14

WW2p121

**Scheme**. Synthesis of *6-ROX labeled $O^6$-[4-(3-amino-1-propynyl)-2-nitrobenzyl]-2'-deoxyguanosine-5'-triphosphate*. (i) 4-iodo-2-nitrobenzyl alcohol, DABCO, DBU, 4Å molecular sieves, anhydrous 1,2-DME, 0°C, then gradually warmed to room temperature, 24 hours, 77%; (ii) *n*-$Bu_4NF$, THF, room temperature, 1.5 hours, 62%; (iii) *N*-propargyltrifluoroacetamide, $Pd(PPh_3)_4$, CuI, $Et_3N$, anhydrous DMF, four hours, 42%; (iv) $POCl_3$, proton sponge, $(MeO)_3PO$, 0°C, one hour; $(n\text{-}Bu_3NH)_2H_2P_2O_7$, *n*-$Bu_3N$, DMF, five minutes; 1 M $HNEt_3HCO_3$, one hour; $NH_4OH$, one hour; (v) 6-ROX-SE, 0.1 M $Na_2CO_3/NaHCO_3$ buffer (pH 9.2), one hour.

*$O^6$-(4-Iodo-2-nitrobenzyl)-3',5'-bis-O-(tert-butyldimethylsilyl)-2-deoxyguanosine (**dG.11**)*

DABCO (90 mg, 0.80 mmol) and 4-iodo-2-nitrobenzyl alcohol (555 mg, 2.00 mmol) were added to a solution of compound **dG.08** (270 mg, 0.40 mmol) and 4 Å molecular sieves (129 mg) in anhydrous 1,2-DME (4 mL) at 0°C. The mixture was warmed to room temperature and stirred for 30 minutes. DBU (91 μL, 0.60 mmol) was added, and the reaction was stirred at room temperature for 24 hours. Ethyl acetate (70 mL) was added, and the organic solution was washed with water (10 mL) and brine (10 mL), dried over $Na_2SO_4$, concentrated *in vacuo*, and purified by silica gel chromatography to give $O^6$-(4-iodo-2-nitrobenzyl)-3',5'-bis-*O*-(*tert*butyldimethylsilyl)-2-deoxyguanosine **dG.11** (233 mg, 77%).

*$^1H$ NMR (400 MHz, $CDCl_3$):* δ 8.40 (d, 1 H, *J* = 1.7 Hz, Ph-H), 7.94 (s, 1 H, H-8), 7.86 (dd, 1 H, *J* = 1.7 and 8.3 Hz, Ph-H), 7.60 (d, 1 H, *J* = 8.3 Hz, Ph-H), 6.31 (t, 1 H, *J* = 6.5 Hz, H-1'), 5.86 (s, 2 H, $NH_2$), 4.87 (s, 2 H, $PhCH_2$), 4.59 (m, 1 H, H-3'), 3.98 (m, 2 H, H-4'), 3.82 (dd, AB, 1 H, *J* = 4.2 and 11.2 Hz, H-5'a), 3.75 (dd, 1 H, *J* = 4.2 and 11.2 Hz, H-5'b), 2.57 (m, 1 H, H-2'a), 2.37 (m, 1 H, H-2'b), 0.91 (s, 18 H, $(CH_3)_3CSi$), 0.10 (s, 6 H, $(CH_3)_2Si$), 0.08 (s, 6 H, $(CH_3)_2Si$).

*$O^6$-(4-Iodo-2-nitrobenzyl)-2-deoxyguanosine (**dG.12**)*

A solution of *n*-$Bu_4NF$ (291 mg, 0.924 mmol) in THF (2 mL) was added to a solution of compound **dG.11** (233 mg, 0.31 mmol) in THF (4 mL) at room temperature. The mixture was stirred for 1.5 hours, *concentrated in vacuo,* and purified by silica gel chromatography to give $O^6$-(4-iodo-2-nitrobenzyl)-2-deoxyguanosine **dG.12** (101 mg, 62%).

*$^1H$ NMR (400 MHz, DMSO-$d_6$):* δ 8.43 (d, 1 H, *J* = 1.8 Hz, Ph-H), 8.16 (dd, 1 H, *J* = 1.8 and 8.2 Hz, Ph-H), 8.13 (s, 1 H, H-8), 7.52 (d, 1 H, *J* = 8.2 Hz, Ph-H), 6.48 (br s, 2 H, $D_2O$ exchangeable, $NH_2$), 6.22 (dd, 1 H, *J* = 6.2 and 7.7 Hz, H-1'), 5.79 (s, 2 H, $PhCH_2$), 5.27 (d, 1 H, $D_2O$ exchangeable, 5'-OH), 4.97 (t, 1 H, $D_2O$ exchangeable, 3'-OH), 4.35 (m, 1 H, H-3'), 3.82 (m, 1 H, H-4'), 3.52 (m, 2 H, H-5'a and H-5'b), 2.58 (m, 1 H, H-2'a), 2.22 (m, 1 H, H-2'b).

*$O^6$-[4-(3-Trifluoroacetamido-1-propynyl)-2-nitrobenzyl]-2'-deoxyguanosine (**dG.13**)*

A solution of compound **dG.12** (95 mg, 0.18 mmol), *N*-propargyltrifluoroacetylamide (82 mg, 0.53 mmol), tetrakis(triphenylphosphine)-palladium(0) (21 mg, 0.018 mmol), CuI (7 mg, 0.036 mmol) and $Et_3N$ (51 μL, 0.36 mmol) in anhydrous DMF (1.4 mL) was stirred at room temperature for four hours. $CH_2Cl_2$ (1 mL), methanol (1 mL), and $NaHCO_3$ (84 mg, 1 mmol) were added, and the mixture was stirred for 20 minutes, concentrated *in vacuo,* and purified by silica gel column chromatography and preparative HPLC to give $O^6$-[4-(3-trifluoroacetamido-1-propynyl)-2-nitrobenzyl]-2'-deoxyguanosine **dG.13** (42 mg, 42%).

*$^1H$ NMR (400 MHz, DMSO-$d_6$):* δ 10.11 (br 1 H, NH), 8.16 (d, 1 H, *J* = 1.7 Hz, Ph-H), 8.13 (s, 1 H, H-8), 7.86 (dd, 1 H, *J* = 1.7 and 8.2 Hz, Ph-H), 7.75 (d, 1 H, *J* = 8.2 Hz, Ph-H), 6.50 (br s, 2 H, $D_2O$ exchangeable, $NH_2$), 6.22 (t, 1 H, *J* = 6.4 Hz, H-1'), 5.87 (s, 2H, $PhCH_2$), 5.27 (d, 1 H, $D_2O$ exchangeable, 5'-OH), 4.97 (t, 1 H, $D_2O$ exchangeable, 3'-OH), 4.35 (m, 1 H, H-3'), 4.33 (s, 2 H, $CH_2$), 3.82 (m, 2 H, H-4'), 3.55 (m, 1 H, H-5'b), 3.51 (m, 1 H, H-5'a), 2.58 (m, 1 H, H-2'a), 2.23 (m, 1 H, H-2'b).

*$O^6$-[4-(3-Amino-1-propynyl)-2-nitrobenzyl]-2'-deoxyguanosine-5'-triphosphate (**dG.14**)*

Compound **dG.13** (33 mg, 0.06 mmol) and proton sponge (26 mg, 0.12 mmol) were evaporated three times from anhydrous pyridine (3 mL) and dissolved in trimethylphosphate (0.3 mL). $POCl_3$ (8 μL, 0.09 mmol) was added, and the mixture was stirred for one hour at 0°C. A solution of bis-tri-*n*-butylammonium pyrophosphate (142 mg, 0.3 mmol)

and tri-*n*-butylamine (60 μL) in anhydrous DMF (0.6 mL) was added. After five minutes of stirring, triethylammonium bicarbonate buffer (1 M, pH 7.5; 10 mL) was added. The reaction was stirred for one hour at room temperature, followed by the dropwise addition of concentrated ammonium hydroxide (5 mL, 27%) at 0°C. The mixture was stirred for one additional hour at room temperature and then lyophilized to dryness. The residue was dissolved in water (10 mL), filtered, and purified with reverse-phase HPLC using a Perkin Elmer OD-300 $C_{18}$ column (4.6 x 250 mm) to yield *O*$^6$-[4-(3-amino-1-propynyl)-2-nitrobenzyl]-2'-deoxyguanosine-5'-triphosphate **dG.14.** Mobile phase: A, 100 mM triethylammonium acetate (TEAA) in water (pH 7.0); B, 100 mM TEAA in water/$CH_3CN$ (30:70). HPLC purification was achieved using a linear gradient of 5-50% B for 20 minutes and then 50-90% B for 10 minutes.

*$^1H$ NMR (400 MHz, $D_2O$):* δ 8.28 (s, H-8), 8.26 (s, 1 H, Ph-H), 7.80 (d, 1 H, *J* = 8.0 Hz, Ph-H), 7.63 (d, 1 H, *J* = 8.0 Hz, Ph-H), 6.38 (t, 1 H, *J* = 6.4 Hz, H-1'), 5.88 (br s, 2 H, Ph-$CH_2$), 4.29 (m, 3 H, H-4', H-5'), 3.67 (s, 2 H, $CH_2$), 2.80 (m, 1 H, H-2'a), 2.56 (m, 1 H, H-2'b);

*$^{31}P$ NMR (162 MHz, $D_2O$):* δ -5.85 (d, *J* = 19.4 Hz), -10.98 (d, *J* = 19.4 Hz), -21.78 (t, *J* = 19.4 Hz);

*ToF-MS (ESI):* For the molecular ion $C_{20}H_{24}N_7O_{15}P_3Na$ [M+Na]+, the calculated mass was 718.0441, and the observed mass was 718.0600.

*6-ROX lableled O$^6$-[4-(3-Amino-1-propynyl)-2-nitrobenzyl]-2'-deoxyguanosine-5'-triphosphate (**WW2p121**)*

A solution of 6-ROX-SE (0.3 mg, 0.47 μmol) in anhydrous DMSO (12 μL) was added to a solution of triphosphate **dG.14** (0.36 μmol) in $Na_2CO_3$/$NaHCO_3$ buffer (0.1 M, pH 9.2; 0.6 mL) and incubated at room temperature for one hour. The reaction was purified with reverse-phase HPLC using a Perkin Elmer OD-300 $C_{18}$ column (4.6 x 250 mm) to yield the 6-ROX labeled triphosphate **WW2p121.** Mobile phase: A, 100 mM TEAA in water (pH 7.0); B, 100 mM TEAA in water/$CH_3CN$ (30:70). HPLC purification was achieved using a linear gradient of 5-50% B for 20 minutes and then 50-90% B for 10 minutes. The concentration of **WW2p121** was estimated by adsorption spectroscopy using the extinction coefficient of the 6-ROX dye *(i.e.,* 82,000 at 575 nm).

**Synthesis of *O*$^6$-[1-(2-nitrophenyl)ethyl]-2'-deoxyguanosine-5'-triphosphate (WW2p143)**

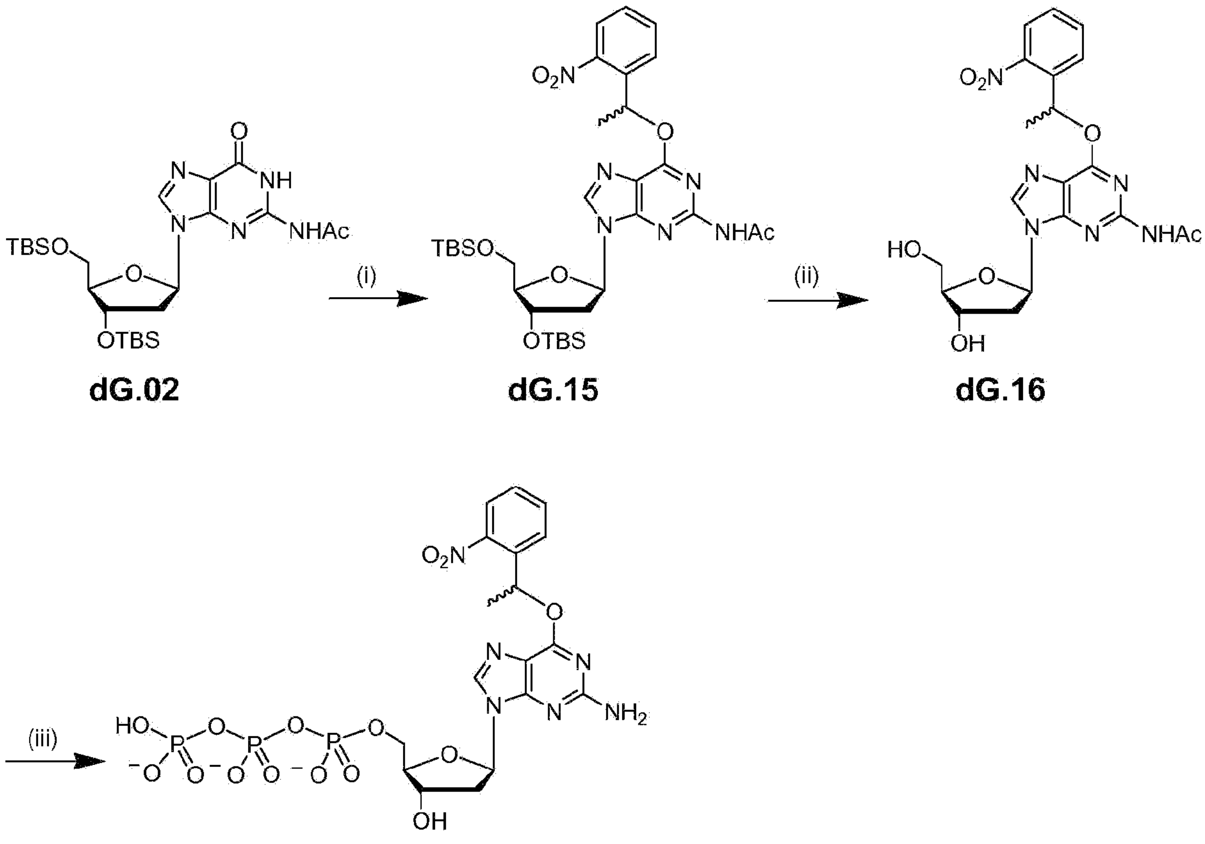


dG.02 dG.15 dG.16

WW2p143

**Scheme**. Synthesis of *$O^6$-[1-(2-nitrophenyl)ethyl]-2'-deoxyguanosine-5'-triphosphate.* (i) 1-(2-nitrophenyl)ethanol, $PPh_3$, DIAD, anhydrous THF, room temperature, six hours, 74%; (ii) *n*-$Bu_4NF$, THF, 0°C, then gradually warmed to room temperature, four hours, 64%; (iii) $POCl_3$, proton sponge, $(MeO)_3PO$, 0°C, two hours; ($n$-$Bu_3NH)_2H_2P_2O_7$, *n*-$Bu_3N$, DMF, five minutes; 1 M $HNEt_3HCO_3$, one hour; $NH_4OH$, 60 °C, three hours.

*$O^6$-[1-(2-Nitrophenyl)ethyl]-$N^2$-acetyl-3',5'-bis-O-(tert-butyldimethylsilyl)-2'-deoxyguanosine (**dG.15**)*

A solution of compound dG.02 (108 mg, 0.2 mmol), 1-(2-nitrophenyl)ethanol (33 mg, 0.23 mmol) and $PPh_3$ (79 mg, 0.3 mmol) in anhydrous THF (2 mL) was treated with diisopropyl azodicarboxylate (DIAD, 59 μL, 0.3 mmol) and stirred for six hours at room temperature. The mixture was diluted with $CH_2Cl_2$ (20 mL), washed once with saturated $NH_4Cl$ solution (10 mL), dried over $Na_2SO_4$, concentrated, and purified by silica gel chromatography to yield $O^6$-[1-(2-nitrophenyl)ethyl]-$N^2$-acetyl-3',5'-bis-O-(*tert*-butyldimethylsilyl)-2'-deoxyguanosine **dG.15** (102 mg, 74%, 1:1 mixture of diastereomers) as a yellow foam.

*$^1H$ NMR (400 MHz, $CDCl_3$) for diastereomers:* δ 8.13 and 8.12 (2 s, 1 H, H-8). 7.89 (d, 1 H, *J* = 8.0 Hz, Ph-H), 7.83 (m, 1 H, Ph-H), 7.74 (br s., 1 H, NH), 7.57 (t, 1 H, *J* = 7.2 Hz, Ph-H), 7.40 (t, 1 H, *J* = 8.0 Hz, Ph-H), 6.69 (m, 1 H, PhCH), 6.36 (t, 1 H, *J* = 6.3 Hz, H-1'), 4.56 (m, 1 H, H-3'), 3.98 (m, 1 H, H-4'), 3.82 (m, 1 H, H-5'a), 3.76 (m, 1 H, H-5'b), 2.50 (m, 1 H, H-2'a), 2.41 (m, 4 H, H-2'b and $CH_3CO$), 1.88 (2 d, *J* = 6.5 Hz, $CH_3$), 0.91 (s, 18 H, $(CH_3)_3CSi$), 0.10 (s, 6 H, $(CH_3)_2Si$), 0.09 (s, 6 H, $(CH_3)_2Si$).

*$O^6$-[1-(2-Nitrophenyl)ethyl]-$N^2$-acetyl-2'-deoxyguanosine (**dG.16**)*

A solution of *n*-$Bu_4NF$ (95mg, 0.36 mmol) in THF (2 mL) was added to a solution of compound **dG.15** (100 mg, 0.15 mmol) in THF (5 mL) at 0°C. The mixture was gradually warmed to room temperature and stirred for four hours. Silica gel (500 mg, 60-200 mesh) was added, and the mixture was evaporated *in vacuo*. The residue was purified by silica gel chromatography to yield $O^6$-[1-(2-nitrophenyl)ethyl]-N'-acetyl-2'-deoxyguanosine **dG.16** (43 mg, 64%, 1:1 mixture of diastereomers).

*$^1H$ NMR (400 MHz, DMSO-$d_6$) for diastereomers:* $\delta$ 10.19 and 10.18 (2 s, 1 H, $D_2O$ exchangeable, NH), 8.43 (2 s, 1 H, H-8), 8.04 (d, *J* = 8.2 Hz, Ph-H), 7.79 - 7.74 (m, 2 H, Ph-H), 7.56 (t, 1 H, *J* = 8.1 Hz, Ph-H), 6.84 (m, 1 H, PhCH), 6.26 (t, 1 H, *J* = 6.8 Hz, H-1'), 5.29 (2 d, 1 H, $D_2O$ exchangeable, 5'-OH), 4.88 (m, 1 H, $D_2O$ exchangeable, 3'-OH), 4.39 (m, 1 H, H-3'), 3.82 (m, 1 H, H-4'), 3.56 (m, 1 H, H-5'b), 3.51 (m, 1 H, H-5'a), 2.56 (m, 1 H, H-2'a), 2.23 (m, 1 H, H-2'b), 2.12 (s, 3 H, $CH_3CO$), 1.79 (2 d, J = 6.4 Hz, $CH_3$);

*ToF-MS* (*ESI*)*:* For the molecular ion $C_{20}H_{21}N_6O_7$ $[M-H]^-$, the calculated mass was 457.1472, and the observed mass was 457.1392.

*$O^6$-[1-(2-Nitrophenyl)ethyl]-2'-deoxyguanosine-5'-triphosphate (**WW2p143**)*

Compound **dG.16** (25 mg, 0.055 mmol) and proton sponge (23 mg, 0.11 mmol) were evaporated three times from anhydrous pyridine (2 mL) and dissolved in trimethylphosphate (0.3 mL). $POCl_3$ (8 $\mu$L, 0.08 mmol) was added, and the mixture was stirred for two hours at 0°C. A solution of bis-tri-*n*-butylammonium pyrophosphate (130 mg, 0.28 mmol) and tri-*n*-butylamine (55 $\mu$L) in anhydrous DMF (0.55 mL) was added. After five minutes of stirring, triethylammonium bicarbonate buffer (1 M, pH 7.5; 10 mL) was added. The reaction was stirred for one hour at room temperature and then lyophilized to dryness. The residue was dissolved in water (10 mL), filtered, and part of the solution was purified with reverse-phase HPLC using a Perkin Elmer OD-300 $C_{18}$ column (4.6 $\times$ 250 mm) to yield $O^6$-[1-(2-nitrophenyl)ethyl]-$N^2$-acetyl-2'-deoxyguanosine-5'-triphosphate. Mobile phase: A, 100 mM triethylammonium acetate (TEAA) in water (pH 7.0); B, 100 mM TEAA in water/$CH_3CN$ (30:70). HPLC purification was achieved using a linear gradient of 5-50% B for 40 minutes and then 50-90% B for 10 minutes. The purified triphosphate was then treated with concentrated ammonium hydroxide (2 mL, 27%) at 60°C for three hours. Purification using reverse-phase HPLC was performed as described above to yield $O^6$-[1-(2-nitrophenyl)ethyl]-2'-deoxyguanosine-5'-triphosphate **WW2p143** (1:1 mixture of diastereomers).

*$^1H$ NMR (400 MHz, $D_2O$) for diastereomers:* 8.26 and 8.25 (2 s, 1 H, H-8), 8.02 (d, *J* = 8.2 Hz, Ph-H), 7.88 (d, 1 H, *J* = 7.8 Hz, Ph-H), 7.72 (t, 1 H, *J* = 7.6 Hz, Ph-H), 7.53 (t, 1 H, *J* = 8.2 Hz, Ph-H), 6.71 (m, 1 H, PhCH), 6.34 (t, 1 H, *J* = 6.8 Hz, H-1'), 4.25-4.16 (m, 3 H, H-4' and H-5'), 2.80 (m, 1 H, H-2'a), 2.51 (m, 1 H, H-2'b), 1.86 (d, 1 H, *J* = 6.4 Hz, $CH_3$);

*$^{31}P$ NMR (162 MHz, $D_2O$) for diastereomers:* $\delta$ -5.18 (d, *J* = 20.4 Hz), -10.25 (d, *J* = 19.3 Hz), -21.07 (t, *J* = 19.8 Hz);

*ToF-MS (ESI):* For the molecular ion $C_{18}H_{22}N_6O_{15}P_3$ $[M-H]^-$, the calculated mass was 655.0356, and the observed mass was 655.0430.

**Synthesis of 6-ROX labeled *O*$^6$-{1-[4-(3-amino-1-propynyl)-2-nitrophenyl]ethyl}-2'-deoxyguanosine-5'-triphosphate (WW3p008)**

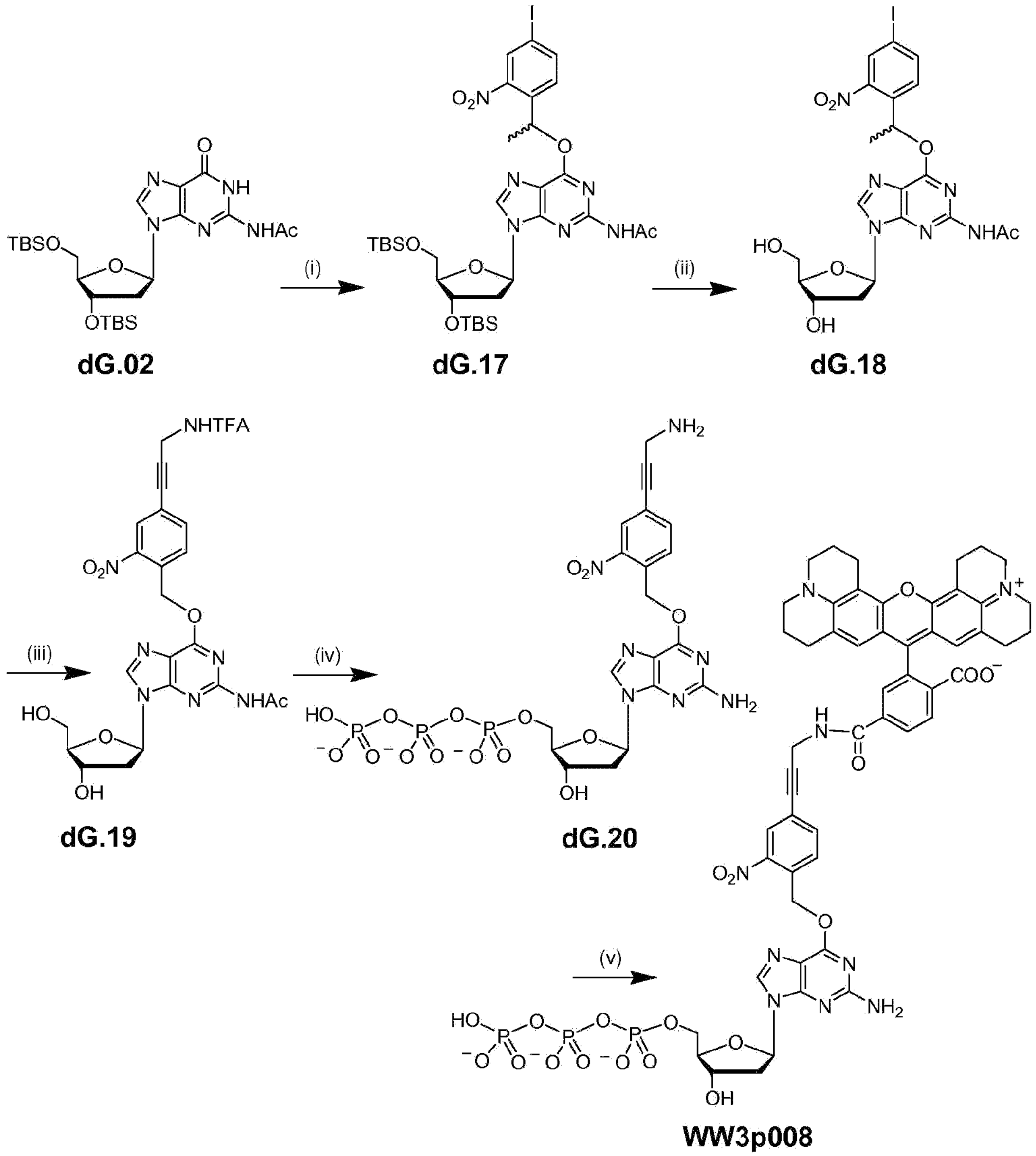


dG.02 dG.17 dG.18 dG.19 dG.20 WW3p008

**Scheme**. Synthesis of *6-ROX labeled $O^6$-{1-[4-(3-amino-1-propynyl)-2-nitrophenyl]ethyl}-2'-deoxyguanosine-5'-triphosphate*. (i) 1-(4-iodo-2-nitrophenyl)ethanol, $PPh_3$, DIAD, anhydrous THF, room temperature, overnight, 76%; (ii) *n*-$Bu_4NF$, THF, 0°C, then gradually warmed to room temperature, TWO hours, 52%; (iii) *N*-propargyltrifluoroacetamide, $Pd(PPh_3)_4$, CuI, $Et_3N$, anhydrous DMF, four hours, 96%; (iv) $POCl_3$, proton sponge, $(MeO)_3PO$, 0°C, two hours; $(n\text{-}Bu_3NH)_2H_2P_2O_7$, *n*-$Bu_3N$, DMF, five minutes; 1 M $HNEt_3HCO_3$, one hour; $NH_4OH$, 60°C, six hours; (v) 6-ROX-SE, 0.1 M $Na_2CO_3/NaHCO_3$ buffer (pH 9.2), one hour.

*$O^6$-[1-(4-Iodo-2-nitrophenyl)ethyl]-$N^2$-acetyl-3',5'-bis-O-(tert-butyldimethylsilyl)-2'-deoxyguanosine (**dG.17**)*

A solution of compound **dG.02** (146 mg, 0.27 mmol), 1-(4-iodo-2-nitrophenyl)ethanol (79 mg, 0.27 mmol) and $PPh_3$ (106 mg, 0.4 mmol) in anhydrous THF (2 mL) was treated with diisopropyl azodicarboxylate (DIAD, 79 μL, 0.4 mmol) and stirred overnight at room temperature. The mixture was diluted with $CH_2Cl_2$ (20 mL), washed once with saturated $NH_4Cl$ solution (10 mL), dried over $Na_2SO_4$, concentrated, and purified by silica gel chromatography to yield $O^6$-[1-(4-iodo-2-nitrophenyl)ethyl]-$N^2$-acetyl-3',5'-bis-*O*-(*tert*-butyldimethylsilyl)-2'-deoxyguanosine **dG.17** (168 mg, 76%, 1:1 mixture of diastereomers) as a yellow foam.

*$^1H$ NMR (400 MHz, $CDCl_3$) for diastereomers:* δ 8.20 (s, 1 H, Ph-H), 8.13 and 8.12 (2 s, 1 H, H-8), 7.87 (d, 1 H, *J* = 8.0 Hz, Ph-H), 7.73 (br s, 1 H, NH), 7.55 (d, 1 H, *J* = 8.0 Hz, Ph-H), 6.62 (m, 1 H, PhCH), 6.35 (t, 1 H, *J* = 6.5 Hz, H-1'), 4.56 (m, 1 H, H-3'), 3.98 (m, 1 H, H-4'), 3.82 (m, 1 H, H-5'a), 3.77 (m, 1 H, H-5'b), 2.50 (m, 1 H, H-2'a), 2.41 (m, 4 H, H-2'b and $CH_3CO$), 1.85 (d, *J* = 6.4 Hz, $CH_3$), 0.91 (s, 18 H, $(CH_3)_3CSi$), 0.09 (2 s, 12 H, $(CH_3)_2Si$).

*$O^6$-[1-(4-Iodo-2-nitrophenyl)ethyl]-$N^2$-acetyl-2'-deoxyguanosine (**dG.18**)*

A solution of *n*-$Bu_4NF$ (125 mg, 0.48 mmol) in THF (1.5 mL) was added to a solution of compound **dG.17** (155 mg, 0.19 mmol) in THF (2 mL) at 0°C. The reaction mixture was gradually warmed to room temperature and stirred for two hours. Silica gel 60 (1 g, 60-200 mesh) was added, and the mixture was evaporated *in vacuo* to dryness. The residue was purified by silica gel column chromatography to yield $O^6$-[1-(4-iodo-2-nitrophenyl)ethyl]-$N^2$-acetyl-2'-deoxyguanosine **dG.18** (58 mg, 52%, 1:1 mixture of diastereomers) as a white foam.

*$^1H$ NMR (400 MHz, DMSO-$d_6$) for diastereomers:* δ 10.21 and 10.20 (2 s, 1 H, $D_2O$ exchangeable, NH), 8.43 (2 s, 1 H, H-8), 8.34 (2 s, Ph-H), 8.08 (2 d, 1 *H, J* = 8.3 Hz, Ph-H), 7.54 (2 d, 1 H, *J* = 8.3 Hz, Ph-H), 6.75 (m, 1 H, PhCH), 6.27 (t, 1 H, *J* = 6.4 Hz, H-1'), 5.30 (m, 1 H, $D_2O$ exchangeable, 5'-OH), 4.89 (m, 1 H, $D_2O$ exchangeable, 3'-OH), 4.39 (m, 1 H, H-3'), 3.82 (m, 1 H, H-4'), 3.56 (m, 1 H, H-5'b), 3.50 (m, 1 H, H-5'a), 2.60 (m, 1 H, H-2'a), 2.22 (m, 1 H, H-2'b), 2.12 (s, 3 H, $CH_3CO$), 1.75 (2 d, *J* = 6.4 Hz, $CH_3$).

*$O^6$-{1-[4-(3-Trifluoroacetamido-1-propynyl)-2-nitrophenyl]ethyl}-$N^2$-acetyl-2'-deoxyguanosine (**dG.19**)*

A solution of compound **dG.18** (58 mg, 0.1 mmol), *N*-propargyl trifluoroacetylamide (45 mg, 0.3 mmol), tetrakis(triphenylphosphine)-palladium(0) (11.5 mg, 0.01 mmol), copper(I) iodide (3.8 mg, 0.02 mmol) and triethylamine (27 μL, 0.19 mmol) was stirred at room temperature for four hours. Methanol (1 mL), $CH_2Cl_2$ (1 mL), and sodium bicarbonate (80 mg, 0.95 mmol) were added, and the mixture was stirred for an additional half hour, then concentrated *in vacuo* and purified by column chromatography on silica gel to yield $O^6$-{1-[4-(3-trifluoroacetamido-1-propynyl)-2-nitrophenyl]ethyl}-$N^2$-acetyl-2'-deoxyguanosine **dG.19** (58 mg, 96%, 1:1 mixture of diastereomers).

*$^1H$ NMR (400 MHz, DMSO-$d_6$) for diastereomers:* δ 10.21 and 10.20 (2 s. 1 H, $D_2O$ exchangeable, NH), 10.08 (br, 1 H, $NHCOCF_3$), 8.43 (2 s, 1 H, H-8), 8.06 (s, 1 H, Ph-H), 7.77 (m, 2 H, Ph-H), 6.78 (m, 1 H, PhCH), 6.28 (t, 1 *H, J* = 6.4 Hz, H-1'), 5.29 (2 d, 1 H, $D_2O$ exchangeable, 5'-OH), 4.89 (t, 1 H, $D_2O$ exchangeable, 3'-OH), 4.39 (m, 1 H, H-3'), 4.29 (d, 2 H, $CH_2$), 3.82 (m, 2 H, H-4'), 3.50 (m, 1 H, H-5'a), 3.44 (m, 1 H, H-5'b), 2.67 (m, 1 H, H-2'a), 2.23 (m, 1 H, H-2'b), 2.11 (s, 3 H, $CH_3$), 1.78 (d, 3 H, *J* = 6.4 Hz, $CH_3$);

*$O^6$-{1-[4-(3-Amido-1-propynyl)-]-nitrophenyl]ethyl}-2'-deoxyguanosine-5'-triphosphate **(dG.20)***

Compound **dG.19** (44 mg, 0.07 mmol) and proton sponge (30 mg, 0.14 mmol) were evaporated three times from anhydrous pyridine (3 mL) and dissolved in trimethylphosphate (0.5 mL). $POCl_3$ (10 μL, 0.11 mmol) was added, and the mixture was stirred for three hours at 0°C. A solution of bis-tri-*n*-butylammonium pyrophosphate (166 mg, 0.35 mmol) and tri-*n*-butylamine (70 μL) in anhydrous DMF (0.7 mL) was added. After five minutes of stirring, triethylammonium bicarbonate buffer (1 M, pH 7.5; 10 mL) was added. The reaction was stirred for one hour at room temperature and then lyophilized to dryness. The residue was dissolved in water (10 mL), filtered, and part of the solution was purified with reverse-phase HPLC using a Perkin Elmer OD-300 $C_{18}$ column (4.6 x 250 mm) to yield $O^6$-{1-[4-(3-tritluoroacetamido-1-propynyl)-2-nitrophenyl]ethyl}-$N^2$-acetyl-2'-deoxyguanosine-5'-triphosphate. Mobile phase: A, 100 mM triethylammonium acetate (TEAA) in water (pH 7.0);

B, 100 mM TEAA in water/$CH_3CN$ (30:70). HPLC purification was achieved usinga linear gradient of 5-50% B for 20 minutes and then 50-90% B for 10 minutes. The purified triphosphate was then treated with concentrated ammonium hydroxide (2 mL, 27%) at 60°C for six hours to yield $O^6$-{1-[4-(3-amido-1-propynyl)-2-nitrophenyl]ethyl}-2'-deoxyguanosine-5'-triphosphate **dG.20** (1:1 mixture of diastereomers).

*$^1H$ NMR (400 MHz, $D_2O$) for diastereomers:* 8.21 and 8.20 (2 s, 1 H, H-8), 7.85 and 7.75 (2 s, 1 H, Ph-H), 7.64 (m, 1 H, Ph-H), 7.45 (m, 1 H, Ph-H), 6.53 (m, 1 H, PhCH), 6.28 (m, 1 H, H-1'), 4.23 - 4.12 (m, 3 H, H-4' and H-5'), 3.97 (s, 2 H, $CH_2$), 2.70 (m, 1 H, H-2'a), 2.50 (m, 1 H, H-2'b), 1.74 (m, 1 H, $CH_3$);

*$^{31}P$ NMR (162 MHz, $D_2O$) for diastereomers:* δ -5.53 (d, *J* = 20.1 Hz), -10.50 (d, *J* = 19.3 Hz), -21.29 (t, *J* = 19.8 Hz);

*ToF-MS (ESI):* For the molecular ion $C_{21}H_{25}N_7O_{15}P_3$ $[M-H]^-$, the calculated mass was 708.0622, and the observed mass was 708.0609.

*6-ROX lableled $O^6$-{1-[4-(3-Amido-1-propynyl)-2-nitrophenyl]ethyl}-2'-deoxyguanosine-5'-triphosphate (**WW3p008**)*

A solution of 6-ROX-SE (3 mg, 4.7 μmol) in anhydrous DMSO (120 μL) was added to a solution of triphosphate **dG.20** (1.45 μmol) in $Na_2CO_3/NaHCO_3$ buffer (0.1 M, pH 9.2; 0.3 mL) and incubated at room temperature for one hour. The reaction was purified with reverse-phase HPLC using a Perkin Elmer OD-300 $C_{18}$ column (4.6 × 250 mm) to yield the 6-ROX labeled triphosphate **WW3p008.** Mobile phase: A, 100 mM TEAA in water (pH 7.0); B, 100 mM TEAA in water/$CH_3CN$ (30:70). HPLC purification was achieved using a linear gradient of 5-50% B for 20 minutes and then 50-90% B for 10 minutes. The concentration of **WW3p008** was estimated by adsorption spectroscopy using the extinction coefficient of the 6-ROX dye *(i.e.,* 82,000 at 575 nm).

**Separation of the two diastereoisomers of *O*⁶-{1-[4-(3-amino-1-propynyl)-2-nitrophenyl]ethyl}-2'-deoxyguanosine-5'-triphosphate (dG.20 ds1 and dG.20 ds2)**

Separation of the two diastereoisomers of **dG.20** was performed by reverse-phase HPLC using a Perkin Elmer OD-300 $C_{18}$ column (4.6 x 250 mm) to yield $O^6$-{(*R or S*)-1-[4-(3-amino-1-propynyl)-2-nitrophenyl]ethyl}-2'-deoxyguanosine triphosphate **dG.20 ds1** (single diastereoisomer, absolute configuration not determined) and $O^6$-{(*S or R)*-1-[4-(3-amino-1-propynyl)-2-nitrophenyl]ethyl}-2'-deoxyguanosine triphosphate **dG.20 ds2** (single diastereoisomer, absolute configuration not determined). Mobile phase: A, 100 mM triethylammonium acetate (TEAA) in water (pH 7.0); B, 100 mM TEAA in water/$CH_3CN$ (30:70). HPLC purification was achieved using a linear gradient of 5-25% B for 70 minutes and then 25-50% B for 30 minutes.

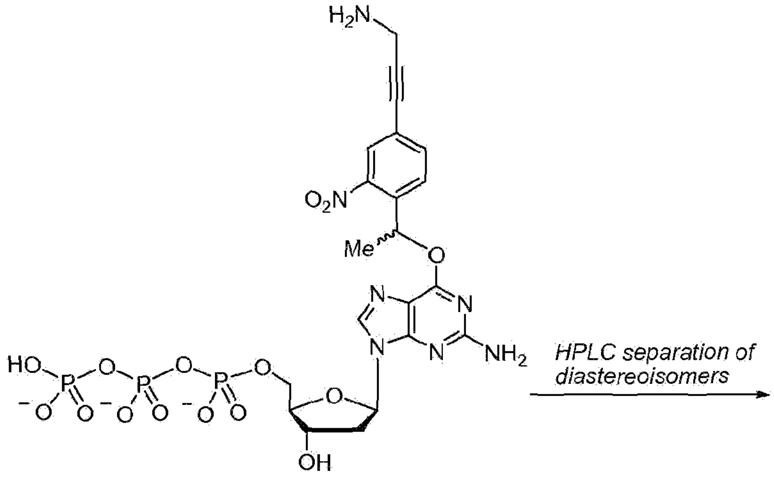


**dG.20**

*1:1 mixture of diastereoisomers*

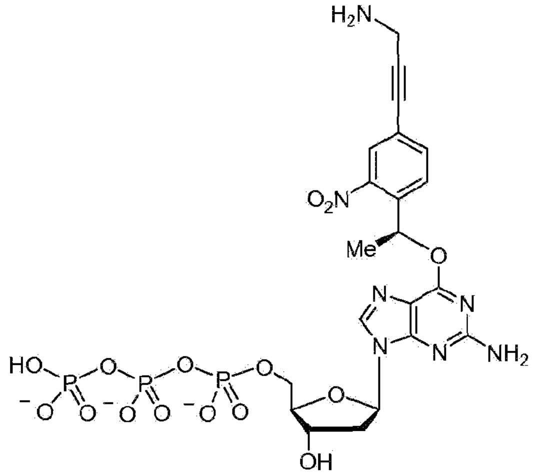

**dG.20 ds1**

*Fast eluting single diastereoisomer, absolute configuration not determined, drawing is representative*

+

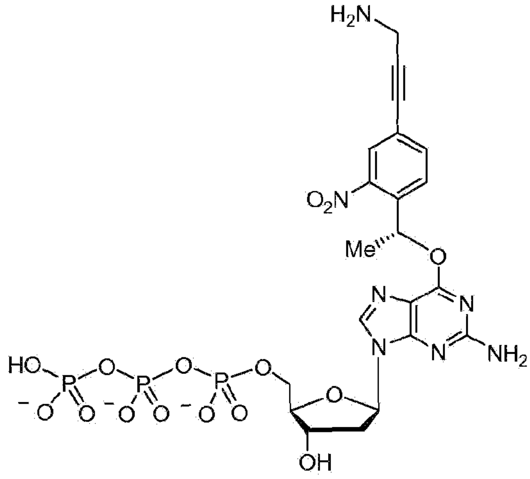

**dG.20 ds2**

*Slow eluting single diastereoisomer, absolute configuration not determined, drawing is representative*

**Synthesis of 6-ROX labeled single diastereoisomer *O*⁶-{(*R or S*)-1-[4-(3-amino-1-propynyl)-2-nitrophenyl)ethyl}-2'-deoxyguanosine-5'-triphosphate (WW3p037)**

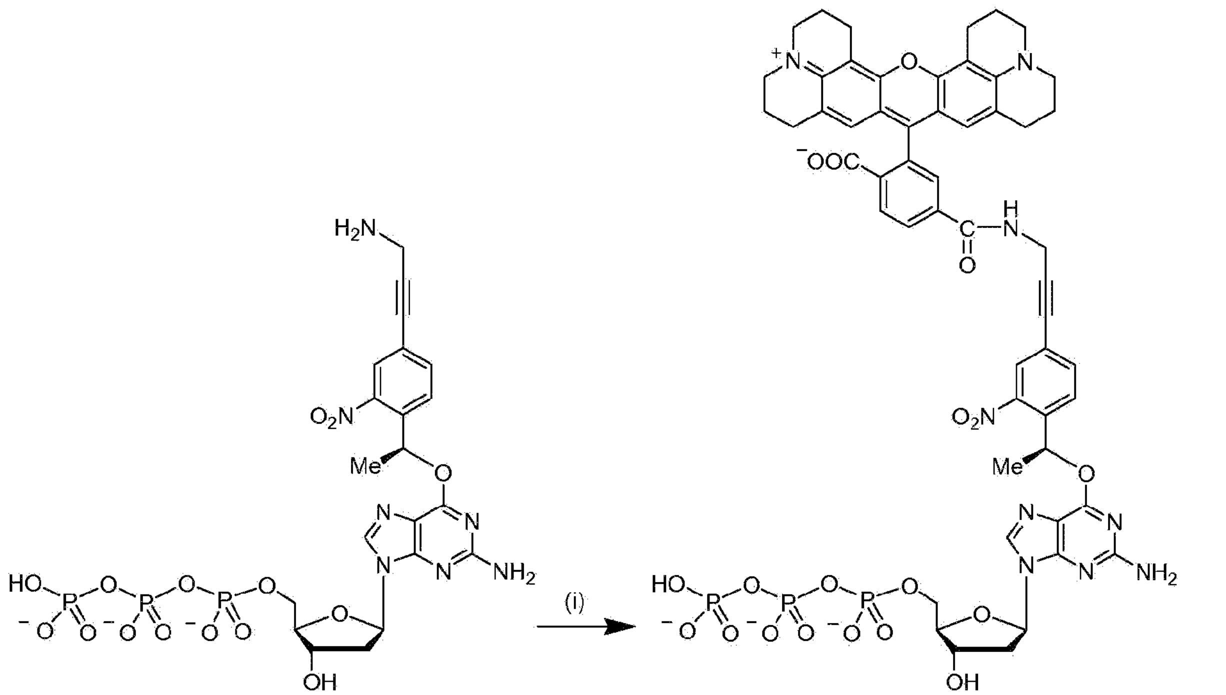


**dG.20 ds1**
*absolute configuration undetermined, drawing is representative*

**WW3p037**
*absolute configuration undetermined, drawing is representative*

**Scheme.** Synthesis of *6-ROX labeled single diastereoisomer $O^6$-{(R or S)-1-[4-(3-amino-1-propynyl)-2-nitrophenyl]ethyl}-2'-deoxyguanosine-5'-triphosphate.* (i) 6-ROX-SE, 0.1 M $NaHCO_3/Na_2CO_3$, pH 9.2, one hour.

*6-ROX labeled single diastereoisomer $O^6$-{(R or S)-1-[4-(3-amino-1-propynyl)-2-nitrophenyl]ethyl}-2'-deoxyguanosine-5'-triphosphate (**WW3p037**)*

A solution of 6-ROX-SE (1.5 mg, 2.38 μmol) in anhydrous DMSO (120 μL) was added to a solution of triphosphate **dG.20 ds1** (0.67 μmol, single diastereoisomer, absolute configuration not determined) in $Na_2CO_3/NaHCO_3$ buffer (0.1 M, pH 9.2; 150 μL) and incubated at room temperature for one hour. The reaction was purified by reverse-phase HPLC using a Perkin Elmer OD-300 $C_{18}$ column (4.6 × 250 mm) to yield the 6-ROX labeled single diastereoisomer triphosphate **WW3p037.** Mobile phase: A, 100 mM triethylammonium acetate (TEAA) in water (pH 7.0); B, 100 mM TEAA in water/$CH_3CN$

(30:70). HPLC purification was achieved using a linear gradient of 5-50% B for 20 minutes and then 50-90% B for 20 minutes. The concentration of **WW3p037** was estimated by adsorption spectroscopy using the extinction coefficient of the 6-ROX dye *(i.e.,* 82,000 at 575 nm).

**Synthesis of 6-ROX labeled single diastereoisomer *$O^6$*-{(*S or R*)-1-[4-(3-amino-1-propynyl)-2-nitrophenyl]ethyl}-2'-deoxyguanosine-5'-triphosphate** (**WW3p039**)

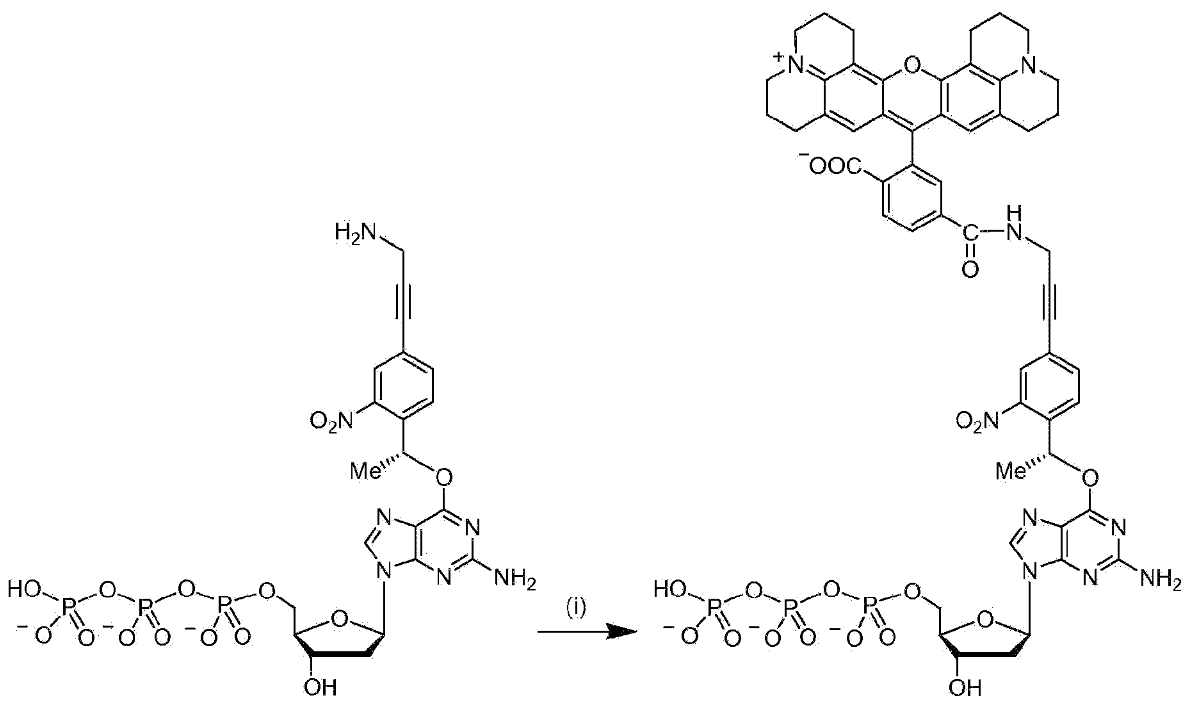


**dG.20 ds2**
*absolute configuration undetermined, drawing is representative*

**WW3p039**
*absolute configuration undetermined, drawing is representative*

**Scheme.** Synthesis of *6-ROX labeled single diastereoisomer $O^6$-{(S or R)-1-[4-(3-amino-1-propynyl)-2-nitrophenyl]ethyl}-2'-deoxyguanosine-5'-triphosphate.* (i) 6-ROX-SE, 0.1 M $NaHCO_3/Na_2CO_3$, pH 9.2, one hour.

*6-ROX labeled single diastereoisomer $O^6$-{(S or R)-1-[4-(3-amino-1-propynyl)-2-nitrophenyl]ethyl}-2'-deoxyguanosine-5'-triphosphate (**WW3p039**)*

A solution of 6-ROX-SE (2.5 mg, 3.96 μmol) in anhydrous DMSO (200 μL) was added to a solution of triphosphate **dG.20 ds2** (0.97 μmol, single diastereoisomer, absolute configuration not determined) in $Na_2CO_3/NaHCO_3$ buffer (0.1 M, pH 9.2; 150 μL) and incubated at room temperature for one hour. The reaction was purified by reverse-phase HPLC using a Perkin Elmer OD-300 $C_{18}$ column (4.6 x 250 mm) to yield the 6-ROX labeled single diastereoisomer triphosphate **WW3p039.** Mobile phase: A, 100 mM triethylammonium acetate (TEAA) in water (pH 7.0); B, 100 mM TEAA in water/$CH_3CN$ (30:70). HPLC purification was achieved using a linear gradient of 5-50% B for 20 minutes and then 50-90% B for 20 minutes. The concentration of **WW3p039** was estimated by adsorption spectroscopy using the extinction coefficient of the 6-ROX dye (*i.e.,* 82,000 at 575 nm).

**Synthesis of 6-ROX labeled single diastereoisomer $O^6$-{(*R or S*)-1-{4-[3-(6-amino-caproyl)amino-1-propynyl]-2-nitrophenyl}ethyl}-2'-deoxyguanosine-5'-triphosphate (WW3p041)**

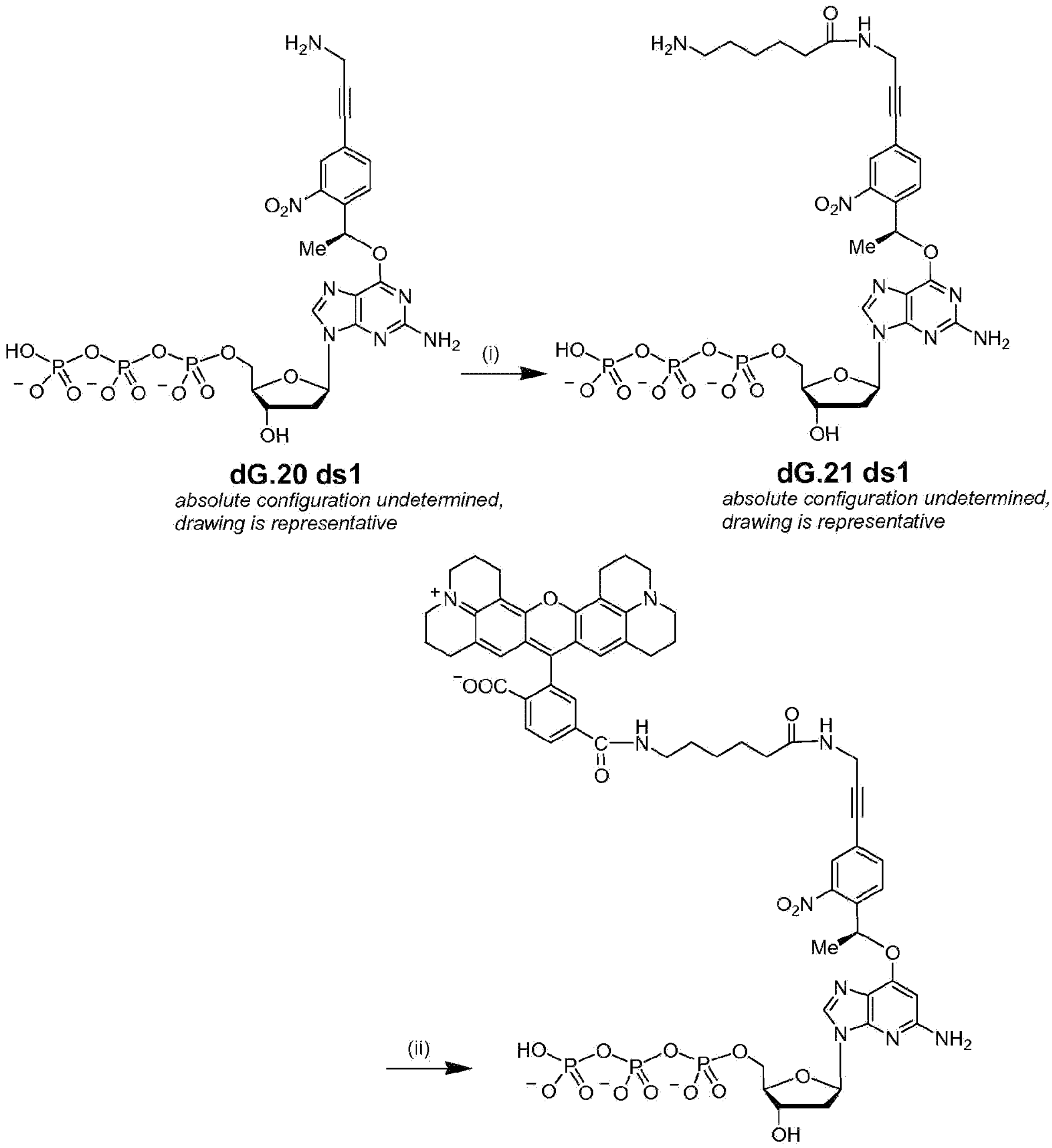


**dG.20 ds1**

*absolute configuration undetermined, drawing is representative*

**dG.21 ds1**

*absolute configuration undetermined, drawing is representative*

**WW3p041**

*absolute configuration undetermined, drawing is representative*

**Scheme.** Synthesis of *6-ROX labeled single diastereoisomer $O^6$-{(R or S)-1-{4-[3-(6-aminocaproyl)amino-1-propynyl]-2-nitrophenyl}ethyl}-2'-deoxyguanosine triphosphate.* (i) 6-*N*-(trifluoroacetyl)aminocaproic acid *N*-succinimidyl ester, 0.1 M $NaHCO_3/Na_2CO_3$, pH 9.2, one hour; $NH_4OH$, one hour; (ii) 6-ROX-SE, 0.1 M $NaHCO_3/Na_2CO_3$, pH 9.2, one hour.

*$O^6$-{(R or S)-1-{4-[3-(6-Aminocaproyl)amino-1-propynyl]-2-nitrophenyl}ethyl}-2'-deoxyguanosine-5'-triphosphate (single diastereoisomer **dG.21 ds1**)*

A solution of 6-*N*-(trifluoroacetyl)aminocaproic acid *N*-succinimidyl ester (1.0 mg, 3.08 μmol) in anhydrous DMSO (20 μL) was added to a solution of triphosphate **dG.20 ds1** (0.89 μmol, single diastereoisomer, absolute configuration not determined) in $Na_2CO_3$/$NaHCO_3$ buffer (0.1 M, pH 9.2; 200 μL) and incubated at room temperature for one hour. Concentrated ammonium hydroxide (25% aq., 0.5 mL) was added, and the mixture was incubated at room temperature for another hour. The reaction was purified by reverse-phase HPLC using a Perkin Elmer OD-300 $C_{18}$ column (4.6 × 250 mm) to yield the triphosphate **dG.21 ds1** (single diastereoisomer, absolute configuration not determined). Mobile phase: A, 100 mM triethylammonium acetate (TEAA) in water (pH 7.0); B, 100 mM TEAA in water/$CH_3CN$ (30:70). HPLC purification was achieved using a linear gradient of 5-50% B for 20 minutes and then 50-90% B for 10 minutes.

*Synthesis of 6-ROX labeled single diastereoisomer $O^6$-{(R or S)-1-{4-[3-(6-amino-caproyl)amino-1-propynyl]-2-nitrophenyl}ethyl}-2'-deoxyguanosine-5'- triphosphate (WW3p041)*

A solution of 6-ROX-SE (1.5 mg, 2.34 μmol) in anhydrous DMSO (120 μL) was added to a solution of triphosphate **dG.21 ds1** (0.59 μmol, single diastereoisomer, absolute configuration not determined) in $Na_2CO_3$/$NaHCO_3$ buffer (0.1 M, pH 9.2; 200 μL) and incubated at room temperature for one hour. The reaction was purified by reverse-phase HPLC using a Perkin Elmer OD-300 $C_{18}$ column (4.6 x 250 mm) to yield the 6-ROX labeled single diastereoisomer triphosphate **WW3p041.** Mobile phase: A, 100 mM triethylammonium acetate (TEAA) in water (pH 7.0); B, 100 mM TEAA in water/$CH_3CN$ (30:70). HPLC purification was achieved using a linear gradient of 5-50% B for 20 minutes and then 50-90% B for 20 minutes. The concentration of **WW3p041** was estimated by adsorption spectroscopy using the extinction coefficient of the 6-ROX dye (*i.e.,* 82,000 at 575 nm).

Example 5- Polymerase End-Point (PEP) assays:

Numerous groups have employed qualitative, Sanger-based assays to estimate relative incorporation efficiencies of terminating nucleotide analogs compared to their natural nucleotide counterparts. Although useful, the ability to assay modified nucleotide analogs in the absence of natural nucleotides is not feasible using these assays. These two limitations led to a quantitative, polymerase end-point (PEP) assay, which is utilized in a high-throughput manner for screening numerous modified nucleotides against a number of well-characterized, commercially available polymerases. Identification of lead compounds with a specific DNA polymerase could then be prioritized for further kinetic studies. The PEP assay is designed with the polymerase concentration in excess of the primer/template complex (*i.e.,* this complex is fully bounded with polymerase at the start of the titration), thereby limiting reaction to nucleotide binding and nucleotyl coupling steps. Limiting amounts of the desired nucleotide are then titrated across the appropriate concentration range (generally three orders of magnitude) to observe extension of a dye-labeled primer by gel electrophoresis. The end-point concentration is determined from a semi-log plot where the number of moles of substrate and product are equal, called the $IC_{50}$ *(i.e.,* incorporation concentration at 50%) value.

For all polymerases evaluated in this study, 40 nM of oligo-template (5'-TACGGAGCA-G<u>**T**</u>ACT-GGCCGTCGTTTTACA, interrogation base is underlined and bolded) was annealed to 5 nM BODIPY-FL labeled primer (5'-TTGTAAAACGACGGCCAGT) in 1xThermoPol buffer (20 mM Tris-HCl, pH 8.8; 10 mM $(NH_4)_2SO_4$; 10 mM KCl; 2 mM $MgSO_4$; 0.1% Triton X-100, New England BioLabs) at 80°C for 30 seconds, 57°C for 30 seconds, and then cooled to 4°C. The primer/template complex is then diluted by one-half (*i.e.,* its final concentration is 2.5 nM in a volume of 10 μL) by the addition of DNA polymerase, nucleotide analog, and ThermoPol buffer. This defines the lower limit of the $IC_{50}$ value for nucleotide titrations to 1.25 nM (*i.e.,* [primer] = [primer +1]). Polymerase reactions were incubated at their appropriate temperature for 10 minutes, then cooled to 4°C and quenched with 10 μL of stop solution (98% deionized formamide; 10 mM $Na_2$EDTA, pH 8.0; 25mg/mL Blue Dextran, MW 2,000,000). Stopped reactions were heated to 90°C for 30 seconds, and then placed on ice. The extension products were analyzed on a 10% Long Ranger (Cambrex) polyacrylamide gel using an AB model 377 DNA sequencer, and the quantitative data are displayed as a linear-log plot of product formation *versus* compound concentration. The PEP assays were performed in triplicate for each DNA polymerase/ nucleotide analog combination to calculate its $IC_{50}$ values ± one standard deviation.

The number of activity units for eight commercially available, 3'-exonuclease deficient (3'-exo-) DNA polymerases was first determined by titration with 2'-deoxyadenosine triphosphate (dATP, concentration range from 0.1 nM to 100 nM) with the goal of reaching the PEP $IC_{50}$ limit of 1.25 nM (data not shown). In general, increasing the number of units reduced the $IC_{50}$ values for dATP towards this limit, with exception with *Taq,* Therminator, and Therminator II. For these enzymes, there was an increase in $IC_{50}$ values for dATP with increasing enzyme concentration, which was not investigated further and presumed to be due to a direct relationship with increasing enzyme concentration and phosphorolysis. For these cases, the number of units used for subsequent PEP assays were those activity units that gave the lowest $IC_{50}$ values for dATP.

*Modified nucleotide titrations:*

WW1p129 and ddATP were then titrated using the PEP assay with the eight DNA polymerases (unit activities previously defined) in the concentration range of either 0.1 nM to 100 nM, 1 nM to 1 μM, 10 nM to 10 μM, or 100 nM to 100 μM (see Table 1). UV-light sensitive compounds were handled at all times in low light conditions to minimize conversion to dATP. These data show in all cases, except that for *Taq*FS*,* that WW1p129 is incorporated more efficiently (i.e., lower $IC_{50}$ value) than ddATP.

Table 1. Summary of PEP assay result for WW1p129 using eight different DNA polymerases

| DNA polymerase | IC50 values | | |
|---|---|---|---|
| | **dATP** | **WW1p129** | **ddATP** |
| *Bst :* 65°C | 1.2 ± 0.1 nM | 21 ± 3 nM | **0.37 ± 0.03 μM** |
| Klenow(3'-exo-): 37°C | 1.6 ± 0.1 nM | 4.3 ± 0.2 nM | 29 ± 5 nM |
| *Taq*: 68°C | 5.5 ± 0.5 nM | **2.1 ± 0.2 μm** | **12.6 ± 0.9 μm** |
| *Taq* FS: 68°C | 5.3 ± 0.1 nM | **0.89 ± 0.06 μm** | 3.3 ± 0.1 nm |
| Therminator: 75°C | 2.3 ± 0.3 nM | 3.1 ± 0.4 nM | 9.7 ± 1.1 nM |
| Therminator II: 75°C | 4.4 ± 0.6 nM | 7.8 ± 0.7 nM | **0.23 ± 0.03 μM** |
| Vent(3'-exo-): 72°C | 1.6 ± 0.2 nM | 2.1 ± 0.2 nM | **0.55 ± 0.04 μM** |
| DeepVent(3'-exo-): 72°C | 2.8 ± 0.2 nM | 11.0 ± 0.6 nM | **3.4 ± 0.4 μM** |

PEP assays have also been performed using a number of photocleavable terminating nucleotides using Bst polymerase and Therminator DNA polymerase (Table 2), which shows the compounds are effectively incorporated. The data in the Table 2 suggests that the compounds according to the invention are excellent substrates.

**Table 2: Comparison of IC50 values with Bst and Therminator polymerases**

| compound | Bst | Therminator |
|---|---|---|
| WW1p129 | 39 ± 9 nm | 2.5 ± 0.4 nm |
| VL3p03085 | 138 ± 38 nm | 1.1 ± 0.1 nm |
| WW2p044 | 57 ± 11 nm | 1.8 ± 0.2 nm |
| WW2p077 | 3.8 ± 0.2 micromolar | 6.9 ± 0.5 nm |
| WW2p050 | n/a | 4.4 ± 0.6 nm |
| WW2p075 | n/a | 3.8 ± 1.1 nm |
| WW2p080 | n/a | 3.0 ± 0.6 nm |
| WW2p121 | n/a | 6.3 ± 0.4 nm |

LABELED NUCLEOTIDES AND NUCLEOSIDES

Example 1: dA compounds

Synthesis of $N^6$-benzyl-2'-deoxyadenosine triphosphate (WW2p062)

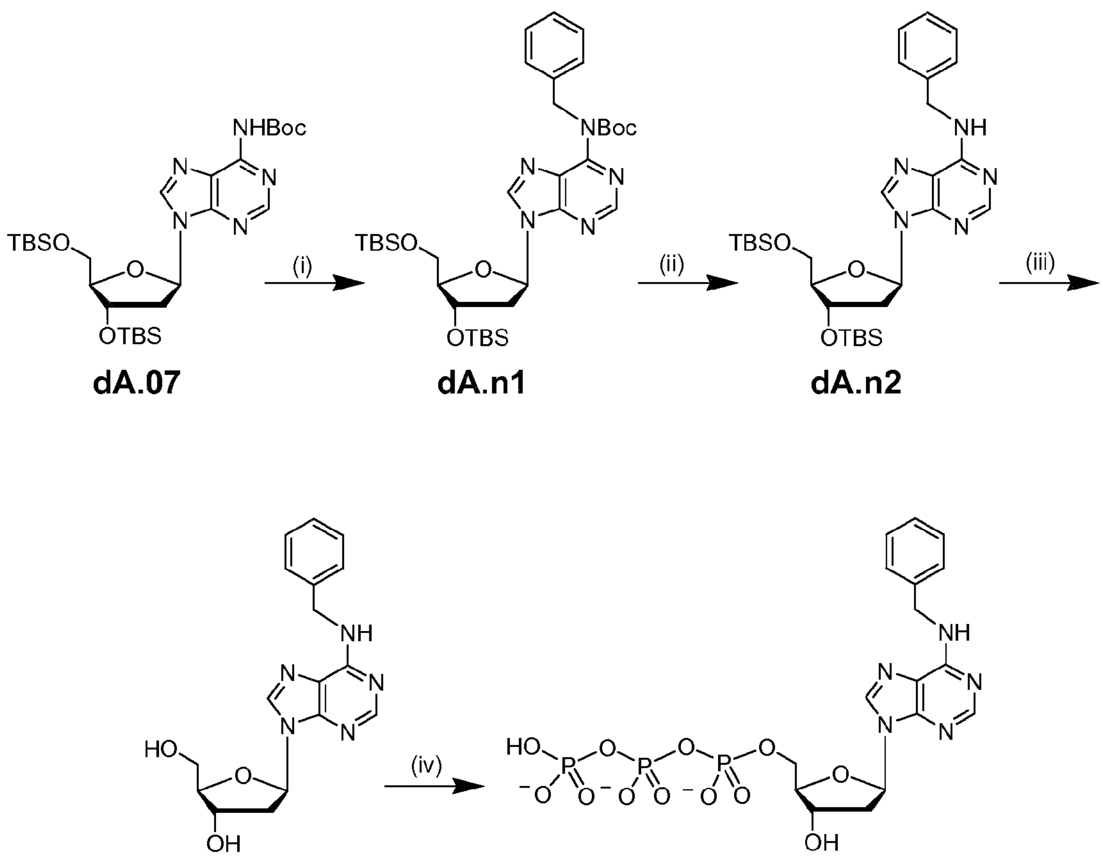


dA.07 dA.n1 dA.n2

dA.n3 WW2p062

**Scheme.** Synthesis of *$N^6$-benzyl-2'-deoxyadenosine-5'-triphosphate*. (i) NaH, DMF, benzyl bromide, 0°C, then gradually warmed to room temperature, 86%; (ii) $SiO_2$, vacuum, 70-80°C, 95%; (iii) *n*-$Bu_4NF$, THF, 99%; (iv) $POCl_3$, $(MeO)_3PO$, minus 20-30°C; $(n\text{-}Bu_3NH)_2H_2P_2O_7$, *n*-$Bu_3N$, DMF; 1 M $HNEt_3HCO_3$; 32%.

*$N^6$-tert-Butyloxycarbonyl-$N^6$-benzyl-3',5'-O-bis-tert-butyldimethylsilyl-2'-deoxyadenosine (**dA.n1**)*

NaH (18 mg, 0.75 mmol, dry) was added to a solution of compound **dA.07** (400 mg, 0.58 mmol) in anhydrous DMF (5 mL) at 0°C and stirred for 30 minutes. A solution of benzyl bromide (149 mg, 0.87 mmol) in anhydrous DMF (2.5 mL) was added dropwise. The mixture was gradually warmed to room temperature and stirred for two hours. DMF was removed *in vacuo,* and the residue was dissolved in ethyl acetate (60 mL), washed twice with saturated $NH_4Cl$ solution (40 mL each) and once with water (40 mL). The combined aqueous layer was extracted with ethyl acetate (10 mL), and the combined organic layer was dried over $Na_2SO_4$, concentrated *in vacuo,* and purified by silica gel column chromatography to yield *$N^6$-tert*-butyloxycarbonyl-*$N^6$*-benzyl-3',5'-*O*-bis-*tert*-butyldimethylsilyl-2'-deoxyadenosine **dA.n1** (398 mg, 86%) as a viscous oil.

*$^1H$ NMR (400 MHz, $CDCl_3$):* δ 8.72 (s, 1 H, H-8), 8.32 (s, 1 H, H-2), 7.39 (m, 2 H, Ph-H), 7.25 (m, 2 H, Ph-H), 7.18 (m, 1 H, Ph-H), 6.49 (t, 1 *H, J* = 6.4 Hz, H-1'), 5.28 (s, 2 H, Ph-$CH_2$), 4.62 (m, 1 H, H-3'), 4.01 (m, 1 H, H-4'), 3.85 (dd, 1 *H, J* = 4.4 and 11.2 Hz, H-5'a), 3.77 (dd, 1 H, *J* = 3.4 and 11.2 Hz, H-5'b), 2.61 (m, 1 H, H-2'a), 2.43 (m, 1 H, H-2'b), 1.65 (s, 9 H, $(CH_3)_3CO$), 0.96 (s, 18 H, $(CH_3)_3CSi$), 0.08 (2 s, 12 H, $(CH_3)_2Si$).

*$N^6$-Benzyl-3',5'-O-bis-tert-butyldimethylsilyl-2'-deoxyadenosine (**dA.n2**)*

Silica gel 60 (3.76 g, 100-200 mesh, activated by heating to 70-80°C under reduced pressure for 24 hours)

was added to a solution of compound **dA.n1** (376 mg, 0.56 mmol) in $CH_2Cl_2$ (20 mL), and the mixture was evaporated *in vacuo* to dryness. The residue was heated to 70-80°C under reduced pressure for two days, washed three times with methanol (30 mL each), and filtered using a buchi funnel. The combined filtrate was concentrated *in vacuo* and purified by silica gel column chromatography to yield $N^6$-benzyl-3',5'-*O*-bis-*tert*-butyldimethylsilyl-2'-deoxyadenosine **dA.n2** (305 mg, 95%) as a yellow foam.

*$^1H$ NMR (400 MHz, $COCl_3$):* $\delta$ 8.41 (s, 1 H, H-8), 8.07 (s, 1 H, H-2), 7.38 (m, 2 H, Ph-H), 7.33 (m, 2 H, Ph-H), 7.28 (m, 1 H, Ph-H), 6.45 (t, 1 H, *J* = 6.4 Hz, H-1'), 6.12 (br s, 1 H, 6-NH), 4.87 (br s, 2 H, Ph-$CH_2$), 4.62 (m, 1 H, H-3'), 4.01 (m, 1 H, H-4'), 3.87 (dd, 1 H, *J* = 4.2 and 11.2 Hz, H-5'a), 3.77 (dd, 1 H, *J* = 3.2 and 11.2 Hz, H-5'b), 2.64 (m, 1 H, H-2'a), 2.44 (m, 1 H, H-2'b), 0.91 (s, 18 H, $(CH_3)_3CSi$), 0.09 (2 s, 12 H, $(CH_3)_2Si$-).

*$N^6$-Benzyl-2'-deoxyadenosine (**dA.n3**)*

A solution of *n*-$Bu_4NF$ (335 mg, 1.28 mmol) in THF (2.5 mL) was added to a solution of compound **dA.n2** (292 mg, 0.51 mmol) in THF (6 mL) at 0°C. The reaction mixture was gradually warmed to room temperature and stirred for two hours. Silica gel 60 (1 g) was added, and the mixture was *evaporated in vacuo* to dryness. The residue was purified by silica gel column chromatography to yield *N*$^6$-benzyl-2'-deoxyadenosine **dA.n3** (173 mg, 99%) as a white foam.

*$^1H$ NMR (400 MHz, $CD_3OD$):* $\delta$ 8.30 (s, 1 H, H-8), 8.25 (s, 1 H, H-2), 7.36 (m, 2 H, Ph-H), 7.31 (m, 2 H, Ph-H), 7.24 (m, 1 H, Ph-H), 6.42 (dd, 1 H, *J* = 6.0 and 7.9 Hz, H-1'), 4.81 (br s, 2 H, Ph-$CH_2$), 4.57 (m, 1 H, H-3'), 4.06 (m, 1 H, H-4'), 3.83 (m, 1 H, *J* = 2.9 and 12.3 Hz, H-5'a), 3.73 (dd, 1 H, *J* = 3.3 and 12.3 Hz, H-5'b), 2.79 (m, 1 H, H-2'a), 2.40 (m, 1 H, H-2'b).

*$N^6$-Benzyl-2'-deoxyadenosine-5'-triphosphate (**WW2p062**)*

$POCl_3$ (22 $\mu$L, 0.24 mmol) was added to a solution of compound **dA.10a** (42 mg, 0.12 mmol) in trimethylphosphate (0.5 mL) and maintained at minus 20-30°C for two hours. A solution of bis-tri-*n*-butylammonium pyrophosphate (285 mg, 0.6 mmol) and tri-n-butylamine (120 $\mu$L) in anhydrous DMF (1.2 mL) was added. After five minutes of stirring, triethylammonium bicarbonate buffer (1 M, pH 7.5; 10 mL) was added. The reaction was stirred at room temperature for one hour and then lyophilized to dryness. The residue was dissolved in water (10 mL), filtered, and purified by anion exchange chromatography using a Q Sepharose FF column (2.5 $\times$ 20 cm) with a linear gradient of $NH_4HCO_3$ (50 mM to 500 mM in 300 minutes) at a flow rate of 4.5 mL/min. The fractions containing triphosphate were combined and lyophilized to give *N*$^6$-benzyl-2'-deoxyadenosine-5'-triphosphate WW2p062 (24 mg, 32%) as a white fluffy solid.

*$^1H$ NMR (400 MHz, $D_2O$):* $\delta$ 8.43 (s, 1 H, H-8), 8.20 (s, 1 H, H-2), 7.39 - 7.30 (m, 5 H, Ph-H), 6.50 (t, 1 H, *J* = 6.4 Hz, H-1'), 4.85 (s, 2 H, Ph-$CH_2$), 4.31 (s, 1 H, H-4'), 4.22 (m, 2 H, H-5'a and H-5'b), 2.82 (m, 1 H, H-2'a), 2.62 (m, 1 H, H-2'b);

*$^{31}P$ NMR (162 MHz, $D_2O$):* $\delta$ -5.72 (d, *J* = 15.9 Hz), -10.78 (d, *J* = 15.4 Hz), -19.16 (t, *J* = 14.9 Hz);

*ToF-MS (ESI):* For the molecular ion $C_{17}H_{20}N_5O_{12}P_3Na$ $[M-2H+Na]^-$, the calculated mass was 602.0219, and the observed mass was 602.0363.

**Synthesis of 6-FAM labeled *N*$^6$-[4-(3-amino-1-propyl)benzyl]-2'-deoxyadenosine triphosphate (WW2p085)**

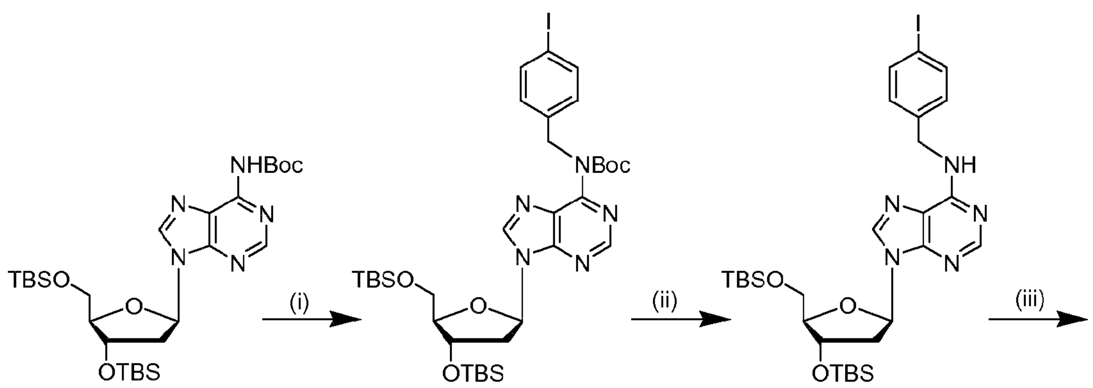


dA.07 dA.n4 dA.n5

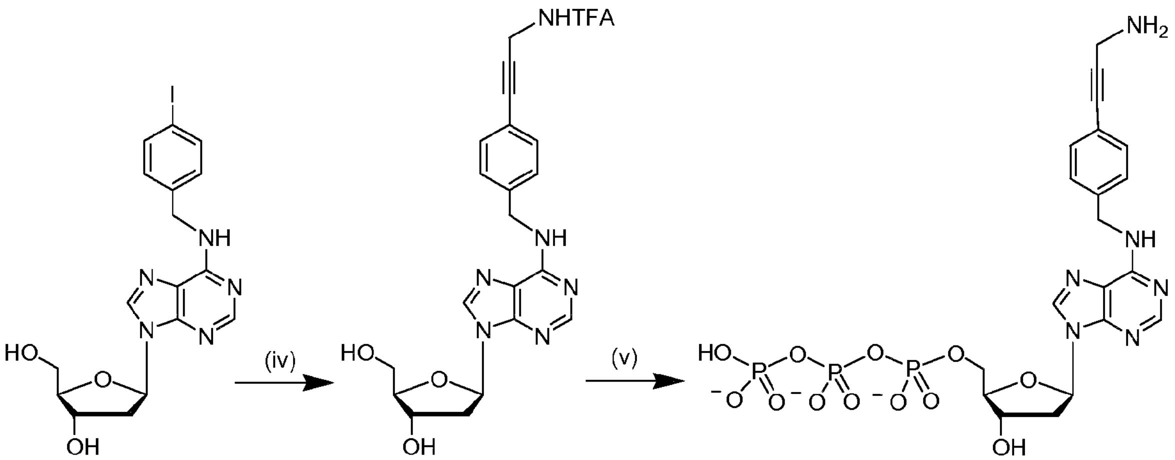


dA.n6 dA.n7 dA.n8

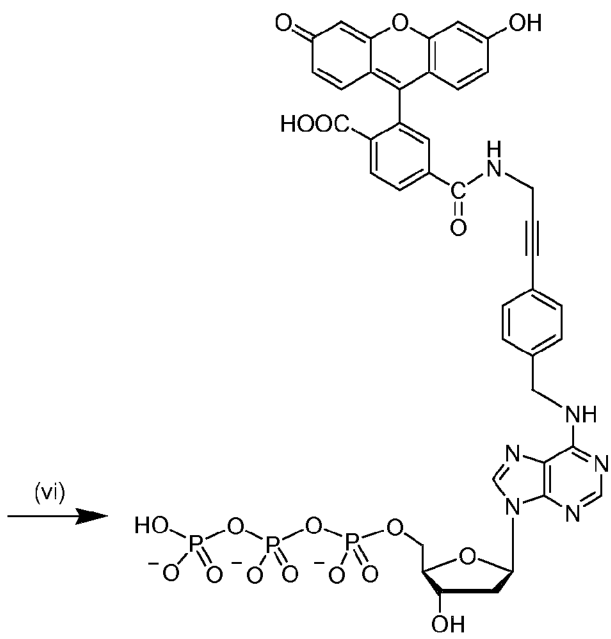


**WW2p085**

**Scheme.** Synthesis of *6-FAM labeled $N^6$-[4-(3-amino-1-propyl)benzyl]-2'-deoxyadenosine triphosphate.* (i) NaH, DMF, 4-iodobenzyl bromide, 0°C, then gradually warmed to room temperature, 99%; (ii) $SiO_2$, vacuum, 70-80°C, 99%; (iii) *n*-$Bu_4NF$, THF, 98%; (iv) *N*-propargyltrifluoroacetamide, $Pd(PPh_3)_4(0)$, CuI, $Et_3N$, anhydrous DMF, 4.5 h, 94%; (v) $POCl_3$, proton sponge, $(MeO)_3PO$, minus 20-30°C, two hours; $(n\text{-}Bu_3NH)_2H_2P_2O_7$, *n*-$Bu_3N$, DMF, five minutes; 1 M $HNEt_3HCO_3$, one hour; $NH_4OH$, one hour; 84 %; (vi) 6-FAM-SE, 0.1 M $NaHCO_3/Na_2CO_3$, pH 9.2.

*$N^6$-tert-Butyloxycarbonyl-$N^6$-(4-iodobenzyl)-3',5'-O-bis-tert-butyldimethylsilyl-2'-deoxyadenosine* ***(dA.n4)***

NaH (23 mg, 0.94 mmol, dry) was added to a solution of compound **dA.07** (500 mg, 0.72 mmol) in anhydrous DMF (6.5 mL) at 0°C and stirred for 30 minutes. A solution of 4-Iodobenzyl bromide (322 mg, 1.08 mmol) in anhydrous DMF (2.5 mL) was added dropwise. The mixture was gradually warmed to room temperature and stirred for two hours. DMF was removed *in vacuo,* and the residue was dissolved in ethyl acetate (60 mL), washed twice with saturated $NH_4Cl$ solution (40 mL each) and once with water (40 mL). The combined aqueous layer was extracted with ethyl acetate (10 mL), and the combined organic layer was dried over $Na_2SO_4$, concentrated *in vacuo,* and purified by silica gel column chromatography to yield $N^6$-*tert*-butyloxycarbonyl-$N^6$-(4-iodobenzyl)-3',5'-*O*-bis-*tert*-butyldimethylsilyl-2'-deoxyadenosine **dA.n4** (565 mg, 99%) as a viscous oil.

*$^1H$ NMR (400 MHz, $CDCl_3$):* δ 8.71 (s, 1 H, H-8), 8.33 (s, 1 H, H-2), 7.58 (d, 2 H, *J* = 8.2 Hz, Ph-H), 7.17 (d, 2 H, *J* = 8.2 Hz, Ph-H), 6.49 (t, 1 H, *J* = 6.4 Hz, H-1'), 5.20 (s, 2 H, Ph-$CH_2$), 4.62 (m, 1 H, H-3'), 4.02 (m, 1 H, H-3'), 3.86 (dd, 1 H, *J* = 4.2 and 11.2 Hz,
H-5'a), 3.78 (dd, 1 H, *J* = 3.2 and 11.2 Hz, H-5'b), 2.63 (m, 1 H, H-2'a), 2.45 (m, 1 H, H-2'b), 1.42 (s, 9 H, $(CH_3)_3CO$), 0.92 (s, 18 H, $(CH_3)_3CSi$), 0.08 (2 s, 12 H, $(CH_3)_2Si$-).

*$N^6$-(4-Iodobenzyl)-3',5'-O-bis-tert-butyldimethylsilyl-2'-deoxyadenosine* ***(dA.n5)***

Silica gel 60 (6.00 g, 100-200 mesh, activated by heating to 70-80°C under reduced pressure for 24 hours) was added to a solution of compound **dA.n4** (565 mg, 0.71 mmol) in $CH_2Cl_2$ (20 mL), and the mixture was evaporated

*in vacuo* to dryness. The residue was heated to 70-80°C under reduced pressure for two days, washed three times with methanol (30 mL each), and filtered using a buchi funnel. The combined filtrate was concentrated *in vacuo* and purified by silica gel column chromatography to yield *N*[6]-(4-iodobenzyl)-3',5'-*O*-bis-*tert*-butyldimethylsilyl-2'-deoxyadenosine **dA.n5** (489 mg, 99%) as a yellow foam.

*1H NMR (400 MHz, $CDCl_3$):* δ 8.38 (s, 1 H, H-8), 8.06 (s, 1 H, H-2), 7.63 (d, 2 H, *J* = 8.2 Hz, Ph-H), 7.11 (d, 2 H, *J* = 8.2 Hz, Ph-H), 6.45 (t, 1 H, *J* = 6.4 Hz, H-1'), 6.34 (t, 1 H, 6-NH), 4.81 (br s, 2 H, Ph-$CH_2$), 4.61 (m, 1 H, H-3'), 4.00 (m, 1 H, H-4'), 3.85 (dd, 1 H, *J* = 4.2 and 11.2 Hz, H-5'a), 3.76 (dd, 1 H, *J* = 3.2 and 11.2 Hz, H-5'b), 2.64 (m, 1 H, H-2'a), 2.44 (m, 1 H, H-2'b), 0.91 (s, 18 H, $(CH_3)_3CSi$), 0.09 (2 s, 12 H, $(CH_3)_2Si$-).

*N[6]-(4-Iodobenzyl)-2'-deoxyadenosine* ***(dA.n6)***

A solution of *n*-$Bu_4NF$ (282 mg, 1.08 mmol) in THF (1.0 mL) was added to a solution of compound **dA.n5** (300 mg, 0.43 mmol) in THF (1.2 mL) at 0°C. The reaction mixture was gradually warmed to room temperature and stirred for two hours. Silica gel 60 (1 g) was added, and the mixture was evaporated *in vacuo* to dryness. The residue was purified by silica gel column chromatography to yield *N*[6]-(4-iodobenzyl)- 2'-deoxyadenosine **dA.n6** (266 mg, 98%) as a white foam.

*1H NMR (400 MHz, DMSO-$d_6$):* 5 8.48 (br s, 1 H, $D_2O$ exchangeable, 6-NH), 8.40 (s, 1 H, H-8), 8.27 (s, 1 H, H-2), 7.68 (d, 2 H, *J* = 8.0 Hz, Ph-H), 7.17 (d, 2 H, *J* = 8.0 Hz, Ph-H), 6.39 (t, 1 H, *J* = 6.4 Hz, H-1'), 5.34 (d, 1 H, $D_2O$ exchangeable, 3'-OH), 5.22 (t, 1 H, $D_2O$ exchangeable, 5'-OH), 4.68 (br s, 2 H, Ph-$CH_2$), 4.44 (m, 1 H, H-4'), 3.91 (m, 1 H, H-3'), 3.64 (m, 1 H, H-5'a), 3.55 (m, 1 H, H-5'b), 2.76 (m, 1 H, H-2'a), 2.31 (m, 1 H, H-2'b).

*N[6]-[4-(3-trifluoroacetamido-1-propynyl)benzyl]-2'-deoxyadenosine* ***(dA.n7)***

A solution of compound **dA.n6** (266 mg, 0.57 mmol), *N*-propargyltrifluoroacetamide (260 mg, 1.72 mmol), CuI (22 mg, 0.11 mmol), tetrakis(triphenylphosphine)-palladium(0) (65 mg, 0.06 mmol), and $Et_3N$ (160 μL, 1.14 mmol) in anhydrous DMF (2.1 mL) was stirred at room temperature for 4.5 hours. The mixture was concentrated *in vacuo* and purified by silica gel column chromatography to yield *N*[6]-[4-(3-trifluoroacetamido-1-propynyl)-benzyl]-2'-deoxyadenosine **dA.n7** (268 mg, 94%) as a waxy solid.

*1H NMR (400 MHz, DMSO-$d_6$):* δ 10.05 (br m, 1 H, $D_2O$ exchangeable, NH), 8.46 (br m, 1 H, $D_2O$ exchangeable, NH), 8.37 (s, 1 H, H-8), 8.19 (s, 1 H, H-2), 7.37 (d, 2 H, *J* = 8.2 Hz, Ph-H), 7.32 (d, 2 H, *J* = 8.2 Hz, Ph-H), 6.35 (dd, 1 H, *J* = 6.4 and 7.5 Hz, H-1'), 5.31 (d, 1 H, $D_2O$ exchangeable, 3'-OH), 5.19 (t, 1 H, $D_2O$ exchangeable, 5'-OH), 4.70 (br s, 2 H, Ph-$CH_2$), 4.41 (m, 1 H, H-3'), 4.26 (d, 2 H, *J* = 4.3 Hz, $CH_2$) 3.88 (m, 1 H, H-4'), 3.61 (m, 1 H, H-5'a), 3.53 (m, 1 H, H-5'b), 2.73 (m, 1 H, H-2'a), 2.25 (m, 1 H, H-2'b).

*N[6]-[4-(3-Amino-1-propyl)benzyl]-2'-deoxyadenosine-5'-triphosphate* ***(dA.n8)***

$POCl_3$ (16 μL, 0.17 mmol) was added to a solution of compound **dA.n7** (56 mg, 0.11 mmol) and proton sponge (37 mg, 0.17 mmol) in trimethylphosphate (0.5 mL) and maintained at minus 20-30°C for two hours. A solution of bis-tri-*n*-butylammonium pyrophosphate (261 mg, 0.55 mmol) and tri-*n*-butylamine (110 μL) in anhydrous DMF (1.1 mL) was added. After five minutes of stirring, triethylammonium bicarbonate buffer (1 M, pH 7.5; 10 mL) was added. The reaction was stirred for one hour at room temperature, followed by the dropwise addition of concentrated ammonium hydroxide (10 mL, 27%) at 0°C. The mixture was stirred for an additional hour at room temperature and then lyophilized to dryness. The residue obtained was dissolved in water (10 mL), filtered, and purified by anion exchange chromatography using a Q Sepharose FF column (2.5 x 20 cm) with a linear gradient of $NH_4HCO_3$ (50 mM to 500 mM in 300 minutes) at a flow rate of 4.5 mL/min. The fractions containing triphosphate were combined and lyophilized to give triphosphate **dA.n8** (63 mg, 84%) as a white fluffy solid.

*1H NMR (400 MHz, $D_2O$):* δ 8.41 (s, 1 H, H-8), 8.19 (s, 1 H, H-2), 7.38 - 7.26 (m, 4 H, Ph-H), 6.47 (dd, 1 H, *J* = 5.5 and 6.6 Hz, H-1'), 4.30 (s, 1 H, H-4'), 4.21 (m, 2 H, H-5'a and H-5'b), 3.63 (s, 2 H, $CH_2$), 2.79 (m, 1 H, H-2'a), 2.60 (m, 1 H, H-2'b).

*31P NMR (162 MHz, $D_2O$):* δ -5.80 (d, *J* = 20.1 Hz), -10.94 (d, *J* = 19.3 Hz), -21.59 (t, *J* = 19.3 Hz);

*ToF-MS (ESI):* For the molecular ion $C_{20}H_{23}N_6O_{12}P_3Na$ $[M-2H+Na]^-$, the calculated mass was 655.0485, and the observed mass was 655.0758.

*6-FAM labeled N[6]-[4-(3-Amino-1-propyl)benzyl]-2'-deoxyadenosine-5'-triphosphates* ***(WW2p085)***

A solution of 6-FAM-SE (3.5 mg, 7.35 μmol) in anhydrous DMSO (70 μL) was added to a solution of triphosphate **dA.18a** (3.5 μmol) in $Na_2CO_3/NaHCO_3$ buffer (0.1 M, pH 9.2; 600 μL) and incubated at room temperature for one hour.

The reaction was purified by reverse-phase HPLC using a Perkin Elmer OD-300 $C_{18}$ column (4.6 x 250 mm) to yield the 6-FAM labeled triphosphate **WW2p085.** Mobile phase: A, 100 mM triethylammonium acetate (TEAA) in water (pH 7.0); B, 100 mM TEAA in water/$CH_3CN$ (30:70). Elution was performed with a linear gradient of 5-20% B for 20 minutes and then 20-90% B for 20 minutes. The concentration of **WW2p085** was estimated by adsorption spectroscopy using the extinction coefficient of the 6-FAM dye (*i.e.,* 68,000 at 494 nm).

*ToF-MS (ESI):* For the molecular ion $C_{41}H_{36}N_6O_{18}P_3$ $[M+H]^+$, the calculated mass was 993.1299, and the observed mass was 993.1520.

**Synthesis of 6-FAM labeled *N*6-{1-[4-(3-amino-1-propynyl)phenyl]ethyl}-2'-deoxyadenosine triphosphate (WW2p093)**

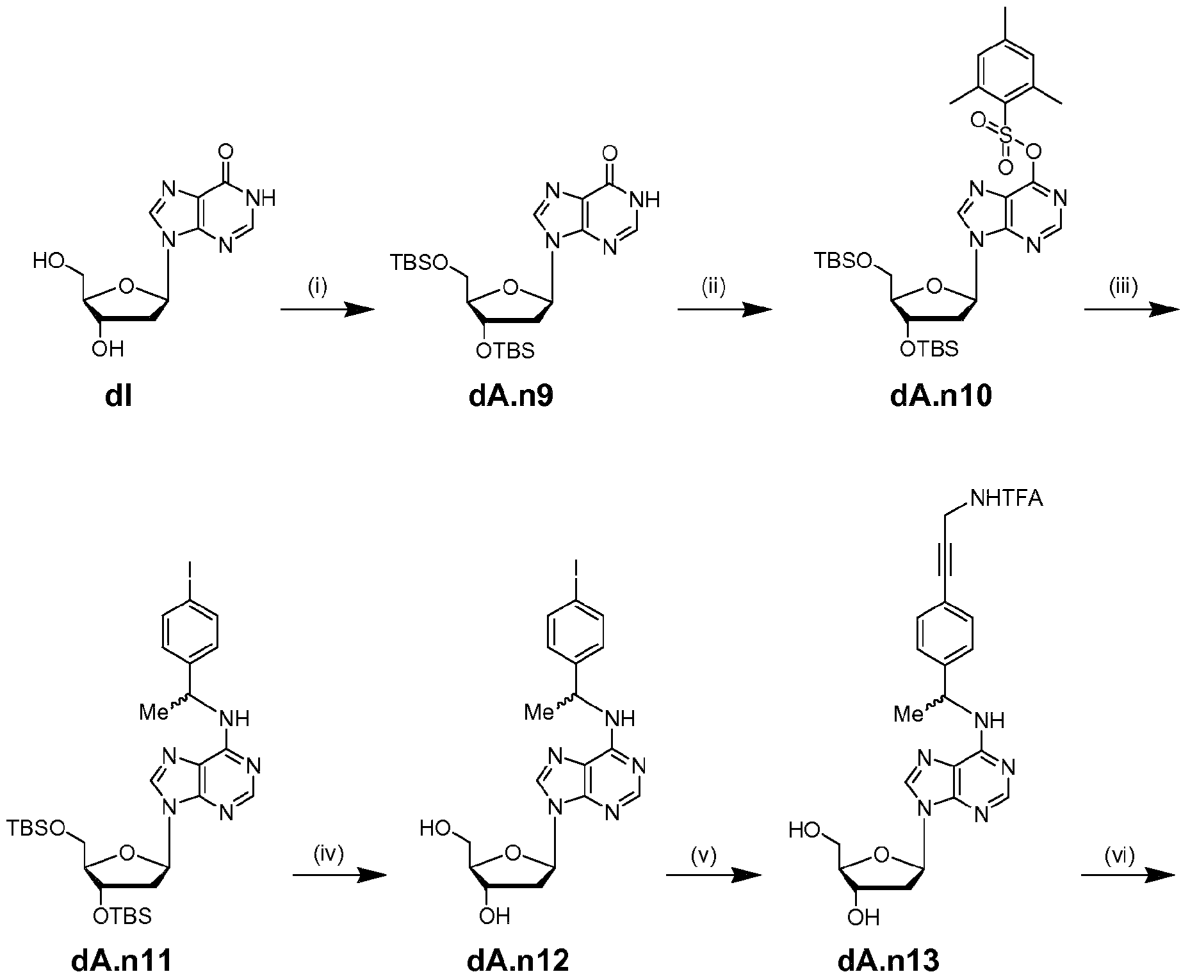


dI dA.n9 dA.n10

dA.n11 dA.n12 dA.n13

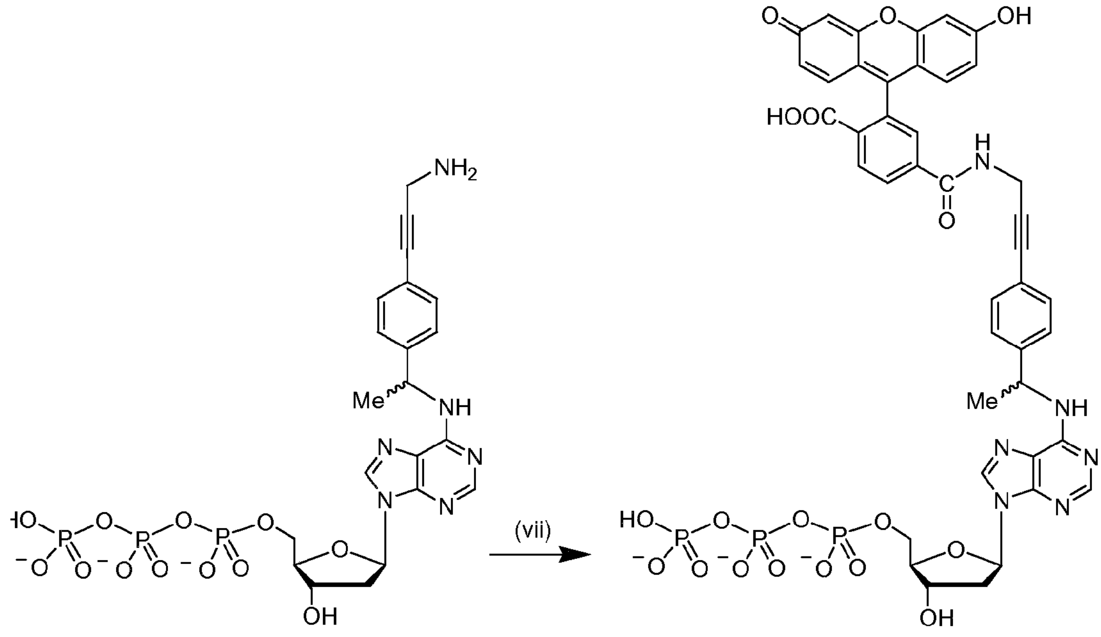


**dA.n14** **WW2p093**

**Scheme.** Synthesis of *6-FAM labeled N⁶-{1-[4-(3-amino-1-propynyl)phenyl]ethyl}-2'-deoxyadenosine triphosphate.* (i) TBSCl, imidazole, anhydrous DMF, 0°C, then gradually warmed to room temperature, 12 hours, 83%; (ii) 2-mesitylenesulfonyl chloride, $Et_3N$, DMAP, anhydrous $CH_2Cl_2$, room temperature, 1.5 hours, 20%; (iii) 1-(4-iodophenyl)ethylamine, molecular sieves, anhydrous 1,4-dioxane, 50°C, 18 hours, 88%; (iv) *n*-$Bu_4NF$, THF, 0°C, then gradually warmed to room temperature, 93%; (v) *N*-propargyltrifluoroacetamide, $Pd(PPh_3)_4(0)$, CuI, $Et_3N$, anhydrous DMF, 4.5 hours, 86%; (vi) $POCl_3$, $(MeO)_3PO$, minus 20-30°C; $(n\text{-}Bu_3NH)_2H_2P_2O_7$, *n*-$Bu_3N$, DMF; 1 M $HNEt_3HCO_3$; 86% (vii) 6-FAM-SE, 0.1 M $NaHCO_3/Na_2CO_3$, pH 9.2, one hour.

*3',5'-O-Bis-tert-butyldimethylsilyl-2'-deoxyinosine* ***(dA.n9)***[1]

A solution of TBSCl (1.91 g, 12.67 mmol) was added to a solution of 2'-deoxyinosine (1.00 g, 3.96 mmol) and imidiazole (1.73 g, 25.34 mmol) in anhydrous DMF (3 mL) at 0°C under nitrogen atmosphere. The reaction mixture was gradually warmed to room temperature and stirred for 12 hours. The mixture was then concentrated *in vacuo,* dissolved in $CH_2Cl_2$ (100 [1]The exact procedure can be found in: Kiselyov, A. S.; Steinbrecher, T.; Harvey, R. G. (1995) "Synthesis of the Fjord-region cis- and trans-Amino Triol Derivatives of the carcinogenic Hydrocarbon Benzo[g]chrysene and Utilization for the Synthesis of a Deoxyadenosine Adduct Linked to the N6-Amino Group" J. Org. Chem., 60: 6129-6134. mL), washed twice with water (50 mL), dried over anhydrous $Na_2SO_4$, concentrated *in vacuo,* and purified by silica gel chromatography to yield 3',5'-*O*-bis-*tert*-butyldimethylsilyl-2'-deoxyinosine **dA.n9** (1.58 g, 83%) as a white powder.

*$^1$H NMR (400 MHz, DMSO-$d_6$):* δ 12.37 (br s, 1 H, $D_2O$ exchangeable, NH), 8.25 (s, 1 H, H-8), 8.04 (d, 1 H, *J* = 3.6 Hz, H-2), 6.29 (t, 1 H, *J* = 6.6 Hz, H-1'), 4.59 (m, 1 H, H-3'), 3.84 (m, 1 H, H-4'), 3.74 (m, 1 H, H-5'a), 3.66 (m, 1 H, H-5'b), 2.76 (m, 1 H, H-2'a), 2.30 (m, 1 H, H-2'b), 0.89 (s, 9 H, $(CH_3)_3CSi$), 0.85 (s, 9 H, $(CH_3)_3CSi$), 0.11 (s, 6 H, $(CH_3)_2Si$), 0.02 (2 s, 6 H, $(CH_3)_2Si$).

*$O^6$-(2-Mesitylenesulfonyl)-3',5'-bis-O-tert-butyldimethylsilyl-2'-deoxyinosine* ***(dA.10)***[1]

2-Mesitylenesulfonyl chloride (0.70 g, 2.12 mmol), $Et_3N$ (0.42 mL, 3.07 mmol), and DMAP (16 mg, 0.13 mmol) were added to a solution of **dA.n9** (1.02 g, 2.12 mmol) in anhydrous $CH_2Cl_2$ (15 mL). The reaction mixture was stirred at room temperature for 1.5 hours, then diluted with ethyl ether (50 mL). The ether solution was washed twice with a saturated solution of $NaHCO_3$ (25 mL each) and then with brine (25 mL). The organic layer was dried over $Na_2SO_4$,

concentrated *in vacuo*, and purified by silica gel chromatography to yield $O^6$-(2-mesitylenesulfonyl)-3',5'-bis-*O*-*tert*-butyldimethylsilyl-2'-deoxyinosine **dA.n10** (279 mg, 20%).

*$^1$H NMR (400 MHz, $CDCl_3$):* δ 8.55 (s, 1 H, H-8), 8.38 (s, 1 H, H-2), 6.99 (s, 2 H, Ph-H), 6.48 (t, 1 H, *J* = 6.4 Hz, H-1'), 4.61 (m, 1 H, H-3'), 4.03 (m, 1 H, H-4'), 3.85 (m, 1 H, H-5'a), 3.76 (m, 1 H, H-5'b), 2.77 (s, 6 H, $CH_3$), 2.61 (m, 1 H, H-2'a), 2.43 (m, 1 H, H-2'b), 2.32 (s, 3 H, $CH_3$), 0.91 (s, 9 H, $(CH_3)_3CSi$), 0.89 (s, 9 H, $(CH_3)_3CSi$), 0.09 (s, 6 H, $(CH_3)_2Si$), 0.08 (2 s, 6 H, $(CH_3)_2Si$).

*$N^6$-[1-(4-Iodophenyl)ethyl]-3',5'-bis-O-tert-butyldimethylsilyl-2'-deoxyadenosine **(dA.n11)***

A solution of 1-(4-iodophenyl)ethylamine (312 mg, 1.26 mmol) in anhydrous 1,4-dioxane (1 mL) was added to a solution of **dA.n10** (279 mg, 0.42 mmol) in anhydrous 1,4-dioxane (2 mL) containing molecular sieves (4 Å, 8-12 Mesh, 0.75 g) at room temperature under nitrogen atmosphere. The mixture was then stirred at 50°C for 18 hours. The solvent was removed *in vacuo*, and the crude product was purified by silica gel column chromatography to yield $N^6$-[1-(4-iodophenyl)ethyl]-3',5'-bis-*O*-tert-butyldimethylsilyl-2'-deoxyadenosine **dA.n11** (263 mg, 88%, 1:1 mixture of diastereoisomers) as a white foam.

*$^1$H NMR (400 MHz, $CDCl_3$) for diastereoisomers:* δ 8.32 (s, 1 H, H-8), 8.08 (s, 1 H, H-2), 7.61 (m, 2 H, Ph-H), 7.15 (m, 2 H, Ph-H), 6.42 (t, 1 H, *J* = 6.4 Hz, H-1'), 6.20 (br m, 1 H, NH), 5.50 (br s, 1 H, Ph-CH), 4.59 (m, 1 H, H-3'), 3.99 (m, 1 H, H-4'), 3.85 (m, 1 H, H-5'a), 3.77 (m, 1 H, H-5'b), 2.60 (m, 1 H, H-2'a), 2.42 (m, 1 H, H-2'b), 1.59 (d, 3 H, *J* = 7.0 Hz, $CH_3$), 0.90 (s, 18 H, $(CH_3)_3CSi$), 0.08 (s, 12 H, $(CH_3)_2Si$);

*$^{13}$C NMR (100 MHz, MeOH-$d_4$) for diastereoisomers:* δ 153.78 (C), 151.94 (CH), 143.78/143.71 (C), 138.36 (CH), 137.57 (CH), 128.17/128.16 (CH), 119.99 (C), 92.41 (C), 87.81/87.79 (CH), 84.28 (CH), 71.78/71.74 (CH), 62.72/62.68 ($CH_2$), 49.40 (br, CH), 41.31 ($CH_2$), 25.96 ($CH_3$), 25.75 ($CH_3$), 22.64 ($CH_3$), 18.41 (C), 17.99 (C), -4.66 ($CH_3$), -4.82 ($CH_3$), -5.39 ($CH_3$), -5.48 ($CH_3$).

*$N^6$-[1-(4-Iodophenyl)ethyl]-2'-deoxyadenosine **(dA.n12)***

A solution of *n*-$Bu_4NF$ (409 mg, 1.30 mmol) in THF (3 mL) was added to a solution of compound **dA.n11** (263 mg, 0.37 mmol) in THF (5 mL) at 0°C. The reaction mixture was gradually warmed to room temperature and stirred for 30 minutes, then concentrated *in vacuo* to dryness. The residue was purified by silica gel column chromatography to yield $N^6$-[1-(4-iodophenyl)ethyl]-2'-deoxyadenosine **dA.n12** (164 mg, 93% 1:1 mixture of diastereoisomers) as a waxy solid.

*$^1$H NMR (400 MHz, DMSO-$d_6$) for diastereomers:* δ 8.35 (s, 1 H, H-8), 8.32 (br s, 1 H, $D_2O$ exchangeable, NH), 8.13 (s, 1 H, H-2), 7.62 (d, 2 H, *J* = 8.2 Hz, Ph-H), 7.22 (d, 2 H, 2 H, *J* = 8.2 Hz, Ph-H), 6.32 (m, 1 H, H-1'), 5.41 (br, 1 H, Ph-CH), 5.31 (d, 1 H, $D_2O$ exchangeable, 3'-OH), 5.19 (m, 1 H, $D_2O$ exchangeable, 5'-OH), 4.35 (m, 1 H, H-4'), 3.85 (m, 1 H, H-4'), 3.58 (m, 1 H, H-5'a), 3.48 (m, 1 H, H-5'b), 2.68 (m, 1 H, H-2'a), 2.22 (m, 1 H, H-2'b), 1.49 (d, 3 H, *J* = 7.0 Hz, $CH_3$).

*$N^6$-{1-[4-(3-Trifluoroacetamido-1-propynyl)phenyl]ethyl}-2'-deoxyadenosine (***dA.n13***)*

A solution of compound **dA.n12** (70 mg, 0.145 mmol), *N*-propargyltrifluoroacetamide (66 mg, 0.44 mmol), CuI (5.5 mg, 0.03 mmol), tetrakis(triphenylphosphine)-palladium(0) (17 mg, 0.015 mmol), and $Et_3N$ (41μL, 0.29 mmol) in anhydrous DMF (2.2 mL) was stirred at room temperature for 5.5 hours. The mixture was concentrated *in vacuo* and purified by silica gel column chromatography to yield $N^6$-{1-[4-(3-trifluoroacetamido-1-propynyl)phenyl]ethyl]-2'-deoxyadenosine **dA.n13** (63 mg, 86%, 1:1 mixture of diastereoisomers) as a waxy solid.

*$^1$H NMR (400 MHz, DMSO-$d_6$) for diastereomers:* δ 10.05 (t, 1 H, *J* = 5.4 Hz, $D_2O$ exchangeable, NH), 8.36 (s, 1 H, H-8), 8.34 (br s, 1 H, $D_2O$ exchangeable, NH), 8.15 (s, 1 H, H-2), 7.43 (d, 2 H, *J* = 8.2 Hz, Ph-H), 7.36 (d, 2 H, 2 H, *J* = 8.2 Hz, Ph-H), 6.33 (dd, 1 H, *J* = 6.4 and 7.5, Hz, H-1'), 5.49 (br, 1 H, Ph-CH), 5.30 (d, 1 H, $D_2O$ exchangeable, 3'-OH), 5.10 (m, 1 H, $D_2O$ exchangeable, 5'-OH), 4.39 (m, 1 H, H-3'), 4.25 (d, 2 H, *J* = 5.4 Hz, $CH_2$), 3.87 (m, 1 H, H-3'), 3.59 (m, 1 H, H-5'a), 3.51 (m, 1 H, H-5'b), 2.72 (m, 1 H, H-2'a), 2.24 (m, 1 H, H-2'b), 1.52 (d, 3 H, *J* = 7.0 Hz, $CH_3$);

*$N^6$-{1-[4-(3-Amino-1-propynyl)phenyl]ethyl}-2'-deoxyadenosine-5'-triphosphate **(dA.n14)***

$POCl_3$ (14 μL, 0.15 mmol) was added to a solution of compound **dA.n14** (51 mg, 0.1 mmol) and proton sponge (32 mg, 0.15 mmol) in trimethylphosphate (0.5 mL) and maintained at minus 20-30°C for two hours. A solution of bis-tri-n-butylammonium pyrophosphate (237 mg, 0.5 mmol) and tri-n-butylamine (100 μL) in anhydrous DMF (1.0 mL) was added. After five minutes of stirring, triethylammonium bicarbonate buffer (1 M, pH 7.5; 10 mL) was added. The reaction was stirred for one hour at room temperature, followed by the dropwise addition of concentrated ammonium hydroxide (10 mL, 27%) at 0°C. The mixture was stirred for an additional hour at room temperature and then lyophilized to dryness.

The residue obtained was dissolved in water (10 mL), filtered, and purified by anion exchange chromatography using a Q Sepharose FF column (2.5 x 20 cm) with a linear gradient of $NH_4HCO_3$ (50 mM to 500 mM in 300 minutes) at a flow rate of 4.5 mL/min. The fractions containing triphosphate were combined and lyophilized to give triphosphate **dA.n14** (60 mg, 86%, 1:1 mixture of diastereoisomers) as a white fluffy solid.

*$^1H$ NMR (400 MHz, $D_2O$) for diastereoisomers:* δ 8.41 (s, 1 H, H-8), 8.14 (2 s, 1 H, H-2), 7.38 (m, 4 H, Ph-H), 6.46 (m, 1 H, H-1'), 5.32 (br, 1 H, Ph-CH), 4.30 (s, 1 H, H-3'), 4.20 (m, 2 H, H-5'a and H-5'b), 3.61 (s, 2 H, $CH_2$), 2.78 (m, 1 H, H-2'a), 2.59 (m, 1 H, H-2'b), 1.60 (d, 3 H, *J* = 6.9 Hz, $CH_3$);
*$^{31}P$ NMR (162 MHz, $D_2O$):* δ -6.02 (d, *J* = 19.4 Hz), -11.19 (d, *J* = 19.4 Hz), -21.77 (t, *J* = 19.4 Hz);
*ToF-MS (ESI):* For the molecular ion $C_{21}H_{25}N_6O_{12}P_3Na$ $[M-2H+Na]^-$, the calculated mass was 669.0641, and the observed mass was 669.0960.

*6-FAMlabeled $N^6$-{1-[4-(3-Amino-1-propynyl)phenyl]ethyl}-2'-deoxyadenosine-5'-triphosphate* ***(WW2p093)***

A solution of 6-FAM-SE (3.5 mg, 7.4 μmol) in anhydrous DMSO (70 μL) was added to a solution of triphosphate **dA.n14** (4.1 μmol) in $Na_2CO_3/NaHCO_3$ buffer (0.1 M, pH 9.2; 600 μl) and incubated at room temperature for one hour. The reaction was purified by reverse-phase HPLC using a Perkin Elmer OD-300 $C_{18}$ column (4.6 x 250 mm) to yield the 6-FAM labeled triphosphate **WW2p093.** Mobile phase: A, 100 mM triethylammonium acetate (TEAA) in water (pH 7.0); B, 100 mM TEAA in water/$CH_3CN$ (30:70). HPLC purification was achieved using a linear gradient of 5-20% B for 20 minutes and then 20-90% B for 20 minutes. The concentration of **WW2p093** was estimated by adsorption spectroscopy using the extinction coefficient of the 6-FAM dye (*i.e.,* 68,000 at 494 nm).

## Claims

1. A compound according to the following formula:

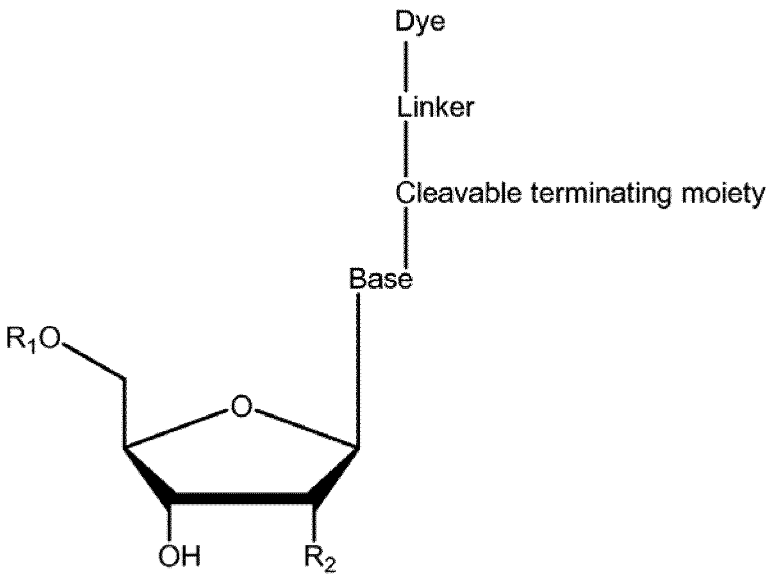

wherein the compound is selected from the group consisting of the compounds represented by formulas I-VII or salts thereof:

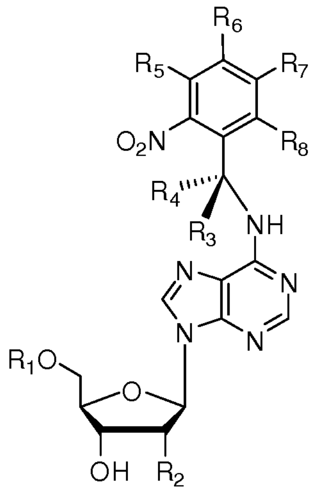

formula I

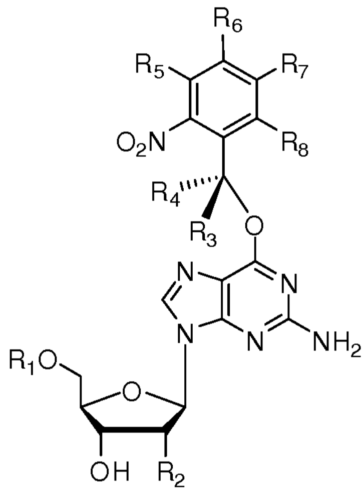

formula II

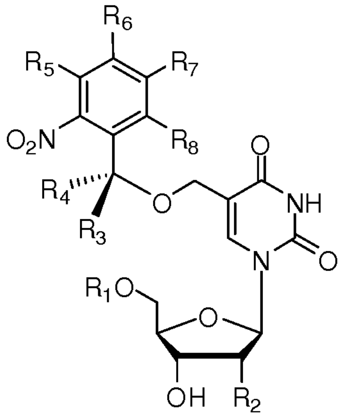

formula III

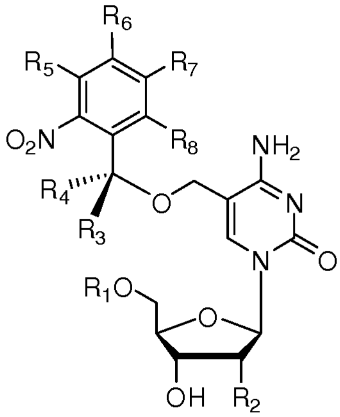

formula IV

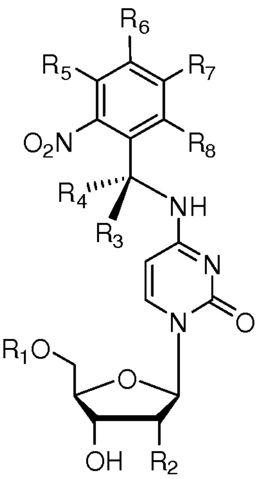

formula V

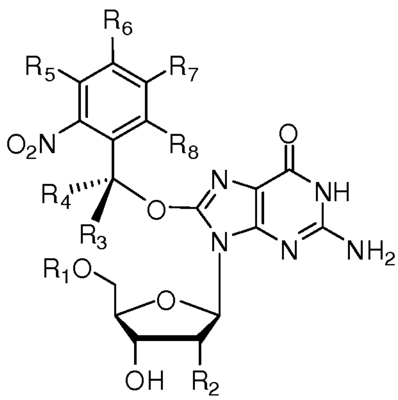

formula VI

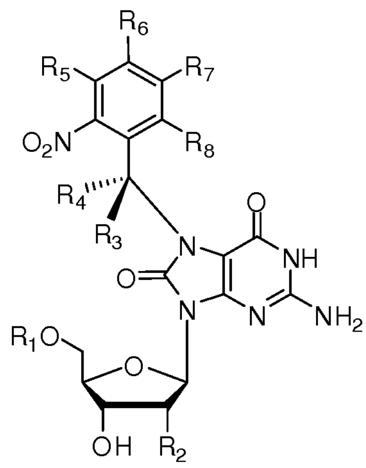

formula VII

wherein

$R_1$ is H, monophosphate, diphosphate, or triphosphate,
$R_2$ is H or OH,
$R_3$ and $R_4$ are selected from H, a $C_1$-$C_{12}$ straight chain or branched alkyl, a $C_2$-$C_{12}$ straight chain or branched alkenyl or polyenyl, a $C_2$-$C_{12}$ straight chain or branched alkynyl or polyalkynyl, and an aromatic group such as a phenyl, naphthyl, or pyridinyl ring, with the proviso that at least one of $R_3$ and $R_4$ is H,
$R_5$, $R_6$, $R_7$, and $R_8$ are each independently selected from H, $OCH_3$, $NO_2$, CN, a halide, a $C_1$-$C_{12}$ straight chain or branched alkyl, a $C_2$-$C_{12}$ straight chain or branched alkenyl or polyenyl, a $C_2$-$C_{12}$ straight chain or branched alkynyl or polyalkynyl, an aromatic group such as a phenyl, naphthyl, or pyridinyl ring, and/or a linker group of the general structure:

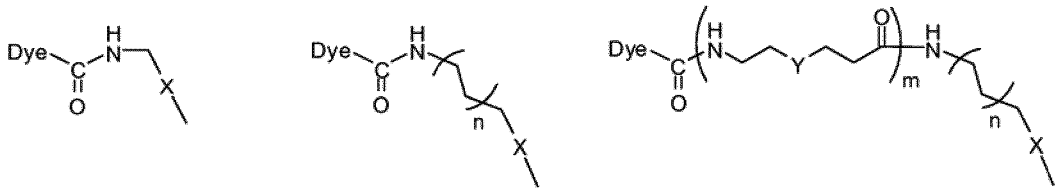

wherein

X = $CH_2$, CH=CH, C≡C, O, S, or NH,
Y = $CH_2$, O, or NH,
n and m are each an integer from 0-12, and

Dye is a fluorophore.

2. A compound according to claim 1, wherein $R_3$ or $R_4$ are selected from $-CH_3$, $-CH_2CH_3$, $-CH_2CH_2CH_3$, isopropyl, tert-butyl, phenyl, 2-nitrophenyl, and 2,6-dinitrophenyl.

3. A compound according to the following formula:

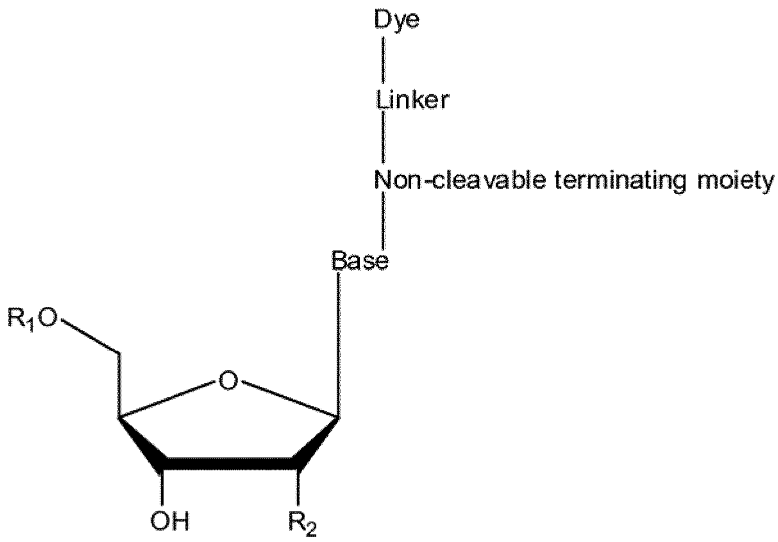

wherein the compound is selected from the group consisting of the compounds represented by formulas VIII-XIV or salts thereof:

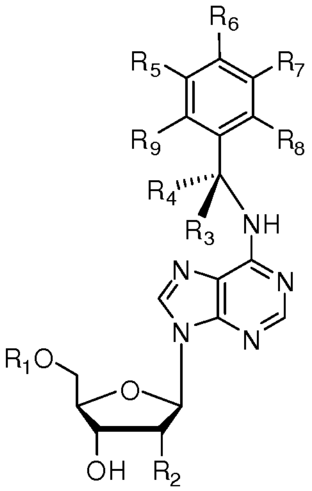

formula VIII

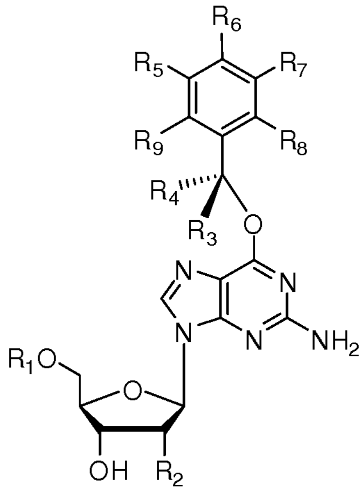

formula IX

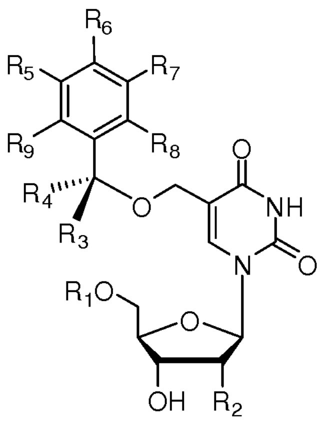

formula X

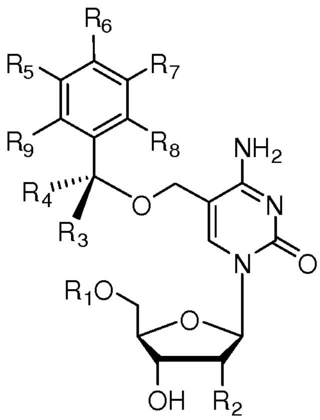

formula XI

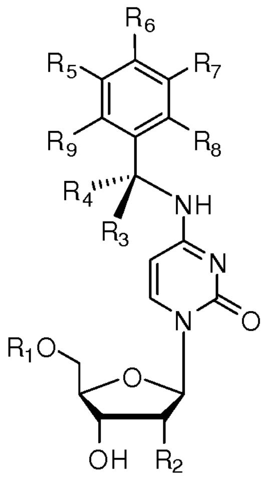

formula XII

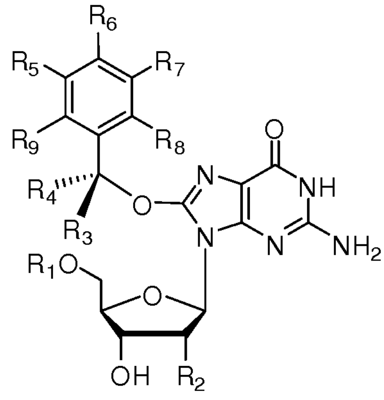

formula XIII

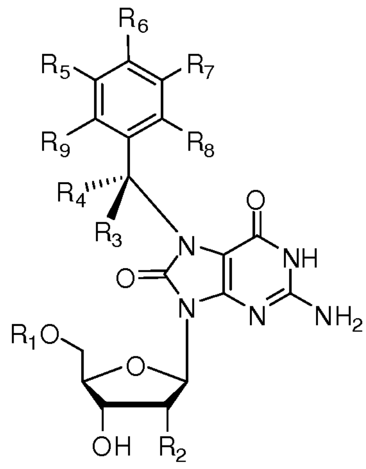

formula XIV

wherein

$R_1$ is H, monophosphate, diphosphate, or triphosphate,
$R_2$ is H or OH,
$R_3$ and $R_4$ are each independently selected from H, a $C_1$-$C_{12}$ straight chain or branched alkyl, a $C_2$-$C_{12}$ straight chain or branched alkenyl or polyenyl, a $C_2$-$C_{12}$ straight chain or branched alkynyl or polyalkynyl, and an aromatic group such as a phenyl, naphthyl, or pyridinyl ring;
$R_5$, $R_6$, and $R_7$, are each independently selected from H, $OCH_3$, $NO_2$, CN, a halide, a $C_1$-$C_{12}$ straight chain or branched alkyl, a $C_2$-$C_{12}$ straight chain or branched alkenyl or polyenyl, a $C_2$-$C_{12}$ straight chain or branched alkynyl or polyalkynyl, an aromatic group such as a phenyl, naphthyl, or pyridinyl ring, and/or a linker group of the general structure:

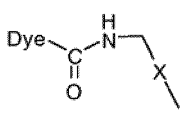

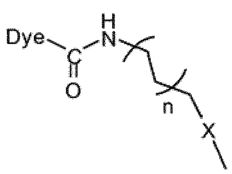

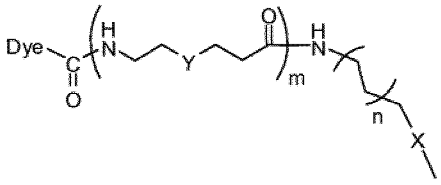

wherein

X = $CH_2$, CH=CH, C≡C, O, S, or NH,

Y = $CH_2$, O, or NH,
n and m are each an integer from 0-12,
Dye is a fluorophore, and
$R_8$ and $R_9$ are as defined above for $R_5$, $R_6$, and $R_7$, with the proviso that $R_8$ and $R_9$ are not $NO_2$.

**4.** A compound according to claim 3, wherein at least one of $R_3$ and $R_4$ is selected from the group consisting of $-CH_3$, $-CH_2CH_3$, $-CH_2CH_2CH_3$, isopropyl, *tert*-butyl, and phenyl.

**5.** A compound according to claim 3, wherein at least one of $R_3$ and $R_4$ is selected from the group consisting of alkyl and aromatic groups, the alkyl or aromatic group optionally containing at least one heteroatom, and further wherein the aromatic group is optionally defined as an aryl or polycyclic group.

**6.** A compound according to claim 1, wherein the compound is selected from the group consisting of:

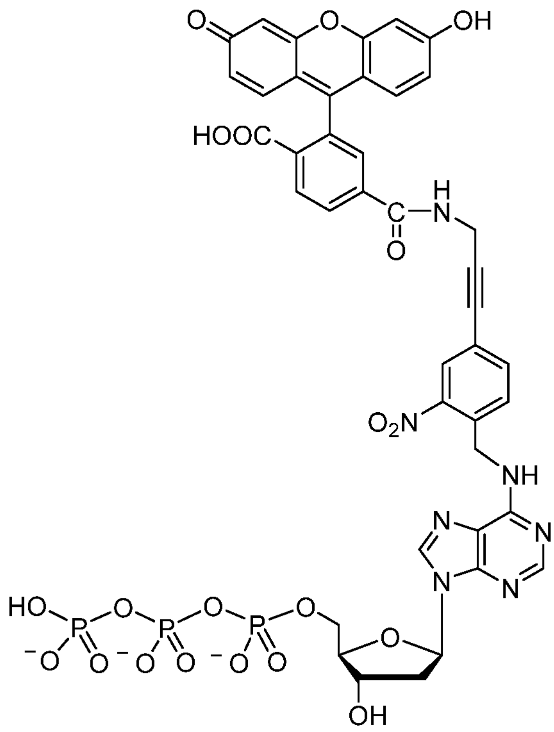

,

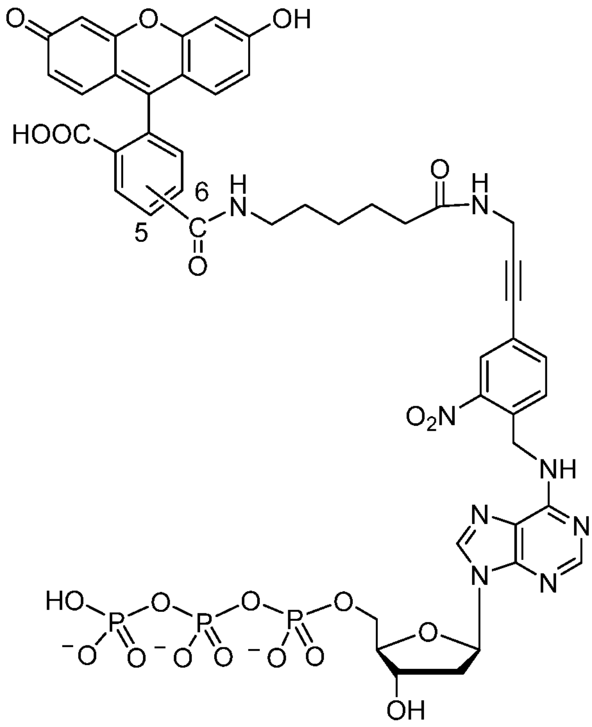

,

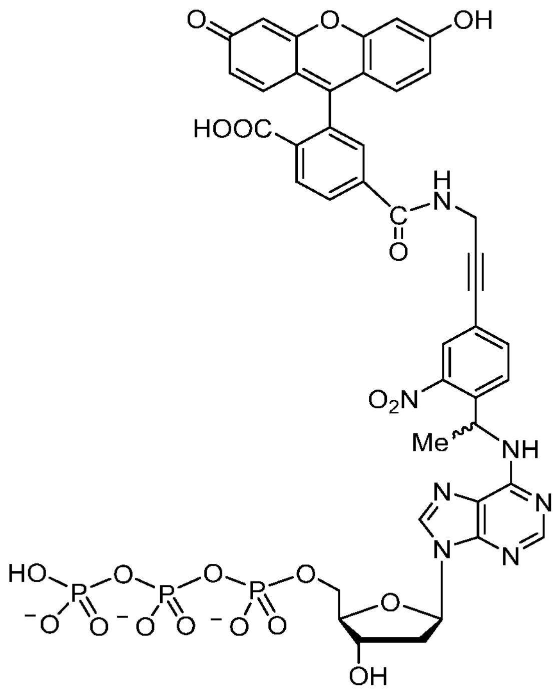

,

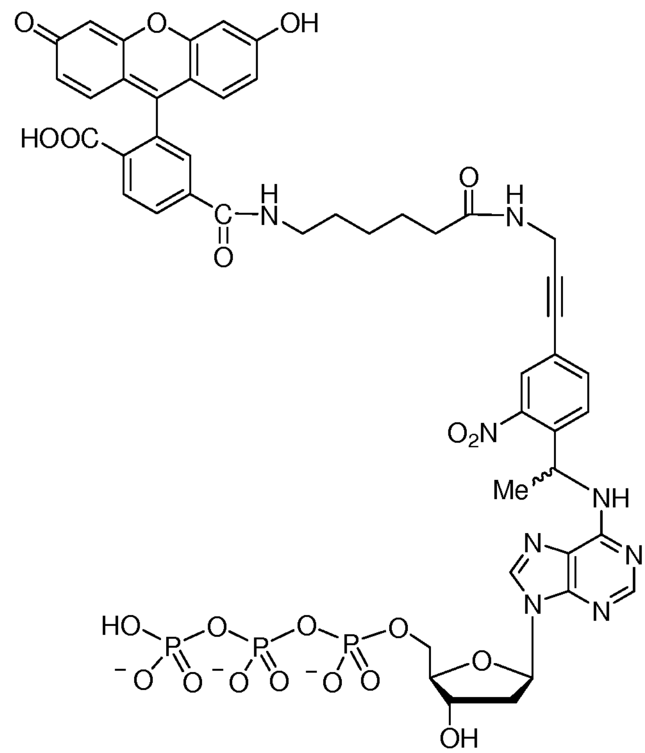

,

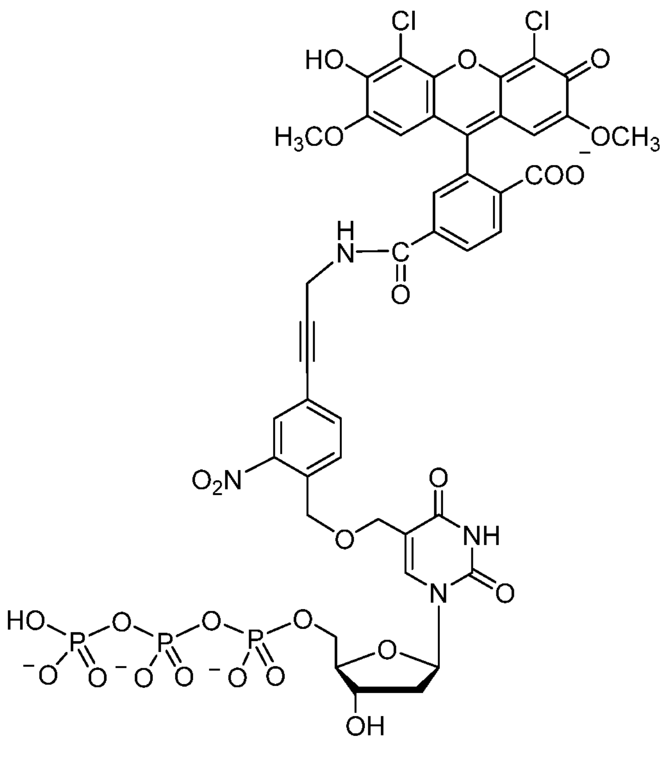

,

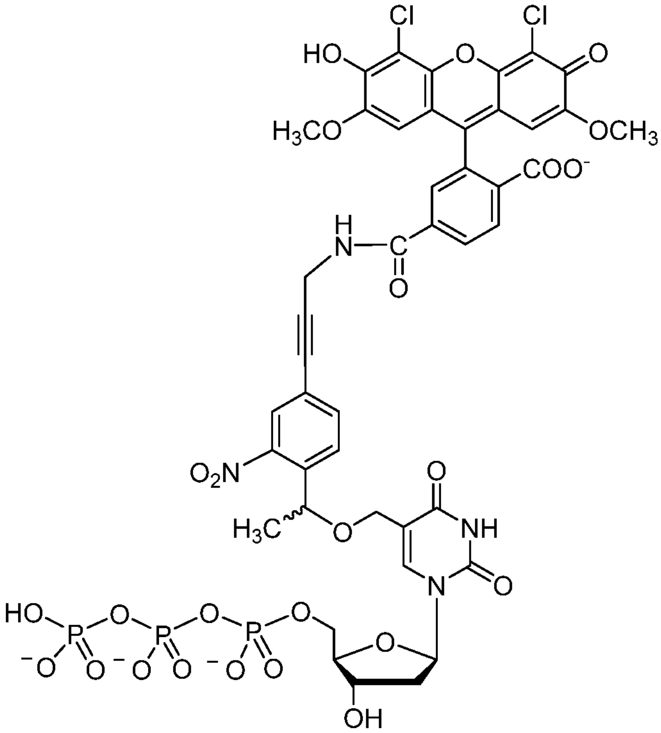

,

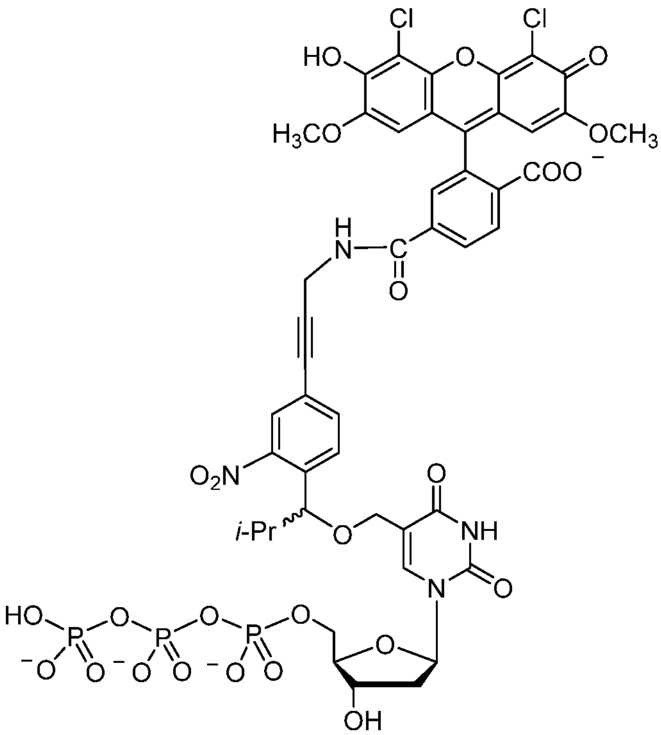

,

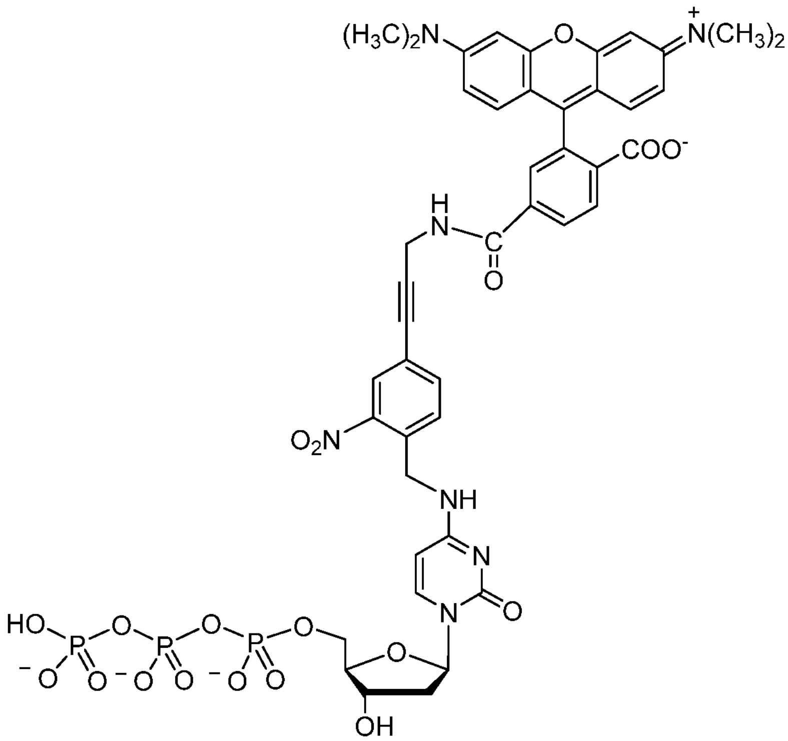

,

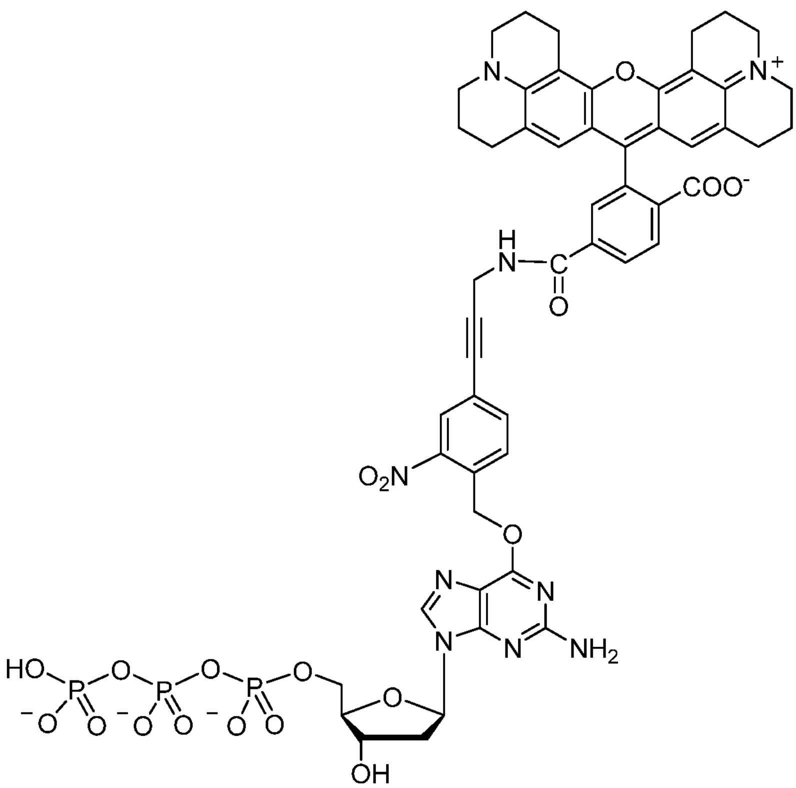

,

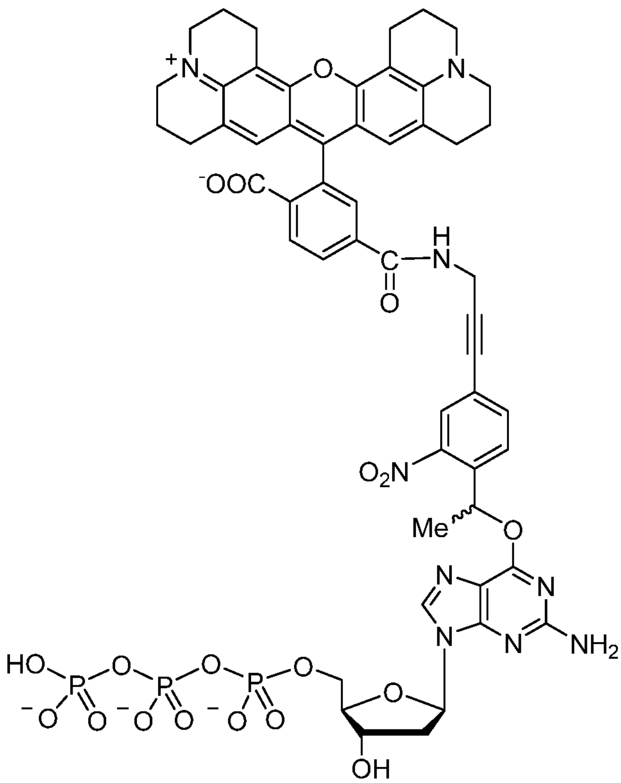

,

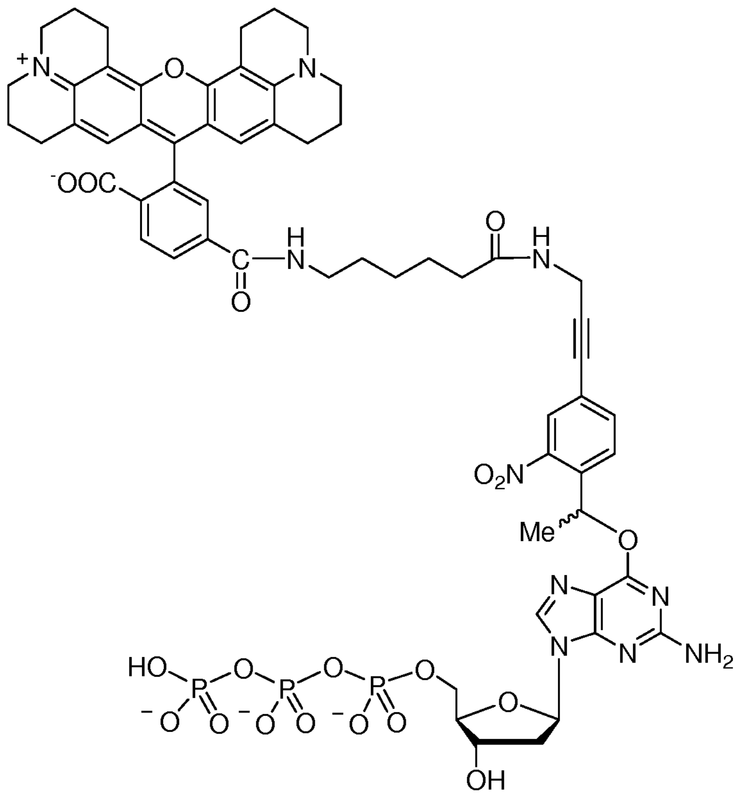

,

and

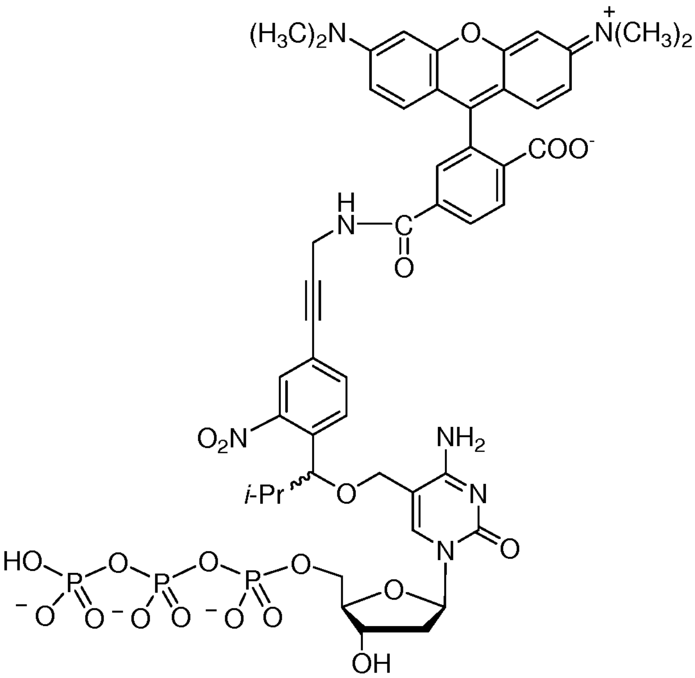

**7.** A compound according to claim 3, wherein the compound is selected from the group consisting of

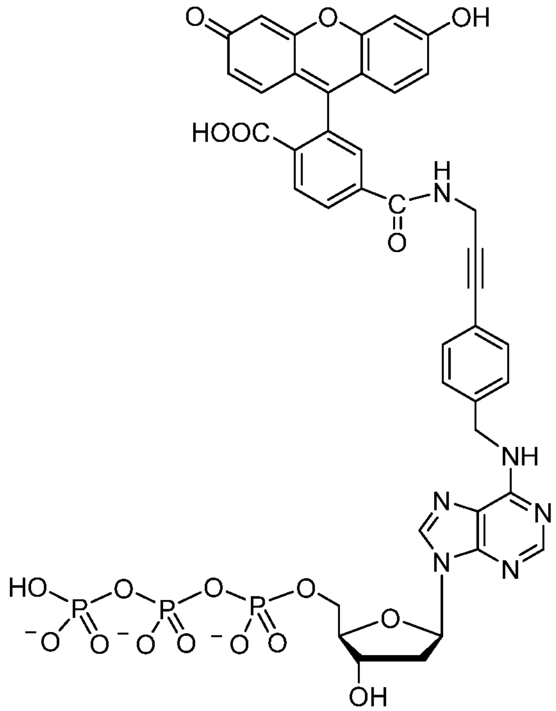

,

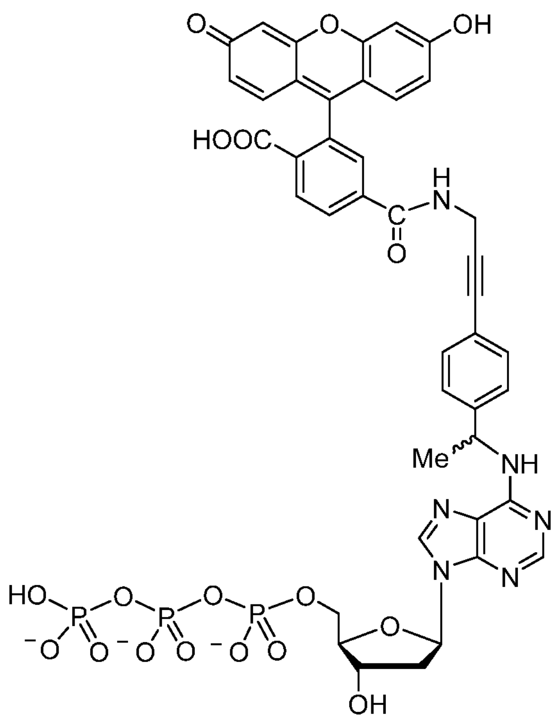

,

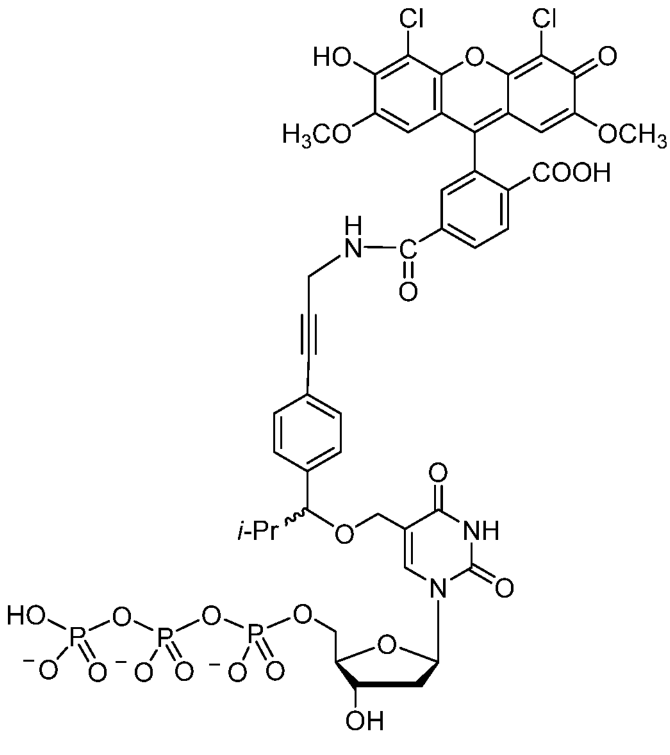

,

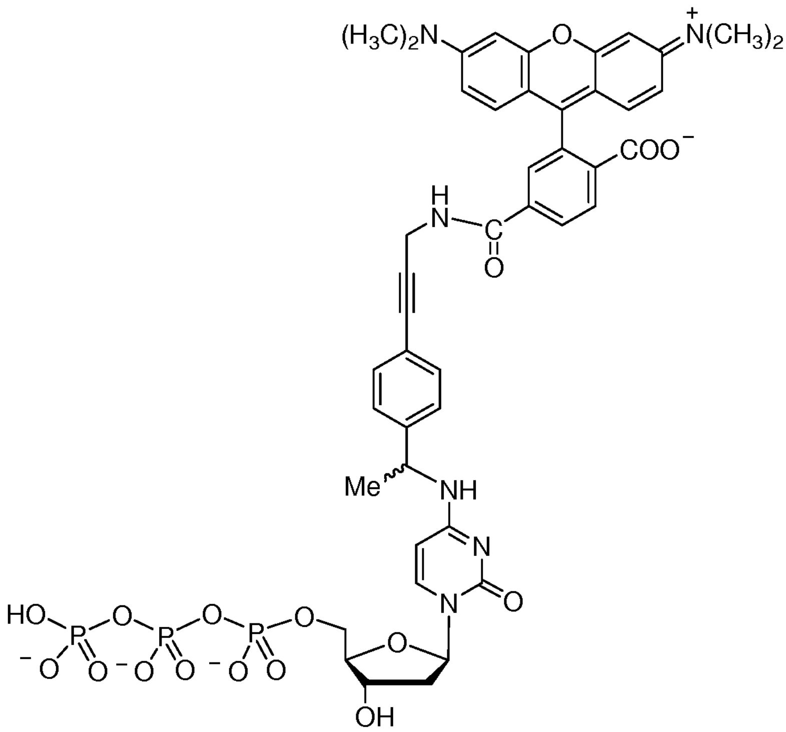

,

and

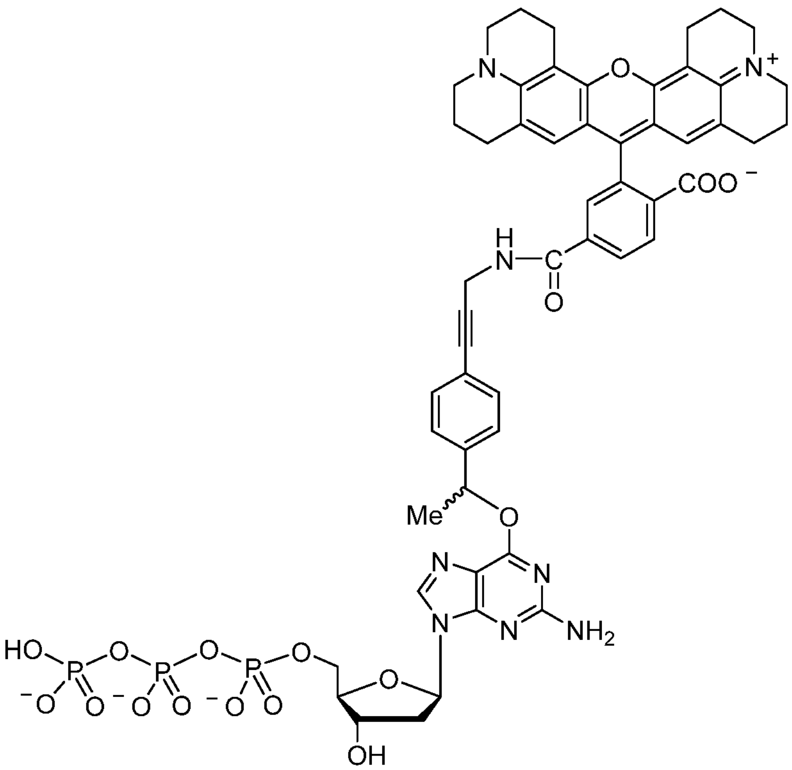

**8.** A compound according to any of claims 1 to 5, wherein at least one of $R_5$, $R_6$, $R_7$, and $R_8$ is an aromatic group consisting of aryl and polycyclic groups.

**9.** A compound according to any of claims 1 to 5, and 8, wherein the fluorophore is selected from BODIPY, fluorescein, rhodamine, coumarin, xanthene, cyanine, pyrene, phthalocyanine, phycobiliprotein, alexa, squarene dye, combinations resulting in energy transfer dyes, and derivatives thereof.

**10.** A method of sequencing a target nucleic acid, the method comprising the following steps:

(i) attaching a primer to a solid surface;
(ii) hybridizing a target nucleic acid to the primer attached to the solid surface;
(iii) adding at least one compound according to claim 1 or 2, wherein if more than one type of base is present, each base is optionally attached to a different fluorophore;
(iv) adding a polymerase to the hybridized primer/target nucleic acid complex to incorporate the at least one compound of step (iii) into a growing primer nucleic acid strand by a polymerase-catalyzed reaction, wherein the at least one incorporated compound of step (iii) terminates the polymerase reaction at an efficiency of between about 70% to about 100%;
(v) optionally washing the solid surface to remove unincorporated components;
(vi) detecting the incorporated fluorophore to identify the at least one incorporated compound of step (iii), wherein the detection is optionally carried out using a pulsed multiline excitation detector for imaging fluorescent dyes;
(vii) optionally adding one or more chemical compounds to permanently cap unextended primers;
(viii) exposing the solid surface to a light source to remove the photo-cleavable terminating moiety resulting in an extended primer nucleic acid with naturally occurring components;
(ix) optionally washing the solid surface to remove the cleaved photo-cleavable terminating moiety;
(x) repeating steps (iii) through (ix) one or more times to identify a plurality of bases in the target nucleic acid.

**11.** The method according to claim 10, wherein incorporation of the at least one compound according to step (iv) occurs at about 70% to about 100% and preferably at about 85% to about 100% of the efficiency of incorporation of a native substrate with the same base in the polymerase reaction.

**12.** The method according to claim 10 or 11, wherein the polymerase a reverse transcriptase, a terminal transferase, or a DNA polymerase, in particular wherein the polymerase is a Taq DNA polymerase, a Klenow(-exo-) DNA polymerase, a Bst DNA polymerase, a Vent(-exo-) DNA polymerase, a Pfu(-exo-) DNA polymerase, or a DeepVent(exo-) DNA polymerase, or wherein the polymerase is a modified polymerase selected from Taq FS DNA polymerase, ThermoSequenase DNA polymerase, ThermoSequenase II DNA polymerase, Therminator DNA polymerase, Therminator II DNA polymerase, and Vent(-exo-) A488L DNA polymerase.

**13.** The method according to any of claims 10 to 12, wherein about 85% to about 100% of photo-cleavable terminating moieties are removed by exposure to the light source in step (viii) and/or wherein incorporation of the at least one compound according to step (iv) is followed by termination of the primer nucleic acid strand growth at an efficiency of from about 90% to about 100%.

**14.** A method of converting a non-naturally occurring component in a nucleic acid molecule into a naturally occurring component, the method comprising the steps of:

(i) incorporating a compound according to claim 1 or 2 into a nucleic acid; and
(ii) exposing the resulting nucleic acid to a light source to remove the photo-cleavable terminating moiety from the nucleic acid.

**15.** Use of a compound according to claim 1 or 2 in a Sanger or Sanger-type sequencing reaction or in a pyrosequencing or pyrosequencing-type sequencing reaction.

**16.** A method of incorporating a non-naturally occurring component into a nucleic acid and optionally determining the sequence of the nucleic acid, the method comprising the steps of:

(i) adding a target nucleic acid to a reaction, and optionally to a Sanger or Sanger-type sequencing reaction;
(ii) adding at least one compound according to claim 3 to the reaction and optionally to the Sanger or Sanger-type sequencing reaction, wherein if more than one type of base is present, each base is optionally attached to a different fluorophore;
(iii) adding a complementary primer and a polymerase enzyme to the reaction; and
(iv) performing a polymerase-catalyzed reaction to incorporate the at least one compound of step (ii) into a growing nucleic acid strand; and optionally
(v) analyzing the result of step (iv) for incorporation of the at least one compound from step (ii) and optionally

for sequence determination,

wherein steps (i)-(iii) can be performed in any order.

17. The method according to claim 16, wherein incorporation of the at least one compound according to step (iv) is followed by termination of strand growth at an efficiency of from about 90% to about 100%, or wherein incorporation of at least one compound according to step (iv) occurs at about 70% to about 100% of the efficiency of incorporation of native substrate with the same base in the polymerase reaction.

18. The method according to claim 16 or 17, wherein the method is a to determine the sequence of a nucleic acid and the polymerase is a reverse transcriptase, a terminal transferase, or a DNA polymerase, in particular wherein the polymerase is a Taq DNA polymerase, a Klenow(exo-) DNA polymerase, a Bst DNA polymerase, a Vent(-exo-) DNA polymerase, a Pfu(exo-) DNA polymerase, or a DeepVent(exo-) DNA polymerase, or wherein the polymerase is a modified polymerase selected from TaqFS DNA polymerase, ThermoSequenase DNA polymerase, ThermoSequenase II DNA polymerase, Therminator DNA polymerase, Therminator II DNA polymerase, and Vent(exo-) A488L DNA polymerase.

19. Use of a compound according to claim 3 in a Sanger or Sanger-type sequencing reaction or in a mini-sequencing or minisequencing-type sequencing reaction.

20. A method of terminating nucleic acid synthesis, the method comprising the step of placing a 3'-OH unprotected nucleotide or nucleoside according to any of claims 1-8 in the environment of a polymerase and allowing incorporation of the 3'-OH unprotected nucleotide or nucleoside into a nucleic acid molecule.

21. The method according to claim 20, wherein the efficiency of termination of DNA synthesis upon incorporation of the 3'-OH unprotected nucleotide or nucleoside ranges from about 90% to about 100%, or wherein the efficiency of incorporation of the 3'-OH unprotected nucleotide or nucleoside ranges from about 70% to about 100% compared to the efficiency of incorporation of a naturally occurring nucleotide or nucleoside with the same base as the 3'-OH unprotected nucleotide or nucleoside.

**Patentansprüche**

1. Eine Verbindung gemäß der folgenden Formel:

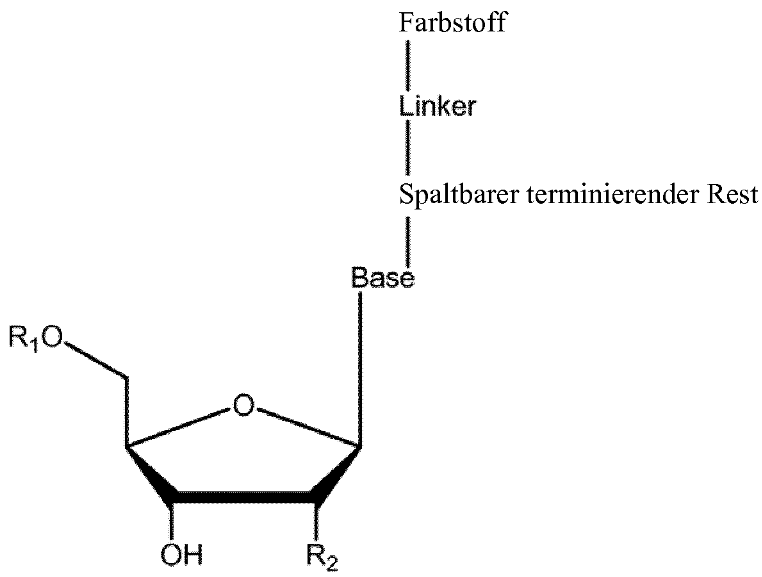

wobei die Verbindung aus der Gruppe ausgewählt ist, die aus Verbindungen besteht, die durch die Formeln 1-VII oder Salzen davon repräsentiert werden:

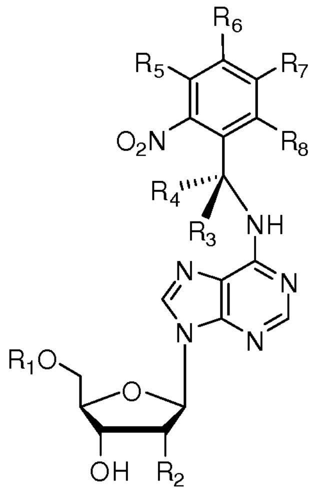

Formel I

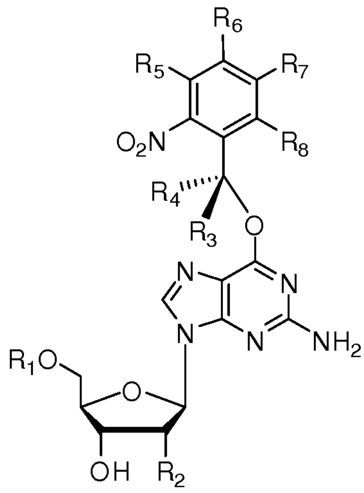

Formel II

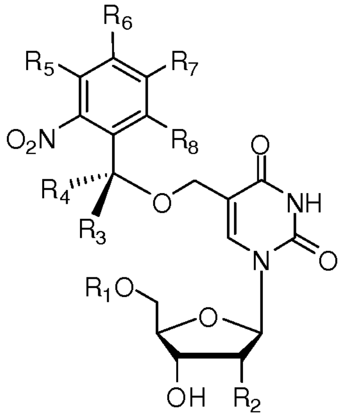

Formel III

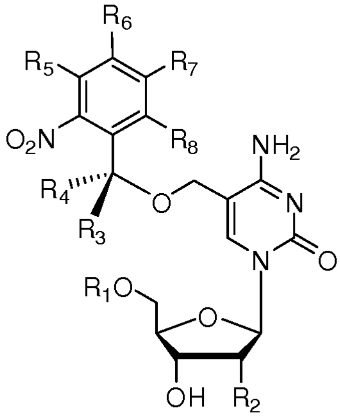

Formel IV

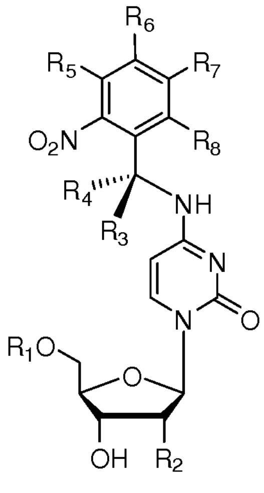

Formel V

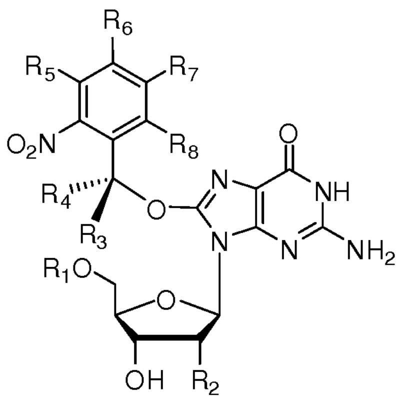

Formel VI

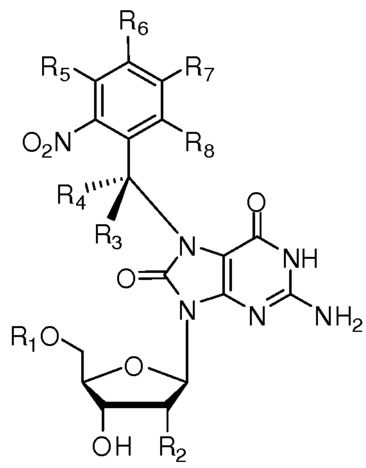

Formel VII

wobei

$R_1$ H, Monophosphat, Diphosphat, oder Triphosphat ist,
$R_2$ H oder OH ist,
$R_3$ und $R_4$ aus H, einem geradkettigen oder verzweigten $C_1$-$C_{12}$-Alkyl, einem geradkettigen oder verzweigten $C_2$-$C_{12}$-Alkenyl oder -Polyenyl, einem geradkettigen oder verzweigten $C_2$-$C_{12}$-Alkynyl oder -Polyalkynyl, und einer aromatischen Gruppe wie einem Phenyl-, Naphthyl-, oder Pyridinylring ausgewählt sind, unter der Voraussetzung, dass mindestens einer von $R_3$ und $R_4$ H ist,
$R_5$, $R_6$, $R_7$, und $R_8$ jeweils unabhängig aus H, $OCH_3$, $NO_2$, CN, einem Halid, einem geradkettigen oder verzweigten $C_1$-$C_{12}$-Alkyl, einem geradkettigen oder verzweigten $C_2$-$C_{12}$-Alkenyl oder -Polyenyl, einem geradkettigen oder verzweigten $C_2$-$C_{12}$-Alkynyl oder -Polyalkynyl, einer aromatischen Gruppe wie einem Phenyl-, Naphthyl-, oder Pyridinylring, und/oder einer Linkergruppe der allgemeinen Struktur:

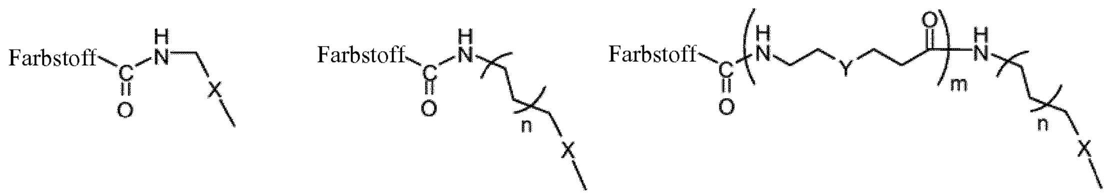

ausgewählt sind, wobei

X = $CH_2$, CH=CH, C≡C, O, S, oder NH,
Y = $CH_2$, O, oder NH,
n und m jeweils eine ganze Zahl von 0-12 sind, und

Farbstoff ein Fluorophor ist.

2. Eine Verbindung gemäß Anspruch 1, wobei $R_3$ oder $R_4$ aus $-CH_3$, $-CH_2CH_3$, $-CH_2CH_2CH_3$, Isopropyl, tert-Butyl, Phenyl, 2-Nitrophenyl, und 2,6-Dinitrophenyl ausgewählt ist.

3. Eine Verbindung gemäß der folgenden Formel:

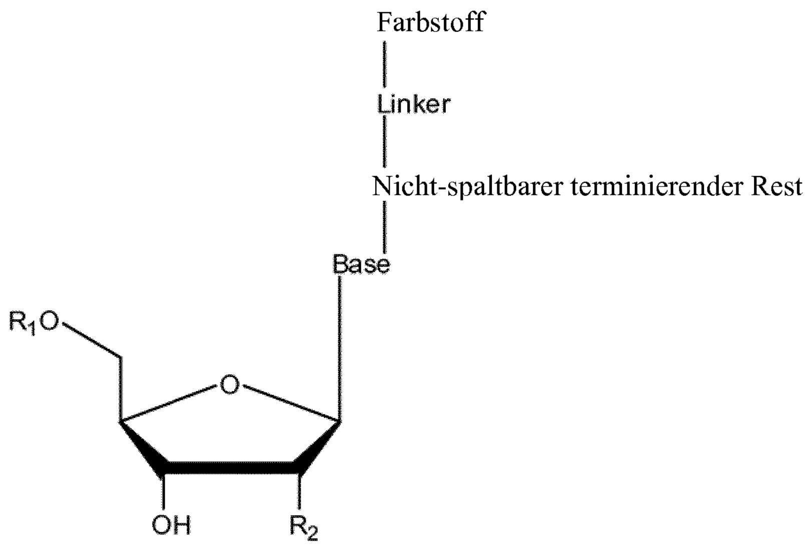

wobei die Verbindung aus der Gruppe ausgewählt ist, die aus Verbindungen besteht, die durch die Formeln VIII-XIV oder Salzen davon repräsentiert werden:

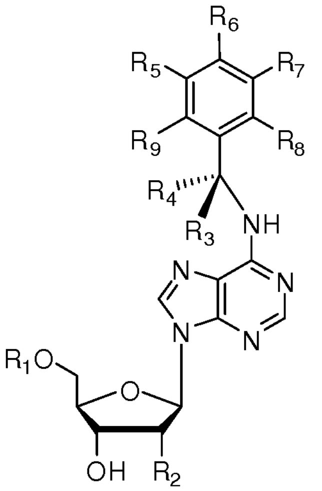

Formel VIII

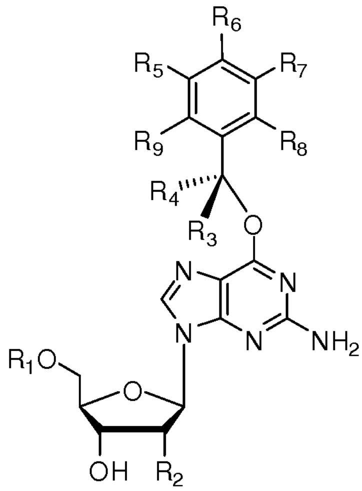

Formel IX

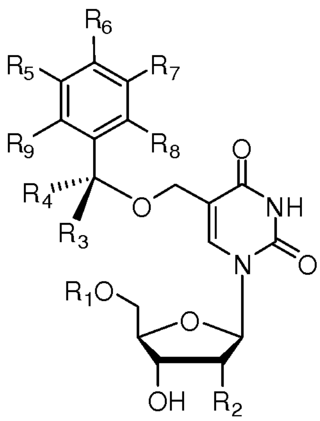

Formel X

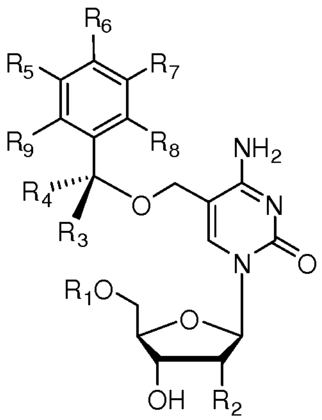

Formel XI

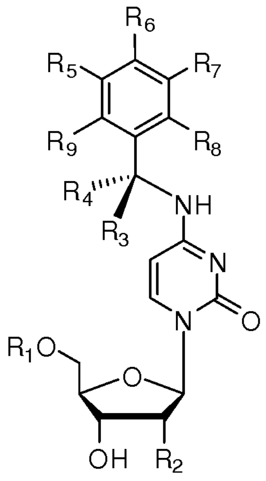

Formel XII

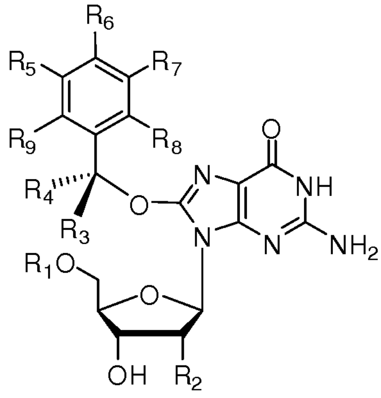

Formel XIII

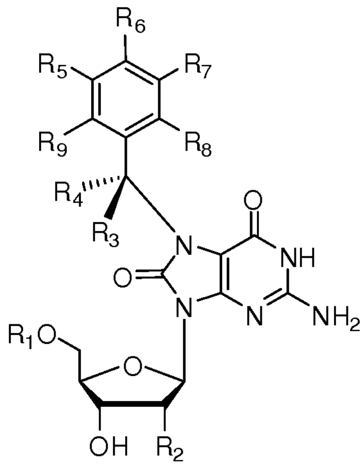

Formel XIV

wobei

$R_1$ H, Monophosphat, Diphosphat, oder Triphosphat ist,
$R_2$ H oder OH ist,
$R_3$ und $R_4$ jeweils unabhängig aus H, einem geradkettigen oder verzweigten $C_1$-$C_{12}$-Alkyl, einem geradkettigen oder verzweigten $C_2$-$C_{12}$-Alkenyl oder -Polyenyl, einem geradkettigen oder verzweigten $C_2$-$C_{12}$-Alkynyl oder Polyalkynyl, und einer aromatischen Gruppe wie einem Phenyl-, Naphthyl-, oder Pyridinylring ausgewählt sind;
$R_5$, $R_6$, und $R_7$, jeweils unabhängig aus H, $OCH_3$, $NO_2$, CN, einem Halid, einem geradkettigen oder verzweigten $C_1$-$C_{12}$-Alkyl, einem geradkettigen oder verzweigten $C_2$-$C_{12}$-Alkenyl oder -Polyenyl, einem geradkettigen oder verzweigten $C_2$-$C_{12}$-Alkynyl oder -Polyalkynyl, einer aromatischen Gruppe wie einem Phenyl-, Naphthyl-, oder Pyridinylring, und/oder einer Linkergruppe der allgemeinen Struktur:

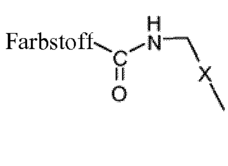
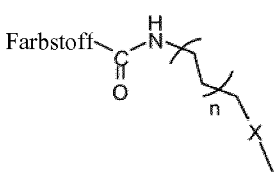
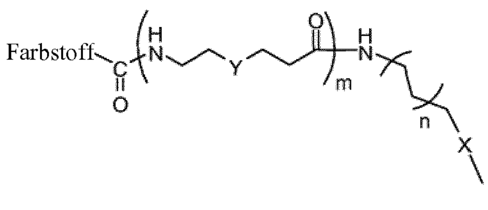

ausgewählt sind, wobei

X = $CH_2$, CH=CH, C≡C, O, S, oder NH,
Y = $CH_2$, O, oder NH,
n und m jeweils eine ganze Zahl von 0-12 sind,
Farbstoff ein Fluorophor ist, und
Rg und $R_9$ wie oben für $R_5$, $R_6$, und $R_7$ definiert sind, mit der Voraussetzung, dass $R_8$ und $R_9$ nicht $NO_2$ sind.

**4.** Eine Verbindung gemäß Anspruch 3, wobei mindestens einer von $R_3$ und $R_4$ aus der Gruppe bestehend aus $-CH_3$, $-CH_2CH_3$, $-CH_2CH_2CH_3$, Isopropyl, *tert*-Butyl, und Phenyl ausgewählt ist.

**5.** Eine Verbindung gemäß Anspruch 3, wobei mindestens einer von $R_3$ und $R_4$ aus der Gruppe bestehend aus Alkyl- und aromatischen Gruppen ausgewählt ist, wobei die Alkyl- oder aromatische Gruppe optional mindestens ein Heteroatom enthält, und wobei ferner die aromatische Gruppe optional als Aryl- oder polyzyklische Gruppe definiert ist.

**6.** Eine Verbindung gemäß Anspruch 1, wobei die Verbindung aus der Gruppe bestehend aus:

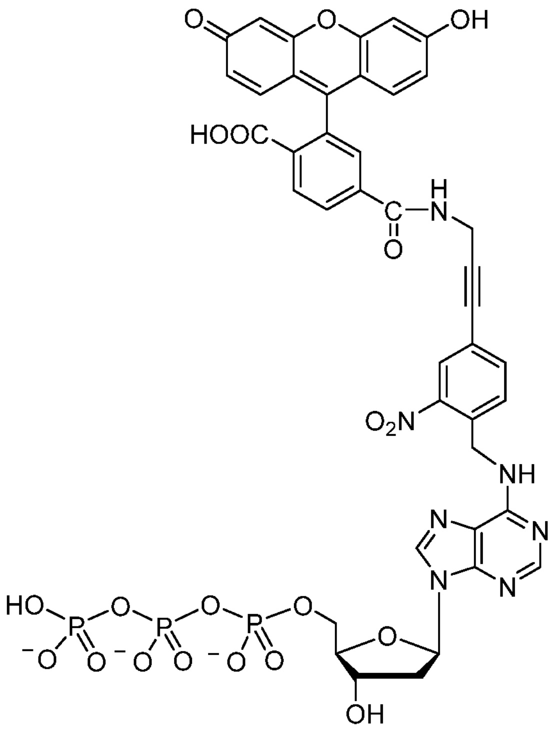

,

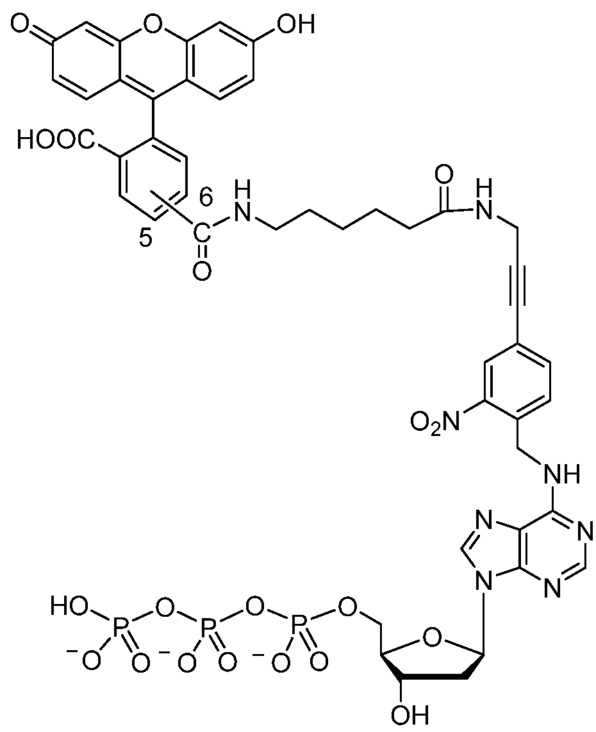

,

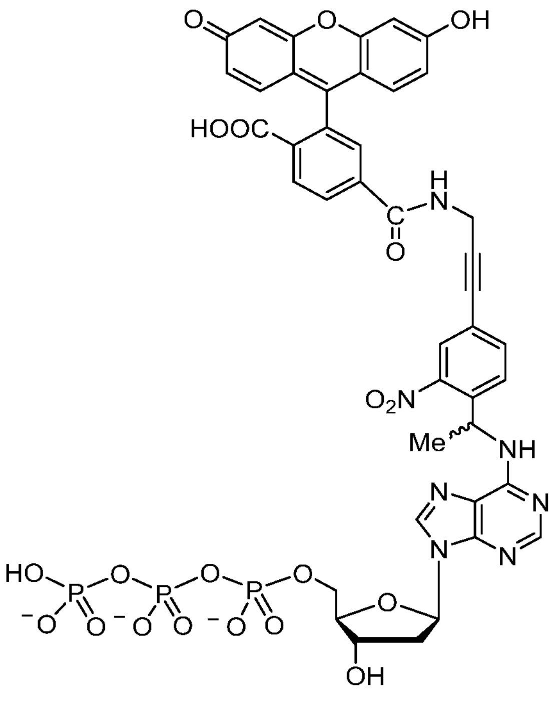

,

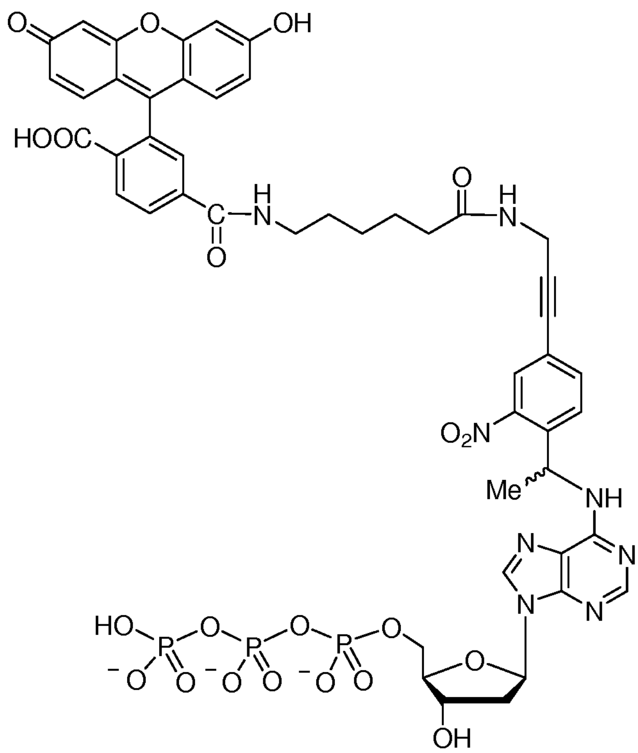

,

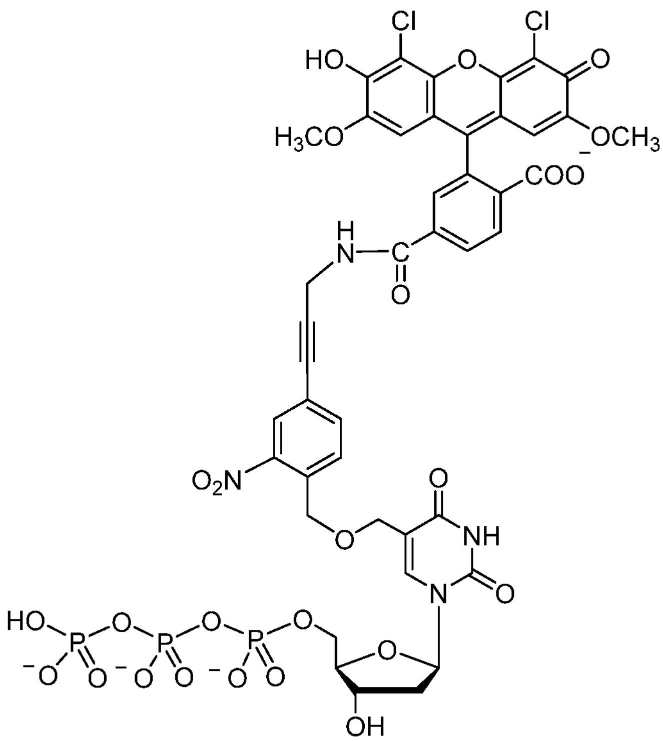

,

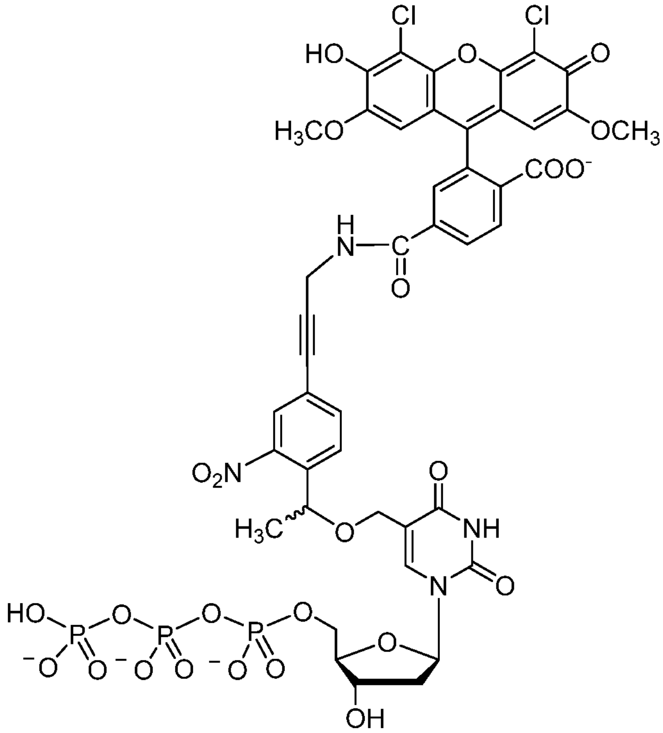

,

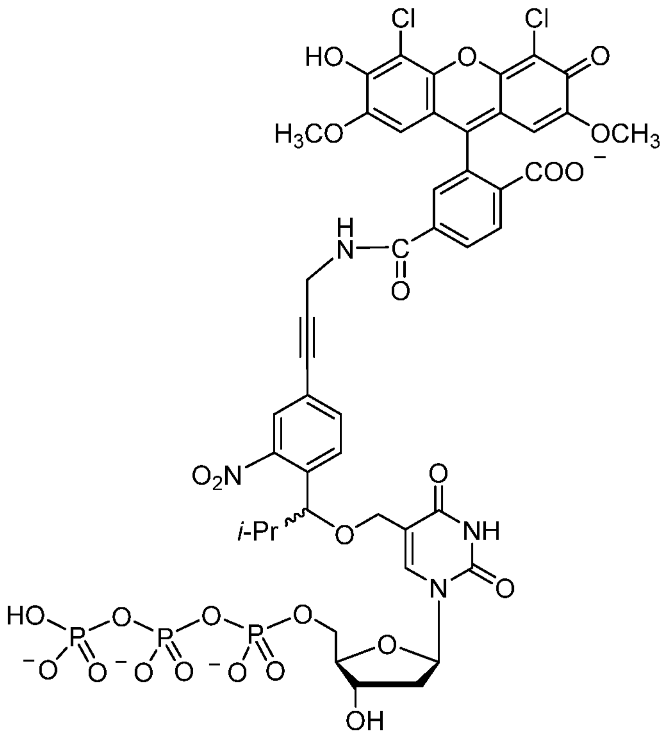

,

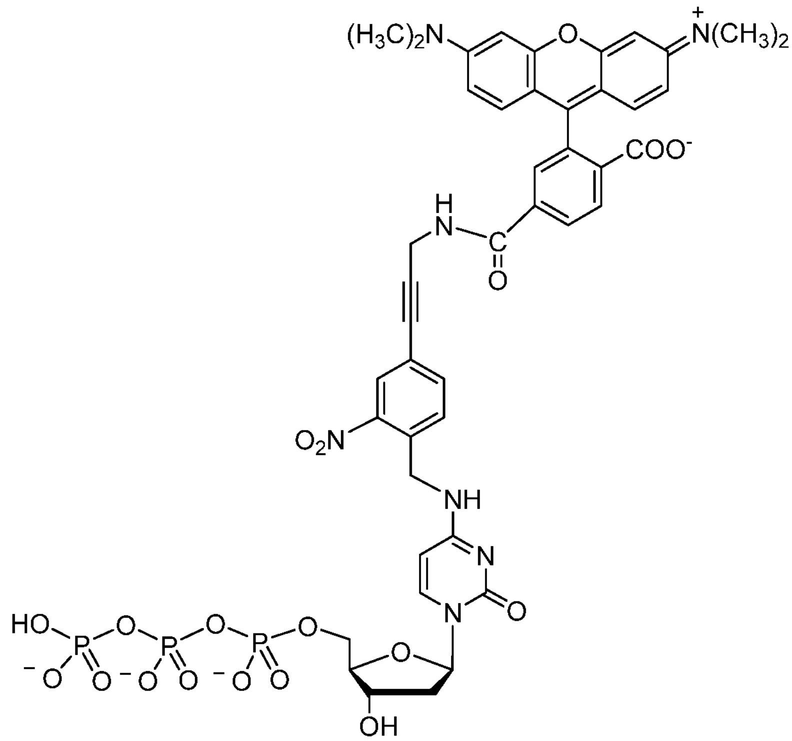

,

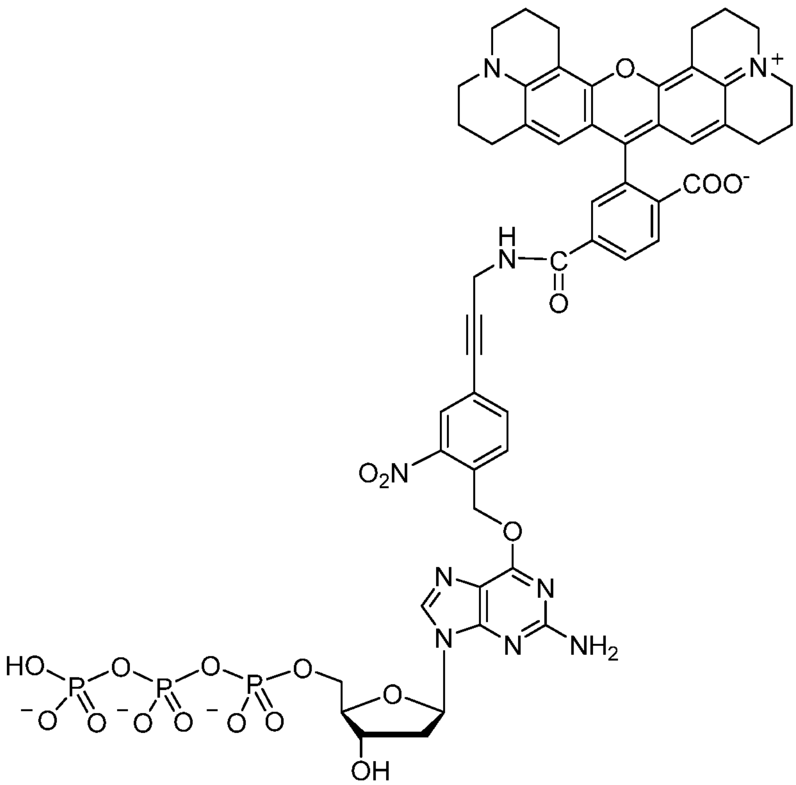

,

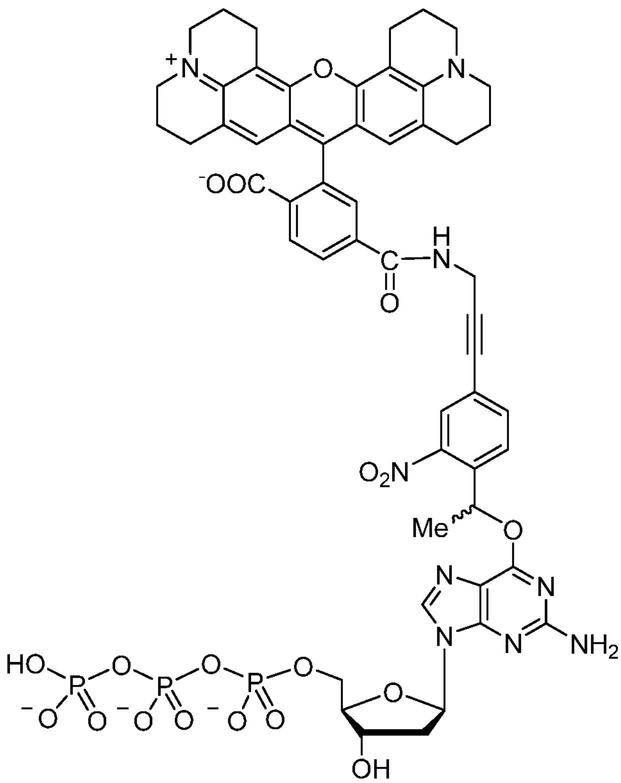

,

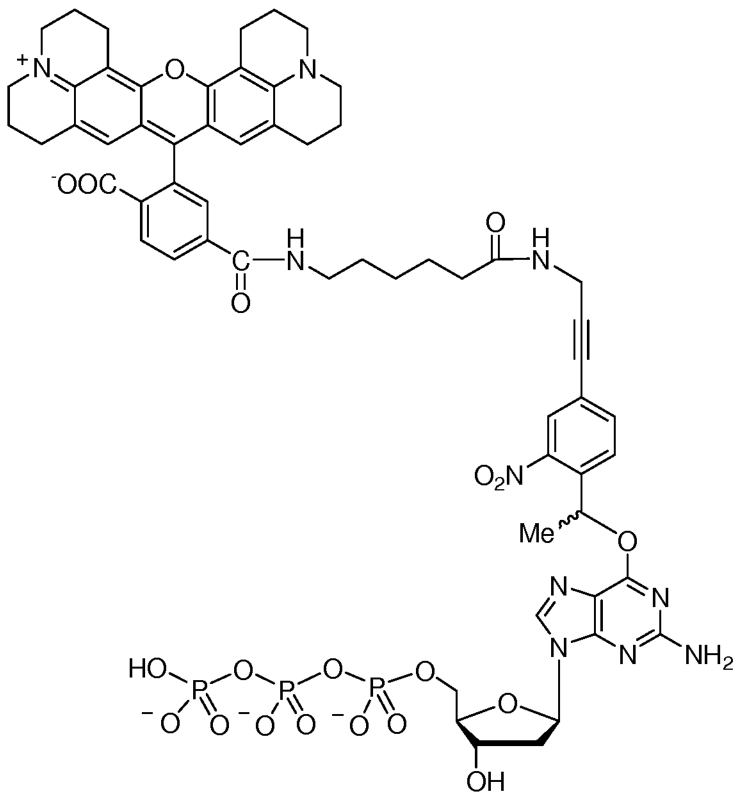

,

und

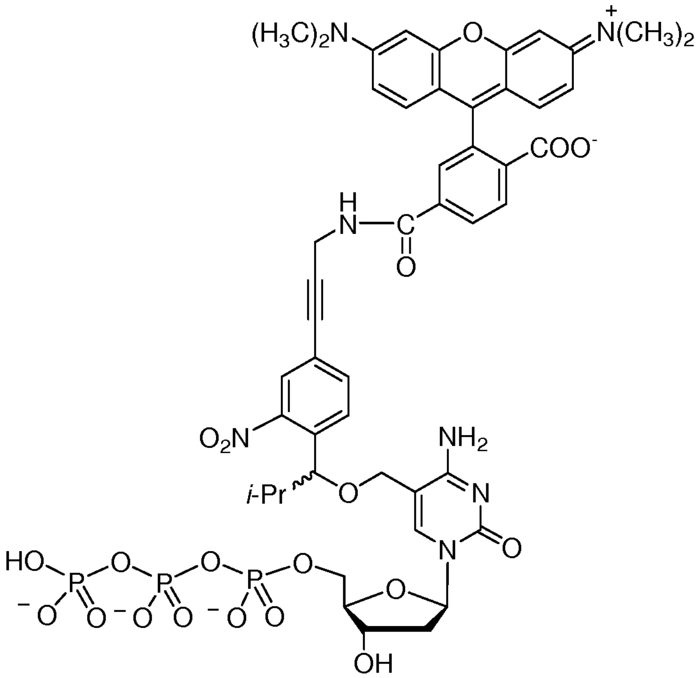

ausgewählt ist.

**7.** Eine Verbindung gemäß Anspruch 3, wobei die Verbindung aus der Gruppe bestehend aus

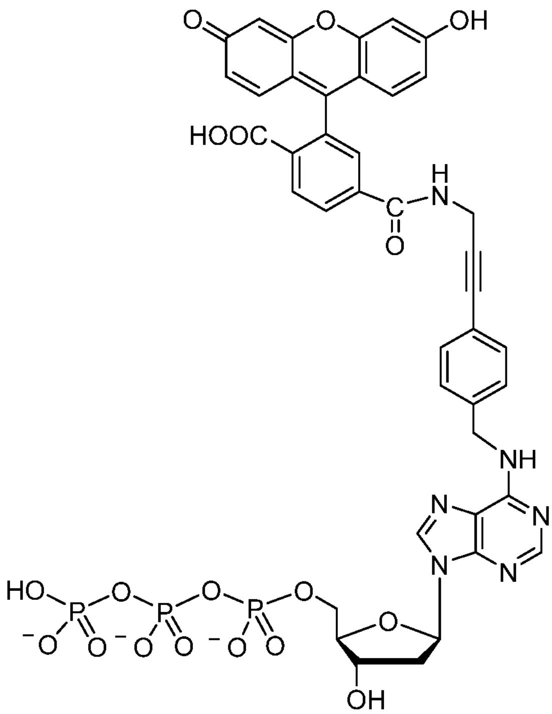

,

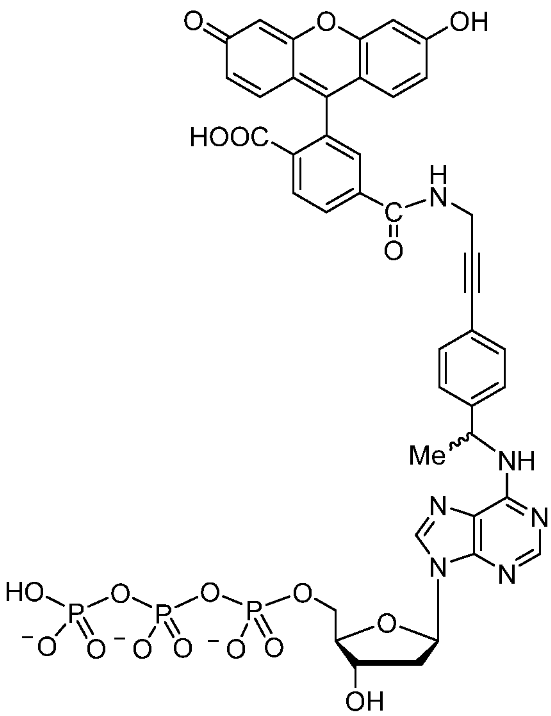

,

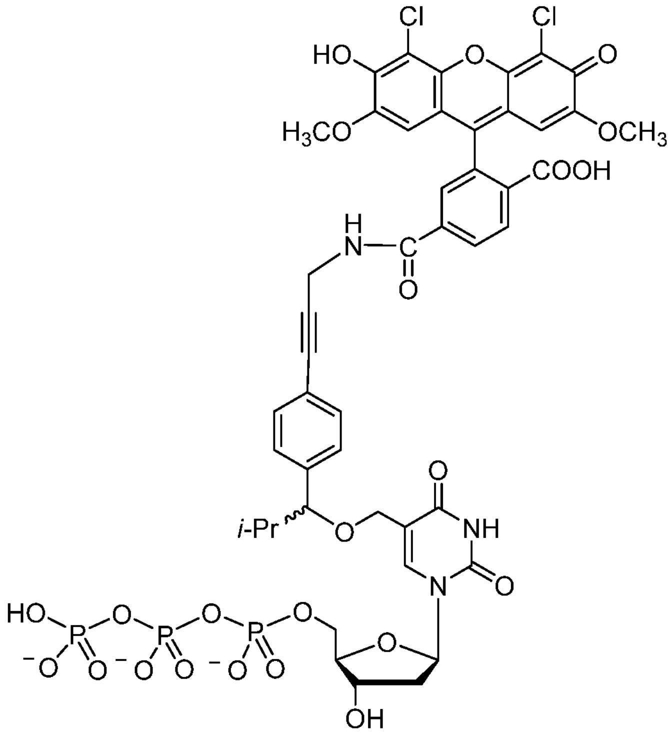

,

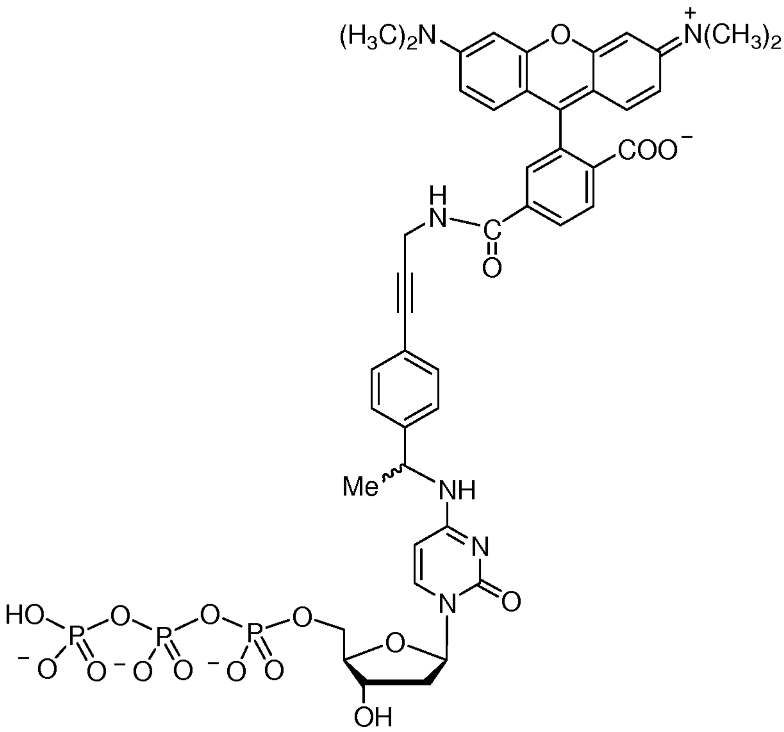

,

und

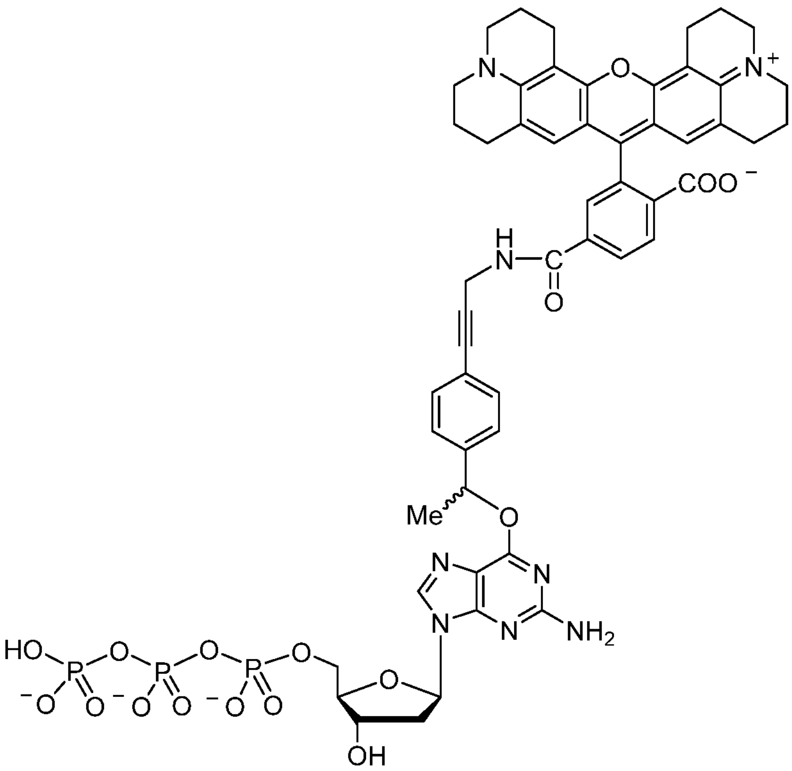

ausgewählt ist.

**8.** Eine Verbindung gemäß einem der Ansprüche 1 bis 5, wobei mindestens einer von $R_5$, $R_6$, $R_7$, und $R_8$ eine aromatische Gruppe bestehend aus Aryl- und polyzyklischen Gruppen ist.

**9.** Eine Verbindung gemäß einem der Ansprüche 1 bis 5, und 8, wobei das Fluorophor aus BODIPY, Fluorescein, Rhodamin, Coumarin, Xanthen, Cyanin, Pyren, Phthalocyanin, Phycobiliprotein, Alexa, Squarene-Farbstoff, Kombinationen, die zu Energie übertragenden Farbstoffen führen, und Derivaten davon ausgewählt ist.

**10.** Ein Verfahren zur Sequenzierung einer Zielnukleinsäure, wobei das Verfahren die folgenden Schritte umfasst:

(i) Anbringen eines Primers an einer festen Oberfläche;
(ii) Hybridisieren einer Zielnukleinsäure an den an der festen Oberfläche angebrachten Primer;
(iii) Zugeben mindestens einer Verbindung nach Anspruch 1 oder 2, wobei jede Base optional an ein anderes Fluorophor angebracht ist, wenn mehr als ein Basentyp vorliegt;
(iv) Zugeben einer Polymerase zu dem hybridisierten Primer/Zielnukleinsäure-Komplex um mindestens eine Verbindung aus Schritt (iii) in den wachsenden Primer-Nukleinsäurestrang mittels einer polymerasekatalysierten Reaktion einzubauen, wobei die mindestens eine eingebaute Verbindung aus Schritt (iii) die Polymerasekettenreaktion mit einer Effizienz zwischen etwa 70% bis etwa 100% abbricht;
(v) optional Waschen der festen Oberfläche, um nicht-eingebaute Komponenten zu entfernen;
(vi) Nachweisen des eingebauten Fluorophors, um die mindestens eine eingebaute Verbindung aus Schritt (iii) zu identifizieren, wobei der Nachweis optional unter Verwendung eines Detektors für gepulste Multilinienanregung zur Bildgebung von Fluoreszenzfarbstoffen durchgeführt wird;
(vii) optional Zugeben einer oder mehrerer chemischen Verbindungen, um die nicht verlängerten Primer permanent mit einem Cap zu versehen;
(viii) Exponieren der festen Oberfläche gegenüber einer Lichtquelle, um den lichtspaltbaren terminierenden Rest zu entfernen, was zu einer verlängerten Primer-Nukleinsäure mit natürlich vorkommenden Komponenten führt;
(ix) optional Waschen der festen Oberfläche, um den gespaltenen, lichtspaltbaren terminierenden Rest zu entfernen;
(x) einmaliges oder mehrfaches Wiederholen der Schritte (iii) bis (ix), um eine Vielzahl an Basen in der Zielnukleinsäure zu identifizieren.

**11.** Das Verfahren gemäß Anspruch 10, wobei der Einbau mindestens einer Verbindung gemäß Schritt (iv) zu etwa 70% bis etwa 100% und vorzugsweise zu etwa 85% bis etwa 100% mit der Effizienz des Einbaus eines nativen Substrats mit derselben Base in der Polymerasereaktion auftritt.

**12.** Das Verfahren gemäß Anspruch 10 oder 11, wobei die Polymerase eine reverse Transkriptase, eine terminale Transferase, oder eine DNA-Polymerase ist, insbesondere wobei die Polymerase eine Taq-DNA-Polymerase, eine Klenow(-exo-) DNA-Polymerase, eine Bst-DNA-Polymerase, eine Vent(-exo-)DNA-Polymerase, eine Pfu(-exo-)-DNA-Polymerase, oder eine DeepVent(exo-) DNA-Polymerase ist, oder wobei die Polymerase eine modifizierte Polymerase ist, die aus Taq FS DNA-Polymerase, ThermoSequenase DNA-Polymerase, ThermoSequenase II DNA-Polymerase, Therminator DNA-Polymerase, Therminator II DNA-Polymerase, und Vent(-exo-) A488L DNA-Polymerase ausgewählt ist.

**13.** Das Verfahren gemäß einem der Ansprüche 10 bis 12, wobei etwa 85% bis etwa 100% der lichtspaltbaren terminierenden Reste durch die Exposition gegenüber der Lichtquelle in Schritt (viii) entfernt werden und/oder wobei sich auf den Einbau mindestens einer Verbindung gemäß Schritt (iv) ein Abbruch des Primer-Nukleinsäurestrangwachstums mit einer Effizienz von etwa 90% bis etwa 100% anschließt.

**14.** Ein Verfahren zum Überführen einer natürlicherweise nicht vorkommenden Komponente in einem Nukleinsäuremolekül in eine natürlicherweise vorkommende Komponente, wobei das Verfahren die Schritte umfasst:

(i) Einbauen einer Verbindung gemäß Anspruch 1 oder 2 in eine Nukleinsäure; und
(ii) Exponieren der gewonnenen Nukleinsäure gegenüber einer Lichtquelle, um den lichtspaltbaren terminierenden Rest von der Nukleinsäure zu entfernen.

**15.** Verwendung einer Verbindung gemäß Anspruch 1 oder 2 in einer Sanger- oder Sanger-artigen Sequenzierungsreaktion oder in einer Pyrosequenzierungs- oder Pyrosequenzierungsartigen Sequenzierungsreaktion.

**16.** Ein Verfahren zum Einbau einer natürlicherweise nicht vorkommenden Komponente in eine Nukleinsäure und optional zum Bestimmen der Sequenz der Nukleinsäure, wobei das Verfahren die Schritte umfasst:

(i) Zugeben einer Zielnukleinsäure zu einer Reaktion, und optional zu einer Sanger- oder Sanger-artigen Sequenzierungsreaktion;
(ii) Zugeben mindestens einer Verbindung gemäß Anspruch 3 zu der Reaktion und optional zu der Sanger- oder Sanger-artigen Sequenzierungsreaktion, wobei jede Base optional an ein anderes Fluorophor angebracht ist, wenn mehr als ein Basentyp vorliegt;
(iii) Zugeben eines komplementären Primers und eines Polymeraseenzyms zu der Reaktion; und
(iv) Durchführen einer polymerasekatalysierten Reaktion, um die mindestens eine Verbindung des Schritts (ii) in einen wachsenden Nukleinsäurestrang einzubauen; und optional
(v) Analysieren des Ergebnisses des Schritts (iv) auf Einbau der mindestens einen Verbindung des Schritts (ii) und optional zur Sequenzbestimmung,

wobei die Schritte (i)-(iii) in jeglicher Reihenfolge durchgeführt werden können.

**17.** Das Verfahren gemäß Anspruch 16, wobei sich auf den Einbau der mindestens einen Verbindung gemäß Schritt (iv) ein Abbruch des Strangwachstums mit einer Effizienz von etwa 90% bis etwa 100% anschließt, oder wobei der Einbau der mindestens einen Verbindung gemäß Schritt (iv) mit einer Effizienz von etwa 70% bis etwa 100% der Einbaueffizienz eines nativen Substrats mit derselben Base in der Polymerasereaktion auftritt.

**18.** Das Verfahren gemäß Anspruch 16 oder 17, wobei das Verfahren dazu dient, die Sequenz einer Nukleinsäure zu bestimmen und wobei die Polymerase eine reverse Transkriptase, eine terminal Transferase, oder eine DNA-Polymerase ist, insbesondere wobei die Polymerase eine Taq-DNA-Polymerase, eine Klenow(exo-)DNA-Polymerase, eine Bst-DNA-Polymerase, eine Vent(-exo-)DNA-Polymerase, eine Pfu(exo-)DNA-Polymerase, oder eine DeepVent(exo-)DNA-Polymerase ist, oder wobei die Polymerase eine modifizierte Polymerase ist, die aus TaqFS-DNA-Polymerase, ThermoSequenase-DNA-Polymerase, ThermoSequenase II-DNA-Polymerase, Therminator DNA-Polymerase, Therminator II-DNA-Polymerase, und Vent(exo-)A488L DNA-Polymerase ausgewählt ist.

**19.** Verwendung einer Verbindung gemäß Anspruch 3 in einer Sanger- oder Sanger-artigen Sequenzierungsreaktion oder in einer Minisequenzierungs- oder Minisequenzierungs-artigen Sequenzierungsreaktion.

**20.** Ein Verfahren zum Abbruch der Nukleinsäuresynthese, wobei das Verfahren den Schritt des Platzierens eines 3'-OH-ungeschützten Nukleotids oder Nukleosids gemäß einem der Ansprüche 1-8 in die Umgebung einer Polymerase und das Gestatten des Einbaus des 3'-OH-ungeschützten Nukleotids oder Nukleosids in ein Nukleinsäuremolekül umfasst.

**21.** Das Verfahren nach Anspruch 20, wobei die Effizienz des Abbruchs der DNA-Synthese nach Einbau des 3'-OH-ungeschützten Nukleotids oder Nukleosids im Bereich von etwa 90% bis etwa 100% liegt, oder wobei die Einbaueffizienz des 3'-OH-ungeschützten Nukleotids oder Nukleosids im Vergleich zur Einbaueffizienz eines natürlicherweise vorkommenden Nukleotids oder Nukleosids mit derselben Base wie das 3'-OH-ungeschützte Nukleotid oder Nukleosid im Bereich von etwa 70% bis etwa 100% liegt.

## Revendications

**1.** Composé selon la formule suivante :

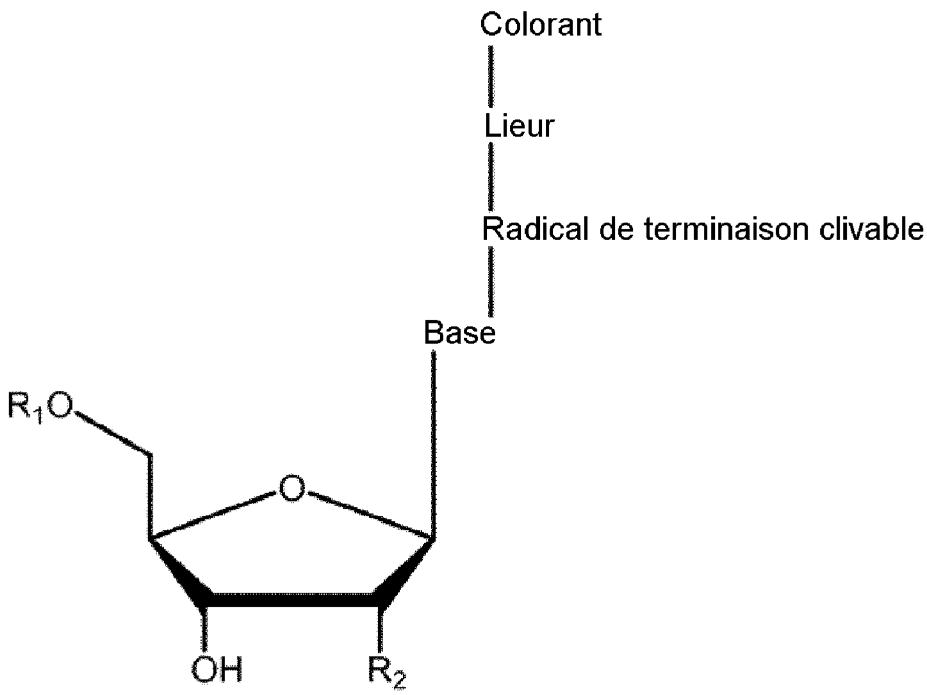


dans laquelle le composé est choisi dans le groupe constitué des composés représentés par les formules I à VII ou leurs sels :

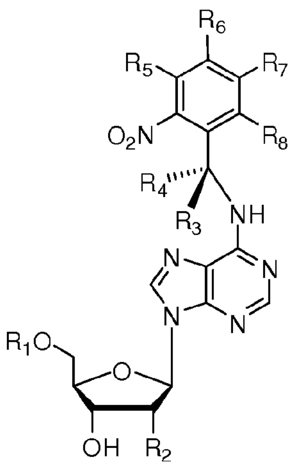

formule I

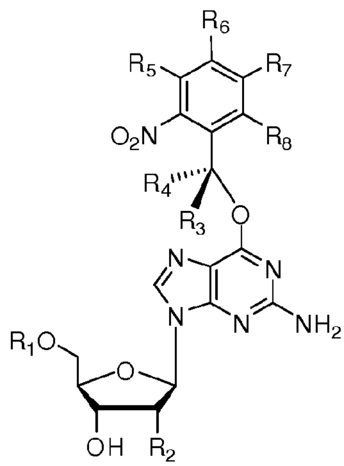

formule II

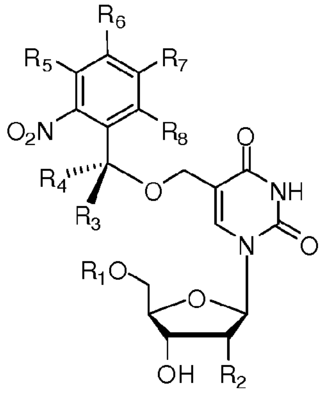

formule III

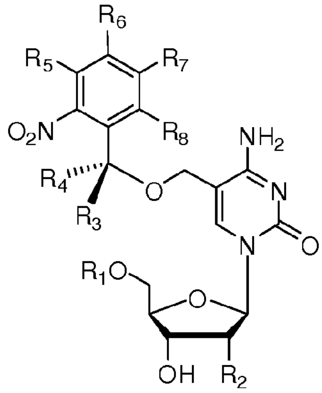

formule IV

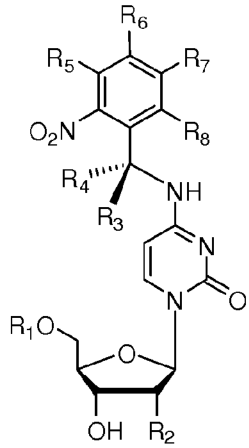

formule V

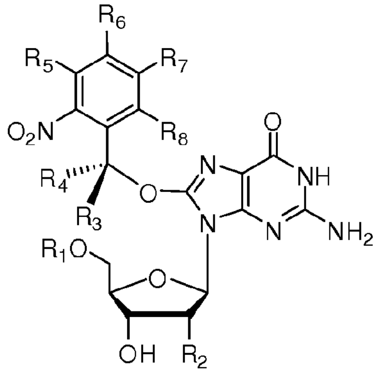

formule VI

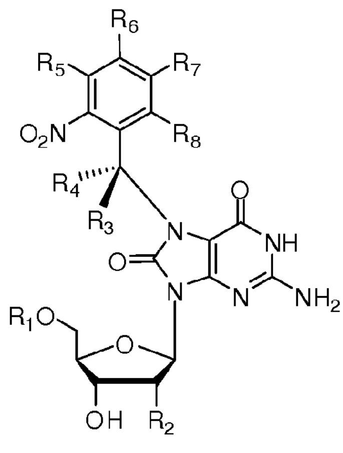

formule VII

dans lesquelles

$R_1$ représente H, un monophosphate, un diphosphate, ou un triphosphate,
$R_2$ représente H ou OH,
$R_3$ et $R_4$ sont choisis parmi H, un groupe alkyle en $C_1$ à $C_{12}$ à chaîne linéaire ou ramifiée, alcényle ou polyényle en $C_2$ à $C_{12}$ à chaîne linéaire ou ramifiée, alcynyle ou polyalcynyle en $C_2$ à $C_{12}$ à chaîne linéaire ou ramifiée, et un groupe aromatique tel qu'un cycle phényle, naphtyle, ou pyridinyle, à condition qu'au moins l'un de $R_3$ et

$R_4$ représente H,
$R_5$, $R_6$, $R_7$ et $R_8$ sont choisis chacun indépendamment parmi H, $OCH_3$, $NO_2$, CN, un halogénure, un groupe alkyle en $C_1$ à $C_{12}$ à chaîne linéaire ou ramifiée, alcényle ou polyényle en $C_2$ à $C_{12}$ à chaîne linéaire ou ramifiée, alcynyle ou polyalcynyle en $C_2$ à $C_{12}$ à chaîne linéaire ou ramifiée, et un groupe aromatique tel qu'un cycle phényle, naphtyle, ou pyridinyle, et/ou un groupe lieur de structure générale :

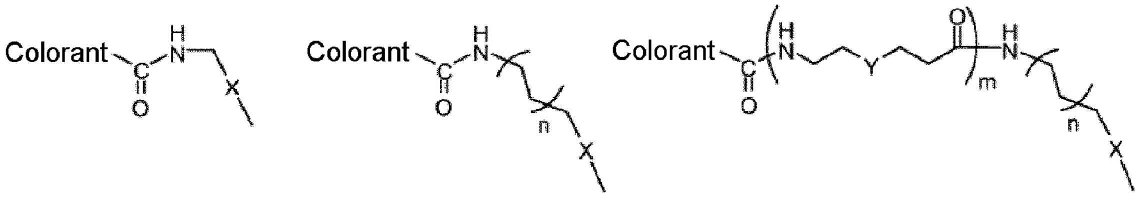

dans lesquelles

X = $CH_2$, CH=CH, C≡C, O, S, ou NH,
Y = $CH_2$, O, ou NH,
n et m sont chacun un nombre entier de 0 à 12, et

Colorant représente un fluorophore.

**2.** Composé selon la revendication 1, dans lequel $R_3$ et $R_4$ sont choisis parmi $-CH_3$, $-CH_2CH_3$, $-CH_2CH_2CH_3$, les groupes isopropyle, tert-butyle, phényle, 2-nitrophényle, et 2,6-dinitrophényle.

**3.** Composé selon la formule suivante :

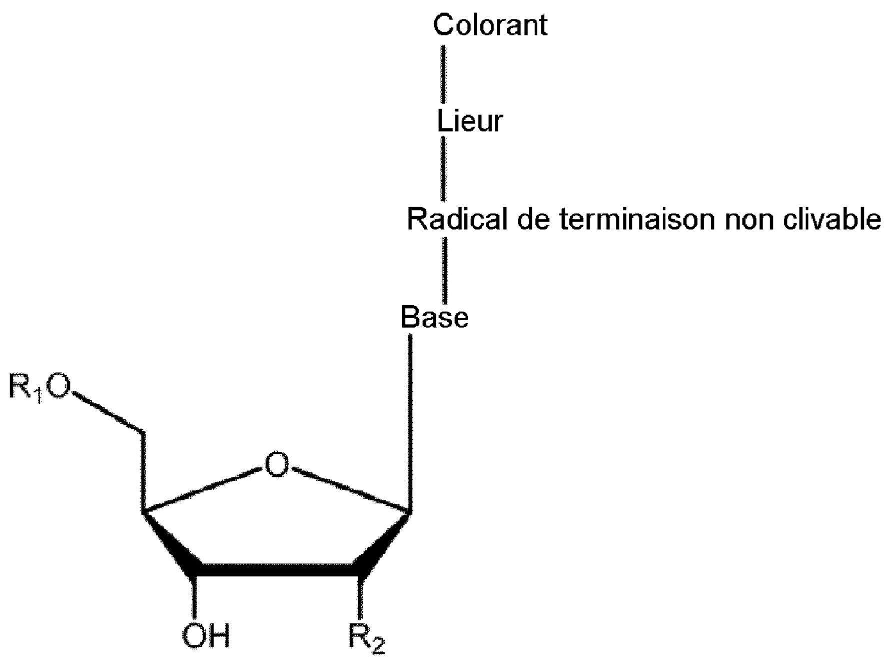

dans laquelle le composé est choisi dans le groupe constitué des composés représentés par les formules VIII à XIV ou leurs sels :

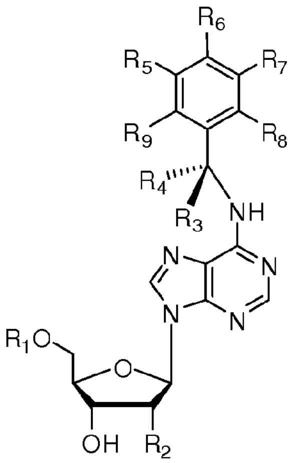

formule VIII

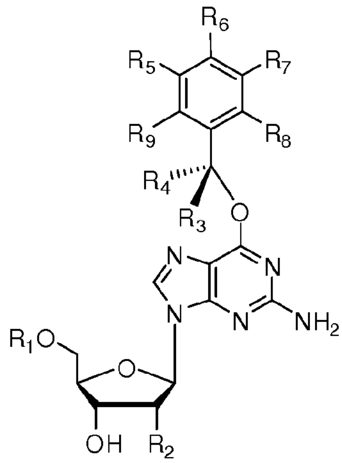

formule IX

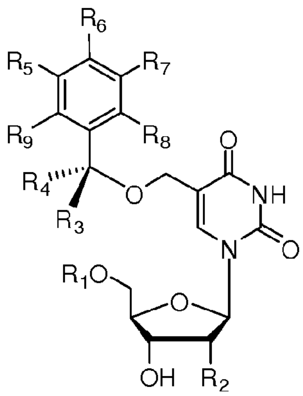

formule X

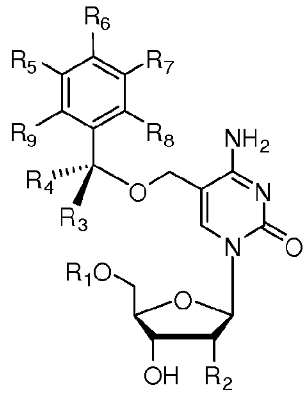

formule XI

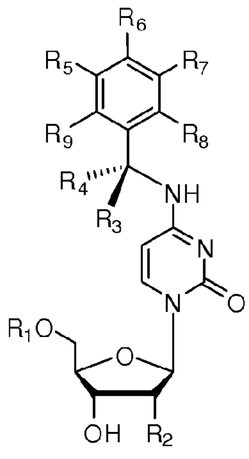

formule XII

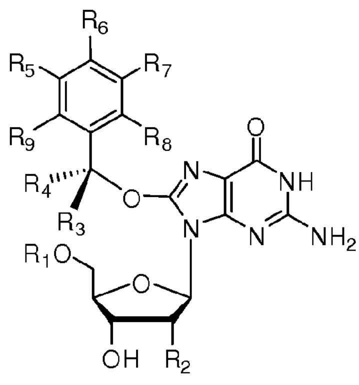

formule XIII

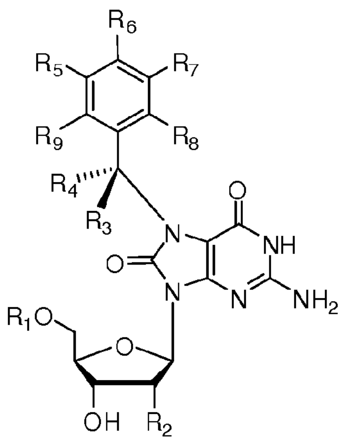

formule XIV

dans lesquelles

$R_1$ représente H, un monophosphate, un diphosphate, ou un triphosphate,

$R_2$ représente H ou OH,

$R_3$ et $R_4$ sont choisis chacun indépendamment parmi H, un groupe alkyle en $C_1$ à $C_{12}$ à chaîne linéaire ou ramifiée, alcényle ou polyényle en $C_2$ à $C_{12}$ à chaîne linéaire ou ramifiée, alcynyle ou polyalcynyle en $C_2$ à $C_{12}$ à chaîne linéaire ou ramifiée, et un groupe aromatique tel qu'un cycle phényle, naphtyle, ou pyridinyle ;

$R_5$, $R_6$, et $R_7$ sont choisis chacun indépendamment parmi H, $OCH_3$, $NO_2$, CN, un halogénure, un groupe alkyle en $C_1$ à $C_{12}$ à chaîne linéaire ou ramifiée, alcényle ou polyényle en $C_2$ à $C_{12}$ à chaîne linéaire ou ramifiée, alcynyle ou polyalcynyle en $C_2$ à $C_{12}$ à chaîne linéaire ou ramifiée, et un groupe aromatique tel qu'un cycle phényle, naphtyle, ou pyridinyle, et/ou un groupe lieur de structure générale :

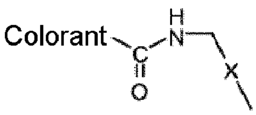

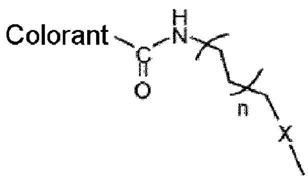

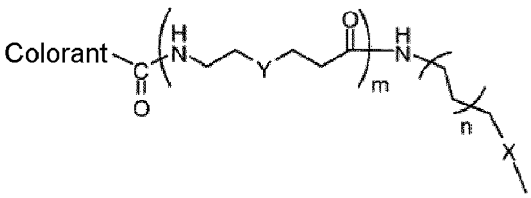

dans lesquelles

X = $CH_2$, CH=CH, C≡C, O, S, ou NH,

Y = $CH_2$, O, ou NH,

n et m sont chacun un nombre entier de 0 à 12,

Colorant représente un fluorophore, et

$R_8$ et $R_9$ sont tels que définis ci-dessus pour $R_5$, $R_6$, et $R_7$, à condition que $R_8$ et $R_9$ ne représentent pas $NO_2$.

**4.** Composé selon la revendication 3, dans lequel au moins l'un de $R_3$ et $R_4$ est choisi dans le groupe constitué de $-CH_3$, $-CH_2CH_3$, $-CH_2CH_2CH_3$, les groupes isopropyle, *tert*-butyle, et phényle.

**5.** Composé selon la revendication 3, dans lequel au moins l'un de $R_3$ et $R_4$ est choisi dans le groupe constitué de groupes alkyle et aromatiques, le groupe alkyle ou aromatique contenant éventuellement au moins un hétéroatome, et en outre dans lequel le groupe aromatique est éventuellement défini comme un groupe aryle ou polycyclique.

**6.** Composé selon la revendication 1, le composé étant choisi dans le groupe constitué de :

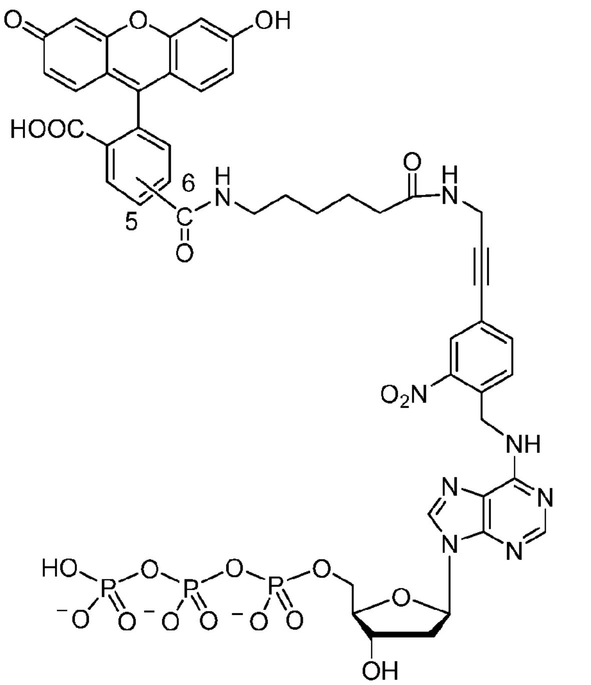

,

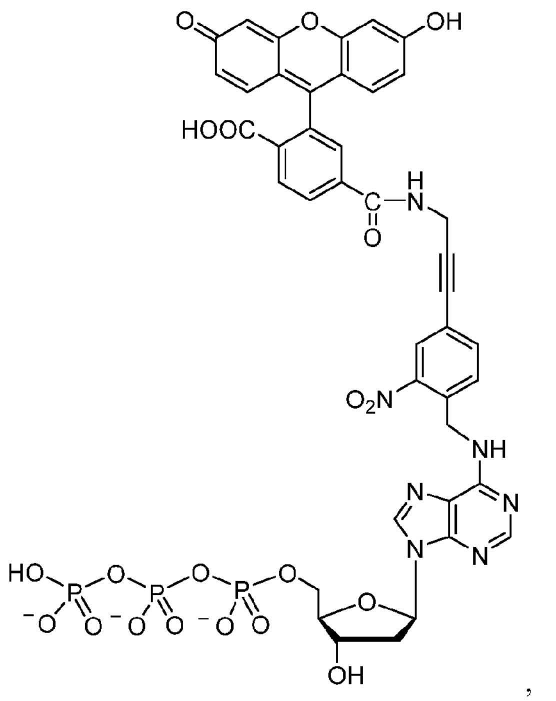

,

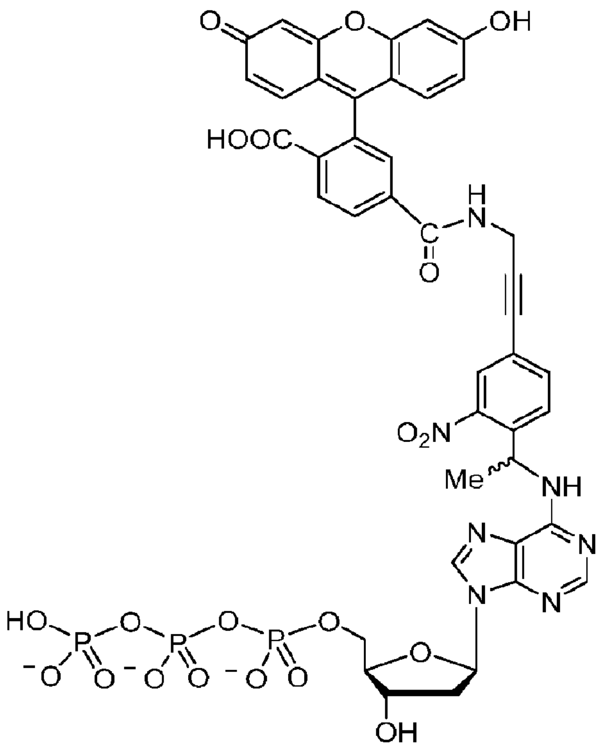

,

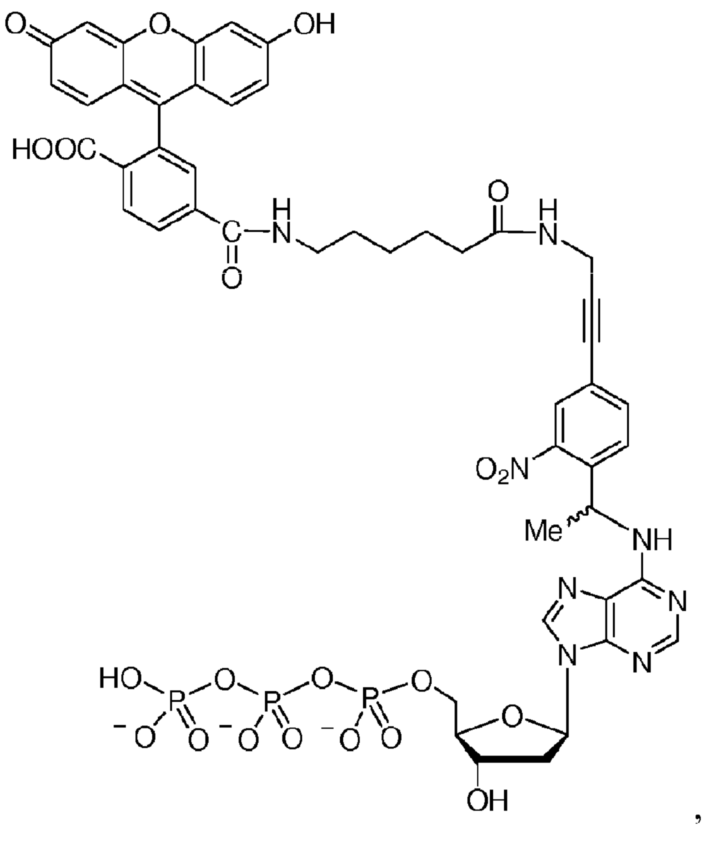

,

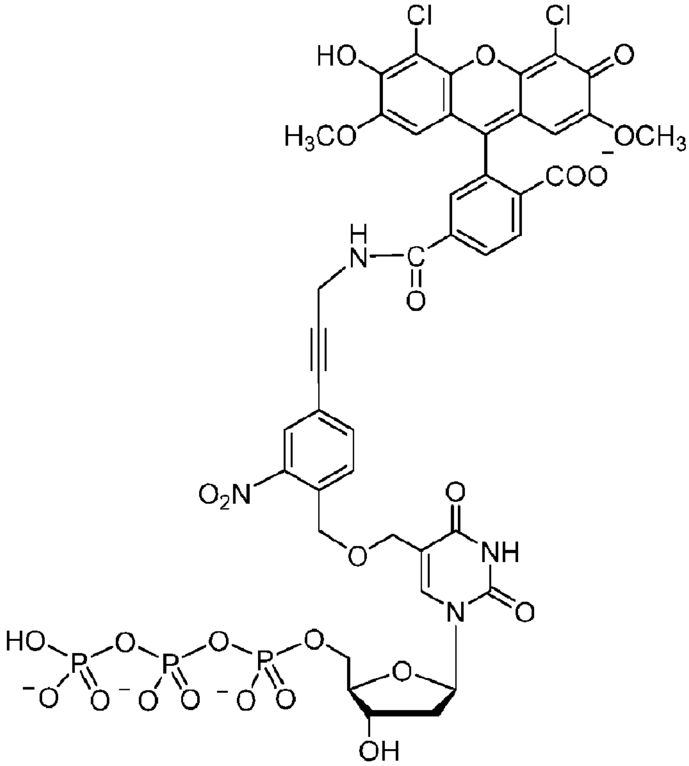

,

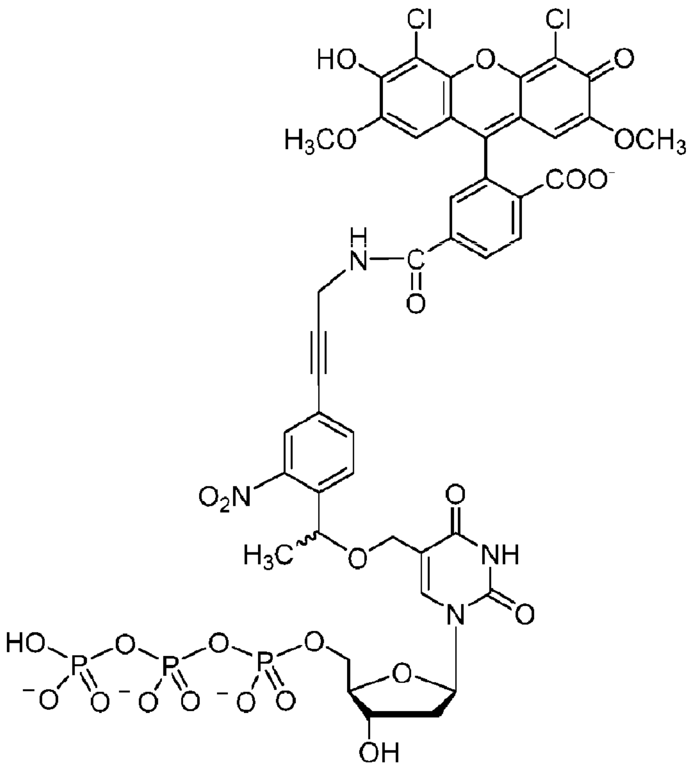

,

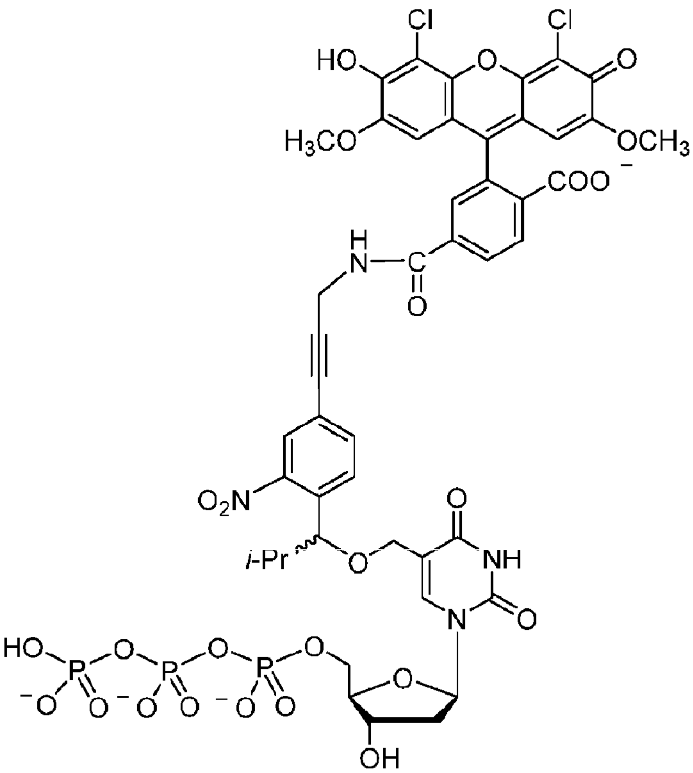

,

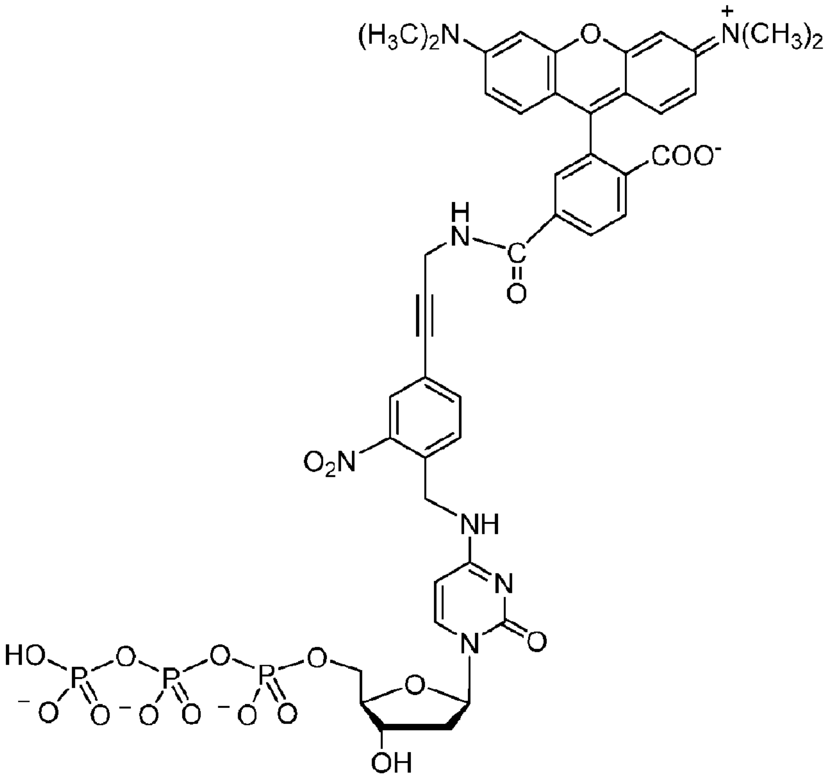

,

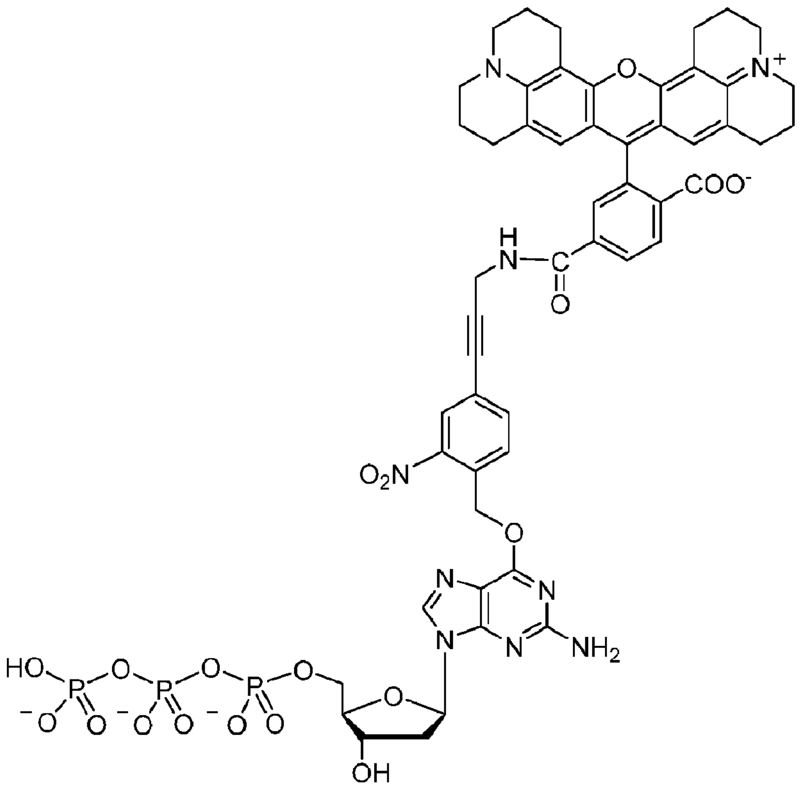

,

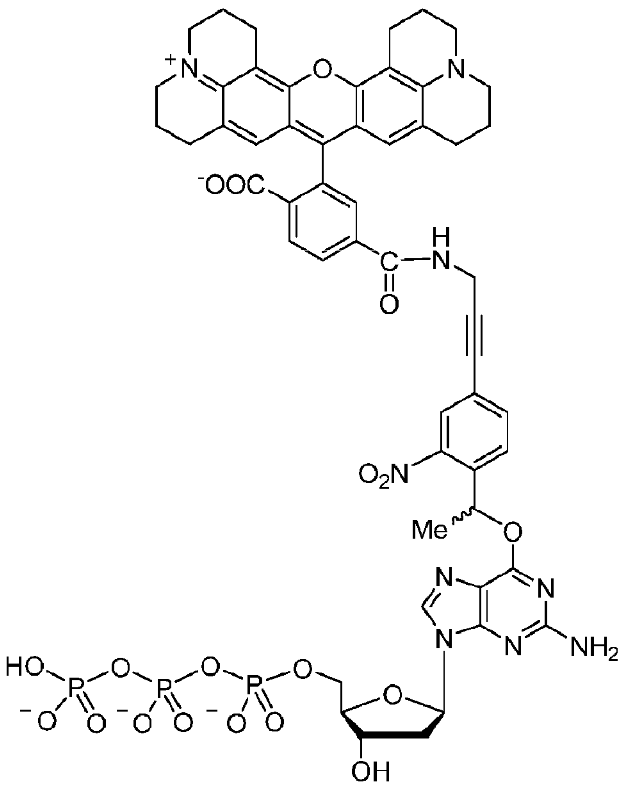

,

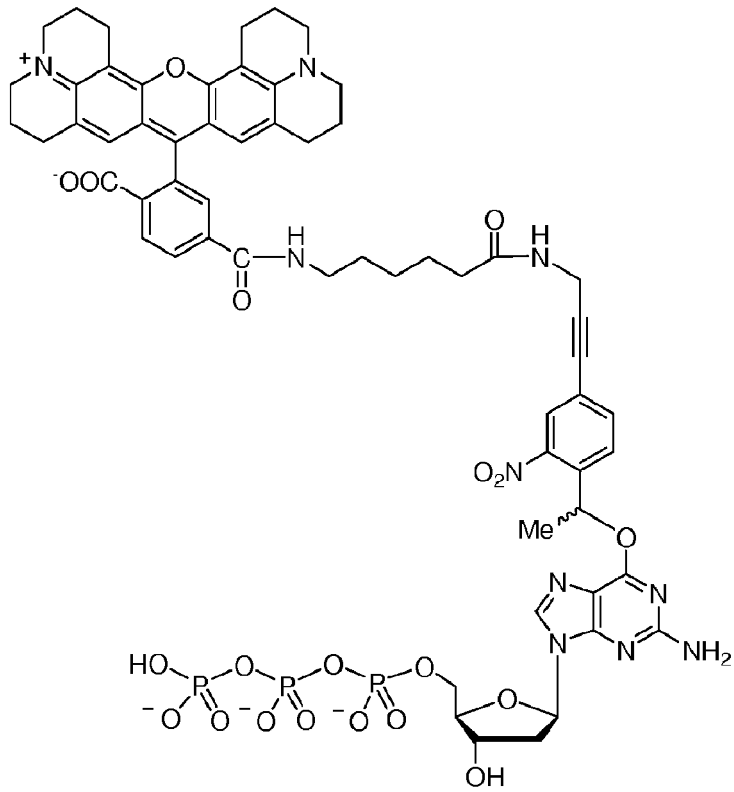

,

et

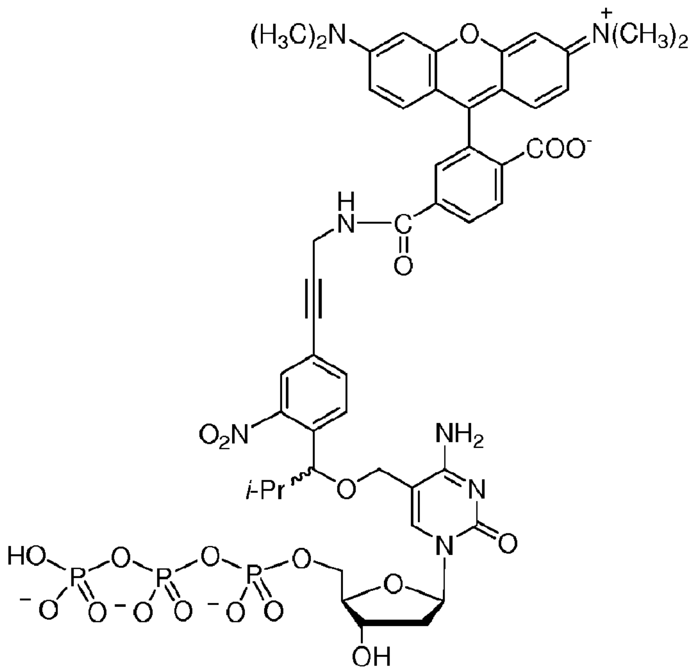

7. Composé selon la revendication 3, le composé étant choisi dans le groupe constitué de

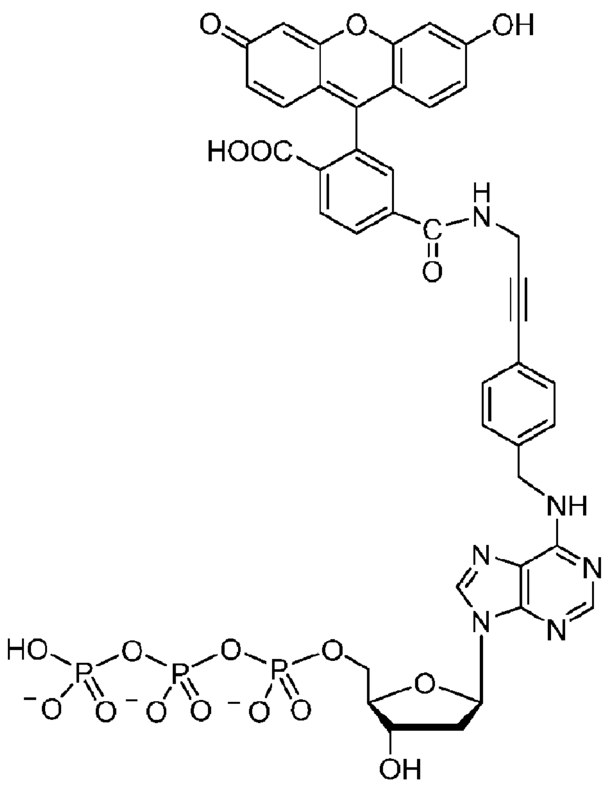

,

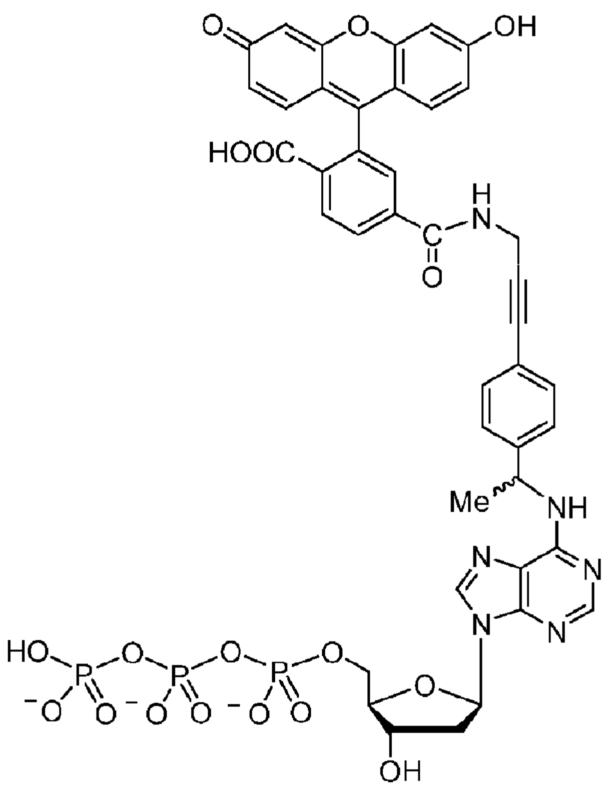

,

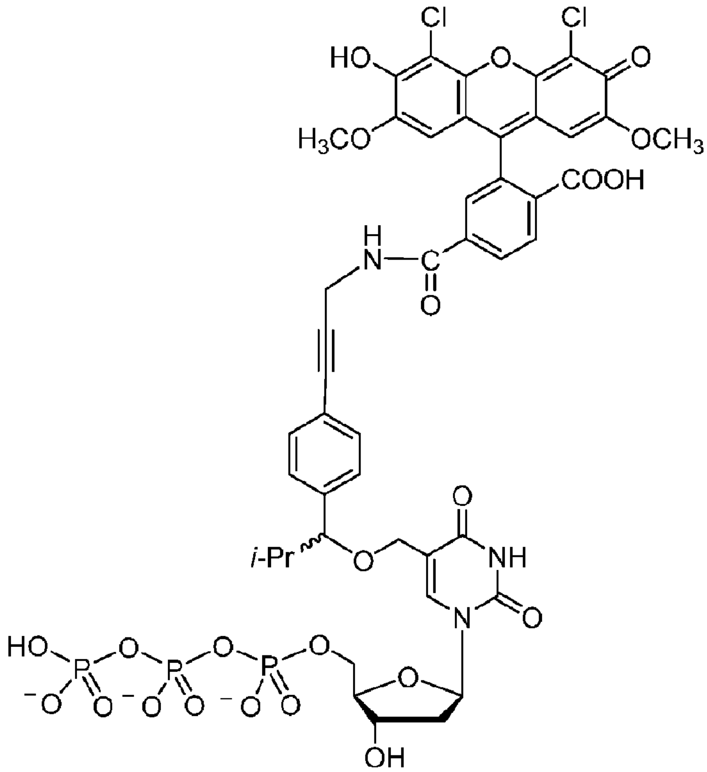

,

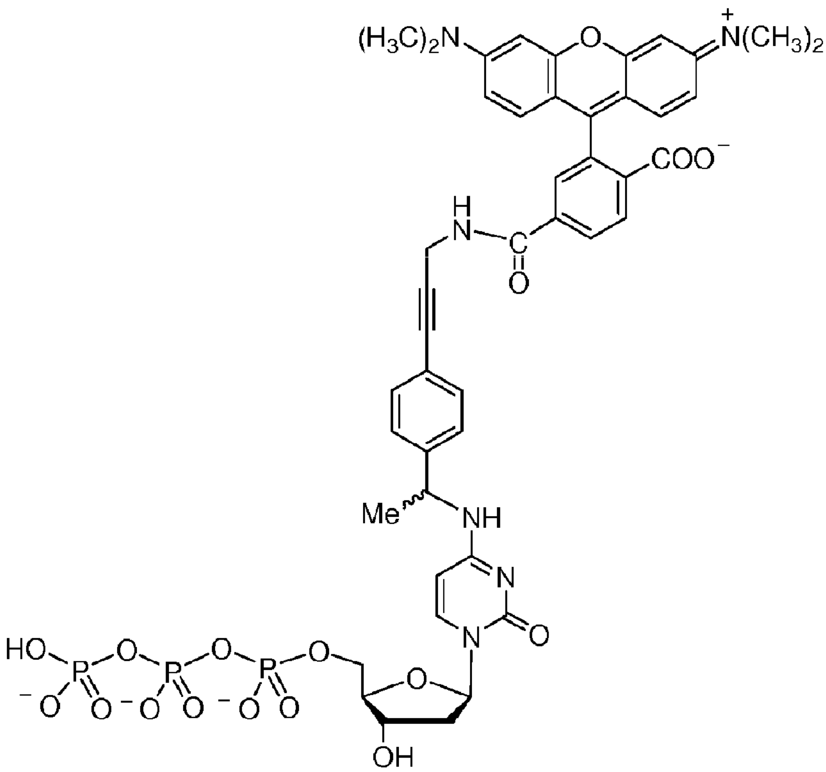

, et

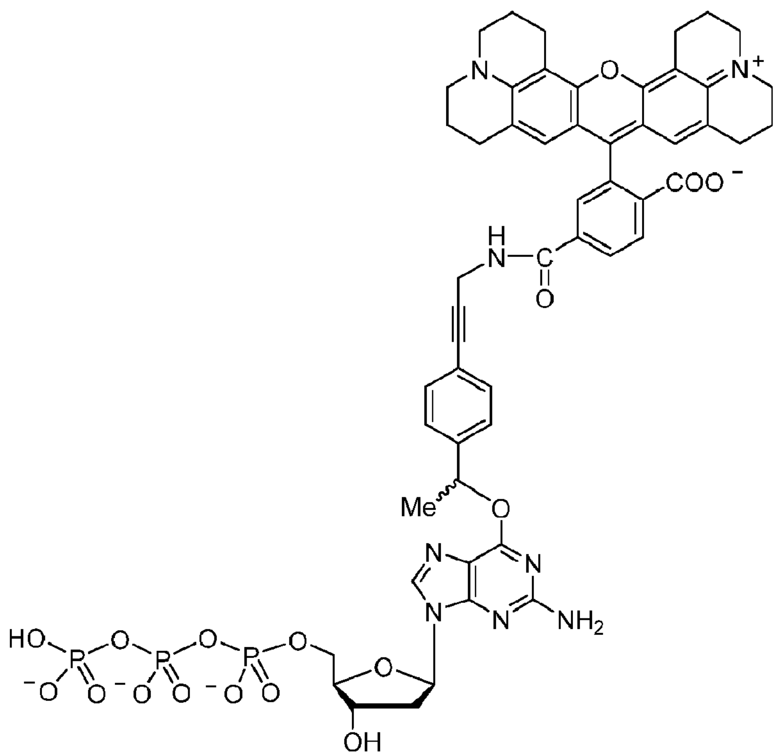

8. Composé selon l'une quelconque des revendications 1 à 5, dans lequel au moins l'un de $R_5$, $R_6$, $R_7$, et $R_8$ représente un groupe aromatique constitué de groupes aryle et polycycliques.

9. Composé selon l'une quelconque des revendications 1 à 5, et 8, dans lequel le fluorophore est choisi parmi BODIPY, la fluorescéine, la rhodamine, la coumarine, le xanthène, la cyanine, le pyrène, la phtalocyanine, la phycobiliprotéine, alexa, un colorant squarène, des combinaisons produisant des colorants à transfert d'énergie, et leurs dérivés.

10. Méthode de séquençage d'un acide nucléique cible, la méthode comprenant les étapes suivantes :

(i) la fixation d'une amorce à une surface solide ;
(ii) l'hybridation d'un acide nucléique cible à l'amorce fixée à la surface solide ;
(iii) l'addition d'au moins un composé selon la revendication 1 ou 2, où si plus d'un type de base est présent, chaque base est éventuellement fixée à un fluorophore différent ;
(iv) l'addition d'une polymérase au complexe hybridé amorce/acide nucléique cible pour incorporer l'au moins un composé de l'étape (iii) dans un brin d'amorce-acide nucléique croissant par une réaction catalysée par une polymérase, où l'au moins un composé incorporé de l'étape (iii) termine la réaction de la polymérase avec une efficacité située entre environ 70 % et environ 100 % ;
(v) le lavage éventuel de la surface solide pour éliminer les composants non incorporés ;
(vi) la détection du fluorophore incorporé pour identifier l'au moins un composé incorporé de l'étape (iii), où la détection est réalisée éventuellement en utilisant un détecteur d'excitation à lignes multiples par impulsions pour l'imagerie des colorants fluorescents ;
(vii)l'addition éventuelle d'un ou de plusieurs composés chimiques pour coiffer de façon permanente les amorces non étendues ;
(viii) l'exposition de la surface solide à une source de lumière pour éliminer le radical de terminaison photo-clivable produisant une amorce-acide nucléique étendue avec des composants existant l'état naturel ;
(ix) le lavage éventuel de la surface solide pour éliminer le radical de terminaison photo-clivable clivé ;
(x) la répétition des étapes (iii) à (ix) une ou plusieurs fois pour identifier une pluralité de bases dans l'acide nucléique cible.

11. Méthode selon la revendication 10, dans laquelle l'incorporation du au moins un composé selon l'étape (iv) se produit à environ 70 % à environ 100 % et de préférence à environ 85 % à environ 100 % de l'efficacité de l'incorporation d'un substrat natif avec la même base dans la réaction de la polymérase.

**12.** Méthode selon la revendication 10 ou 11, dans laquelle la polymérase est une transcriptase inverse, une transférase terminale, ou une ADN polymérase, en particulier dans lequel la polymérase est une Taq ADN polymérase, une Klenow (-exo-) ADN polymérase (-exo-), une Bst ADN polymérase, une Vent (-exo-) ADN polymérase, une Pfu (-exo-) ADN polymérase, ou une DeepVent (-exo-) ADN polymérase, ou dans laquelle la polymérase est une polymérase modifiée choisie parmi la Taq FS ADN polymérase, la ThermoSequenase ADN polymérase, la ThermoSequenase II ADN polymérase, la Therminator ADN polymérase, la Therminator II ADN polymérase, et la Vent (-exo-) A488L ADN polymérase.

**13.** Méthode selon l'une quelconque des revendications 10 à 12, dans laquelle environ 85 % à environ 100 % des radicaux de terminaison photo-clivables sont éliminés par exposition à la source de lumière dans l'étape (viii) et/ou dans laquelle l'incorporation du au moins un composé selon l'étape (iv) est suivie d'une terminaison de la croissance du brin d'amorce-acide nucléique avec une efficacité d'environ 90 % à environ 100 %.

**14.** Méthode de conversion d'un composant n'existant pas à l'état naturel dans une molécule d'acide nucléique en un composant existant l'état naturel, la méthode comprenant les étapes suivantes :

(i) l'incorporation d'un composé selon la revendication 1 ou 2 dans un acide nucléique ; et
(ii) l'exposition de l'acide nucléique résultant à une source de lumière pour éliminer le radical de terminaison photo-clivable de l'acide nucléique.

**15.** Utilisation d'un composé selon la revendication 1 ou 2 dans une réaction de séquençage de Sanger ou de type Sanger ou dans une réaction de séquençage par pyroséquençage ou de type pyroséquençage.

**16.** Méthode d'incorporation d'un composant n'existant pas à l'état naturel dans un acide nucléique et de détermination éventuelle de la séquence de l'acide nucléique, la méthode comprenant les étapes suivantes :

(i) l'addition d'un acide nucléique cible à une réaction, et éventuellement à une réaction de séquençage de Sanger ou de type Sanger ;
(ii) l'addition d'au moins un composé selon la revendication 3 à la réaction et éventuellement à la réaction de séquençage de Sanger ou de type Sanger, où si plus d'un type de base est présent, chaque base est éventuellement fixée à un fluorophore différent ;
(iii) l'addition d'une amorce complémentaire et d'une enzyme polymérase à la réaction ; et
(iv) la réalisation d'une réaction catalysée par la polymérase pour incorporer l'au moins un composé de l'étape (ii) dans un brin d'acide nucléique croissant ; et éventuellement
(v) l'analyse du résultat de l'étape (iv) pour l'incorporation du au moins un composé issu de l'étape (ii) et éventuellement pour la détermination de la séquence,

dans laquelle les étapes (i) à (iii) peuvent être effectuées dans un ordre quelconque.

**17.** Méthode selon la revendication 16, dans laquelle l'incorporation du au moins un composé selon l'étape (iv) est suivie d'une terminaison de la croissance du brin avec une efficacité d'environ 90 % à environ 100 %, ou dans laquelle l'incorporation du au moins un composé selon l'étape (iv) survient à environ 70 % à environ 100 % de l'efficacité de l'incorporation d'un substrat natif avec la même base dans la réaction de la polymérase.

**18.** Méthode selon la revendication 16 ou 17, où la méthode consiste à déterminer la séquence d'un acide nucléique et la polymérase est une transcriptase inverse, une transférase terminale, ou une ADN polymérase, en particulier dans lequel la polymérase est une Taq ADN polymérase, une Klenow (-exo-) ADN polymérase (-exo-), une Bst ADN polymérase, une Vent (-exo-) ADN polymérase, une Pfu (-exo-) ADN polymérase, ou une DeepVent (-exo-) ADN polymérase, ou dans laquelle la polymérase est une polymérase modifiée choisie parmi la Taq FS ADN polymérase, la ThermoSequenase ADN polymérase, la ThermoSequenase II ADN polymérase, la Therminator ADN polymérase, la Therminator II ADN polymérase, et la Vent (-exo-) A488L ADN polymérase.

**19.** Utilisation d'un composé selon la revendication 3 dans une réaction de séquençage de Sanger ou de type Sanger ou dans une réaction de séquençage par mini-séquençage ou de type miniséquençage.

**20.** Méthode de terminaison de la synthèse d'un acide nucléique, la méthode comprenant l'étape de placement d'un nucléotide ou d'un nucléoside non protégé en 3'-OH selon l'une quelconque des revendications 1 à 8 dans l'environnement d'une polymérase et permettant l'incorporation du nucléotide ou du nucléoside non protégé en 3'-OH

dans une molécule d'acide nucléique.

**21.** Méthode selon la revendication 20, dans laquelle l'efficacité de la terminaison de la synthèse de l'ADN lors de l'incorporation du nucléotide ou du nucléoside non protégé en 3'-OH se situe dans la plage d'environ 90 % à environ 100 %, ou dans laquelle l'efficacité de l'incorporation du nucléotide ou du nucléoside non protégé en 3'-OH se situe dans la plage d'environ 70 % à environ 100 % comparativement à l'efficacité de l'incorporation d'un nucléotide ou d'un nucléoside existant à l'état naturel avec la même base que le nucléotide ou le nucléoside non protégé en 3'-OH.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2003021212 A
- US 20030058440 A
- US 7125660 B


### Non-patent literature cited in the description


- **SANGER, F. ; NICKLEN, S. ; COULSON, A. R.** DNA sequencing with chain-terminating inhibitors. *Proc. Natl. Acad. Sci. USA,* 1977, vol. 74, 5463-5467
- **GIBBS, R. A. ; NGUYEN, P.-N. ; MCBRIDE, L. J. ; KOEPF, S. M. ; CASKEY, C. T.** Identification of mutations leading to the Lesch-Nyhan syndrome by automated direct DNA sequencing of in vitro amplified cDNA. *Pro. Natl. Acad. Sci. USA,* 1989, vol. 86, 1919-1923
- **HUNKAPILLER, T. ; KAISER, R. J. ; KOOP, B. F. ; HOOD, L.** Large-scale and automated DNA sequencing Determination. *Science,* 1991, vol. 254, 59-67
- International Human Genome Sequencing Consortium. Initial sequencing and analysis of the human genome. *Nature,* 2001, vol. 409, 860-921
- **METZKER, M.L.** *Genome Rex,* 2005, vol. 15, 1767-1776
- **PILLAI, V. N. R.** Photoremovable Protecting Groups in Organic Synthesis. *Synthesis,* 1980, 1-26
- **OHTSUKA, E. ; TANAKA, S. ; IKEHARA, M.** Studies on transfer ribonucleic acids and related compounds. IX(1) Ribooligonucleotide synthesis using a photosensitive o-nitrobenzyl protection at the 2'-hydroxyl group. *Nucleic Acids Res.,* 1974, vol. 1, 1351-1357
- **CHAULK, S. G. ; MACMILLAN, A. M.** Caged RNA: photo-control of a ribozyme. *Nucleic Acids Res.,* 1998, vol. 26, 3173-3178
- **PEASE, A. C. ; SOLAS, D. ; SULLIVAN, E. J. ; CRONIN, M. T. ; HOLMES, C. P. ; FODOR, S. P. A.** Light-generated oligonucleotide arrays for rapid DNA sequence analysis. *Proc. Natl. Acad. Sci. USA,* 1994, vol. 91, 5022-5026
- **METZKER, M. L. ; RAGHAVACHARI, R. ; RICHARDS, S. ; JACUTIN, S. E. ; CIVITELLO, A. ; BURGESS, K. ; GIBBS, R. A.** Termination of DNA synthesis by novel 3'-modified deoxyribonucleoside triphosphates. *Nucleic Acids Res.,* 1994, vol. 22, 4259-4267
- **BARTHOLOMEW, D. G. ; BROOM, A. D.** One-step Chemical Synthesis of Ribonucleosides bearing a Photolabile Ether Protecting Group. *J. Chem. Soc. Chem. Commun.,* 1975, 38
- **FURRER, E. ; GIESE, B.** On the distance-independent hole transfer over long (A·T)n-sequences in DNA. *Helvetica Chimica Acta,* 2003, vol. 86, 3623-3632
- **KISELYOV, A. S. ; STEINBRECHER, T. ; HARVEY, R. G.** Synthesis of the Fjord-region cis- and trans-Amino Triol Derivatives of the carcinogenic Hydrocarbon Benzo[g]chrysene and Utilization for the Synthesis of a Deoxyadenosine Adduct Linked to the N6-Amino Group. *J. Org. Chem.,* 1995, vol. 60, 6129-6134